(19) Europäisches Patentamt
European Patent Office
Office européen des brevets

(11) EP 2 189 449 A1

(12) **EUROPEAN PATENT APPLICATION**
published in accordance with Art. 153(4) EPC

(43) Date of publication:
**26.05.2010 Bulletin 2010/21**

(21) Application number: **08826977.4**

(22) Date of filing: **06.08.2008**

(51) Int Cl.:
*C07D 231/38* (2006.01) *A61K 31/415* (2006.01)
*A61K 31/4155* (2006.01) *A61K 31/445* (2006.01)
*A61K 31/501* (2006.01) *A61P 3/00* (2006.01)
*A61P 3/04* (2006.01) *A61P 3/06* (2006.01)
*A61P 3/10* (2006.01) *A61P 9/10* (2006.01)
*A61P 9/12* (2006.01) *A61P 25/24* (2006.01)
*A61P 25/28* (2006.01) *C07D 401/12* (2006.01)
*C07D 401/14* (2006.01) *C07D 403/12* (2006.01)
*C07D 405/12* (2006.01) *C07D 409/14* (2006.01)
*C07D 413/14* (2006.01)

(86) International application number:
**PCT/JP2008/064085**

(87) International publication number:
**WO 2009/020137 (12.02.2009 Gazette 2009/07)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **06.08.2007 JP 2007204739**
**24.03.2008 JP 2008074895**

(71) Applicant: **Dainippon Sumitomo Pharma Co., Ltd.**
**Osaka 541-8524 (JP)**

(72) Inventors:
- **HORIUCHI, Yoshihiro**
  **Suita-shi**
  **Osaka 564-0053 (JP)**
- **NUNAMI, Noriko**
  **Suita-shi**
  **Osaka 564-0053 (JP)**
- **TATAMIDANI, Hiroto**
  **Suita-shi**
  **Osaka 564-0053 (JP)**
- **OHATA, Eiko**
  **Suita-shi**
  **Osaka 564-0053 (JP)**

(74) Representative: **Vossius & Partner**
**Siebertstraße 4**
**81675 München (DE)**

(54) **AMINOPYRAZOLE AMIDE DERIVATIVE**

(57) Disclosed is a compound represented by the formula (1) below or a pharmaceutically acceptable salt thereof, which is useful as an agent for prevention and/or treatment of diabetes and the like.

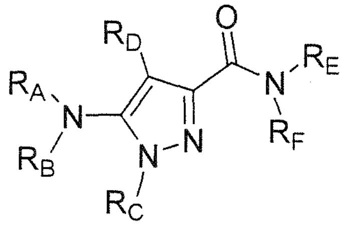

(1)

(In the formula, $R_A$ and $R_B$ independently represent an optionally substituted alkyl group or the like; $R_C$ represents an optionally substituted alkyl group or the like; $R_D$ represents a hydrogen atom or the like; $R_E$ represents a hydrogen atom or the like; and $R_F$ represents a group selected from those represented by the formulae (G1) below:

EP 2 189 449 A1

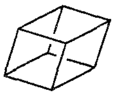 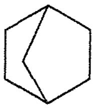 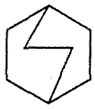 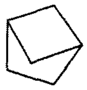 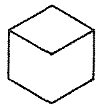 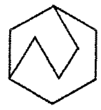

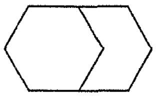 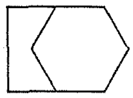 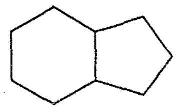 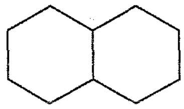 (G1)

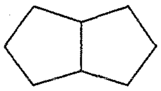 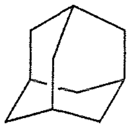 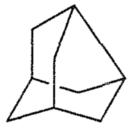 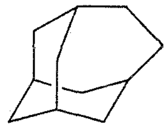 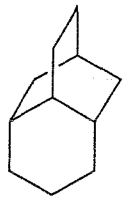 ,

wherein one hydrogen atom serves as a bonding hand, or the like.)

## Description

TECHNICAL FIELD

The invention relates to a pyrazole amide compound useful as a medicament. More specifically, the invention relates to a therapeutic or preventive agent for conditions related to glucocorticoid, or a pyrazole amide compound which is effective as an 11β hydroxysteroid dehydrogenase type 1 enzyme (referred to as 11βHSD hereinafter) inhibitor or 11βHSD modulator. The invention further relates to a therapeutic agent for diabetes that the active ingredient is a pyrazole amide compound which is effective as an 11βHSD1 inhibitor or 11βHSD1 modulator.

BACKGROUND ART

Glucocorticoid regulates peripheral glucose metabolism and amino acid metabolism. In human being, glucocorticoid is produced in adrenal gland and is metabolized in peripheral tissues including adipose tissue or liver. Since 11βHSD1 is an enzyme converting inactive cortisone into activated cortisol and is mainly expressed in adipose tissue or liver, 11βHSD1 is believed to be related to glucocorticoid activation in adipose tissue or liver. Cortisol shows promoting activities for fat accumulation in adipocyte or gluconeogenesis in liver, and hence, 11βHSD1 is believed to contribute to the maintenance of systemic homeostasis by adjusting glucose and/or lipid metabolism in periphery. On the other hand, in human insulin resistance patients, 11βHSD1 in adipose tissues was significantly increased in the activity, and the 11βHSD1 activity in visceral fat is remarkably higher than that in subcutaneous fat. In 11βHSD1 gene defect mice, development of visceral fat accumulation, glucose and/or lipid metabolism abnormality is suppressed on high-fat food feeding, and mice overexpressing adipocyte-specific 11βHSD1 show remarkable visceral fat-type obesity, or glucose and/or lipid metabolism abnormality. This indicates that an overactivation of 11βHSD1 is intimately related to development of visceral fat accumulation and/or metabolic syndrome in both human and mice. Specifically, advantageous effects including suppression of gluconeogenesis in liver and fat accumulation in adipocyte as well as improvement of systemic glucose and/or lipid metabolism are expected by inhibiting the enzyme activity.
As far the improvement of glucose metabolism, it has been reported that the 11βHSD1 activity in pancreatic β cells could relate to the suppression of insulin secretion and the 11βHSD1 activity could be involved in the suppression of glucose uptake in human muscle cells. Thus, an 11βHSD1 inhibitor has potential to improve hyperglycemia directly.
Additionally, 11βHSD1 has been shown to function in nerve cells or immunocytes, and the 11βHSD1 inhibitor is also expected to have therapeutic effects on diseases caused by the above abnormalities.
Various 11βHSD1 inhibitors have been reported. For example, it is reported that derivatives with pyrazole ring in Patent Document 1, and amide derivatives in Patent Document 2 are effective as 11βHSD1 inhibitor.

[Patent Document 1] WO2005/016877 pamphlet
[Patent Document 2] WO2004/089470 pamphlet

DISCLOSURE OF INVENTION

PROBLEMS TO BE RESOLVED BY THE INVENTION

A development of a pharmaceutically satisfiable compound which shows 11βHSD1 inhibitory effect as a therapeutic agent for preventing and/or treating diseases, including type II diabetes, abnormal glucose tolerance, hyperglycemia, insulin resistance, hypo-HDL-emia, hyper-LDL-emia, dyslipidemia, hyperlipidemia, hypertriglyceridemia, hypercholesterolemia, hypertension, arteriosclerosis, angiostenosis, atherosclerosis, obesity, cognitive disorder, glaucoma, retinopathy, dementia, Alzheimer disease, osteoporosis, immune disorder, syndrome X, depression, cardiovascular disease, neurodegenerative disease, has now been desired.

MEANS OF SOLVING THE PROBLEMS

Until now, the following [1] pyrazole-3-carboxylic acid amide derivatives of formula (1) has not been prepared for 11βHSD1 inhibitor, and the inhibitory activity thereof has been completely unknown. As a result of extensive studies of the derivatives in order to achieve the subject, the inventors have found that pyrazole-3-carboxylic acid amide derivatives of formula (1), which are substituted with alkyl etc. at 1-position and dialkylamino etc. at 5-position, have high 11βHSD1 inhibitory activity.
The inventors have found that pyrazole-3-carboxylic acid amide derivatives of formula (1) or pharmaceutically acceptable salts thereof if needed, which are referred to as inventive compounds hereinafter, have an excellent 11βHSD1 inhibitory activity, and have achieved this invention.

Specifically, the invention relates to the following embodiments:

[1] A compound of formula (1):

[Chemical Formula 1]

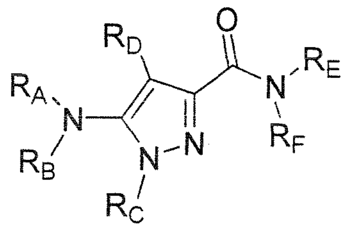

(1)

wherein

$R_A$ and $R_B$ are each independently optionally substituted alkyl, optionally substituted cycloalkyl, optionally substituted heterocycloalkyl, or a group of formula: -Rw-Rx-Ry-Rz;
Rw is, independently when it exists more than one, optionally substituted alkylene or optionally substituted cycloalkylene;
Rx is, independently when it exists more than one, a single bond, oxygen atom, or a group of formula: $-S(O)_n-$, -C(O)-, $-NR^3-$, -OC(O)-, -C(O)O-, $-CONR^3-$, $-NR^3CO-$, $-SO_2N^3-$, $-NR^3SO_2-$ or - $NR^3CONR^4-$;
Ry is, independently when it exists more than one, a single bond or optionally substituted alkylene;
Rz is, independently when it exists more than one, optionally substituted alkyl, optionally substituted cycloalkyl, optionally substituted aryl, optionally substituted heteroaryl or optionally substituted heterocycloalkyl;
$R^3$ and $R^4$ are each independently hydrogen atom or optionally substituted alkyl;
n is 0, 1 or 2;
$R_C$ is optionally substituted alkyl, optionally substituted cycloalkyl or optionally substituted cycloalkylalkyl;
$R_D$ is hydrogen atom, halogen atom, cyano or optionally substituted alkyl;
$R_E$ is hydrogen atom or optionally substituted alkyl;
$R_F$ is a group selected from the following formulae (G1):

[Chemical Formula 2]

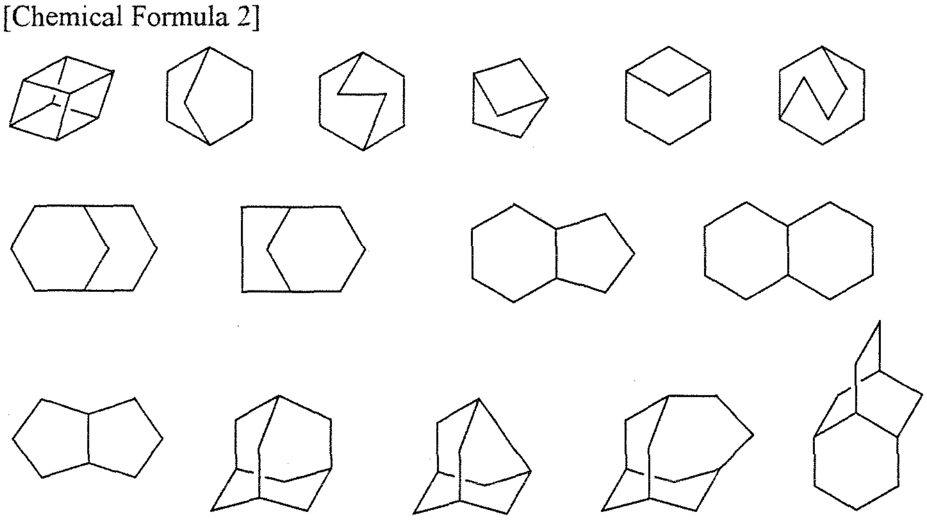

(G1)

,

wherein one of hydrogen atoms is a bond, which may be optionally substituted;
provided that if both $R_A$ and $R_B$ are selected from the following group X, then $R_F$ is a group of the following formula (2):

[Chemical Formula 3]

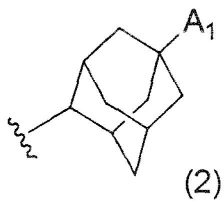

(2),

$A_1$ is $COOR^1$, $CONR^1R^2$, $SO_2NR^1R^2$, $COOR^1$-substituted alkyl, $CONR^1R^2$-substituted alkyl, or $SO_2NR^1R^2$-substituted alkyl, $R^1$ and $R^2$ are each independently hydrogen atom or optionally substituted alkyl, or $R^1$ and $R^2$ may combine each other together with the adjacent nitrogen atom to form optionally substituted saturated heterocycle;
the group X is optionally substituted alkyl, optionally substituted piperidinyl, optionally substituted pyrrolidinyl, optionally substituted arylalkyl, optionally substituted heteroarylalkyl, optionally substituted piperidinylalkyl or optionally substituted pyrrolidinylalkyl, wherein the substituent is hydroxyl, oxo, halogen atom, cyano, nitro, alkyl, alkoxy, amino which may be optionally substituted by alkyl or arylalkyl, methylenedioxy, trihalomethyl, or trihalomethoxy; or a pharmaceutically acceptable salt thereof;

[2] The compound of [1] of formula (3):

[Chemical Formula 4]

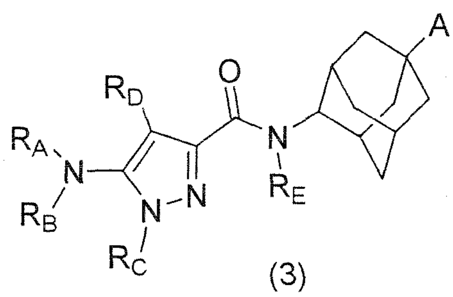

(3)

wherein

$R_A$ and $R_B$ are each independently optionally substituted alkyl, optionally substituted cycloalkyl, optionally substituted heterocycloalkyl, or a group of formula: -Rw-Rx-Ry-Rz;
Rw is, independently when it exists more than one, optionally substituted alkylene or optionally substituted cycloalkylene;
Rx is, independently when it exists more than one, a single bond, oxygen atom, or a group of formula: $-S(O)_n-$, -C(O)-, $-NR^3-$, -OC(O)-, -C(O)O-, $-CONR^3-$, $-NR^3CO-$, $-SO_2NR^3-$, $-NR^3SO_2-$ or $-NR^3CONR^4-$;
Ry is, independently when it exists more than one, a single bond or optionally substituted alkylene;
Rz is, independently when it exists more than one, optionally substituted alkyl, optionally substituted cycloalkyl, optionally substituted aryl, optionally substituted heteroaryl or optionally substituted heterocycloalkyl;
$R^3$ and $R^4$ are each independently hydrogen atom or optionally substituted alkyl;
n is 0, 1 or 2;
$R_C$ is optionally substituted alkyl, optionally substituted cycloalkyl or optionally substituted cycloalkylalkyl;
$R_D$ is hydrogen atom, halogen atom, cyano or optionally substituted alkyl;
$R_E$ is hydrogen atom or optionally substituted alkyl;
A is hydrogen atom, halogen atom, hydroxyl, cyano, or a group of formula: $COOR^1$, $CONR^1R^2$, $SO_2NR^1R^2$, $COOR^1$-substituted alkyl, $CONR^1R^2$-substituted alkyl, or $SO_2NR^1R^2$-substituted alkyl, $R^1$ and $R^2$ are each independently hydrogen atom or optionally substituted alkyl, or $R^1$ and $R^2$ may combine each other together with the adjacent nitrogen atom to form optionally substituted saturated heterocycle;
provided that if both $R_A$ and $R_B$ are selected from the following group X, then A is $COOR^1$, $CONR^1R^2$, $SO_2NR^1R^2$, $COOR^1$-substituted alkyl, $CONR^1R^2$-substituted alkyl, or $SO_2NR^1R^2$-substituted alkyl;

the group X is optionally substituted alkyl, optionally substituted piperidinyl, optionally substituted pyrrolidinyl, optionally substituted arylalkyl, optionally substituted heteroarylalkyl, optionally substituted piperidinylalkyl, or optionally substituted pyrrolidinylalkyl, wherein the substituent is hydroxyl, oxo, halogen atom, cyano, nitro, alkyl, alkoxy, amino which may be optionally substituted by alkyl or arylalkyl, methylenedioxy, trihalomethyl, or trihalomethoxy; or a pharmaceutically acceptable salt thereof;

[3] The compound of [2], wherein $R_C$ is optionally substituted alkyl, $R_D$ is hydrogen atom, halogen atom or optionally substituted alkyl, $R_E$ is hydrogen atom, A is halogen atom, hydroxyl, cyano, or a group of formula: $COOR^1$, $CONR^1R^2$, $SO_2NR^1R^2$, $COOR^1$-substituted alkyl, $CONR^1R^2$-substituted alkyl or $SO_2NR^1R^2$-substituted alkyl, $R^1$ and $R^2$ are each independently hydrogen atom or optionally substituted alkyl, or $R^1$ and $R^2$ may combine each other together with the adjacent nitrogen atom to form optionally substituted saturated heterocycle, or a pharmaceutically acceptable salt thereof;
[4] The compound of either [2] or [3], wherein $R_A$ and $R_B$ are each independently optionally substituted alkyl, optionally substituted cycloalkyl, optionally substituted cycloalkylalkyl or optionally substituted heterocycloalkyl, A is a group of formula: $COOR^1$, $CONR^1R^2$ or $SO_2NR^1R^2$, $R^1$ and $R^2$ are each independently hydrogen atom or optionally substituted alkyl, $R_A$ is optionally substituted cycloalkyl or optionally substituted cycloalkylalkyl, $R_B$ is optionally substituted alkyl, optionally substituted cycloalkyl, optionally substituted heterocycloalkyl, or a group of formula: -Rw-Rx-Ry-Rz wherein Rw, Rx, Ry and Rz are the same as defined in [2]; or $R_A$ is optionally substituted alkyl, $R_B$ is a group of formula: -Rw-Rx-Ry-Rz wherein Rw, Rx, Ry and Rz are the same as defined in [2], or a pharmaceutically acceptable salt thereof;
[5] The compound of any one of [2] to [4], wherein $R_A$ and $R_B$ are each independently optionally substituted alkyl, optionally substituted cycloalkyl or optionally substituted cycloalkylalkyl, A is a group of formula: $COOR^1$, $CONR^1R^2$ or $SO_2NR^1R^2$, $R^1$ and $R^2$ are each independently hydrogen atom or optionally substituted alkyl, or a pharmaceutically acceptable salt thereof;
[6] The compound of either [4] or [5], wherein A is a group of formula: $CONR^1R^2$, $R^1$ and $R^2$ are each independently hydrogen atom or alkyl which may be optionally substituted by hydroxyl, alkoxy, benzenesulfonyl or pyridyl, or a pharmaceutically acceptable salt thereof;
[7] The compound of [6], wherein A and nitrogen atom on which adamantyl group is substituted are arranged in *E*-configuration, or a pharmaceutically acceptable salt thereof;

[8] The compound of any one of [2] to [4], wherein $R_A$ is optionally substituted cycloalkyl or optionally substituted cycloalkylalkyl, $R_B$ is optionally substituted alkyl, optionally substituted cycloalkyl, optionally substituted heterocycloalkyl, or a group of formula: -Rw-Rx-Ry-Rz wherein Rw, Rx, Ry and Rz are the same as defined in [2], or a pharmaceutically acceptable salt thereof;
[9] The compound of [8], wherein $R_B$ is optionally substituted alkyl, optionally substituted heterocycloalkyl, or a group of formula: -Rw-Rx-Ry-Rz wherein Rw is optionally substituted alkylene, Rx is a single bond, oxygen atom, or a group of formula: $-S(O)_n-$, Ry is a single bond, Rz is optionally substituted aryl or optionally substituted heterocycloalkyl, or a pharmaceutically acceptable salt thereof;

[10] The compound of any one of [2] to [4], wherein $R_A$ is optionally substituted alkyl, $R_B$ is a group of formula: -Rw-Rx-Ry-Rz wherein Rw, Rx, Ry and Rz are the same as defined in [2], or a pharmaceutically acceptable salt thereof;
[11] The compound of [10], wherein Rx is a group of formula: $-S(O)_n-$, -C(O)-, $-NR^3-$, OC(O)-, -C(O)O-, $-CONR^3-$, $-NR^3CO-$, $-SO_2NR^3-$, $-NR^3SO_2-$ or $-NR^3CONR^4-$, $R^3$ and $R^4$ are each independently hydrogen atom or optionally substituted alkyl, n is 0, 1 or 2, or a pharmaceutically acceptable salt thereof;
[12] The compound of [11], wherein Rw is optionally substituted alkylene, Rx is a group of formula: $-S(O)_n$, Ry is a single bond, Rz is optionally substituted alkyl, or a pharmaceutically acceptable salt thereof;
[13] The compound of [10], wherein Rx is oxygen atom, Rz is optionally substituted cycloalkyl, optionally substituted aryl, optionally substituted heteroaryl or optionally substituted heterocycloalkyl, or a pharmaceutically acceptable salt thereof;
[14] The compound of [13], wherein Rw is optionally substituted alkylene, Ry is a single bond, Rz is optionally substituted aryl or optionally substituted heterocycloalkyl, or a pharmaceutically acceptable salt thereof;
[15] The compound of [10], wherein Rx is a single bond, Rz is optionally substituted cycloalkyl or optionally substituted

heterocycloalkyl, or a pharmaceutically acceptable salt thereof;

[16] The compound of [15], wherein Rw is optionally substituted alkylene, Ry is a single bond, Rz is optionally substituted cycloalkyl or optionally substituted heterocycloalkyl, or a pharmaceutically acceptable salt thereof;

[17] The compound of [10], wherein Rx is a single bond, Rz is substituted aryl, substituted heteroaryl or substituted heterocycloalkyl, in which the substituent is $-COR^5$, $-S(O)_nR^5$, $-NR^{7a}COR^5$,$-SO_2NR^{7a}R^{7b}$, $-NR^{7a}CONR^{7b}R^5$, $-OR^6$ or $-(CH_2)_mR^6$, $R^5$ is alkyl, cycloalkyl, aryl, heteroaryl or heterocycloalkyl, $R^6$ is cycloalkyl, aryl, heteroaryl or heterocycloalkyl, the alkyl, cycloalkyl, aryl, heteroaryl and heterocycloalkyl groups in $R^5$ and $R^6$ may be further optionally substituted by halogen atom, haloalkyl, haloalkoxy, alkyl, hydroxyl, alkoxy, $-NR^{8a}R^{8b}$, alkylsulfonyl, cyano, cycloalkyl, cycloalkylsulfonyl, alkoxyalkoxy, hydroxyalkoxy, cycloalkyloxyalkyl, cycloalkyloxy, haloalkoxyalkyl, hydroxyalkyl, alkoxyalkyl, $NR^{8a}R^{8b}$-substituted alkyl, alkylsulfonylalkyl, cyanoalkyl, cycloalkylalkyl, cycloalkylsulfonylalkyl, alkoxyalkoxyalkyl, hydroxyalkoxyalkyl or nitrogen-containing saturated heterocycle, $R^{7a}$, $R^{7b}$, $R^{8a}$ and $R^{8b}$ are each independently hydrogen atom or alkyl, n and m are each independently 0, 1 or 2, or a pharmaceutically acceptable salt thereof;

[18] The compound of [17], wherein Rw is optionally substituted alkylene, Ry is a single bond, Rz is substituted aryl or substituted heterocycloalkyl, in which the substituent is $-COR^5$ or $-S(O)_nR^5$, or a pharmaceutically acceptable salt thereof;

[19] The compound of [10], wherein Rw is optionally substituted cycloalkylene, Rx is a single bond, Ry is a single bond, Rz is optionally substituted aryl, or a pharmaceutically acceptable salt thereof;

[20] The compound of [2], wherein $R_A$ is tetrahydropyranyl, $R_B$ is alkyl or cycloalkyl, or a pharmaceutically acceptable salt thereof;

[21] The compound of [2] of formula (4):

[Chemical Formula 5]

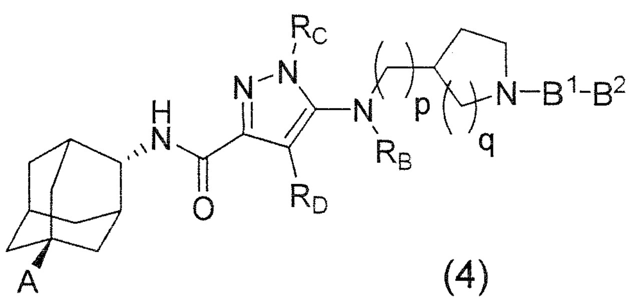

(4)

wherein p is 0, 1 or 2, q is 1 or 2, $B^1$ is a single bond, carbonyl or sulfonyl, $B^2$ is optionally substituted alkyl, optionally substituted cycloalkyl, optionally substituted heterocycloalkyl, optionally substituted aryl, optionally substituted heteroaryl, optionally substituted alkylamino, optionally substituted dialkylamino, optionally substituted cycloalkylamino, optionally substituted heterocycloalkylamino, optionally substituted arylamino or optionally substituted heteroarylamino, provided that $B^1$ is a single bond, then $B^2$ is optionally substituted aryl or optionally substituted heteroaryl, or a pharmaceutically acceptable salt thereof;

[22] The compound of [21], wherein $B^1$ is a single bond, $B^2$ is optionally substituted aryl or optionally substituted heteroaryl, or a pharmaceutically acceptable salt thereof;

[23] The compound of [22], wherein $B^2$ is optionally substituted aryl, or a pharmaceutically acceptable salt thereof;

[24] The compound of [22], wherein $B^2$ is optionally substituted heteroaryl, or a pharmaceutically acceptable salt thereof;

[25] The compound of [24], wherein $B^2$ is optionally substituted pyridyl, or a pharmaceutically acceptable salt thereof;

[26] The compound of [21], wherein $B^1$ is carbonyl, $B^2$ is optionally substituted aryl, optionally substituted alkyl, optionally substituted cycloalkyl or optionally substituted heteroaryl, or a pharmaceutically acceptable salt thereof;

[27] The compound of [26], wherein the optionally substituted alkyl group in $B^2$ is optionally substituted benzyl, or a pharmaceutically acceptable salt thereof;

[28] The compound of [26], wherein the optionally substituted cycloalkyl group in $B^2$ is cyclopropyl or cyclobutyl

substituted by optionally substituted aryl, or a pharmaceutically acceptable salt thereof;

[29] The compound of [26], wherein $B^2$ is optionally substituted aryl, or a pharmaceutically acceptable salt thereof;

[30] The compound of [26], wherein $B^2$ is optionally substituted heteroaryl, or a pharmaceutically acceptable salt thereof;

[31] The compound of [26], wherein the optionally substituted heteroaryl group in $B^2$ is optionally substituted pyridyl, or a pharmaceutically acceptable salt thereof;

[32] The compound of [26], wherein $B^2$ is fluorine-substituted alkyl, or a pharmaceutically acceptable salt thereof;

[33] The compound of [21], wherein $B^1$ is sulfonyl, $B^2$ is optionally substituted alkyl, optionally substituted cycloalkyl, optionally substituted heterocycloalkyl, optionally substituted aryl or optionally substituted heteroaryl, or a pharmaceutically acceptable salt thereof;

[34] The compound of [33], wherein $B^2$ is optionally substituted aryl, or a pharmaceutically acceptable salt thereof;

[35] The compound of [33], wherein the optionally substituted alkyl group in $B^2$ is fluorine-substituted alkyl, or a pharmaceutically acceptable salt thereof;

[36] The compound of [33], wherein the optionally substituted heteroaryl group in $B^2$ is optionally substituted pyridyl, or a pharmaceutically acceptable salt thereof;

[37] The compound of [33], wherein the optionally substituted alkyl group in $B^2$ is optionally substituted benzyl, or a pharmaceutically acceptable salt thereof;

[38] The compound of [21], wherein $B^1$ is carbonyl, $B^2$ is optionally substituted alkylamino, optionally substituted dialkylamino, optionally substituted cycloalkylamino, optionally substituted heterocycloalkylamino, optionally substituted arylamino or optionally substituted heteroarylamino, or a pharmaceutically acceptable salt thereof;

[39] The compound of [38], wherein $B^2$ is optionally substituted arylamino or optionally substituted heteroarylamino, or a pharmaceutically acceptable salt thereof;

[40] The compound of [38], wherein $B^2$ is optionally substituted arylamino, or a pharmaceutically acceptable salt thereof;

[41] The compound of [38], wherein the optionally substituted heteroarylamino group in $B^2$ is optionally substituted pyridylamino, or a pharmaceutically acceptable salt thereof;

[42] The compound of [38], wherein the optionally substituted alkylamino group in $B^2$ is optionally substituted benzylamino, or a pharmaceutically acceptable salt thereof;

[43] The compound of any one of [21] to [42], wherein p is 0 and q is 1, or a pharmaceutically acceptable salt thereof;

[44] The compound of any one of [21] to [42] of formula (5):

[Chemical Formula 6]

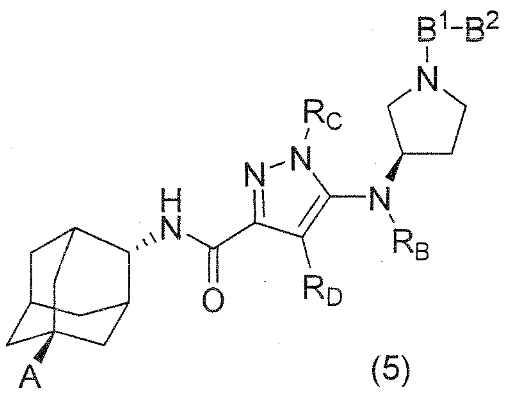

(5)

,

or a pharmaceutically acceptable salt thereof;

[45] The compound of any one of [21] to [42] of formula (6):

[Chemical Formula 7]

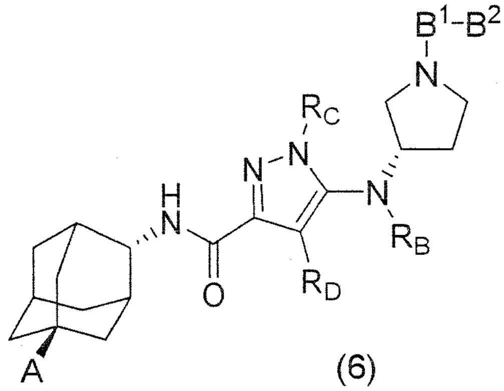

(6)

,

or a pharmaceutically acceptable salt thereof;

[46] The compound of any one of [21] to [42], wherein p is 1 and q is 2, or a pharmaceutically acceptable salt thereof;
[47] The compound of any one of [21] to [42], wherein p is 2 and q is 2, or a pharmaceutically acceptable salt thereof;
[48] The compound of any one of [21] to [42], wherein p is 0 and q is 2, or a pharmaceutically acceptable salt thereof;
[49] The compound of any one of [21] to [42] of formula (7):

[Chemical Formula 8]

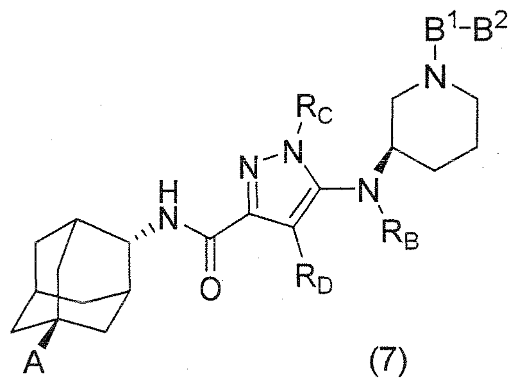

(7)

,

or a pharmaceutically acceptable salt thereof;

[50] The compound of any one of [21] to [42] of formula (8):

[Chemical Formula 9]

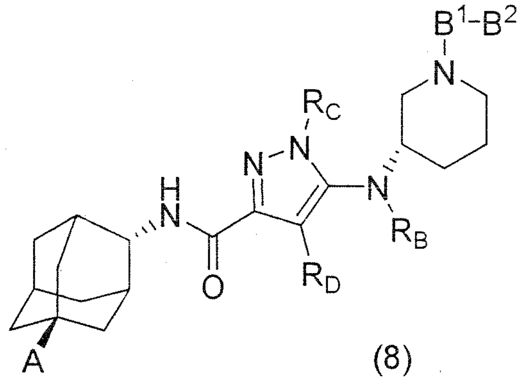

(8)

,

or a pharmaceutically acceptable salt thereof;

[51] The compound of any one of [21] to [50], wherein $R_B$ is methyl or ethyl, or a pharmaceutically acceptable salt thereof;

[52] The compound of any one of [8] to [51], wherein A is hydroxyl, or a pharmaceutically acceptable salt thereof;

[53] The compound of any one of [8] to [51], wherein A is carbamoyl, or a pharmaceutically acceptable salt thereof;

[54] The compound of any one of [4] to [53], wherein $R_D$ is chlorine atom, fluorine atom or methyl, or a pharmaceutically acceptable salt thereof;

[55] The compound of [54], wherein $R_C$ is alkyl, or a pharmaceutically acceptable salt thereof;

[56] The compound of [54], wherein $R_C$ is methyl or ethyl, or a pharmaceutically acceptable salt thereof;

[57] The compound of [56], wherein $R_E$ is hydrogen atom, or a pharmaceutically acceptable salt thereof;

[58] The compound of any one of [4] to [57], wherein A and nitrogen atom on which adamantyl group is substituted are arranged in *E*-configuration, or a pharmaceutically acceptable salt thereof;

[59] A medicament, comprising as the active ingredient the compound of any one of [1] to [58] or a pharmaceutically acceptable salt thereof;

[60] A therapeutic agent for type II diabetes, abnormal glucose tolerance, hyperglycemia, insulin resistance, dyslipidemia, hypertension, arteriosclerosis, angiostenosis, obesity, cognitive disorder, dementia, Alzheimer disease, syndrome X, depression, cardiovascular disease or atherosclerosis, comprising as the active ingredient the compound of any one of [1] to [58] or a pharmaceutically acceptable salt thereof;

[61] A therapeutic agent for diabetes, insulin resistance or type II diabetes, comprising as the active ingredient the compound of any one of [1] to [58] or a pharmaceutically acceptable salt thereof;

[62] A therapeutic agent for arteriosclerosis or atherosclerosis, comprising as the active ingredient the compound of any one of [1] to [58] or a pharmaceutically acceptable salt thereof;

[63] A therapeutic agent for syndrome X, comprising as the active ingredient the compound of any one of [1] to [58] or a pharmaceutically acceptable salt thereof;

[64] A therapeutic agent for obesity, comprising as the active ingredient the compound of any one of [1] to [58] or a pharmaceutically acceptable salt thereof;

[65] A therapeutic agent for cognitive disorder, dementia, Alzheimer disease or depression, comprising as the active ingredient the compound of any one of [1] to [58] or a pharmaceutically acceptable salt thereof;

[66] A therapeutic agent for dyslipidemia, comprising as the active ingredient the compound of any one of [1] to [58] or a pharmaceutically acceptable salt thereof; or

[67] A therapeutic agent for hypertension, comprising as the active ingredient the compound of any one of [1] to [58] or a pharmaceutically acceptable salt thereof.

The invention also relates to the following embodiments:

[68] A compound of formula (1):

[Chemical Formula 10]

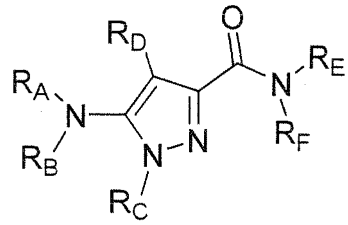

(1)

wherein

$R_A$ and $R_B$ are each independently optionally substituted alkyl, optionally substituted cycloalkyl, optionally substituted heterocycloalkyl, or a group of formula: -Rw-Rx-Ry-Rz;
Rw is, independently when it exists more than one, optionally substituted alkylene;
Rx is, independently when it exists more than one, a single bond, oxygen atom, or a group of formula: $-S(O)_n-$, -C(O)-, $-NR^3-$, -OC(O)-, -C(O)O-, $-CONR^3-$, $-NR^3CO-$, $-SO_2NR^3-$, $-NR^3SO_2-$ or - $NR^3CONR^4-$;
Ry is, independently when it exists more than one, a single bond or optionally substituted alkylene;
Rz is, independently when it exists more than one, optionally substituted alkyl, optionally substituted cycloalkyl, optionally substituted aryl, optionally substituted heteroaryl or optionally substituted heterocycloalkyl;
$R^3$ and $R^4$ are each independently hydrogen atom or optionally substituted alkyl;
n is 0, 1 or 2;
$R_C$ is optionally substituted alkyl, optionally substituted cycloalkyl or optionally substituted cycloalkylalkyl;
$R_D$ is hydrogen atom, halogen atom, cyano, optionally substituted alkyl or optionally substituted cycloalkyl;
$R_E$ is hydrogen atom or optionally substituted alkyl;
$R_F$ is a group selected from the following formulae (G1):

[Chemical Formula 11]

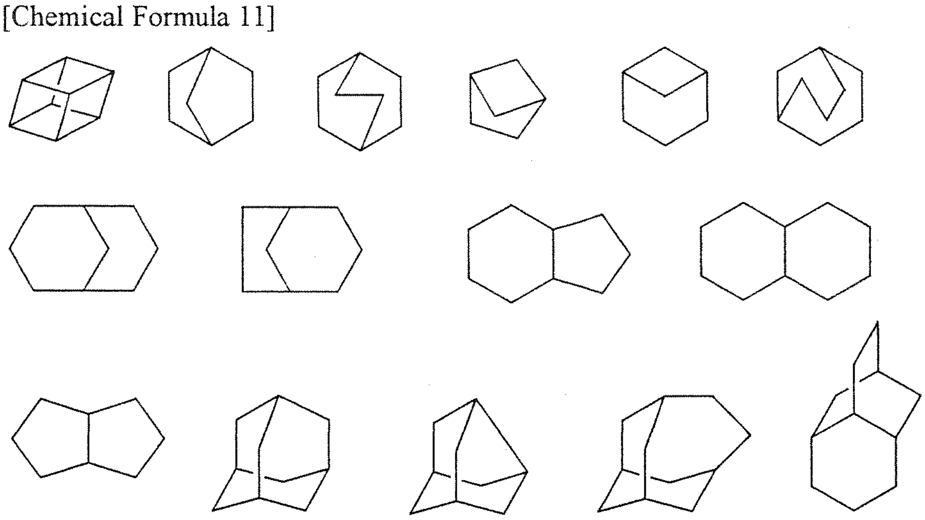

(G1)

,

wherein one of hydrogen atoms is a bond, which may be optionally substituted;
provided that if both $R_A$ and $R_B$ are selected from the following group X, then $R_F$ is a group of the following formula (2):
[Chemical Formula 12]

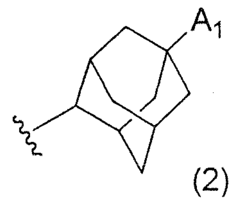

(2),

$A_1$ is $COOR^1$, $CONR^1R^2$, $SO_2NR^1R^2$, $COOR^1$-substituted alkyl, $CONR^1R^2$-substituted alkyl, or $SO^2NR^1R^2$-substituted alkyl, $R^1$ and $R^2$ are each independently hydrogen atom or optionally substituted alkyl, or $R^1$ and $R^2$ may combine each other together with the adjacent nitrogen atom to form an optionally substituted saturated heterocycle;

the group X is optionally substituted alkyl, optionally substituted piperidinyl, optionally substituted pyrrolidinyl, optionally substituted arylalkyl, optionally substituted heteroarylalkyl, optionally substituted piperidinylalkyl or optionally substituted pyrrolidinylalkyl, wherein the substituent is hydroxyl, oxo, halogen atom, cyano, nitro, alkyl, alkoxy, amino which may be optionally substituted by alkyl or arylalkyl, methylenedioxy, trihalomethyl, or trihalomethoxy; or a pharmaceutically acceptable salt thereof;

[69] The compound of [68] of formula (3):

[Chemical Formula 13]

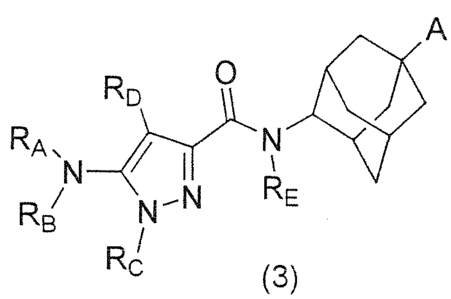

(3)

wherein $R_A$ and $R_B$ are each independently optionally substituted alkyl, optionally substituted cycloalkyl, or a group of formula: -Rw-Rx-Ry-Rz;

Rw is, independently when it exists more than one, optionally substituted alkylene;

Rx is, independently when it exists more than one, a single bond, oxygen atom, or a group of formula: $-S(O)_n-$, -C (O)-, $-NR^3-$, -OC(O)-, -C(O)O-, $-CONR^3-$, $-NR^3CO-$, $-SO_2NR^3-$, $-NR^3SO_2-$ or - $NR^3CONR^4-$;

Ry is, independently when it exists more than one, a single bond or optionally substituted alkylene;

Rz is, independently when it exists more than one, optionally substituted alkyl, optionally substituted cycloalkyl, optionally substituted aryl, optionally substituted heteroaryl or optionally substituted heterocycloalkyl;

$R^3$ and $R^4$ are each independently hydrogen atom or optionally substituted alkyl;

n is 0, 1 or 2;

$R_C$ is optionally substituted alkyl, optionally substituted cycloalkyl or optionally substituted cycloalkylalkyl;

$R_D$ is hydrogen atom, halogen atom, cyano, optionally substituted alkyl or optionally substituted cycloalkyl;

$R_E$ is hydrogen atom or optionally substituted alkyl;

A is hydrogen atom, halogen atom, hydroxyl, cyano, or a group of formula: $COOR^1$, $CONR^1R^2$, $SO_2NR^1R^2$, $COOR^1$-substituted alkyl, $CONR^1R^2$-substituted alkyl, or $SO_2NR^1R^2$-substituted alkyl, $R^1$ and $R^2$ are each independently hydrogen atom or optionally substituted alkyl, or $R^1$ and $R^2$ may combine each other together with the adjacent nitrogen atom to form optionally substituted saturated heterocycle;

provided that if both $R_A$ and $R_B$ are selected from the following group X, then A is $COOR^1$, $CONR^1R^2$, $SO_2NR^1R^2$, $COOR^1$-substituted alkyl, $CONR^1R^2$-substituted alkyl, or $SONR^1R^2$-substituted alkyl;

the group X is optionally substituted alkyl, optionally substituted piperidinyl, optionally substituted pyrrolidinyl, optionally substituted arylalkyl, optionally substituted heteroarylalkyl, optionally substituted piperidinylalkyl, or optionally substituted pyrrolidinylalkyl, wherein the substituent is hydroxyl, oxo, halogen atom, cyano, nitro, alkyl, alkoxy, amino which may be optionally substituted by alkyl or arylalkyl, methylenedioxy, trihalomethyl, or trihalomethoxy; or a pharmaceutically acceptable salt thereof;

[70] The compound of [69], wherein $R_C$ is optionally substituted alkyl, $R_D$ is hydrogen atom, halogen atom or optionally substituted alkyl, $R_E$ is hydrogen atom, A is halogen atom, hydroxyl, cyano, or a group of formula: $COOR^1$, $CONR^1R^2$, $SO_2NR^1R^2$, $COOR^1$-substituted alkyl, $CONR^1R^2$-substituted alkyl or $SO_2NR^1R^2$-substituted alkyl, $R^1$ and $R^2$ are each independently hydrogen atom or optionally substituted alkyl, or $R^1$ and $R^2$ may combine each other together with the adjacent nitrogen atom to form an optionally substituted saturated heterocycle, or a pharmaceutically acceptable salt thereof;

[71] The compound of either [69] or [70], wherein $R_A$ and $R_B$ are each independently optionally substituted alkyl, optionally substituted cycloalkyl or optionally substituted cycloalkylalkyl, A is a group of formula: $COOR^1$, $CONR^1R^2$ or $SO_2NR^1R^2$, $R^1$ and $R^2$ are each independently hydrogen atom or optionally substituted alkyl, or a pharmaceutically acceptable salt thereof;

[72] The compound of [71], wherein A is a group of formula: $CONR^1R^2$, $R^1$ and $R^2$ are each independently hydrogen atom, or a pharmaceutically acceptable salt thereof;

[73] The compound of [72], wherein A and nitrogen atom on which adamantyl group is substituted are arranged in *E*-configuration, or a pharmaceutically acceptable salt thereof;

[74] The compound of either [69] or [70], wherein $R_A$ is optionally substituted cycloalkyl or optionally substituted cycloalkylalkyl, $R_B$ is optionally substituted alkyl, optionally substituted cycloalkyl, or a group of formula: -Rw-Rx-Ry-Rz wherein Rw, Rx, Ry and Rz are the same as defined in [69], or a pharmaceutically acceptable salt thereof;

[75] The compound of either [69] or [70], wherein $R_A$ is optionally substituted alkyl, $R_B$ is a group of formula: -Rw-Rx-Ry-Rz wherein Rw, Rx, Ry and Rz are the same as defined in [69], or a pharmaceutically acceptable salt thereof;

[76] The compound of [75], wherein Rx is a group of formula: $-S(O)_n-$, -C(O)-, $-NR^3-$, - OC(O)-, -C(O)O-, $-CONR^3-$, $-NR^3CO-$, $-SO_2NR^3-$, $-NR^3SO_2-$ or $-NR^3CONR^4-$, $R^3$ and $R^4$ are each independently hydrogen atom or optionally substituted alkyl, n is 0, 1 or 2, or a pharmaceutically acceptable salt thereof;

[77] The compound of [75], wherein Rx is oxygen atom, Rz is optionally substituted cycloalkyl, optionally substituted aryl, optionally substituted heteroaryl or optionally substituted heterocycloalkyl, or a pharmaceutically acceptable salt thereof;

[78] The compound of [75], wherein Rx is a bond, Rz is optionally substituted cycloalkyl or optionally substituted heterocycloalkyl, or a pharmaceutically acceptable salt thereof;

[79] The compound of [75], wherein Rx is a bond, Rz is substituted aryl, substituted heteroaryl or substituted heterocycloalkyl, in which the substituent is $-COR^5$, $-S(O)_nR^5$, $-NR^{7a}COR^5$, - $SO_2NR^{7a}R^{7b}$, $-NR^{7a}CONR^{7b}R^5$, $-OR^6$ or $-(CH_2)_mR^6$, $R^5$ is alkyl, cycloalkyl, aryl, heteroaryl or heterocycloalkyl, $R^6$ is cycloalkyl, aryl, heteroaryl or heterocycloalkyl, the alkyl, cycloalkyl, aryl, heteroaryl and heterocycloalkyl groups in $R^5$ and $R^6$ may be further optionally substituted by halogen atom, haloalkyl, haloalkoxy, alkyl, hydroxyl, alkoxy, $-NR^{8a}R^{8b}$, alkylsulfonyl, cyano, cycloalkyl, cycloalkylsulfonyl, alkoxyalkoxy, hydroxyalkoxy, cycloalkyloxyalkyl, cycloalkyloxy, haloalkoxyalkyl, hydroxyalkyl, alkoxyalkyl, $NR^{8a}R^{8b}$-substituted alkyl, alkylsulfonylalkyl, cyanoalkyl, cycloalkylalkyl, cycloalkylsulfonylalkyl, alkoxyalkoxyalkyl, hydroxyalkoxyalkyl or nitrogen-containing saturated heterocycle, $R^{7a}$, $R^{7b}$, $R^{8a}$ and $R^{8b}$ are each independently hydrogen atom or alkyl, n and m are each independently 0, 1 or 2, or a pharmaceutically acceptable salt thereof;

[80] The compound of any one of [74] to [79], wherein A is hydroxyl, or a pharmaceutically acceptable salt thereof;

[81] The compound of any one of [74] to [79], wherein A is carbamoyl, or a pharmaceutically acceptable salt thereof;

[82] The compound of either [80] or [81], wherein $R_D$ is chlorine atom, or a pharmaceutically acceptable salt thereof;

[83] The compound of [82], wherein $R_C$ is alkyl, or a pharmaceutically acceptable salt thereof;

[84] The compound of [82], wherein $R_C$ is methyl or ethyl, or a pharmaceutically acceptable salt thereof;

[85] The compound of any one of [80] to [84], wherein A and nitrogen atom on which adamantyl group is substituted are arranged in *E*-configuration, or a pharmaceutically acceptable salt thereof;

[86] A medicament, comprising as the active ingredient the compound of any one of [68] to [85] or a pharmaceutically acceptable salt thereof;

[87] A therapeutic agent for type II diabetes, abnormal glucose tolerance, hyperglycemia, insulin resistance, dyslipidemia, hypertension, arteriosclerosis, angiostenosis, obesity, cognitive disorder, dementia, Alzheimer disease, syndrome X, depression, cardiovascular disease or atherosclerosis, comprising as the active ingredient the compound of any one of [68] to [85] or a pharmaceutically acceptable salt thereof;

[88] A therapeutic agent for diabetes, insulin resistance or type II diabetes, comprising as the active ingredient the compound of any one of [68] to [85] or a pharmaceutically acceptable salt thereof;

[89] A therapeutic agent for arteriosclerosis or atherosclerosis, comprising as the active ingredient the compound of any one of [68] to [85] or a pharmaceutically acceptable salt thereof;

[90] A therapeutic agent for syndrome X, comprising as the active ingredient the compound of any one of [68] to [85] or a pharmaceutically acceptable salt thereof;

[91] A therapeutic agent for obesity, comprising as the active ingredient the compound of any one of [68] to [85] or a pharmaceutically acceptable salt thereof;
[92] A therapeutic agent for cognitive disorder, dementia, Alzheimer disease or depression, comprising as the active ingredient the compound of any one of [68] to [85] or a pharmaceutically acceptable salt thereof;
[93] A therapeutic agent for dyslipidemia, comprising as the active ingredient the compound of any one of [68] to [85] or a pharmaceutically acceptable salt thereof; or
[94] A therapeutic agent for hypertension, comprising as the active ingredient the compound of any one of [68] to [85] or a pharmaceutically acceptable salt thereof.

ADVANTAGEOUS EFFECT OF INVENTION

The compound of the invention is useful as a therapeutic and/or preventive agent for diseases including type II diabetes, abnormal glucose tolerance, hyperglycemia, insulin resistance, hypo-HDL-emia, hyper-LDL-emia, dyslipidemia, hyperlipidemia, hypertriglyceridemia, hypercholesterolemia, hypertension, arteriosclerosis, angiostenosis, atherosclerosis, obesity, cognitive disorder, glaucoma, retinopathy, dementia, Alzheimer disease, osteoporosis, immune disorder, syndrome X, depression, cardiovascular disease, neurodegenerative disease, etc..

BEST MODE FOR CARRYING OUT THE INVENTION

The invention is illustrated in more detail as below.
The number of substituents of "optionally substituted" or "substituted" groups herein is one or more without limitation if substitution is acceptable. Each definition of each group is applied to any groups which constitute a part of other groups or a substituent thereof, unless it is specified.
The term "halogen atom" includes fluorine atom, chlorine atom, bromine atom and iodine atom, preferably fluorine atom or chlorine atom.
The term "alkyl" includes $C_1$-$C_5$ straight- and branched-chain alkyl, specifically methyl, ethyl, propyl, 1-methylethyl, butyl, 1-methylpropyl, 2-methylpropyl, 1,1-dimethylethyl, pentyl, 2,2-dimethylpropyl, etc..
The alkyl moiety of cycloalkylalkyl, arylalkyl, heteroarylalkyl, alkylsulfonyl, etc. includes the same as defined in the above alkyl.
The term "alkoxy" includes $C_1$-$C_5$ alkoxy, specifically methoxy, ethoxy, propoxy, 1-methylethoxy, butoxy, 1-methylpropoxy, 2-methylpropoxy, 1,1-dimethylethoxy, pentyloxy, 2,2-dimethylpropoxy, etc..
The alkoxy moiety of alkoxyalkyl, etc. includes the same as defined in the above alkoxy,
**[0031]** The term "trihalomethyl" includes methyl substituted by three halogen atoms.
The term "trihalomethoxy" includes methoxy substituted by three halogen atoms.
The term "haloalkyl" includes alkyl substituted by halogen atom.
The term "haloalkoxy" includes alkoxy substituted by halogen atom.
The term "alkylene" includes $C_1$-$C_5$ straight- and branched-chain alkylene, specifically methylene, ethylene, trimethylene, tetramethylene, etc..
The term "cycloalkyl" includes $C_3$-$C_8$ cycloalkyl, specifically cyclopropyl, cyclobutyl, cyclopentyl, cyclohexyl, cycloheptyl or cyclooctyl.
The cycloalkyl may have any double bonds in any substituent positions.
The cycloalkyl moiety of cycloalkyloxy, cycloalkylalkyl, etc. includes the same as defined in the above cycloalkyl.
The cycloalkyl includes any groups which are allowed to be fused with aryl or heteroaryl, for example any groups of the following formulae (B1):
[Chemical Formula 14]

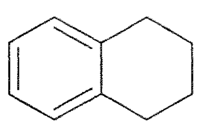
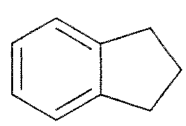
(B1)

wherein any hydrogen atom of non-aromatic ring moiety is replaced with a bond.
The term "cycloalkylene" includes $C_3$-$C_8$ cycloalkane, or any groups of the above formulae (B1) wherein two hydrogen atoms of non-aromatic ring moieties are replaced with bonds. The $C_3$-$C_8$ cycloalkane specifically includes cyclopropane, cyclobutane, cyclopentane, cyclohexane, cycloheptane or cyclooctane.
The term "aryl" includes $C_6$-$C_{10}$ aryl, specifically phenyl, 1-naphthyl, 2-naphthyl or indenyl. A preferable aryl

includes phenyl.

The term "heteroaryl" includes 5 to 10-membered mono and multi-cyclic group containing one or more (e.g., 1 to 4) heteroatoms selected from nitrogen atom, sulfur atom or oxygen atom. Specifically, it includes furyl, thienyl, pyrrolyl, azepinyl, pyrazolyl, imidazolyl, oxazolyl, isoxazolyl, thiazolyl, isothiazolyl, 1,2,4-thiadiazolyl, 1,2,4-oxadiazolyl, 1,3,4-oxadiazolyl, 1,2,4-triazolyl, 1,2,3-triazolyl, pyranyl, pyridyl, pyridazinyl, pyrimidyl, pyrazinyl, indolyl, benzothienyl, benzofuryl, quinolyl, isoquinolyl, quinazolyl, quinoxalinyl, benzoxazolyl, benzothiazolyl, pyrazyl, triazinyl, tetrazolyl, imidazo[1,2-a]pyridyl, dibenzofuranyl, benzimidazolyl, cinnolyl, indazolyl, naphthyridyl, quinolonyl or isoquinolonyl. 5 to 6-membered cyclic group containing 1 to 3 heteroatoms selected from nitrogen atom, sulfur atom or oxygen atom is preferable, specifically pyridyl, pyrazinyl, thienyl, oxazolyl, 1,2,4-oxadiazolyl or pyridazinyl.

The aryl moiety of aryloxy, etc. includes the same as defined in the above aryl. The heteroaryl moiety of heteroaryloxy includes the same as defined in the above aryl.

The term "heterocycloalkyl" includes 5 to 6-membered ring heterocycloalkyl containing one or more (e.g., 1 to 3) heteroatoms selected from nitrogen atom, sulfur atom or oxygen atom, specifically pyrrolidinyl, imidazolidinyl, piperidinyl, morpholinyl, thiomorpholinyl, dioxothiomorpholinyl, hexamethyleneiminyl, oxazolidinyl, thiazolidinyl, imidazolidinyl, oxoimidazolidinyl, dioxoimidazolidinyl, oxooxazolidinyl, dioxooxazolidinyl, dioxothiazolidinyl, tetrahydropyridinyl, tetrahydrofuranyl or tetrahydropyranyl.

The term "heterocycloalkyl" also includes any groups wherein any hydrogen atom of thiomorpholin-1-oxide, morpholin-3-one, thiomorpholin-3-one, piperidin-4-one, piperidin-3-one, piperazine-2,6-dione, morpholin-2-one, piperazine, piperazin-2-one, piperazine-2,3-dione, piperazine-2,5-dione, tetrahydropyrimidin-2(1H)-one, 1,3-oxazinan-2-one, 1,3-oxazolidine, 1,3-thiazolidine, imidazolidin-2-one, 1,3-oxazolidin-2-one, 2,5-dihydro-1H-pyrrole, imidazolidine-2,4-dione, imidazolidin-4-one, 1,4-diazepane, 1,4-oxazepan, tetrahydro-2H-pyrane, tetrahydro-2H-thiopyrane, tetrahydro-2H-thiopyrane-1-oxide, tetrahydro-2H-thiopyrane-1,1-dioxide, 1,4-diazepan-3-one, 1,4-oxazepan-3-one, aziridine, azetidine, azetidine, pyrrolidine, azepane, azocane, pyrrolidin-2-one, piperidin-2-one, azepan-2-one, azocan-2-one, 1,5-dihydro-2H-pyrrol-2-one, 5,6-dihydropyridin-2(1H)-one, 1,5,6,7-tetrahydro-2H-azepin-2-one, 1,5,6,7-tetrahydro-2H-azepin-2-one, 5,6,7,8-tetrahydroazocin-2(1H)-one, 1,2,3,4-tetrahydropyridine, 1,2,3,6-tetrahydropyridine, 2,3,4,7-tetrahydro-1H-azepine, 1,2,3,4,5,8-hexahydroazocine, tetrahydrofuran, tetrahydrothiophene, 1,2-oxathiolane, etc. are replaced with bonds.

A preferable heterocycloalkyl includes pyrrolidyl, piperidyl, morpholinyl, thiomorpholinyl, dioxothiomorpholinyl, oxazolidinyl, more preferably pyrrolidyl or piperidyl.

The term "heterocycloalkyl" also includes any groups fused with aryl or heteroaryl, for example any groups wherein any hydrogen atoms of non-aromatic cyclic moieties of the following formulae (B2) or (B3) are replaced with bonds.

The term "nitrogen-containing saturated heterocycle" includes 5 to 6-membered nitrogen-containing saturated heterocycle, etc. which contain 1 to 2 nitrogen atoms and may contain oxygen atoms or sulfur atoms, specifically pyrrolidinyl, imidazolidinyl, piperidinyl, morpholinyl, thiomorpholinyl, dioxothiomorpholinyl, hexamethyleneiminyl, oxazolidinyl, thiazolidinyl, imidazolidinyl, oxoimidazolidinyl, dioxoimidazolidinyl, oxooxazolidinyl, dioxooxazolidinyl, dioxothiazolidinyl or tetrahydropyridinyl. A preferable one includes pyrrolidinyl, piperidinyl, thiomorpholinyl, dioxothiomorpholinyl, morpholinyl.

The term "aralkyl" includes $C_7$-$C_{12}$ aralkyl wherein alkyl is substituted by aryl, specifically benzyl, 2-phenylethyl or 1-naphthylmethyl.

The aralkyl moiety of aralkyloxy includes the same as defined in the above aralkyl.

The substituents of "substituted alkyl", "substituted alkoxy" and "substituted cycloalkyl" include halogen atom, hydroxyl, nitro, cyano, $-OR^{10}$, $-OCOR^{10}$, $-COR^{10}$, $-COOR^{10}$, $C_3$-$C_6$ cycloalkyl, amino, carboxy, carbamoyl, $-NHR^{10}$, $-NR^{10}R^{11}$, $-NR^{12}COR^{10}$, $-CONR^{10}R^{11}$, $-NR^{12}CONR^{10}R^{11}$, - $NR^{12}SO_2R^{10}$ or $-SO_2R^{10}$ (wherein $R^{10}$ and $R^{11}$ are each independently cycloalkyl, $C_1$-$C_4$ alkyl, $C_6$-$C_{10}$ aryl, heteroaryl or $C_7$-$C_{12}$ aralkyl, which may further substituted by hydroxyl, halogen atom, $C_1$-$C_4$ alkoxy, cycloalkoxy, $C_1$-$C_4$ alkyl, cycloalkyl, haloalkyl, haloalkoxy, amino, $C_1$-$C_4$ alkylamino or $C_1$-$C_4$ dialkylamino, or $R^{10}$ and $R^{11}$ may combine each other together with the adjacent nitrogen atom to form an optionally substituted saturated heterocycle; $R^{12}$ is hydrogen atom or alkyl). A preferable one includes halogen atom, hydroxyl, alkyl, haloalkoxy, alkylsulfonyl and alkoxy. More preferable one includes halogen atom and alkoxy.

The substituent of "substituted cycloalkyl" also includes alkyl which may be optionally substituted by aryl, alkoxy or halogen atom.

The substituent of the substituted cycloalkyl also includes optionally substituted aryl and optionally substituted heteroaryl.

The substituents of "substituted aryl" and "substituted heteroaryl" include halogen atom, hydroxyl, nitro, cyano, nitrogen-containing saturated heterocycle, cycloalkyl, cycloalkyloxy, $C_1$-$C_4$ alkyl (wherein alkyl may be substituted by halogen atom, hydroxyl, amino, cycloalkyloxy, haloalkoxy, alkoxyalkoxy, cycloalkyl, alkoxy, alkylsulfonyl, cycloalkylsulfonyl, hydroxyalkoxy, etc.), $C_1$-$C_4$ alkoxy (wherein alkoxy may be substituted by halogen atom, hydroxyl, alkoxy, etc.), $-COR^{10}$, $-OCOR^{10}$, - $COOR^{10}$, carboxy, amino, $-NHR^{10}$, $-NR^{10}R^{11}$, $-NHCOR^{10}$, $-CONH_2$, $-CONHR^{10}$, $-CONR^{10}R^{11}$, $-SO_2NH_2$, $-SO_2NHR^{10}$, $SONR^{10}R^{11}$, $C_6$-$C_{10}$ aryl, $C_6$-$C_{10}$ aryloxy, $C_7$-$C_{12}$ aralkyloxy (wherein aryl, aryloxy or aralkyloxy may be substituted by hydroxyl, halogen atom, $C_1$-$C_4$ alkoxy, etc.), $-SO_2R^{10}$, cycloalkylsulfonyl (wherein $R^{10}$ and $R^{11}$

are the same as defined above), etc.

A preferable substituent includes nitrogen-containing saturated heterocycle, alkylsulfonyl, halogen atom, hydroxyl, alkyl (which may be optionally substituted by alkoxy or halogen atom), or alkoxy (which may be optionally substituted by alkoxy or halogen atom), etc. More preferable one includes halogen atom, alkylsulfonyl, alkyl (which may be optionally substituted by alkoxy or halogen atom), or alkoxy (which may be optionally substituted by halogen atom).

The substituent of the substituted aryl also includes $C_1$-$C_3$ alkylenedioxy such as methylenedioxy or ethylenedioxy.

The term "substituted aryl" includes any groups fused with cycloalkyl and cycloheteroalkyl, for example any groups of the above formulae (B1) and the following formulae (B2):

[Chemical Formula 15]

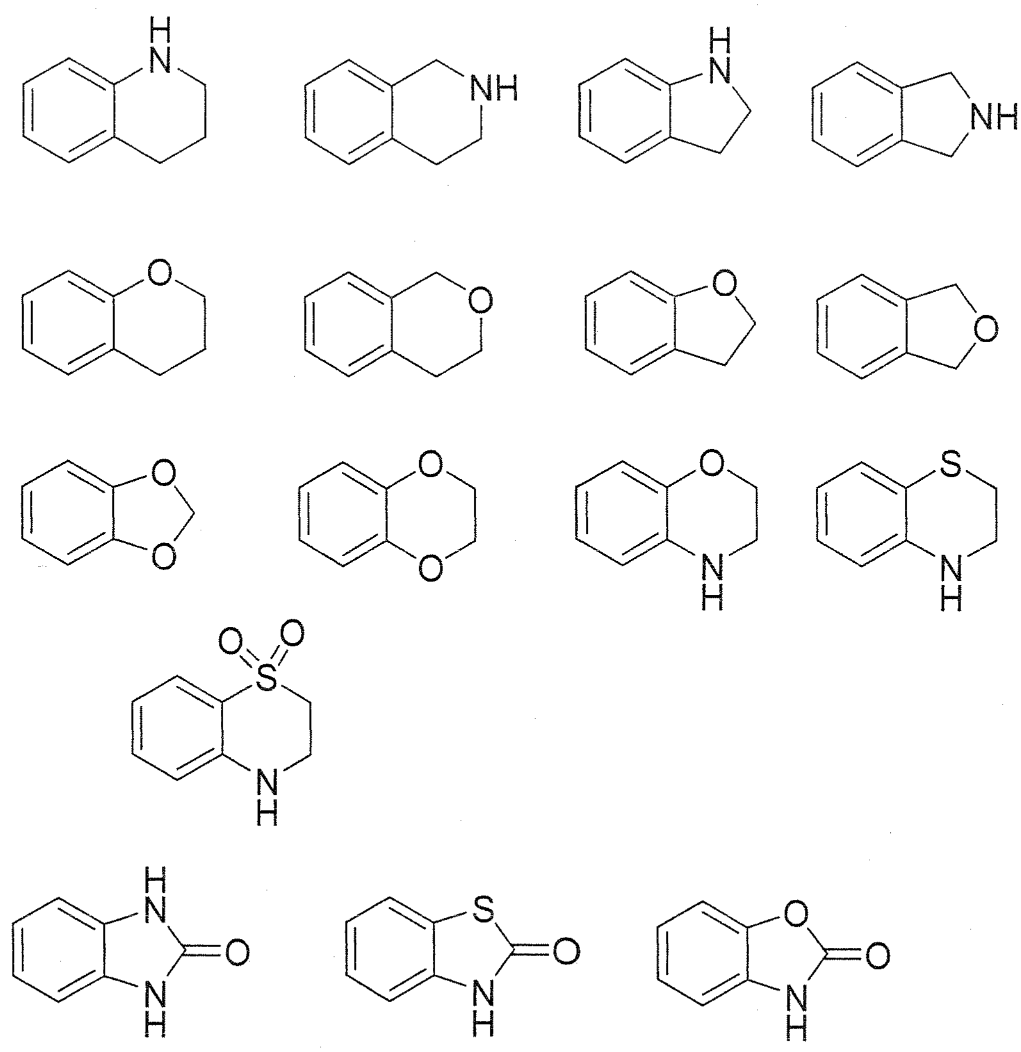

(B2)

wherein any hydrogen atoms of aromatic ring moieties are replaced with bonds, which may be further optionally substituted by the above listed substituents.

The term "substituted heteroaryl" includes any groups fused with cycloalkyl and cycloheteroalkyl, for example any groups of the following formula (B3):

[Chemical Formula 16]

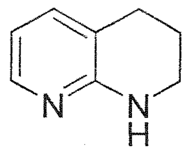 (B3)

wherein any hydrogen atoms of aromatic ring moiety are replaced with bonds, which may be further optionally substituted by the above listed substituents.

The substituents of aryl and heteroaryl moieties of "substituted aralkyl" and "substituted heteroarylalkyl" include any groups listed as the substituents of "substituted aryl" and "substituted heteroaryl".

The substituent of alkyl moiety of "substituted aralkyl" includes any groups listed as the substituents of "substituted alkyl".

The substituent of "substituted heterocycloalkyl" or "substituted nitrogen-containing saturated heterocycle" includes $C_1$-$C_4$ alkyl (which may be optionally substituted by aryl, alkoxy or halogen atom), optionally substituted aryl, optionally substituted heteroaryl, $-OR^{10}$, $-OCOR^{10}$, $-COR^{10}$, $-COOR^1$, $C_3$-$C_6$ cycloalkyl, amino carboxy, carbamoyl, $-NHR^{10}$, $-NR^{10}R^{11}$, $-NR^{12}COR^{10}$, $-CONR^{10}R^{11}$, $NR^{12}CONR^{10}R^{11}$, $-NR^{12}SO_2R^{10}$ or $-SO_2R^{10}$ (wherein $R^{10}$ and $R^{11}$ are each independently cycloalkyl, $C_1$-$C_4$ alkyl, $C_6$-$C_{10}$ aryl, heteroaryl or $C_7$-$C_{12}$ aralkyl, which may be further optionally substituted by hydroxyl, halogen atom, $C_1$-$C_4$ alkoxy, cycloalkoxy, $C_1$-$C_4$ alkyl, cycloalkyl, haloalkyl, haloalkoxy, aryl, heteroaryl, amino, $C_1$-$C_4$ alkylamino or $C_1$-$C_4$ dialkylamino, or $R^{10}$ and $R^{11}$ may combine each other together with the adjacent nitrogen atom to form an optionally substituted saturated heterocycle; $R^{12}$ is hydrogen atom or alkyl). A preferable substituent includes alkyl, $C_6$-$C_{10}$ aryl, heteroaryl, $-COR^{10}$, $-$ $CONR^{10}R^{11}$ or $-SO_2R^{10}$.

A preferable substituent of alkyl of $R^5$ or $R^6$ includes halogen atom, hydroxyl or alkoxy.

A preferable substituent of cycloalkyl, aryl, heteroaryl and heterocycloalkyl of $R^5$ or $R^6$ includes halogen atom, hydroxyl, alkyl (which may be optionally substituted by hydroxyl, alkoxy or halogen atom), and alkoxy (which may be optionally substituted by hydroxyl, alkoxy or halogen atom).

A preferable substituent of $R^1$ or $R^2$ includes halogen atom, hydroxyl, alkoxy, arylsulfonyl or pyridyl.

Alkylamino means amino group substituted by alkyl group.

Dialkylamino means amino group substituted by the same or different two alkyl groups.

Cycloalkylamino means amino group substituted by cycloalkyl group as well as cyclic amino group including pyrrolidino or piperidino.

Heterocycloalkylamino means amino group substituted by heterocycloalkyl group and also includes cyclic amino group including morpholino or thiomorpholino.

Arylamino is amino substituted by aryl group.

Heteroarylamino is amino substituted by heteroaryl group.

The substituent of "substituted alkylamino", "substituted dialkylamino", "substituted cycloalkylamino", "substituted heterocycloamino", "substituted arylamino" or "substituted heteroarylamino" includes any groups listed as the substituents of "substituted alkyl", "substituted diallyl", "substituted cycloalkyl", "substituted heterocycloalkyl", "substituted aryl" or "substituted heteroaryl".

A group selected from (G2) preferably includes adamantyl.

Adamantyl may be optionally substituted, and a preferable substituent position includes a position where A is bonded in the following formula:

[Chemical Formula 17]

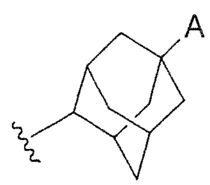

A group, wherein the substituent A and nitrogen atom, on which the adamantyl group is substituted, are arranged in

*E*-configuration is more preferable.

[Chemical Formula 18]

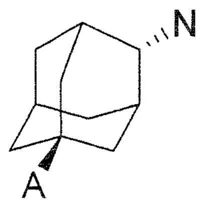

*E*-configuration

The "pharmaceutically acceptable salt" includes alkali metal salt such as potassium salt or sodium salt, alkaline earth metal salt such as calcium salt or magnesium salt, ammonium salt, a watersoluble amine addition salt such as ammonium salt or N-methylglucamine (meglumine), or a lower alkanolammonium salt of an organic amine; and, for example, hydrochloride, hydrobromide, hydroiodide, nitrate, sulfate, hydrogen sulfate, phosphate, acetate, lactate, citrate, tartrate, hydrogen tartrate, succinate, maleate, fumarate, gluconate, saccharate, benzoate, methanesulfonate, ethanesulfonate, benzenesulfonate, para-toluenesulfonate, or pamoate [1,1'-methylene-bis-2-hydroxy-3-naphthoate)], etc..

A resultant salt form of the inventive compound may be directly purified to give a salt of the inventive compound, or a free form of the inventive compound may be dissolved or suspended in an appropriate organic solvent to form a salt thereof by the addition of an acid or a base in a conventional manner.

The inventive compound and a pharmaceutically acceptable salt thereof may exist in the form of adducts with water or various solvents which are included in the invention. The invention includes all tautomers, all possible stereoisomers and all crystalline forms of the inventive compound.

The inventive compound or a pharmaceutically acceptable salt thereof may be orally or parenterally administered (e.g., intravenous, subcutaneous, or drops, intramuscular injection, subcutaneous injection, internal nasal formulation, eye-drop, suppository, transdermal administration formulation including ointment, cream or lotion, etc.) for medical use. A dosage form for oral administration includes tablet, capsule, pill, granule, powder, solution, syrup and suspension, etc. and a dosage form for parenteral administration includes aqueous or oil preparation for injection, ointment, cream, lotion, aerosol, suppository, patch, etc..

The preparation may be formulated by using conventional known techniques and comprise a conventionally acceptable carrier, excipient, binder, stabilizer, lubricant, disintegrant, etc.. The preparation for injection may further comprise an acceptable buffer, solubilizing agent, isotonic agent, etc.. The preparation may also optionally comprise flavoring agent.

The excipient may include an organic excipient including sugar derivative such as lactose, sucrose, glucose, mannitol, sorbitol; starch derivative such as corn starch, potato starch, alpha-starch, dextrin, carboxymethyl starch; cellulose derivative such as crystalline cellulose, low-substituted hydroxypropyl cellulose, hydroxypropyl methylcellulose, carboxymethylcellulose, carboxymethylcellulose calcium, internally-crosslinked carboxymethylcellulose sodium; gum arabic; dextran; pullulan; and an inorganic excipient including silicate derivative such as light anhydrous silicic acid, synthetic aluminum silicate, magnesium aluminometasilicate; phosphate such as calcium phosphate; carbonate such as calcium carbonate; sulfate such as calcium sulfate.

The lubricant may include stearic acid, metal stearate such as calcium stearate, magnesium stearate; talc; colloid silica; wax such as VEEGUM®, spermaceti; boric acid; adipic acid; sulfate such as sodium sulfate; glycol; fumaric acid; sodium benzoate; DL-leucine; fatty acid sodium salt; lauryl sulfate such as sodium lauryl sulfate, magnesium lauryl sulfate; silicic acid such as anhydrous silicic acid, silicic acid hydrate; and the above starch derivative, etc..

The binder may include polyvinylpyrrolidone, macrogol, and the above substances listed as the excipient.

The disintegrant may include the above substances listed as the excipient and chemically modified starch-cellulose such as croscarmellose sodium, sodium carboxymethyl starch or cross-linked polyvinylpyrrolidone.

The stabilizer may include paraoxybenzoic acid ester such as methylparaben, propylparaben; alcohol such as chlorobutanol, benzyl alcohol, phenylethyl alcohol; benzalkonium chloride; phenols such as phenol, cresol; thimerosal; dehydroacetic acid; and sorbic acid.

The flavoring agent may include conventionally-used sweetener, acidulant, perfume, etc..

A tablet for oral administration may comprise an excipient together with various disintegrants as well as granulating binders. A lubricant is often very useful for tablet formulation. The similar type of the solid composition may be used as a bulking agent of a gelatin capsule which may be combined by any ingredients, preferably lactose or milk sugar, or high-molecular-weight polyethyleneglycol.

The active ingredient of aqueous suspension and/or elixir for oral administration may be combined with a diluent together with various sweetening agents, flavoring agents, coloring agents or dyes, or if desired, emulsifiers and/or suspending agents. The diluent includes water, ethanol, propylene glycol, glycerin and a mixture thereof. It is conveniently included in feed or drinking water for animal in a concentration of 5-5000 ppm, preferably 25-5000 ppm.
A solution of the active ingredient for sterile injection may be usually prepared for parenteral administration (intramuscular, intraperitoneal, subcutaneous and intravenous use). A solution of the inventive compound in sesame oil or peanut oil or aqueous propylene glycol may be used. The aqueous solution should be appropriately adjusted and buffered preferably in more than 8 of pH, if needed, to firstly prepare an isotonic solution of a liquid diluent. The aqueous solution is suitable for intravenous injection. The oil solution is suitable for intra-articular, intramuscular and subcutaneous injections. All solutions may be easily prepared under sterile conditions by using typical formulation techniques known to those skilled in the art.

The inventive compound or a pharmaceutically acceptable salt thereof for the intranasal or inhalation administration may be provided in the solution or suspension form squeezed out or released by a patient from a pump spray vessel, or as an aerosol spray from a pressurized vessel or a nebulizer with using an appropriate propellant including dichlorodifluoromethane, trichlorofluoromethane, dichlorotetrafluoroethane or carbon dioxide or using other appropriate gas. A dosage unit in the pressurized aerosol may be determined by a bulb which provides a certain measured amount of the active ingredient. A solution or suspension of the active compound may be contained in the pressurized vessel or nebulizer.
A capsule and cartridge for an inhaler or insufflator (e.g., prepared from gelatin) may be formulated to contain the inventive compound and a powder composition of appropriate powder bases including lactose or starch.
The inventive compound or a pharmaceutically acceptable salt thereof may be also formulated in a composition for the anus such as a suppository or retension enema comprising conventional suppository bases including cacao butter or other glycerides.
A usage of the inventive compound or a pharmaceutically acceptable salt thereof depends on conditions, ages, administration methods, etc., and for example, it is 0.01 mg, preferably 1 mg, as a lower limit and 5000 mg, preferably 500 mg, as a upper limit per day at one time or in several divided doses for adults for oral administration, preferably depending on conditions. It is expected to be effective in 0.01 mg, preferably 0.1 mg, as a lower limit and 1000 mg, preferably 30 mg, as an upper limit per day at one time or in several divided doses for adults for intravenous administration depending on conditions.

The inventive compound may be used in combination with a drug, referred to as a combination drug hereinafter, including a therapeutic agent for diabetes or diabetic complication, anti-hyperlipidemia, antihypertensive, anti-obesity agent, diuretic, etc. for the purpose of enhancement of efficacy. The inventive compound may be administered to a subject simultaneously with a combination drug or at intervals without limitation. The inventive compound may be formulated with a combination drug to prepare a drug combination. A dosage of a combination drug may be optionally selected on the basis of clinically acceptable doses. A compounding ratio of the inventive compound and a combination drug may be optionally selected depending on administration subjects, administration routes, intended diseases, conditions and a combination thereof. For example, 0.01-100 parts of a combination drug to 1 part of the inventive compound by weight may be administered for human.

The therapeutic agent for diabetes includes insulin formulations (e.g., animal insulin formulations extracted from bovine or swine pancreas; human insulin formulations genetically engineered by using E. coli or yeast cells, etc.), insulin resistance improving agents (e.g., pioglitazone or a hydrochloride salt thereof, troglitazone, rosiglitazone or a maleate salt thereof, GI-262570, JTT-501. MCC-555, YM-440, KRP-297, CS-011, etc.), alpha-glucosidase inhibitors (e.g., voglibose, acarbose, miglitol, emiglitate, etc.), biguanides (e.g., metformin, etc.), insulin secretion stimulators (e.g., sulfonylurea agents such as tolbutamide, glibenclamide, gliclazide, chlorpropamide, tolazamide, acetohexamide, glyclopyramide, glimepiride: repaglinide, senaglinide, nateglinide, mitiglinide, etc.), dipeptidyl peptidase-IV (DPP-IV) inhibitors (e.g., sitagliptin or a phosphate salt thereof, vildagliptin, alogliptin or a benzoate salt thereof, denagliptin or a tosylate salt thereof, etc.), GLP-1, GLP-1 analogs (exenatide, liraglutide, SUN-E7001, AVE010, BIM-51077, CJC1131, etc.), protein tyrosine phosphatase inhibitors (e.g., vanadic acid, etc.), β3 agonists (e.g., GW-427353B, N-5984, etc.).

The therapeutic agent for diabetic complication includes aldose reductase inhibitors (e.g., tolrestat, epalrestat, zenarestat zopolrestat, minalrestat, fidarestat, ranirestat, SK-860, CT-112, etc.), neurotrophic factors (e.g., NGF, NT-3, BDNF, etc.), PKC inhibitors (e.g., LY-333531, etc.), AGE inhibitors (e.g., ALT946, pimagedine, piratoxatin, N-phenacylthiazolium bromide (ALT766), etc.), active oxygen removers (e.g., thioctic acid, etc.), cerebral blood-vessel dilators (e.g., tiapride, mexiletine, etc.). The anti-hyperlipidemia includes HMG-CoA reductase inhibitors (e.g., pravastatin, simvastatin, lovastatin, atorvastatin, fluvastatin, pitavastatin or a sodium salt thereof, etc.), squalene synthetase inhibitors, ACAT inhibitors, etc.. The antihypertensive includes angiotensin-converting enzyme inhibitors (e.g., captopril, enalapril, alacepril, delapril, lisinopril, imidapril, benazepril, cilazapril, temocapril, trandolapril, etc.), angiotensin II antagonists (e.g., olmesartan, medoxomil, candesartan, cilexetil, losartan, eprosartan, valsartan, telmisartan, irbesartan, tasosartan, etc.), calcium antagonists (e.g., nicardipine hydrochloride, manidipine hydrochloride, nisoldipine, nitrendipine, nilvadipine,

amlodipine, etc.), etc..

The anti-obesity agent includes central anti-obesity agents (e.g., phentermine, sibutramine, amfepramone, dexamphetamine, mazindol, SR-141716A, etc.), pancreatic lipase inhibitors (e.g., orlistat, etc.), peptidic anorexiants (e.g., leptin, CNTF (ciliary neurotrophic factor), etc.), cholecystokinin agonists (e.g., lintitript, FPL-15849, etc.), etc.. The diuretic includes xanthin derivative (e.g., sodium salicylate and theobromine, calcium salicylate and theobromine, etc.), thiazide preparations (e.g., ethiazide, cyclopenthiazide, trichlormethiazide, hydrochlorothiazide, hydroflumethiazide, bentyl hydrochlorothiazide, penflutizide, polythiazide, methyclothiazide, etc.), anti-aldosterone preparations (e.g., spironolactone, triamterene, etc.), carbonic anhydrase inhibitors (e.g., acetazolamide, etc.), chlorobenzenesulfonamide preparations (e.g., chlortalidone, mefruside, indapamide, etc.), azosemide, isosorbide, ethacrynic acid, piretanide, bumetanide, flosemide, etc..

The combination drug preferably includes GLP-1, GLP-1 analogs, alpha-glucosidase inhibitors, biguanides, insulin secretagogues, insulin resistance improving agents, DPP-IV inhibitors. The two or more combination drugs may be combined in any proportions.

The inventive compound may be combined with a combination drug to reduce dosages thereof within safe limits in terms of side effects of drugs. For example, biguanides may be reduced in lower doses than usual ones. Thus, side effects caused by the drugs may be safely prevented. In addition, dosages of a therapeutic agent for diabetic complication, anti-hyperlipidemia, antihypertensive, etc. may be reduced, and hence, side effects caused by the drugs may be effectively prevented.

Specific examples of the inventive compound of the general formula (1) may include the following compounds.

[Chemical Formula 19]

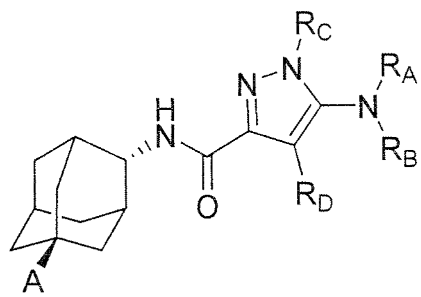

| A | $R_C$ | $R_D$ | $-NR_AR_B$ |
|---|---|---|---|
| $CONH_2$ | Et | H | 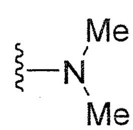 |
| $CONH_2$ | Et | Cl | 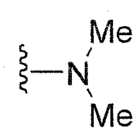 |
| $CONH_2$ | Me | Me | 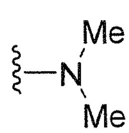 |

(continued)

| A | $R_C$ | $R_D$ | $-NR_AR_B$ |
|---|---|---|---|
| $CONH_2$ | Me | Cl | 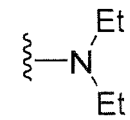 |
| $CONH_2$ | Me | Cl | 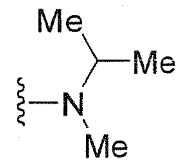 |
| $CONH_2$ | Me | Cl | 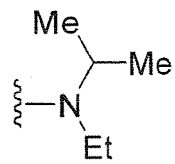 |
| $CONH_2$ | Me | Cl | 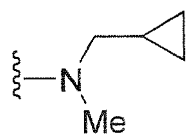 |
| OH | Me | Cl | 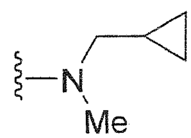 |
| $CONH_2$ | Me | Cl | 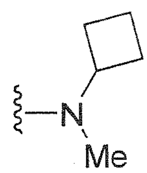 |

[Chemical Formula 20]

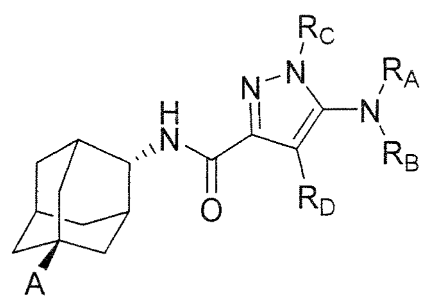

| A | $R_C$ | $R_D$ | $-NR_AR_B$ |
|---|---|---|---|
| OH | Me | Cl | 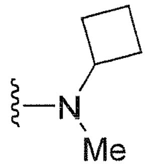 |
| $CONH_2$ | Me | Cl | 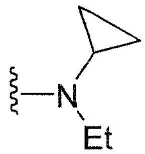 |
| OH | Me | Cl | 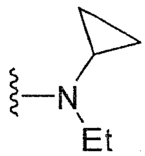 |
| $CONH_2$ | Me | Cl | 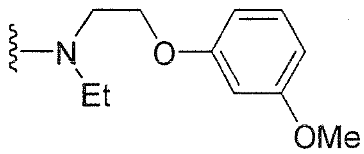 |
| OH | Me | Cl | 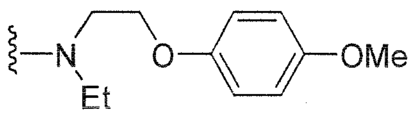 |
| OH | Me | Cl | 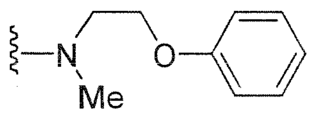 |
| $CONH_2$ | Me | Cl | 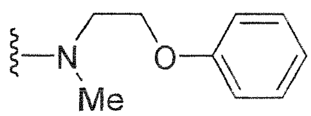 |
| $CONH_2$ | Me | Cl | 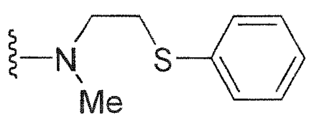 |
| OH | Me | Cl | 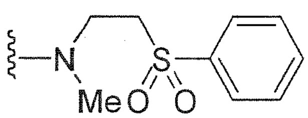 |

[Chemical Formula 21]

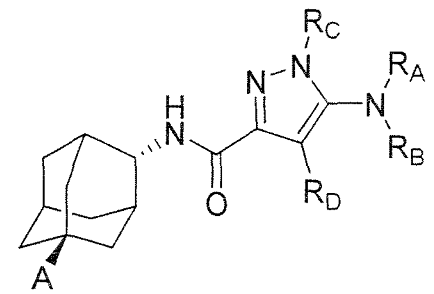

| A | $R_C$ | $R_D$ | $-NR_AR_B$ |
|---|---|---|---|
| OH | Me | Cl | 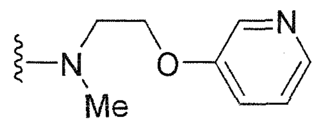 |
| $CONH_2$ | Me | H | 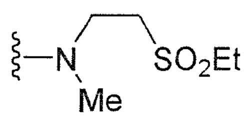 |
| $CONH_2$ | Me | Cl | 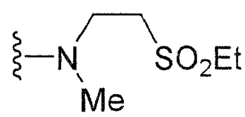 |
| $CONH_2$ | Me | H | 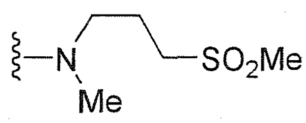 |
| OH | Me | H | 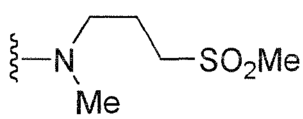 |
| $CONH_2$ | Me | H | 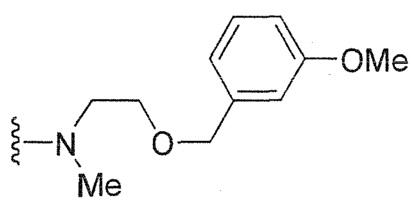 |
| OH | Me | Cl | 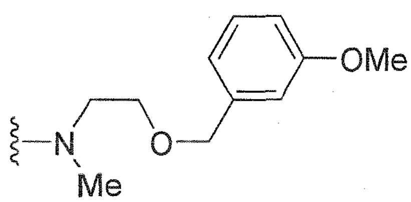 |

A preparation method of the inventive compound of formula (1) is illustrated by an example as follows, but the invention is not limited thereto.

A compound of formula (1) may be synthesized by the following methods.

Preparation 1

Among a compound of formula (1), a compound of formula (A-8) or a salt thereof may be prepared by the following methods.

[Chemical Formula 22]

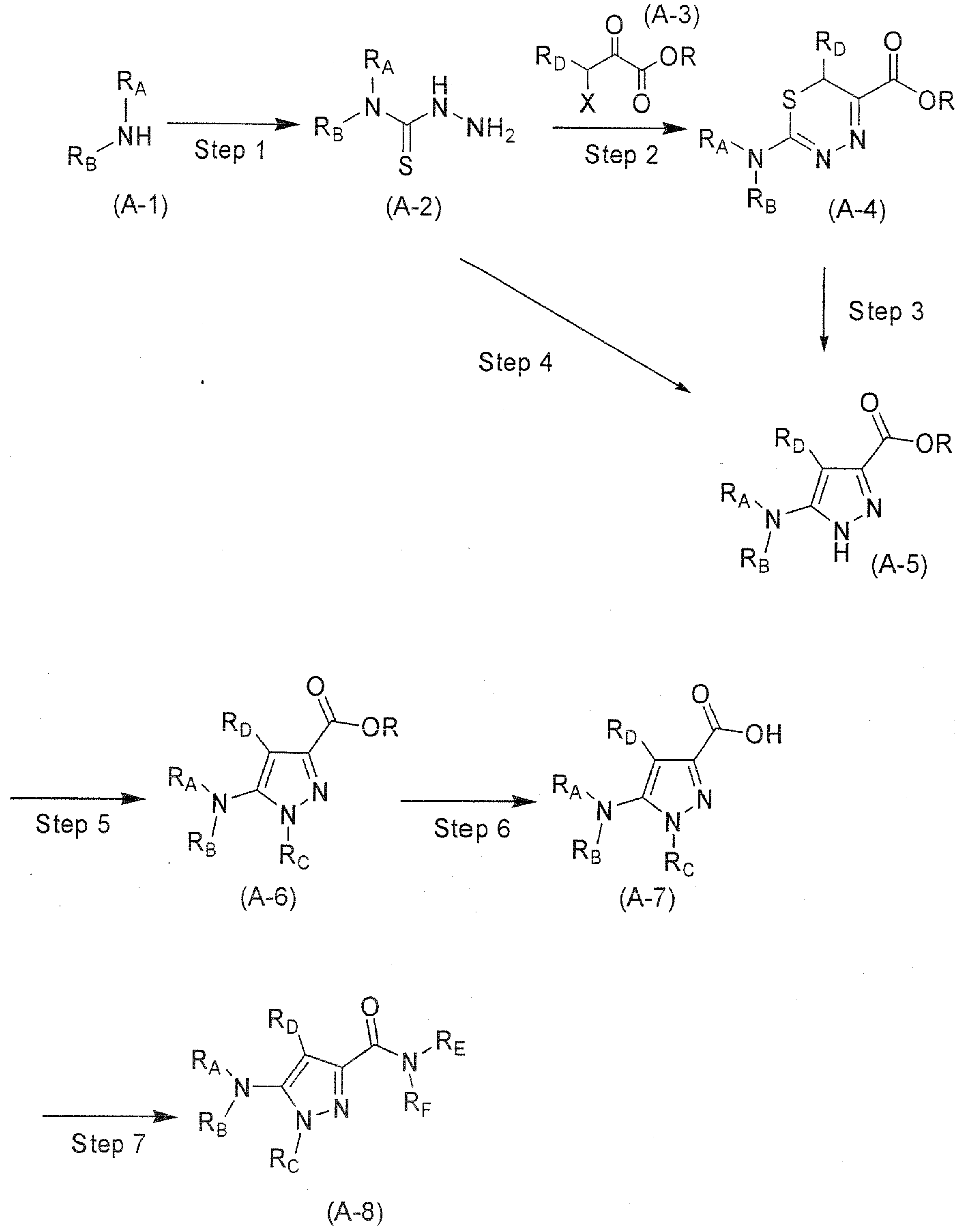


(In the above scheme, $R_A$, $R_B$, $R_C$, $R_D$, $R_E$ and $R_F$ are the same as defined above. R is methyl, ethyl or benzyl, etc.. X is halogen atom, etc.. Provided that $R_D$ is not halogen atom.)

Step 1:

$R_AR_BNH$ (A-1) gives thiosemicarbazide (A-2) in the step.

Amine (A-1) may be reacted with 1,1'-thlocarbonyldiimidazole or thiophosgene in an inert solvent usually at -10˚C to 50˚C for 0.5 to 48 hours, and then, further reacted with hydrazine or hydrazine monohydrate usually at -10˚C to reflux temperature for 0.5 to 8 hours to give thiosemicarbazide (A-2). The inert solvent includes ether type solvents such as tetrahydrofuran, diethylether, dioxane or 1,2-dimethoxyethane, hydrocarbon solvents such as toluene or benzene, polar organic solvents such as N,N-dimethylformamide, NN-dimethylacetamide, N-methyl-2-pyrrolidinone, 1,3-dimethyl-2-imidazolidinone or dimethylsulfoxide, halogenated hydrocarbon solvents such as dichloromethane, chloroform or 1.2-dichloroethane, or a mixed solvent thereof.

Alternatively, amine (A-1) is reacted with aryl halothioformate in an inert solvent usually at - 40˚C to 50˚C for 0.5 to 24 hours in the presence of a base. The obtained thiocarbamate may be reacted with hydrazine or hydrazine monohydrate in an inert solvent usually at -10˚C to reflux temperature for 0.5 to 24 hours to give thiosemicarbazide (A-2). The inert solvent includes ether type solvents such as tetrahydrofuran, diethylether, dioxane or 1,2-dimethoxyethane, hydrocarbon solvents such as toluene or benzene, halogenated hydrocarbon solvents such as dichloromethane, chloroform or 1,2-dichloroethane, polar organic solvents such as N,N-dimethylformamide, N,N-dimethylacetamide, N-methyl-2-pyrrolidinone, 1,3-dimethyl-2-imidazolidinone or dimethylsulfoxide, water, or a mixed solvent thereof. The base may be optionally selected from nitrogen-containing organic bases such as triethylamine, diisopropylethylamine, tributylamine, 1,5-diazabicyclo[4.3,0]non-5-ene (DBN), 1,4-diazabicyclo[2.2.2]octane (DABCO), 1,8-diazabicyclo[5.4.0]undec-7-ene (DBU), pyridine, dimethylaminopyridine, picoline or N-methylmorpholine (NMM), etc., or inorganic bases such as sodium bicarbonate, potassium bicarbonate, sodium carbonate, potassium carbonate, sodium hydroxide or potassium hydroxide, etc..

Step 2:

Thiosemicarbazide (A-2) may be reacted with alpha-halo ketoester (A-3) in an inert solvent usually at -10˚C to reflux temperature for 0.5 to 48 hours to give Compound (A-4). In the reaction, nitrogen-containing organic bases such as triethylamine, diisopropylethylamine, tributylamine, 1,5-diazabicyclo[4.3.0]non-5-ene (DBN), 1,4-diazabicyclo [2.2.2]octane (DABCO), 1,8-diazabicyclo[5.4.0]undec-7-ene (DBU), pyridine, dimethylaminopyridine, picoline or N-methylmorpholine (NMM), or inorganic bases such as sodium bicarbonate, potassium bicarbonate, sodium carbonate, potassium carbonate, sodium hydroxide or potassium hydroxide may be optionally added to the reaction mixture. The inert solvent includes ether type solvents such as tetrahydrofuran, diethylether, dioxane or 1,2-dimethoxyethane, hydrocarbon solvents such as toluene or benzene, polar organic solvents such as dimethylsulfoxide, alcoholic solvents such as methanol, ethanol or 2-propanol, halogenated hydrocarbon solvents such as dichloromethane, chloroform or 1,2-dichloroethane, water, or a mixed solvent thereof, etc..

Step 3:

Compound (A-4) may be treated with an organic acid such as propionic acid, acetic acid, formic acid, methanesulfonic acid, toluenesulfonic acid or trifluoroacetic acid, or a mineral acid such as hydrogen chloride, sulfuric acid or hydrogen bromide, etc. in an inert solvent or in neat usually at -10˚C to reflux temperature for 0.5 to 48 hours to give pyrazole (A-5). The inert solvent includes ether type solvents such as tetrahydrofuran, diethylether, dioxane or 1,2-dimethoxyethane, hydrocarbon solvents such as toluene or benzene, polar organic solvents such as dimethylsulfoxide, alcoholic solvents such as methanol, ethanol or 2-propanol, water, or a mixed solvent thereof, and any stable solvents under the reaction condition may be used among them.

Step 4:

Compound (A-2) gives pyrazole (A-5) in the step without isolating or purifying Compound (A-4).

The reaction system of Step 2 or a concentration residue thereof may be treated with the acid listed in Step 3 at -10˚C to reflux temperature for 0.5-48 hours to give pyrazole (A-5). The reaction may be also carried out with removing a solvent from the reaction system to give pyrazole (A-5) in the step. The solvent in an addition of acid may be selected from ether type solvents such as tetrahydrofuran, diethylether, dioxane or 1,2-dimethoxyethane, hydrocarbon solvents such as toluene or benzene, polar organic solvents such as dimethylsulfoxide, alcoholic solvents such as methanol, ethanol or 2-propanol, water, or a mixed solvent thereof, which may be stable under the reaction condition.

Step 5:

Compound (A-5) is treated with a base, followed by treating with an alkylating agent such as dialkyl sulfate or alkyl halide at -78˚C to reflux temperature to give a compound of formula (A-6) in the step.

The base includes inorganic bases such as potassium carbonate, sodium carbonate, potassium bicarbonate, sodium bicarbonate, lithium carbonate, sodium hydroxide or potassium hydroxide, metal hydrides such as sodium hydride, lithium hydride or potassium hydride, metal alkoxides such as sodium methoxide, potassium methoxide, sodium ethoxide, potassium ethoxide, sodium tertiary-butoxide or potassium tertiary-butoxide, potassium hexamethyldisilazide, sodium hexamethyldisilazide, lithium hexamethyldisilazide, or lithium diisopropylamide. The solvent includes ether type solvents such as diethylether, diisopropylether, tetrahydrofuran or 1,4-dioxane, N,N-dimethylformamide, N,N-dimethylacetamide, N-methyl-2-pyrrolidinone, 1,3-dimethyl-2-imidazolidinone, or dimethylsulfoxide.

Step 6:

An ester group of Compound (A-6) is deprotected to give a carboxylic acid compound (A-7) in the step. The step may be carried out according to methods described in Greene's Protective Groups in Organic Synthesis, John Wiley & Sons Inc., 1981.
Specifically, the following methods are carried out in the step.

(A) Compound (A-6) wherein R is methyl, ethyl, etc. may be converted to a corresponding carboxylic acid by alkali hydrolysis or acid hydrolysis. Specifically, Compound (A-6) may be treated in the presence of a hydroxide of alkali metal or alkaline-earth metal such as sodium hydroxide, potassium hydroxide, lithium hydroxide or magnesium hydroxide in water, or water and alcoholic solvents such as methanol, ethanol, 2-propanol or butanol, ether type solvents such as diethylether, diisopropylether, tetrahydrofuran or 1,4-dioxane, aromatic hydrocarbon solvents such as benzene, toluene or xylene, or a mixed solvent thereof usually at room temperature to reflux temperature for 0.5 to 48 hours to give Compound (A-7).

(B) Compound (A-6) wherein R is benzyl may be reacted in the presence of a metal catalyst such as palladium/carbon, palladium hydroxide, platinum, platinum oxide or nickel, etc. with the addition of hydrogen chloride, ammonium formate, if needed, under hydrogen gas to give Compound (A-7). The solvent includes alcoholic solvents such as methanol, ethanol, 2-propanol or butanol, ether type solvents such as diethylether, diisopropylether, tetrahydrofuran or 1,4-dioxane, aromatic hydrocarbon solvents such as benzene, toluene or xylene, ester type solvents such as ethyl acetate or methyl acetate, organic acids such as acetic acid, or a mixed solvent thereof.

Step 7:

Carboxyl group of Compound (A-7) is activated, followed by reacting with amine $R_ER_FNH$ or a salt thereof to give Compound (A-8) in the step.

The activation method of carboxy group includes a method wherein carboxy group is converted to acid anhydride, mixed acid anhydride, acid halide, activated ester or acid azide, or a method wherein a condensing agent is used. Using the acid halide method, Compound (A-7) may be reacted with a halogenating agent such as oxalyl chloride, thionyl chloride, phosphorus oxychloride or phosphorus pentachloride to give an acid halide, followed by reacting with amine $R_ER_FNH$ or a salt thereof in the presence of a base to give Compound (X-8). The base includes organic bases such as triethylamine, diisopropylethylamine, tributylamine, 1,5-diazabicyclo[4.3.0]non-5-ene (DBN), 1,4-diazabicyclo[2.2.2]octane (DABCO), 1,8-diazabicyclo[5.4.0]undec-7-ene (DBU), pyridine, dimethylaminopyridine, picoline or N-methylmorpholine (NMM), or inorganic bases such as sodium bicarbonate, potassium bicarbonate, sodium carbonate, potassium carbonate, sodium hydroxide or potassium hydroxide, without any limitation. Any solvents which may be stable under the reaction condition may be used in the step. For example, such solvents include halogenated hydrocarbon solvents such as dichloromethane, chloroform, 1,2-dichloroethane or carbon tetrachloride, ether type solvents such as diethylether, diisopropylether, tetrahydrofuran or 1,4-dioxane, aromatic hydrocarbon solvents such as benzene, toluene or xylene, ester type solvents such as ethyl acetate or methyl acetate, water, or a mixture thereof. The reaction temperature is in the range of -80˚C to reflux temperature, usually at -20˚C to ice-cooling temperature. The reaction time is in the range of 10 minutes to 48 hours.
Using the mixed acid anhydride method, Compound (A-7) may be reacted with an acid halide in the presence of a base to give a mixed acid anhydride, followed by reacting with amine $R_ER_FNH$ or a salt thereof to give Compound (A-8). The acid halide includes methoxycarbonyl chloride, ethoxycarbonyl chloride, isopropyloxycarbonyl chloride, isobutyloxycarbonyl chloride, paranitrophenoxy carbonyl chloride or t-butylcarbonyl chloride. The base includes organic bases such

as triethylamine, diisopropylethylamine, tributylamine, 1.5-diazabicyclo[4.3.0]non-5-ene (DBN), 1,4-diazabicyclo[2.2.2] octane (DABCO), 1.8-diazabicyclo[5.4.0]undec-7-ene (DBU), pyridine, dimethylaminopyridine, picoline or N-methylmorpholine (NMM), or inorganic bases such as sodium bicarbonate, potassium bicarbonate, sodium carbonate or potassium carbonate, without any limitation. Any solvents which may be stable under the reaction condition may be used in the step. For example, such solvents include halogenated hydrocarbon solvents such as dichloromethane, chloroform, 1,2-dichloroethane or carbon tetrachloride, ether type solvents such as diethylether, diisopropylether, tetrahydrofuran or 1.4-dioxane, aromatic hydrocarbon solvents such as benzene, toluene or xylene, ester type solvents such as ethyl acetate or methyl acetate, water, or a mixture thereof. The reaction temperature is in the range of -80˚C to reflux temperature, usually at -20˚C to ice-cooling temperature. The reaction time is in the range of 30 minutes to 48 hours.

Compound (A-7) may be reacted with amine $R_ER_FNH$ or a salt thereof using a condensing agent in the presence or absence of a base to give Compound (A-8). The condensing agent includes substances listed in The Experimental Chemistry (Jikken Kagaku Koza), edited by The Chemical Society of Japan, Maruzen, Vol. 22, e.g., phosphoric acid esters such as diethyl cyanophosphate or diphenyl phosphoryl azide, carbodiimides such as 1-ethyl-3-(3-dimethylaminopropyl)-carbodiimide hydrochloride or dicyclohexylcarbodiimide, combinations of disulfides such as 2,2'-dipyridyl disulfide with phosphines such as triphenylphosphine, phosphorus halides such as N,N'-bis(2-oxo-3-oxazolidinyl)phosphinic chloride, combinations of azodicarboxylic acid diesters such as diethyl azodicarboxylate with phosphines such as triphenylphosphine, 2-halo-1-lower alkylpyridinium halides such as 2-chloro-1-methylpyridinium iodide, 1,1'-carbonyldiimidazole, diphenyl phosphoryl azide (DPPA), diethylphosphoryl cyanide (DEPC), dicyclohexylcarbodiimide (DCC), carbonyldiimidazole (CDI), 1-ethyl-3-(3-dimethylaminopropyl)carbodiimide hydrochloride (EDC•HCl), O-(1H-benzotriazol-1-yl)-1,1,3,3-tetramethyl-uronium tetrahydroborate (TBTU), O-(1H-benzothazol-1-yl)-N,N,N',N'-tetramethyl-uronium hexafluorophosphate (HBTU), or (benzotriazol-1-yloxy)tris(dimethylamino)phosphonium hexafluorophosphate. Any solvents which may be stable under the reaction condition may be used in the step without any limitation. Specifically, the same solvents used in the acid-halide method, or aprotic polar solvents such as N,N-dimethylformamide, N,N-dimethylacetamide, N-methyl-2-pyrrolidinone, 1,3-dimethyl-2-imidazolidinone or dimethylsulfoxide, water, or a mixed solvent thereof may be used. The base includes organic bases such as triethylamine, diisopropylethylamine, tributylamine, l,5-diazabicyclo[4.3.0]non-5-ene (DBN), 1,4-diazabicyclo[2.2.2]octane (DABCO), 1,8-diazabicyclo[5.4.0]undec-7-ene (DBU), pyridine, dimethylaminopyridine, picoline or N-methylmorpholine (NMM) without any limitation. The reaction is usually carried out at -10˚C to reflux temperature. The reaction time is usually 0.5 to 48 hours depending mainly on reaction temperatures, starting materials and solvents.

The invention encompasses the following embodiments [PC1]-[PC13].

[PC1] A process for preparing pyrazole (A-5), wherein a reaction system with a base is applied before the addition of an acid in the step in which thiosemicarbazide (A-2) is treated with alpha-halo ketoester (A-3) to give pyrazole (A-5) with or without isolating Compound (A-4).

[PC2] The process for preparing of [PC1], wherein the base added in the reaction is an inorganic base.

[PC3] The process for preparing of [PC1], wherein the inorganic base added in the reaction is one or more combinations selected from sodium bicarbonate, potassium bicarbonate, lithium bicarbonate, sodium carbonate, potassium carbonate or lithium carbonate.

[PC4] A process for preparing pyrazole (A-5), wherein the reaction system contains water before the addition of an acid in the step in which thiosemicarbazide (A-2) is treated with alpha-halo ketoester (A-3) to give pyrazole (A-5) with or without isolating Compound (A-4).

[PC5] A process for preparing pyrazole (A-5), wherein the reaction system is concentrated before the addition of an acid in the step in which thiosemicarbazide (A-2) is treated with alpha-halo ketoester (A-3) to give pyrazole (A-5) with or without isolating Compound (A-4).

[PC6] A process for preparing pyrazole (A-5), wherein the reaction is carried out with removing solvents from the reaction system after the addition of an acid in the steps in which thiosemicarbazide (A-2) is treated with alpha-halo ketoester (A-3) to give pyrazole (A-5).

[PC7] A process for preparing pyrazole (A-5), wherein the reaction is carried out with evaporating solvents from the reaction system after the addition of an acid in the steps in which thiosemicarbazide (A-2) is treated with alpha-halo ketoester (A-3) to give pyrazole (A-5).

[PC8] A process for preparing pyrazole (A-5), wherein the added acid is an organic acid or inorganic acid in the step in which thiosemicarbazide (A-2) is treated with alpha-halo ketoester (A-3) to give pyrazole (A-5) with or without isolating Compound (A-4).

[PC9] The process for preparing of [PC8], wherein the added acid is one or more combinations selected from hydrochloric acid, hydrobromic acid, sulfuric acid, propionic acid, acetic acid, formic acid, methanesulfonic acid,

toluenesulfonic acid or trifluoroacetic acid.
[PC 10] The process for preparing of [A8], wherein the added acid is acetic acid.
[PC11] A process for preparing pyrazole (A-5), comprising one to four combinations selected from [PC1] to [PC3], [PC4], [PC5] to [PC7], [PC8] to [PC10] in the step in which thiosemicarbazide (A-2) is treated with alpha-halo ketoester (A-3) to give pyrazole (A-5) with or without isolating Compound (A-4).
[PC12] A process for preparing pyrazole (A-5), comprising a combination selected from [PC3], [PC4], [PC5] or [PC7], and [PC10] in the steps in which thiosemicarbazide (A-2) is treated with alpha-halo ketoester (A-3) to give pyrazole (A-5) with or without isolating Compound (A-4).
[PC13] A process for preparing pyrazole (A-5) of [PC11] or [PC12], wherein $R_A$ and/or $R_B$ of thiosemicarbazide (A-2) contain the same or different one or more groups selected from Cbz, Boc, tetrahydrofuranyl, tetrahydropyranyl, cyclopropyl, cyclobutyl, optionally substituted benzyloxy or optionally substituted benzylamino as a partial structure.

Preparation 2

A compound of formula (A-12) or a salt thereof among a compound of formula (I) is, for example, prepared according to the following methods.
[Chemical Formula 23]

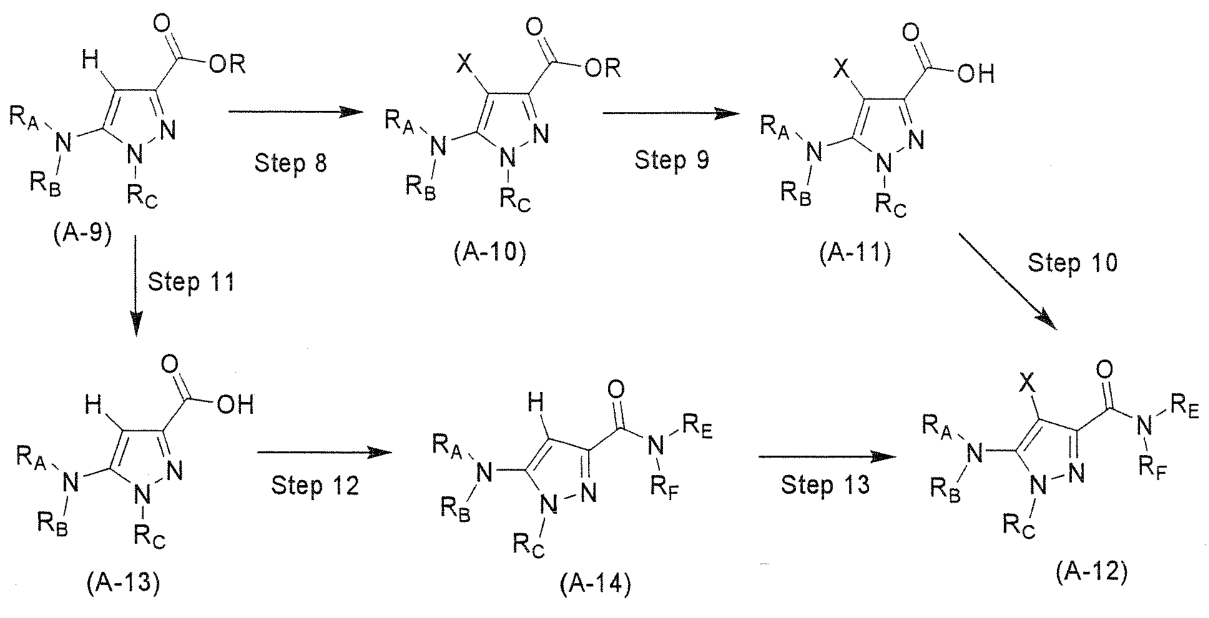


(In the above scheme, $R_A$, $R_E$, $R_C$, $R_E$ and $R_F$ are the same as defined above. R is methyl, ethyl, benzyl, etc.. X is halogen atom, etc..)

Step 8:

Halogen (X) is introduced at 4-position of pyrazole ring in Compound (A-9) to give Compound (A-10) in the step.

Halogen atom may be introduced at 4-position in Compound (A-9) by adding a halogenating agent such as N-chlorosuccinimide, N-bromosuccinimide, chlorine, bromine, iodine, iodine chloride, sulfuryl chloride, SELECTFLUOR®, 1-fluoro-4-hydroxy-1,4-diazoniabicyclo[2.2.2]octane bis(tetrafluoroborate), N-fluorobenzenesulfonimide, N-fluoro-o-benzenedisulfonimide, 1-fluoropyridinium triflate or 1-fluoro-2,6-dichloropyridinium tetrafluoroborate in the presence or absence of an acid. The acid includes hydrogen halides such as hydrogen chloride or hydrogen bromide, or organic acids such as acetic acid or propionic acid. The reaction may be also carried out using a base instead of an acid. The base includes inorganic bases such as sodium bicarbonate, potassium bicarbonate, sodium carbonate or potassium carbonate. Any solvents which may be inert under the reaction condition may be used in the step, e.g., ester type solvents

such as ethyl acetate or methyl acetate, halogenated hydrocarbon solvents such as dichloromethane, chloroform, 1,2-dichloroethane or carbon tetrachloride, aprotic polar solvents such as N,N-dimethylformamide, N,N-dimethylacetamide, N-methyl-2-pyrrolidinone or 1,3-dimethyl-2-imidazolidinone, water, or a mixed solvent thereof, The reaction temperature is usually in the range of -10˚C to reflux temperature. The reaction time is usually in the range of 0.5 to 48 hours.

Compound (A-10) may be treated by Steps 9-10 of the similar method to Preparation 1 to give Compound (A-12).

Compound (A-9) may be treated by Steps 11-12 of the similar method to Preparation 1 to give Compound (A-14).

Compound (A-14) may be treated by the similar method to Step 8 to give Compound (A-12).

Preparation 3

A compound of formula (A-17) or a salt thereof among a compound of formula (1) is prepared according to the following method.

[Chemical Formula 24]

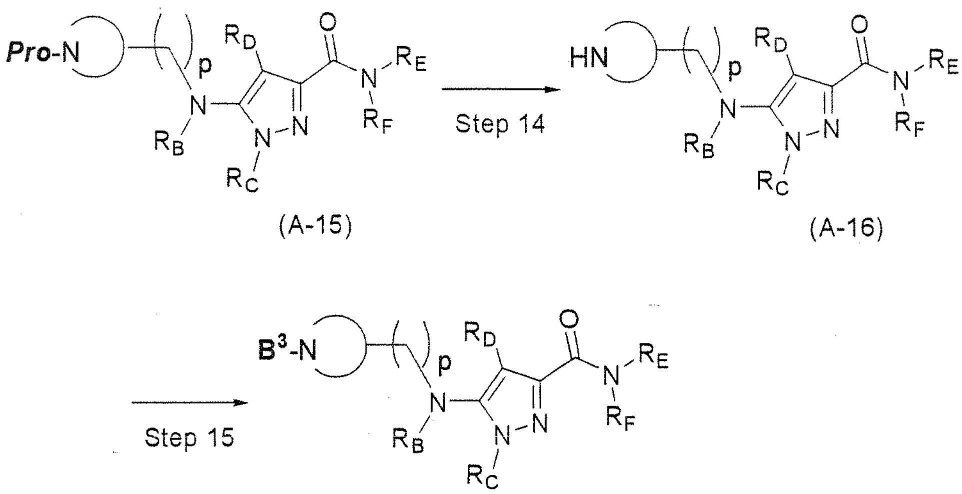


(A-15) (A-16)

(A-17)

(In the above scheme, $R_B$, $R_C$, $R_D$, $R_E$, $R_F$ and p are the same as defined above. *Pro* is a protective group of nitrogen atom. $B^3$ is acyl or sulfonyl.)

Step 14:

Compound (A-15) wherein *Pro* is benzyloxycarbonyl may be treated in the following manner to give Compound (A-16). Compound (A-15) may be treated with hydrogen in an inert solvent usually at ambient temperature to 50˚C for 0.5 to 24 hours in the presence of palladium/carbon to give Compound (A-16). Hydrogen may be used at normal pressure or with pressurized. The inert solvent includes halogenated hydrocarbon solvents such as dichloromethane, chloroform, 1,2-dichloroethane or carbon tetrachloride, ether type solvents such as diethylether, dissopropylether, tetrahydrofuran or 1,4-dioxane, aromatic hydrocarbon solvents such as benzene, toluene or xylene, ester type solvents such as ethyl acetate or methyl acetate, water, or a mixed solvent thereof. Ammonium formate may be used instead of hydrogen.

Step 15:

Acylation or sulfonylation of a deprotected amine of Compound (A-16) may give Compound (A-17) in the step.

The acylation may be carried out in the similar manner to Step 7 of Preparation 1 by using acid halide or carboxylic acid compound to give Compound (A-17) as an amide derivative.

The sulfonylation may be carried out in the similar manner to the acid-halide method of Step 7 of Preparation 1 by using sulfonyl halide such as arylsulfonyl halide to give Compound (A-17) as a sulfoneamide derivative.

Preparation 4

A compound of formula (A-18) or a salt thereof among a compound of formula (1) is prepared by the following method.

[Chemical Formula 25]

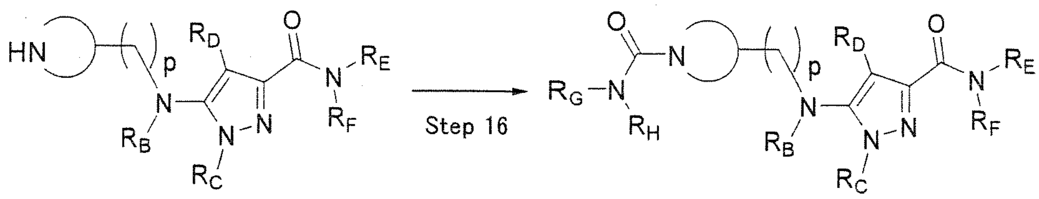


(A-16) (A-18)

(In the above scheme, $R_B$, $R_C$, $R_D$, $R_E$, $R_F$ and p are the same as defined above. $R_G$ and $R_H$ are each hydrogen atom, optionally substituted alkyl, optionally substituted aryl, optionally substituted heteroaryl, optionally substituted cycloalkyl or optionally substituted heterocycloalkyl. Alternatively; $R_G$ and $R_H$ may combine each other together with the adjacent nitrogen atom to form an optionally substituted saturated heterocycle.)

Compound (A-16) is treated with amine $R_GR_HNH$ or a salt thereof to give Compound (A-18) in the step. Amine $R_GR_HNH$ is reacted with 1,1'-carbonyldiimidazole, triphosgene, diphosgene or phosgene in an inert solvent usually at -10°C to 30°C for 0.5 to 6 hours, followed by reacting with Compound (A-16) at -10°C to reflux temperature for 0.5 to 8 hours. Compound (A-16) may be also treated earlier than amine $R_GR_HNH$. Consequently, Compound (A-18) may be prepared in this manner.

Amine $R_GR_HNH$ may be also reacted with para-nitrophenyl chloroformate or trichloromethyl chloroformate in the presence of a base in an inert solvent usually at -10°C to 30°C, followed by reacting with Compound (A-16) usually at -10°C to reflux temperature to give Compound (A-18). Compound (A-16) may be also treated earlier than amine $R_GR_HNH$. The base includes nitrogen-containing organic bases such as triethylamine, diisopropylethylamine, tributylamine, 1,5-diazabicyclo[4.3.0]non-5-ene (DBN), 1,4-diazabicyclo[2.2.2]octane (DABCO), 1,8-diazabicyclo[5.4.0]undec-7-ene (DBU), pyridine, dimethylaminopyridine, picoline or N-methylmorpholine (NMM), or inorganic bases such as potassium carbonate, sodium carbonate or sodium bicarbonate.

The inert solvent includes ether type solvents such as tetrahydrofuran, diethylether, dioxane or 1,2-dimethoxyethane, hydrocarbons such as toluene or benzene, halogenated hydrocarbon solvents such as dichloromethane, chloroform or 1,2-dichloroethane, aprotic polar solvents such as dimethylsulfoxide, N,N-dimethylformamide, N,N-dimethylacetamide, N-methyl-2-pyrrolidinone or 1,3-dimethyl-2-imidazolidinone, a mixed solvent thereof, or a mixed solvent of these solvents with water.

Compound (A-16) may be also treated with isocyanate $R_GNCO$, wherein $R_G$ is not hydrogen atom, to give Compound (A-18).

Compound (A-16) may be treated with isocyanate $R_GNCO$ usually at -10°C to reflux temperature in an inert solvent or neat in the presence or absence of a base to give Compound (A-18). The base includes nitrogen-containing organic bases such as triethylamine, diisopropylethylamine, tributylamine, 1,5-diazabicyclo[4.3.0]non-5-ene (DBN), 1,4-diazabicyclo[2.22]octane (DABCO), 1,8-diazabicyclo[5.4.0]undec-7-ene (DBU), pyridine, dimethylaminopyridine, picoline or N-methylmorpholine (NMM), or inorganic bases such as potassium carbonate, sodium carbonate or sodium bicarbonate. The inert solvent includes ether type solvents such as tetrahydrofuran, diethylether, dioxane or 1,2-dimethoxyethane, hydrocarbons such as toluene or benzene, halogenated hydrocarbon solvents such as dichloromethane, chloroform or 1,2-dichloroethane, aprotic polar solvents such as dimethylsulfoxide, N,N-dimethylformamide, N,N-dimethylacetamide, N-methyl-2-pyrrolidinone or 1,3-dimethyl-2-imidazolidinone, a mixed solvent thereof, or a mixed solvent of these solvents with water.

Preparation 5

[Chemical Formula 26]

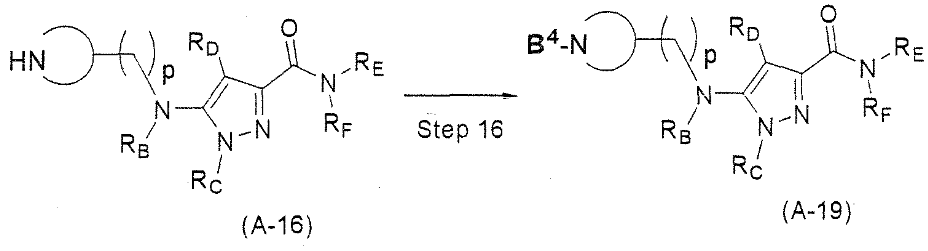


(A-16) (A-19)

(In the above scheme, $R_B$, $R_C$, $R_D$, $R_E$, $R_F$ and p are the same as defined above. $B^4$ is aryl or heteroaryl.)

Compound (A-16) may be treated with halogenated aryl or halogenated heteroaryl ($B^4$-Br, $B^4$-I, $B^4$-Cl, etc.) or aryl metal compound or heteroaryl metal compound ($B^4$-Mtl) to give Compound (A-19), in which -Mt1 is a boronic acid group $-B(OH)_2$, $-B(OMe)_2$ as a boronic acid ester group, -ZnCl as a zinc halide group, etc..

Compound (A-16) may be treated with halogenated aryl, halogenated heteroaryl, aryl metal compound or heteroaryl metal compound usually at room temperature to reflux temperature in the presence or absence of a palladium, copper or nickel metal catalyst such as tetrakis(triphenylphosphine)paliadium, dichiorodi(tris-o-tolylphosphine)palladium, tris (dibenzylideneacetone)dipalladium, copper acetate, copper iodide, nickel di(cyclooctadienyl) or nickel-carbon in the presence of a base such as sodium tertiary-butoxide, potassium carbonate, sodium bicarbonate or lithium hexamethyldisilazide, or a phosphorus ligand such as 2,2'-bis(diphenylphosphino)-1,1'-binaphthyl or triphenylphosphine, if needed, in an inert solvent or neat to give Compound (A-19). The solvent includes ether type solvents such as diethylether, diisopropylether, 1,2-dimethoxyethane, tetrahydrofuran or 1,4-dioxane, aromatic hydrocarbon solvents such as benzene, toluene or xylene, aprotic polar solvents such as dimethylsulfoxide, N,N-dimethylformamide, N,N-dimethylacetamide, N-methyl-2-pyrrolidinone or 1,3-dimethyl-2-imidazolidinone, water, or a mixture thereof. The reaction time is usually in the range of 30 minutes to 48 hours.

If any functional groups except for the intended reaction sites may be affected under the reaction conditions or be inappropriate to carry out the reactions in the above Preparations, such groups except for the intended reaction sites may be protected to carry out the reactions, followed by deprotecting to give the desired compounds. The protective group includes conventional protective groups described in the Protective Groups in Organic Synthesis as mentioned above, and specifically, the protective group for amine includes ethoxycarbonyl, t-butoxycarbonyl, acetyl or benzyl, and that of hydroxyl includes tri-lower alkyl silyl, acetyl or benzyl.

An introduction or deprotection of a protective group may be carried out according to a conventional method in the organic synthetic chemistry (see, for example, the Protective Groups in Organic Synthesis), or with some modification thereof.

Any functional groups of any intermediates or final products may be also optionally modified to give other compounds encompassed in the invention in the above Preparations. The modification of functional groups may be carried out by a conventional method (see, for example, R.C. Larock, Comprehensive Organic Transformations, 1989).

Each intermediate and the desired compound may be isolated and/or purified by a conventional purification method in the organic synthetic chemistry, e.g. neutralization, filtration, extraction, washing, drying, concentration, recrystallization, various chromatography, etc., in each Preparation. Each intermediate may be also used in the next reaction without purification.

Any optical isomers may be isolated in any steps in the above Preparations by a conventional isolating method including a method using an optically-active column or a fractionated crystallization. Any optically-active starting materials may be also used in the Preparations.

The invention encompasses any possible isomers including optical isomers, stereoisomers, tautomers such as ketoenol, and/or geometrical isomers, and a mixture thereof.

Any starting materials and intermediates may be known compounds, or be synthesized therefrom by a conventional method in the Preparations.

A configuration of two substituents on adamantane group in the inventive compound is defined as Z or E relative configuration according to C. D. Jones, M. Kaselj, et al. J. Org. Chem. 63: 2758-2760, 1998.

The invention is illustrated by the following Reference Examples, Examples and Test Examples in more detail, but is not limited thereto. Compound names do not necessarily follow IUPAC nomenclature in the following Reference Examples and Examples.

The following abbreviations may be used in the Reference Examples and Examples.

THF: tetrahydrofuran

$NaBH(OAc)_3$: sodium triacetoxyborohydride

$(Boc)_2O$: di-tert-butyldicarbonate
$Pd(OH)_2$: palladium hydroxide
DMF: N,N-dimethylformamide
WSCI•HCl: 1-(3-dimethylaminopropyl)-3-ethylcarbodiimide hydrochloride
HOBt•$H_2O$: 1-hydroxybenzotriazole monohydrate
NMP: 1-methyl-2-pyrrolidinone
Me: methyl
Et: ethyl
Boc: tert-butoxycarbonyl
Cbz: benzyloxycarbonyl
N: normal (e.g., 2N HCl is 2-normal hydrochloric acid.)
M: molar concentration (mol/L) (e.g., 2M methylamine is 2 mol/L methylamine solution.)
$t_R$: retention time

A reverse-phase preparative purification was carried out as below.
A purification was carried out by using Gilson HPLC System. YMC CombiPrep ODS-A column (5 μm, 50x20 min I.D.) was used, and a mixed solvent system of $CH_3CN$ (containing 0.035% TFA) with water (containing 0.05% TFA) was used. UV was detected in each wavelength of 210 nm, 220 nm and 254 nm.
Elution conditions were as follows.

Preparative instrument: Gilson HPLC System
Column: YMC CombiPrep ODS-A 50x20 min I.D.
Solvent: $CH_3CN$ (containing 0.035% TFA), water (containing 0.05% TFA)
Flow rate: 35 mL/min
Gradient: linear gradient from 1:99 (v/v) $CH_3CN$/water to 95:5 (v/v) $CH_3CN$/water within 13 min at 35 mL/min
obsMS [M+1]: observed protonated molecules
min: minute

LC/MS analytic conditions for identifying compounds in reverse-phase preparative purifications were as follows.
Measurement method SA:

Detection device: Detector Perkin-Elmer Sciex API150EX Mass spectrometer (40 eV)
HPLC: Shimadzu LC 10ATVP
Column: Shiseido CAPCELL PAK C18 ACR (S-5 um, 4.6x50 mm)
Solvent: Solution A: 0.35%TFA/$CH_3CN$, Solution B: 0.05%TFA/$H_2O$
Gradient condition: 0.0-0.5 min A 10%, 0.5-4.8 min Linear gradient from A 10% to 99%, 4.8-5.0 min A 99%
Flow rate: 3.5 mL/min
UV:254 nm

Measurement method SB:

Detection device: Agilent 1100 series for API series, manufactured by Applied Biosystems
HPLC: API150EX LC/MS system, manufactured by Applied Biosystems
Column: YMC CombiScreen ODS-A (S-5 μm, 12 nm, 4.6x50 mm)
Solvent: Solution A: 0.05% TFA/$H_2O$, Solution B: 0.035% TFA/MeCN
Gradient condition: 0.0-0.5 min A 90%, 0.5-4.2 min Linear gradient from A 90% to 1%, 4.2-4.4 min Linear gradient from A 1% to 99%
Flow rate: 3.5 mL/min
UV: 220 nm

## EXAMPLES

### Reference Example 1

Methyl 4-aminoadamantane-1-carboxylate hydrochloride

[Chemical Formula 27]

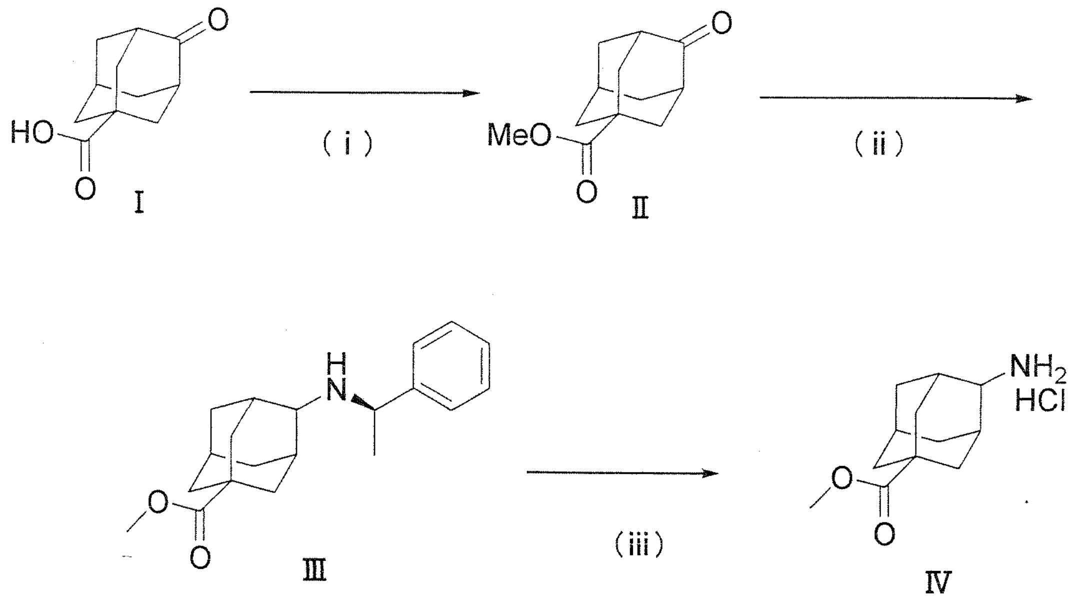


I II III IV

Step (i):

To a solution of Compound I (40.0 g) (see The Journal of Organic Chemistry, 1983, Vol. 48, page 1099) in methanol (500 mL) was added thionyl chloride (22.7 mL). The mixture was heated to reflux and stirred for 3 hours. Then, the mixture was concentrated *in vacuo*, and then extracted with saturated sodium bicarbonate water and ethyl acetate. The organic layer was dried over sodium sulfate and concentrated *in vacuo* to give Compound II (44.0 g).

Step (ii):

Compound II (55.4 g) was dissolved in dichloromethane (1.25 L), and thereto were added (R)-(+)-1-phenetylarnine (32.2 g), $NaBH(OAc)_3$ (82.0 g) and acetic acid (10 mL). The mixture was stirred at room temperature overnight. The mixture was treated with 6N hydrochloric acid, and then basified by 2N sodium hydroxide solution and extracted with chloroform. The organic layer was dried over sodium sulfate and concentrated *in vacuo*. The residue was purified by silica gel column chromatography (eluent: chloroform/methanol = 100/0 to 98/2) to give Compound III (73.6 g).

Step (iii):

To a solution of Compound III (12.6 g) in acetic acid (200 mL) was added palladium hydroxide (6.0 g), and the mixture was stirred under hydrogen (3 atm) for 9 hours. The palladium was filtered off, and then the filtrate was concentrated *in vacuo*. The residue was dissolved in saturated sodium bicarbonate water and THF, and thereto was added $(Boc)_2O$ (9.65 g). The mixture was stirred at room temperature for 1.5 hours. The reaction solution was extracted with ethyl acetate and saturated sodium bicarbonate water. The organic layer was dried over sodium sulfate and concentrated *in vacuo*. The residue was purified by silica gel column chromatography (eluent: chloroform/methanol = 19/1), and dissolved in chloroform (150 mL). Then, thereto was added 4N hydrochloric acid-dioxane (50 mL), and the mixture was stirred at room temperature overnight. The mixture was concentrated *in vacuo*, and the resulting white solid was filtered and concentrated *in vacuo* to give the titled Compound IV (7.0 g).
$^1$H-NMR (DMSO-$d_6$) δ 1.50 (m, 1H), 1.70-1.80 (m, 4H), 1.87-2.06 (m, 6H), 2.06-2.10 (m, 3H), 3.31 (s, 3H), 8.17 (bs, 3H)

Reference Example 2

5-(Dimethylamino)-1-methyl-1H-pyrazole-3-carboxylic acid

[Chemical Formula 28]

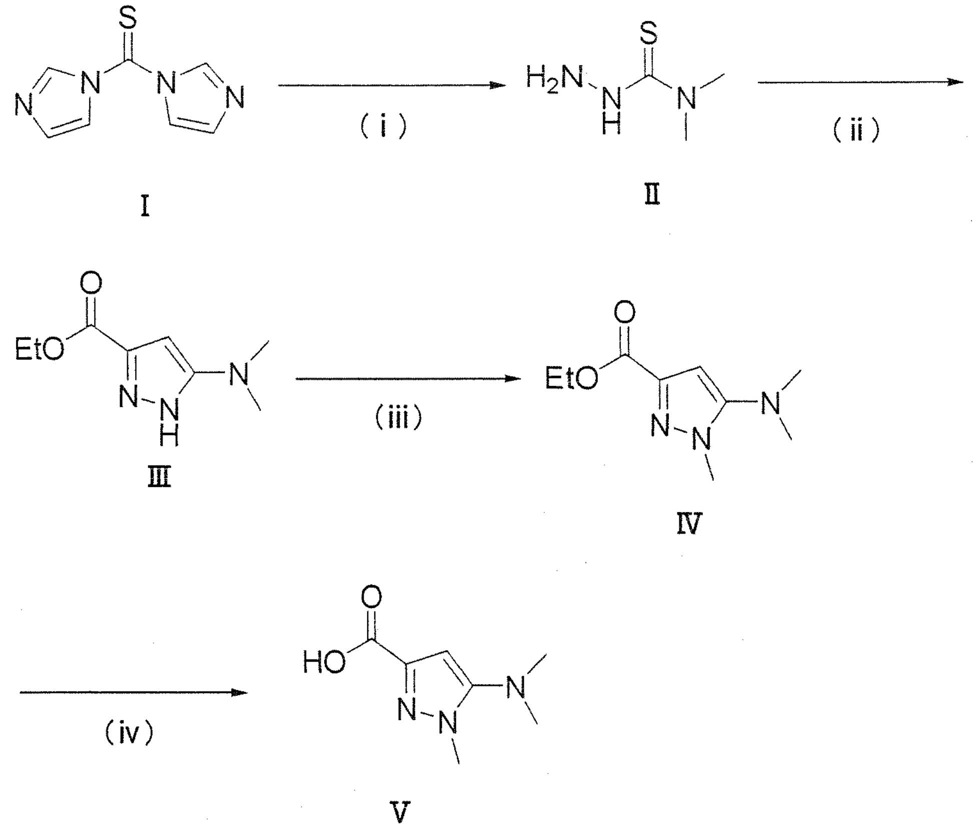


I II III IV V

Step (i):

To a solution of Compound I (20.0 g) in THF (400 mL) was added dropwise 2N dimethylamine-THF solution (56 mL), and the mixture was stirred at room temperature for 2 hours. Then, thereto was added dropwise hydrazine monohydrate (24 mL), and the mixture was stirred under reflux for 3 hours. The mixture was concentrated *in vacuo*, and then thereto was added saturated sodium bicarbonate water. The mixture was extracted with ethyl acetate, and the organic layer was dried over sodium sulfate and concentrated *in vacuo* to give Compound II (21.0 g).

Step (ii):

Compound II was dissolved in a mixed solvent of ethanol (100 mL) and THF (200 mL), and thereto were added sodium bicarbonate (16.2 g) and ethyl bromopyruvate (38.4 g). The mixture was stirred at 60°C for 3 hours. Then, thereto was added 4N hydrochloric acid-dioxane (50 mL), and the mixture was stirred at 70°C for 3 hours. The mixture was concentrated *in vacuo*, and then thereto was added saturated sodium bicarbonate water. The mixture was extracted with ethyl acetate. The organic layer was dried over sodium sulfate and concentrated *in vacuo* to give Compound III (21.0 g).

Step (iii):

Compound III (21.0 g) was dissolved in THF (1 L), and thereto was added sodium hydride (5.8 g) at 0°C. The mixture was stirred at 0°C to room temperature for 1 hour. Then, thereto was added methyl iodide (8.2 mL) at 0°C, and the mixture was stirred at room temperature overnight. Thereto was added water, and then the mixture was concentrated *in vacuo*. Thereto was added saturated sodium bicarbonate water, and the mixture was extracted with ethyl acetate. The organic layer was dried over sodium sulfate and concentrated *in vacuo*. The residue was purified by silica gel chromatography (eluent: hexane/ethyl acetate = 1/1) to give Compound IV (6.5 g).

Step (iv):

Compound IV (6.5 g) was dissolved in methanol (300 mL), and thereto was added 2N sodium hydroxide solution (30 mL) and the mixture was stirred at room temperature overnight. The reaction solution was concentrated *in vacuo*, and then acidified by 1N hydrochloric acid and extracted with ethyl acetate. The organic layer was dried over sodium sulfate and concentrated *in vacuo* to give Compound V (4.4 g) as a yellow oil.
$^1$H-NMR (DMSO-$d_6$) δ 2.62 (s, 6H), 3.69 (s, 3H), 6.21 (s, 1H), 12.4 (bs, 1H)

Reference Example 3

(*E*)-4-Aminoadamantan-1-ol hydrochloride

[Chemical Formula 29]

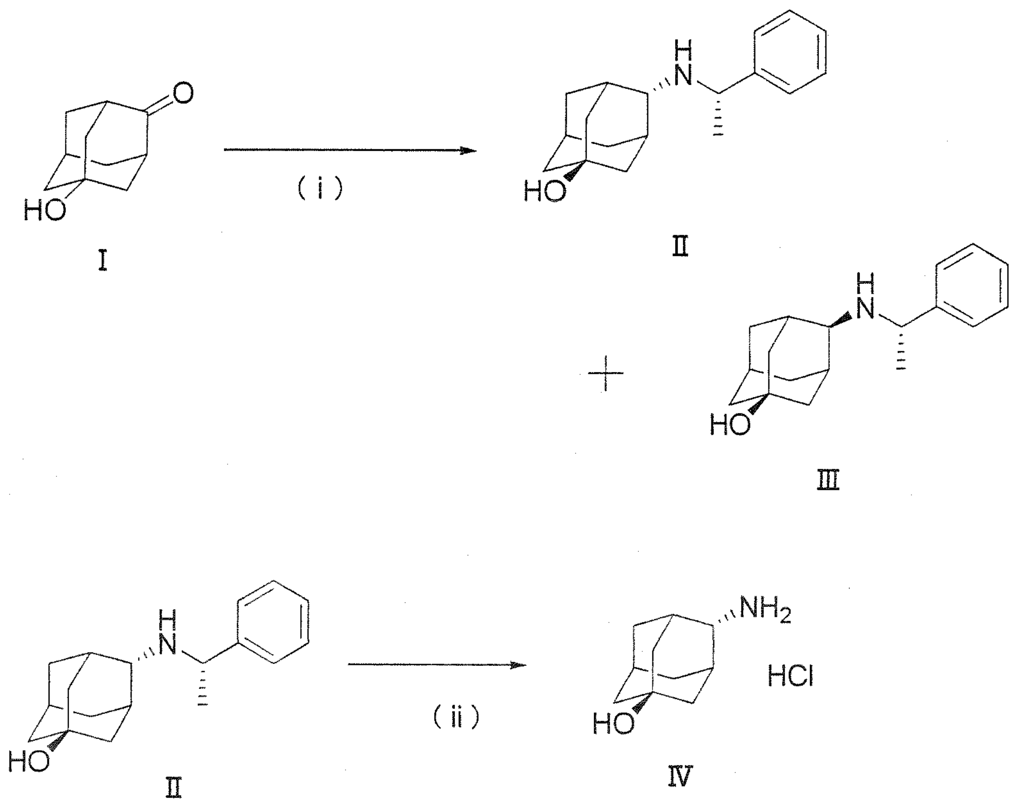


I II III II IV

Step (i):

To a solution of 5-hydroxy-2-adamantanone (10.0 g) in dichloromethane (200 mL) were added (S)-(-)-1-phenetylamine (7.2 g), $NaBH(OAc)_3$ (19 g) and acetic acid (2 mL), and the mixture was stirred at room temperature for 4 hours. Thereto was added 1N hydrochloric acid, and the mixture was washed with chloroform, and then the aqueous layer was basified by 2N sodium hydroxide solution. The mixture was extracted with chloroform, dried over sodium sulfate, and concentrated *in vacuo.* The residue was purified by silica gel chromatography (eluent: chloroform/methanol = 10/1) to give Compound II (5.9 g) as a low-polar ingredient and Compound III (4.2 g) as a high-polar one.

Step (ii):

Compound II (5.9 g) was dissolved in acetic acid (80 mL), and thereto was added palladium hydroxide (3.0 g) and the mixture was stirred under hydrogen (3 atm) for 8.5 hours. The resulting solid was filtered through Celite®, and then the filtrate was concentrated. The residue was dissolved in THF (100 mL) and saturated sodium bicarbonate water (50 mL), and thereto was added $(Boc)_2O$ (4.7 g) and the mixture was stirred at room temperature for 4 hours. The

reaction solution was extracted with ethyl acetate. The organic layer was dried over sodium sulfate and concentrated *in vacuo*. The residue was purified by silica gel chromatography (eluent: hexane/ethyl acetate = 1/1) and dissolved in chloroform (100 mL), and thereto was added 4N hydrochloric acid-dioxane (20 mL) and the mixture was stirred at room temperature for 3 hours. The mixture was concentrated *in vacuo* and azeotroped with toluene to give Compound IV (4.9 g) as a white solid.
$^1$H-NMR (DMSO-$d_6$) δ 1.35-1.39 (m, 2H), 1.59-1.69 (m, 7H), 1.86-1.90 (m, 2H), 2.01 (m, 1H), 2.06-2.12 (m, 2H), 4.50 (bs, 1H), 8.07 (bs, 3H)

Reference Example 4

*N*-(3-Methoxybenzyl)cyclopropaneamine

[Chemical Formula 30]

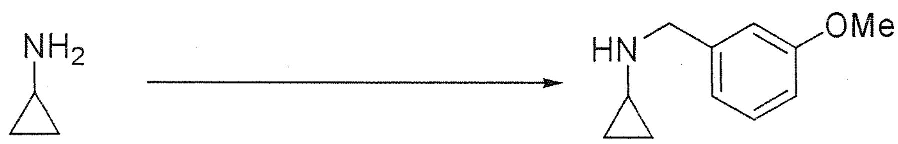

I

II

Cyclopropaneamine (1.5 g) was dissolved in dichloromethane (50 mL), and thereto were added 3-methoxybenzaldehyde (3.5 g), $NaBH(OAc)_3$ (6.7 g) and acetic acid (1 mL) and the mixture was stirred at room temperature overnight. Thereto was added water, and then the mixture was extracted with chloroform. The organic layer was dried over sodium sulfate and concentrated *in vacuo*. The residue was purified by silica gel chromatography (eluent: chloroform/methanol = 10/1) to give Compound II (2.8 g) as a colorless oil.
$^1$H-NMR ($CDCl_3$) δ 0.36-0.41 (m, 2H), 0.43-0.47 (m, 2H), 2.17 (m, 1H), 3.82 (s, 3H), 3.83 (s, 2H), 6.80 (m, 1H), 6.87-6.92 (m, 2H), 7.24 (m, 1H)

Reference Example 5

Ethyl-5-[cyclopropyl(3-methoxybenzyl)amino]-1-methyl-1H-pyrazole-3-carboxylate

[Chemical Formula 31]

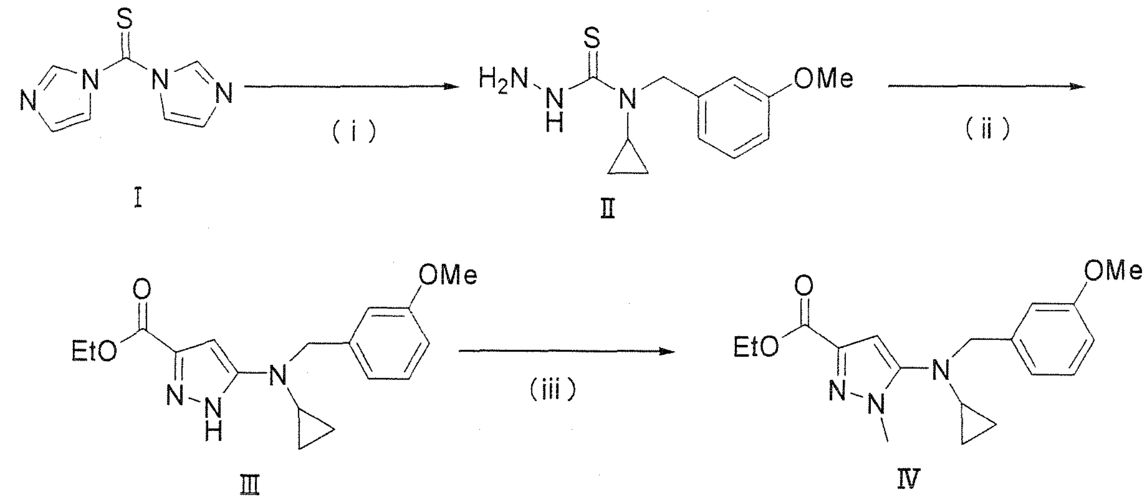


I II III IV

Step (i):

To a solution of Compound I (2.8 g) in THF (150 mL) was added dropwise N-(3-methoxybenzyl)cyclopropaneamine (2.8 g), and the mixture was stirred at room temperature for 5 hours. Then, thereto was added dropwise hydrazine monohydrate (8 mL), and the mixture was stirred under reflux for 6 hours. The mixture was concentrated *in vacuo*, and then thereto was added saturated sodium bicarbonate water and the mixture was extracted with ethyl acetate. The organic layer was dried over sodium sulfate and concentrated *in vacuo*. The resulting white solid was filtered, washed with water and dried *in vacuo* to give Compound II (3.5 g).

Step (ii):

Compound II (1.3 g) was dissolved in ethanol (10 mL) and THF (10 mL), and thereto were added sodium bicarbonate (0.54 g) and ethyl bromopyruvate (1.5 g) and the mixture was stirred at 70˚C for 3 hours. Then, thereto was added acetic acid (1.2 mL) and the mixture was stirred at 60˚C for 4.5 hours. The mixture was concentrated *in vacuo*, and thereto was added saturated sodium bicarbonate water and the mixture was extracted with ethyl acetate. The organic layer was dried over sodium sulfate and concentrated *in vacuo* to give Compound III (62 mg).

Step (iii):

Compound III (62 mg) was dissolved in THF (5 mL), and thereto was added sodium hydride (9.4 mg) at 0˚C. The mixture was stirred at 0˚C to room temperature for 1 hour. Then, thereto was added methyl iodide (13 μL) at 0˚C and the mixture was stirred at room temperature for 3 hours. Thereto was added water, then saturated sodium bicarbonate water, and the mixture was extracted with ethyl acetate. The organic layer was dried over sodium sulfate and concentrated *in vacuo*. The residue was purified by preparative TLC (eluent: hexane/ethyl acetate = 1/1) to give Compound IV (24 mg). $^1$H-NMR ($CDCl_3$) δ 0.71-0.74 (m, 2H), 0.76-0.80 (m, 2H), 1.38 (t, J=7.12Hz, 3H), 2.54 (m, 1H), 3.58 (s, 3H), 3.78 (s, 3H), 4.36 (q, J=7.12Hz, 2H), 4.53 (s, 2H), 6.43 (s, 1H), 6.76-6.85 (m, 3H), 7.20 (m, 1H)

Reference Example 6

N-[2-(4-Fluorophenoxy)ethyl]-N-methylhydrazinecarbothioamide

[Chemical Formula 32]

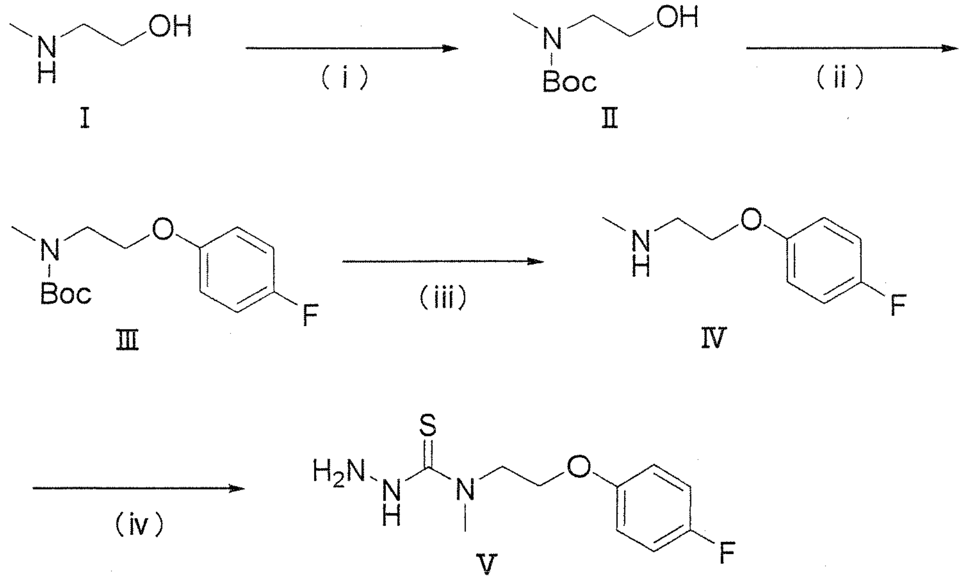


Step (i):

Compound I (41.3 g) was dissolved in THF (100 mL), and thereto were added saturated sodium bicarbonate water (100 mL) and $(Boc)_2O$ (120 g) and the mixture was stirred at room temperature overnight. Thereto was added water, and the mixture was extracted with ethyl acetate. The organic layer was dried over sodium sulfate and then concentrated *in vacuo* to give Compound II (94.4 g).

Step (ii):

Compound II (5.0 g) was dissolved in THF (200 mL), and thereto were added 4-fluorophenol (3.2 g) and triphenylphosphine (7.5 g), then added dropwise diisopropyl azodicarboxylate (5.5 g). The mixture was stirred at room temperature overnight and the reaction solvent was concentrated *in vacuo*. The residue was purified by silica gel chromatography (eluent: hexane/ethyl acetate = 3/1) to give Compound III (2.3 g).

Step (iii):

Compound III (2.3 g) was dissolved in chloroform (100 mL), and thereto was added 4N hydrochloric acid-dioxane solution (30 mL) and the mixture was stirred at room temperature for 6 hours. The reaction solvent was concentrated *in vacuo*, and thereto was added 2N sodium hydroxide solution and the mixture was extracted with chloroform. The organic layer was dried over sodium sulfate and then concentrated *in vacuo* to give Compound IV (1.8 g).

Step (iv):

1,1'-Thiocarbonyl diimidazole (2.0 g) was dissolved in THF (70 mL), and thereto was added Compound IV (1.8 g) and the mixture was stirred at room temperature for 1 hour. Then, thereto was added hydrazine monohydrate (10 mL) and the mixture was stirred under reflux for 1 hour. The mixture was concentrated *in vacuo*, and thereto was added water and the mixture was extractd with ethyl acetate. The organic layer was dried over sodium sulfate and concentrated *in vacuo* to give the titled Compound V (1.8 g).
$^1$H-NMR ($CDCl_3$) δ 1.24 (t, J=8.0Hz, 2H), 2.03 (s, 3H), 4.10 (q, J=8.0Hz, 2H), 4.20 (m, 1H), 6.56-6.66 (m, 4H), 7.12-7.14 (m, 2H)

Reference Example 7

4-Chloro-5-[cyclobutyl(2,2,2-trifluoroethyl)amino]-1-methyl-1H-pyrazole-3-carboxylic acid

[Chemical Formula 33]

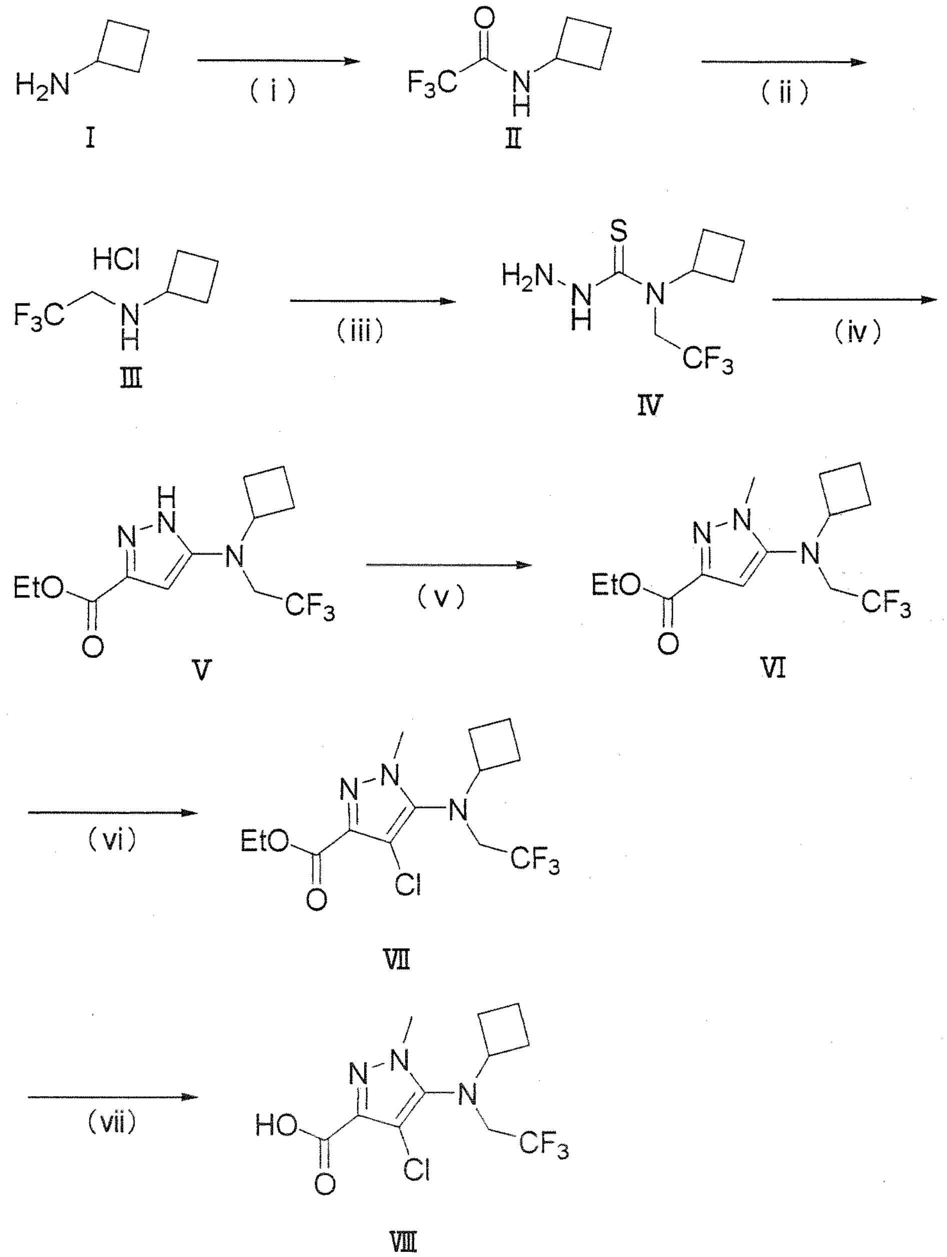


Step (i):

Cyclobutylamine (7.1 g) was dissolved in dichloromethane (400 mL), and thereto was added anhydrous trifluoroacetic acid (17 mL) and the mixture was stirred at room temperature overnight. The mixture was concentrated *in vacuo* to give Compound II (10.5 g).

Step (ii):

To a solution of borane-dimethyl sulfide complex (21.5 g) in THF (300 mL) was added dropwise a solution of Compound II (10.5 g) in THF (50 mL) at 50°C, and the mixture was stirred at 50°C overnight. Thereto was added methanol (150 mL) at 0°C, and the mixture was stirred at room temperature for 1 hour. Then, thereto was added 4N hydrochloric acid-ethanol solution (100 mL) and the mixture was stirred at room temperature for 1 hour. The reaction solution was concentrated *in vacuo*, and the residue was washed with ethyl acetate-hexane. The resulting white solid was filtered and dried *in vacuo* to give Compound III (10.9 g).

Step (iii):

Compound III (1.9 g) was dissolved in THF (20 mL), and thereto was added saturated sodium bicarbonate water (10 mL) and the mixture was stirred at room temperature for 30 minutes. Thereto was added dropwise a solution of 4-chlorophenyl chlorothioformate (2.3 g) in THF (5 mL) at 0˚C, and the mixture was stirred at room temperature for 4 hours. Thereto was added brine, and the mixture was extracted with ethyl acetate. The organic layer was dried over sodium sulfate, concentrated *in vacuo* and dissolved in NMP (12 mL), and thereto was added hydrazine monohydrate (1.5 mL) and the mixture was stirred at room temperature for 1 hour. Thereto was added water, and then the mixture was extracted with ethyl acetate. The organic layer was washed with brine, and then dried over sodium sulfate and concentrated *in vacuo*. The residue was purified by silica gel chromatography (eluent: hexane/ethyl acetate = 1/1) to give Compound IV (2.0 g).

Step (iv):

Compound IV (2.0 g) was dissolved in a mixed solvent of ethanol (20 mL) with THF (20 mL), and thereto were added sodium bicarbonate (765 mg) and ethyl bromopyruvate (1.8 g) and the mixture was stirred at 80˚C for 3 hours. Thereto was added 4N hydrochloric acid-ethanol solution (3 mL), and the mixture was stirred at 60˚C for 12 hours. The mixture was concentrated *in vacuo*, and then thereto was added saturated sodium bicarbonate water and the mixture was extracted with ethyl acetate. The organic layer was dried over sodium sulfate and concentrated *in vacuo*. The residue was purified by silica gel chromatography (eluent; hexane/ethyl acetate = 1/1) to give Compound V (780 mg).

Step (v):

To a solution of sodium hydride (140 mg) in THF (10 mL) was added dropwise a solution of Compound V (778 mg) in THF (5 mL) at 0˚C, and the mixture was stirred at room temperature for 1 hour. Then, thereto was slowly added methyl iodide (200 μL) at 0˚C, and the mixture was stirred at room temperature overnight. Thereto was added water, and then the mixture was concentrated *in vacuo*. Thereto was added brine, and the mixture was extracted with ethyl acetate. The organic layer was dried over sodium sulfate and concentrated *in vacuo*. The residue was purified by silica gel chromatography (eluent: hexane/ethyl acetate = 1/1) to give Compound VI (590 mg).

Step (vi):

Compound VI (340 mg) was dissolved in DMF (4.5 mL), and thereto was added N-chlorosuccinimide (178 mg) and the mixture was stirred at 60˚C for 4 hours. Thereto was added water, and then the mixture was extracted with ethyl acetate. The organic layer was washed with brine. The organic layer was dried over sodium sulfate and concentrated *in vacuo*. The residue was purified by silica gel chromatography (eluent: hexane/ethyl acetate = 3/1) to give Compound VII (324 mg).

Step (vii):

Compound VII (320 mg) was dissolved in ethanol (4 mL), and thereto was added 5N sodium hydroxide solution (560 μL) and the mixture was stirred at room temperature overnight. The reaction solution was concentrated *in vacuo*, and then acidified by 1N hydrochloric acid and extracted with ethyl acetate. The organic layer was dried over sodium sulfate and concentrated *in vacuo* to give the titled Compound VIII (287 mg) as a white solid.
$^1$H-NMR ($CDCl_3$) δ 1.51-1.69 (m, 2H), 1.75-1.88 (m, 2H), 2.05-2.20 (m, 2H), 3.52-3.80 (m, 2H), 3.83 (s, 3H), 3.84-3.95 (m, 2H)

Reference Example 8

Ethyl 1,4-dimethyl-5-[methyl(2,2,2-trifluoroethyl)amino]-1H-pyrazole-3-carboxylate

[Chemical Formula 34]

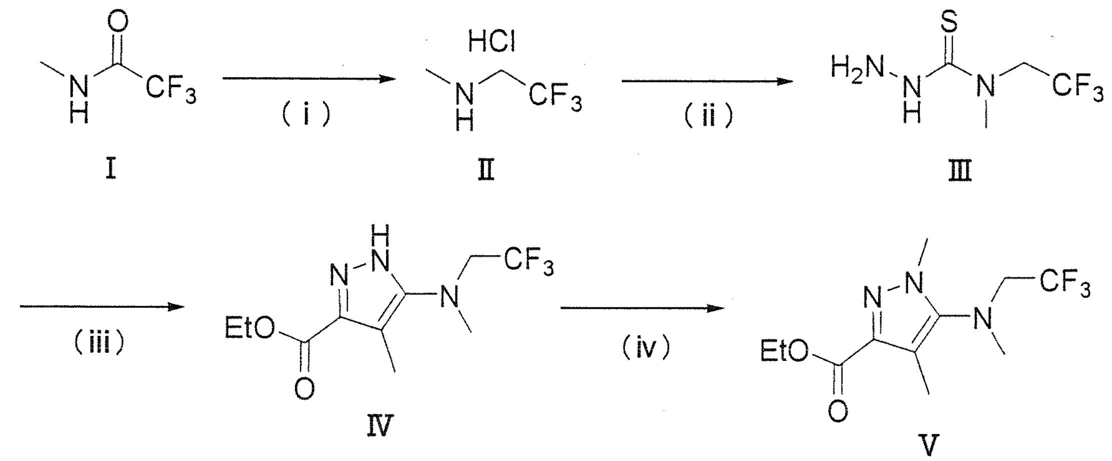


I II III IV V

Step (i):

To a solution of borane-dimethyl sulfide complex (23.9 g) in THF (300 mL) was added dropwise a solution of Compound I (10.0 g) in THF (50 mL) at 50°C and the mixture was stirred at 50°C overnight. Thereto was added methanol (150 mL) at 0°C, and the mixture was stirred at room temperature for 1 hour. Then, thereto was added 4N hydrochloric acid-ethanol solution (100 mL) and the mixture was stirred at room temperature for 1 hour. The reaction solution was concentrated *in vacuo*, and the residue was washed with ethyl acetate-hexane. The resulting white solid was filtered and dried *in vacuo* to give Compound II (10.0 g).

Step (ii):

Compound II (5.5 g) was dissolved in THF (40 mL), and thereto was added triethylamine (5.1 mL) and the mixture was stirred at room temperature for 30 minutes. The mixture was added to a solution of 1,1'-thiocarbonyl diimidazole (7.6 g) in THF (40 mL) and stirred at room temperature for 1 hour. Then, thereto was added hydrazine monohydrate (5.4 mL) and the mixture was stirred at room temperature for 1 hour. The mixture was concentrated *in vacuo*, and thereto was added brine and the mixture was extracted with ethyl acetate. The organic layer was dried over sodium sulfate and then concentrated *in vacuo* to give Compound III (6.1 g).

Step (iii):

Compound III (1.4 g) was dissolved in a mixed solvent of ethanol (20 mL) with THF (20 mL), and thereto were added sodium bicarbonate (670 mg) and ethyl 3-bromo ketobutanoate (2.2 g), and the resulting mixture was stirred at 70°C for 3 hours. The reaction solution was concentrated *in vacuo*, and then thereto was added 4N hydrochloric acid-ethanol solution (5 mL) and the mixture was stirred at 90°C overnight. The mixture was concentrated *in vacuo*, and then thereto was added saturated sodium bicarbonate water and the mixture was extracted with ethyl acetate. The organic layer was dried over sodium sulfate and concentrated *in vacuo*. The residue was purified by silica gel chromatography (eluent: hexane/ethyl acetate = 2/1) to give Compound IV (1.1 g) as a white solid.

Step (iv):

To a solution of sodium hydride (155 mg) in THF (12 mL) was added dropwise a solution of Compound IV (854 mg) in THF (8 mL) at 0°C and the mixture was stirred at room temperature for 1 hour. Then, thereto was slowly added methyl iodide (240 μL) at 0°C and the mixture was stirred at room temperature for 3 hours. Thereto was added water, and then the mixture was concentrated *in vacuo*. Thereto was added brine, and the mixture was extracted with ethyl acetate. The organic layer was dried over sodium sulfate and concentrated *in vacuo*. The residue was purified by silica gel chromatography (eluent: hexane/ethyl acetate = 2/1) to give the titled Compound V (445 mg).
$^1$H-NMR ($CDCl_3$) δ 1.40 (t, J=8.0Hz, 3H), 2.24 (s, 3H), 2.93 (s, 3H), 3.52-3.61 (m, 2H), 3.80 (s, 3H), 4.39 (q, J=8.0Hz, 2H)

Reference Example 9

*N*-(3-Methoxypropyl)cyclopropaneamine hydrochloride

[Chemical Formula 35]

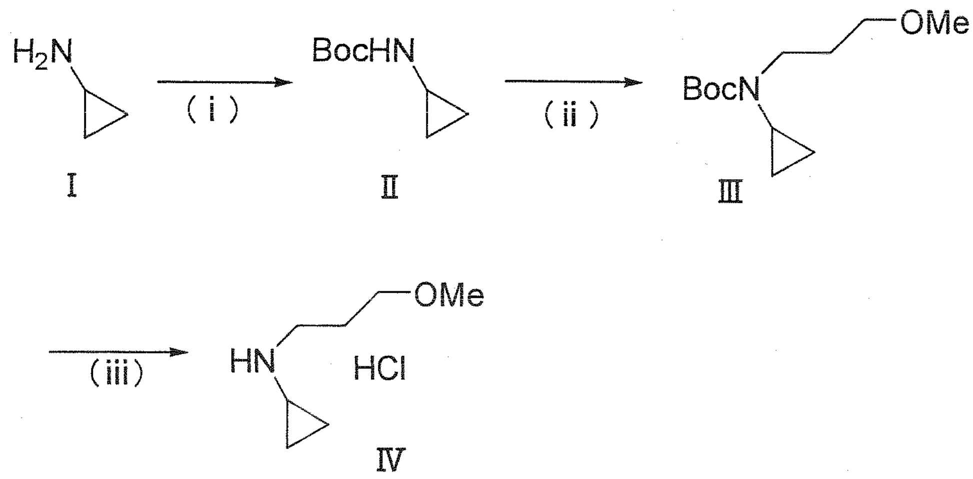


I II III IV

Step (i):

To an ice-cooled mixed solution of cyclopropylamine (5.0 g), saturated sodium bicarbonate water (20 mL) and THF (200 mL) was added $(Boc)_2O$ (19.1 g), and the mixture was stirred at room temperature overnight. The organic layer was separated and the aqueous layer was extracted with ethyl acetate. The organic layer was dried over sodium sulfate and concentrated *in vacuo* to give Compound II (13.0 g).

Step (ii):

To an ice-cooled mixed solution of sodium hydride (1.7 g) and DMF (70 mL) was added dropwise a solution of Compound II (5.0 g) in DMF (5 mL). The mixture was stirred at room temperature for 1 hour and cooled to 0˚C again. Thereto was added dropwise 1-bromo-3-methoxypropane (7.3 g), and then the mixture was stirred at room temperature overnight. Thereto was added water, and the mixture was extracted with ethyl acetate. The organic layer was dried over sodium sulfate and concentrated *in vacuo* to give Compound III.

Step (iii):

Compound III obtained in Step (ii) was dissolved in dioxane (25 mL), and thereto was added 4N hydrochloric acid-dioxane solution (25 mL) and the mixture was stirred at room temperature for 4 hours. The mixture was concentrated *in vacuo*, and the residue was washed with dioxane and hexane to give the titled Compound IV (4.3 g).
$^1$H-NMR ($CDCl_3$) δ 0.67-0.74 (m, 2H), 0.82-0.86 (m, 2H), 1.82-1.88 (m, 2H), 2.64-2.69 (m, 1H), 2.97-3.05 (m, 2H), 3.23 (s, 3H), 3.33-3.39 (m, 2H), 8.92 (bs, 2H)

Reference Example 10

*N*-(2-Methoxyethyl)cyclopropaneamine hydrochloride

The titled compound was synthesized by using 1-bromo-2-methoxyethane in the similar manner to Reference Example 9.

[Chemical Formula 36]

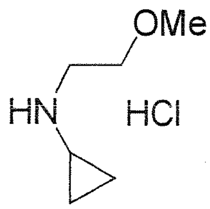

$^1$H-NMR ($CDCl_3$) δ 0.68-0.73 (m, 2H), 0.82-0.86 (m, 2H), 2.63-2.69 (m, 1H), 3.13-3.16 (m, 2H), 3.29 (s, 3H), 3.59-3.62 (m, 2H), 9.06 (bs, 2H)

Reference Example 11

*N*-Cyclopropylcyclopropaneamine hydrochloride

[Chemical Formula 37]

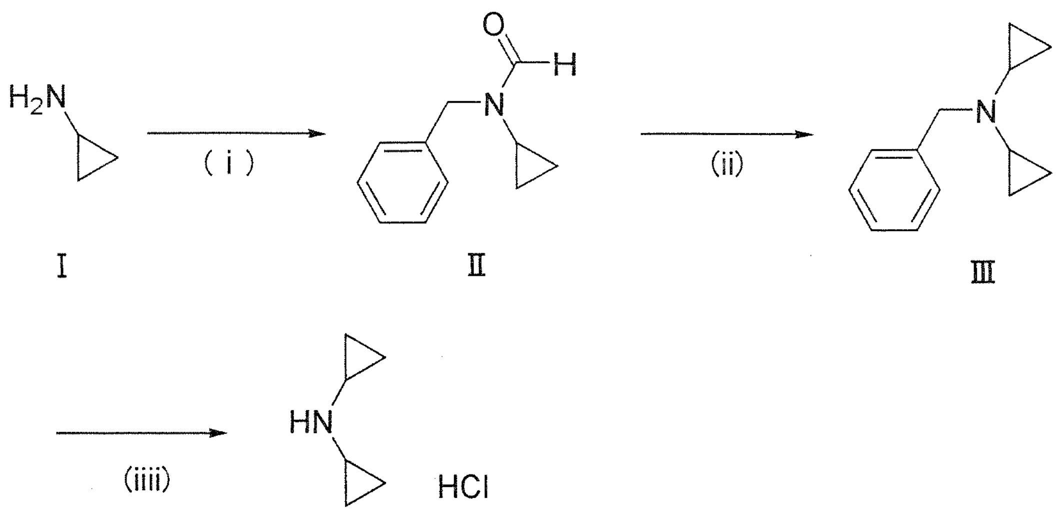


I II III IV

Step (i):

To a solution of cyclopropylamine (3.0 g) and benzaldehyde (5.6 g) in ethylene chloride (200 mL) was added $NaBH(OAc)_3$ (12.3 g), and the mixture was stirred at room temperature overnight. Thereto was added water, and the aqueous layer was extracted with chloroform. The organic layer was washed with brine, dried over sodium sulfate and concentrated *in vacuo*. The residue was dissoved in butyl formate (100 mL) and stirred at 150˚C overnight. The mixture was concentrated *in vacuo*, and the residue was purified by silica gel chromatography (eluent: hexane/ethyl acetate = 3/2) to give Compound II (4.4 g).

Step (ii):

To a solution of ethylmagnesium bromide (0.96M solution in THF, 60 mL) in THF (170 mL) at -70˚C was added dropwise a solution of titanium tetraisopropoxide (9.3 g) in THF (20 mL) over 3 minutes, and the mixture was stirred for 2 minutes. Then, thereto was added dropwise a solution of Compound II (4.4 g) in THF (10 mL) over 3 minutes, and the mixture was stirred for 5 minutes. The mixture was warmed up to room temperature and stirred overnight. To the reaction

solution were added saturated aqueous ammonium chloride solution (150 mL) and water (50 mL), and the mixture was stirred at room temperature for 3 hours. The white precipitate was filtered, and the filtrate was adjusted to pH 10 with 2M aqueous sodium hydroxide solution and extracted with ethyl acetate. The organic layer was washed with brine, dried over sodium sulfate and concentrated *in vacuo*. The residue was purified by silica gel chromatography (eluent: hexane/ethyl acetate = 100/1) to give Compound III (2.7 g).

Step (iii):

A mixed solution of Compound III (2.7 g), methanol (60 mL), 4N hydrochloric acid-dioxane (7.5 mL) and 10% palladium-carbon (300 mg) was stirred at room temperature under hydrogen (3 atm) for 4.5 hours. The reaction solution was filtered through Celite®, and then the filtrate was concentrated to give the titled Compound IV (2.0 g).
$^1$H-NMR ($CDCl_3$) δ 0.71-0.78 (m, 4H), 0.80-0.91 (m, 4H), 2.73 (bs, 2H), 9.35 (bs, 2H)

Reference Example 12

*N*-Methyl-1-(1-phenylcyclobutyl)methaneamine hydrochloride

[Chemical Formula 38]

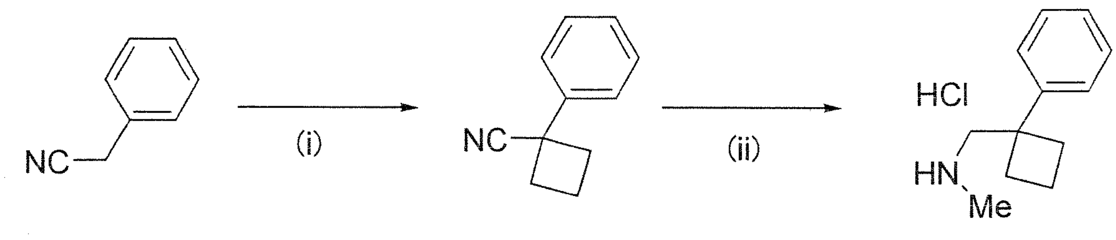


(II) (III)

Step (i):

To a mixed solvent of toluene (135 mL) and water (10 mL) were added benzyl cyanide (5.9 g), potassium hydroxide (26.4 g), 1,3-dibromopropane (10.1 g) and tetrabutylammonium bromide (0.16 g), and the mixture was heated with stirring at 100˚C. After dissolving potassium hydroxide, the reaction vessel was soaked in a water bath and vigorously stirred for 10 minutes. Then, the mixture was heated with stirring at 110˚C for 5 hours. Thereto was added water, and the aqueous layer was extracted with ethyl acetate. The organic layer was washed with brine, dried over sodium sulfate and concentrated *in vacuo*. The residue was purified by silica gel chromatography (eluent: hexane/ethyl acetate = 20/1) to give Compound II (4.1 g).

Step (ii):

Lithium aluminum hydride (3.8 g) was suspended in THF (120 mL), and thereto was added dropwise a solution of Compound II in THF (5 mL) at room temperature. After completion of dropping, the reaction solution was heated at reflux for 5 hours. Thereto were added water (4 mL), aqueous sodium hydroxide solution (15%, 4 mL) and water (12 mL) under ice cooling, and the resulting precipitate was filtered off. The organic layer was concentrated *in vacuo*. To the residue was added water, and the mixture was extracted with methyl acetate. The organic layer was washed with brine, dried over sodium sulfate and concentrated *in vacua*. The residue was dissolved in ethyl formate (50 mL) and stirred at 100˚C overnight. The mixture was concentrated *in vacuo*, and then the residue was dissolved in THF (20 mL) and the solution was added dropwise to a suspension of lithium aluminum hydride (3.8 g) in THF (120 mL) at room temperature. After completion of dropping, the mixture was heated at reflux for 1 hour and stirred at room temperature overnight. Thereto was added sodium sulfate decahydrate until ceasing of gas generation, and then thereto was added anhydrous sodium sulfate. The precipitate was filtered off and the filtrate was concentrated *in vacua* to give Compound III (4.7 g).
$^1$H-NMR ($CDCl_3$) δ 1.72-1.82 (m, 1H), 2.01-2.13 (m, 1H), 2.26-2.40 (m, 4H), 2.43-2.45 (m, 3H), 3.28-3.31 (m, 2H), 7.22-7.30 (m, 3H), 7.36-7.39 (m, 2H), 8.23 (bs, 2H)

Reference Example 13

Benzyl 4-[[3-(ethoxycarbonyl)-1*H*-pyrazol-5-yl](methyl)amino]piperidine-1-carboxylate

[Chemical Formula 39]

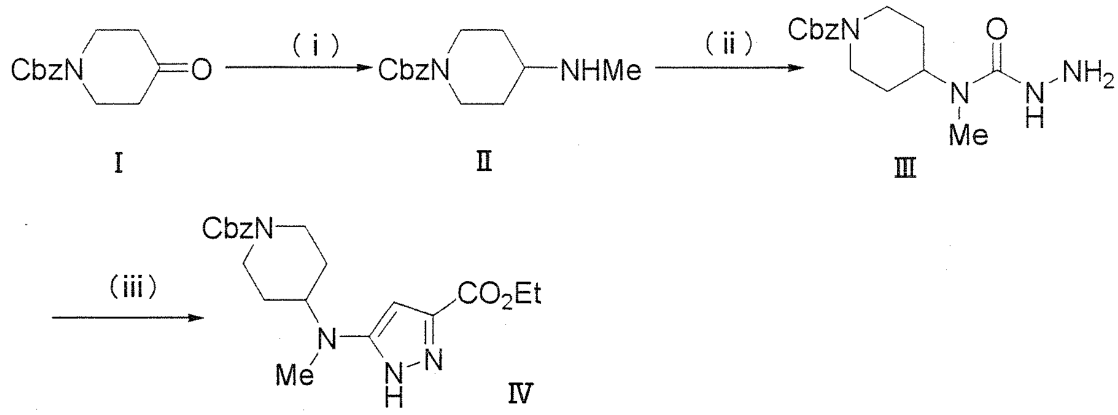


Step (i):

To a solution of Compound I (7.1 g) in dichloromethane (400 mL) was added a solution of methylamine in THF (110 mL, 2M). After ice-cooling, thereto was added acetic acid (43 mL), then $NaBH(OAc)_3$ (35.3 g) in small portions. The mixture was stirred at room temperature overnight, and thereto were added water (200 mL) and potassium carbonate. After the completion of gas generation, the organic layer was separated. The aqueous layer was extracted with dichloromethane. The organic layer was combined to be dried over sodium sulfate and concentrated *in vacuo* to give Compound II (quantitative).

Step (ii):

To an ice-cooled mixture of Compound II obtained in Step (i), THF (200 mL), water (100 mL) and sodium bicarbonate (19.8 g) was added dropwise a solution of 4-chlorophenyl chlorothioformate (17.6 mL) in THF (100 mL), and the mixture was stirred at room temperature for 4 hours. The organic layer was separated, and then the aqueous layer was extracted with ethyl acetate. The organic layer was combined to be dried over sodium sulfate and concentrated *in vacuo*. To the residue was added DMF (200 mL) with ice cooling, and then thereto was added hydrazine monohydrate (12.6 mL) and the mixture was stirred at room temperature for 2 hours. Thereto was added brine, and then the mixture was extracted with ethyl acetate. The organic layer was washed with brine, and then dried over sodium sulfate and concentrated *in vacua*. The residue was purified by silica gel chromatography (eluent: hexane/ethyl acetate = 1/1 to chloroform/methanol = 10/1) to give Compound III (23.75 g).

Step (iii):

To an ice-cooled mixture of Compound III (23.75 g), sodium bicarbonate (12.37 g), 95% ethanol (250 mL) and THF (100 mL) was added ethyl bromopyruvate (11.6 mL). The mixture was stirred at room temperature for 30 minutes and then stirred at 90˚C. After 2 hours, thereto was added acetic acid (150 mL) and the mixture was stirred at 125˚C while removing solvents with a Dean-Stark apparatus. The mixture was stirred overnight, and then cooled to room temperature and concentrated *in vacuo*. To the residue was added saturated sodium bicarbonate water, and the mixture was extracted with ethyl acetate. The organic layer was dried over sodium sulfate and concentrated *in vacua*, The residue was purified by silica gel chromatography (eluent: hexane/ethyl acetate = 1/1 to chloroform/methanol = 10/1) to give Compound III (14.8 g).
$^1$H-NMR ($CDCl_3$) δ 1.38 (t, J=4Hz, 3H), 1.60-1.80 (m, 4H), 2.74 (s, 3H), 2.87 (m, 2H), 3.80 (m, 1H), 4.20-4.41 (m, 4H), 5.14 (s, 2H), 6.16 (s, 1H), 7.31-7.40 (m, 5H), 9.76 (br, 1H)

Reference Example 14

Benzyl 4-{[[3-(ethoxycarbonyl)-1*H*-pyrazol-5-yl](methyl)amino]methyl}piperidine-1-carboxylate

[Chemical Formula 40]

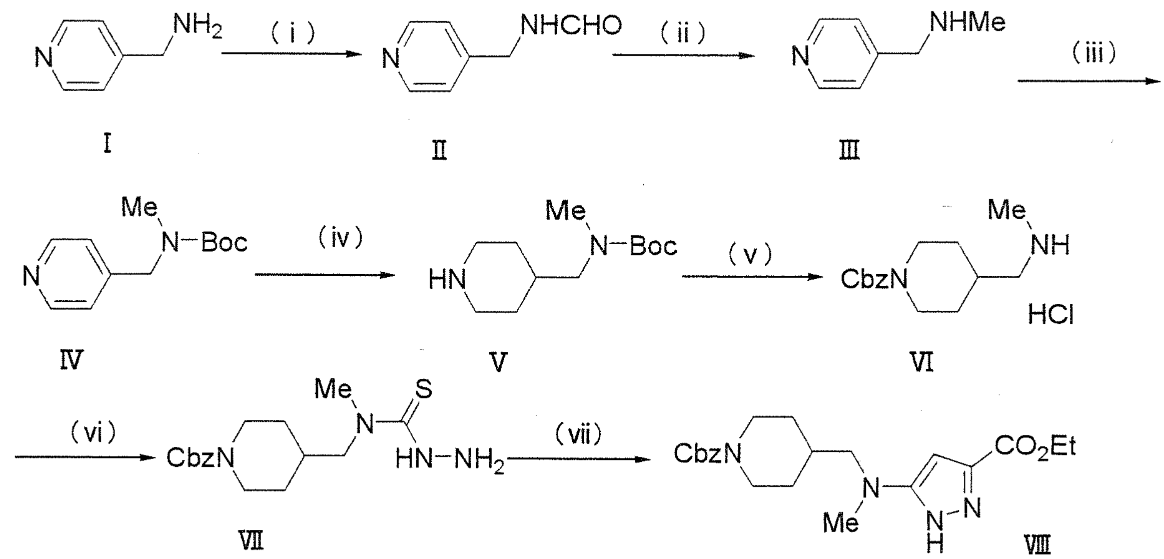


I II III IV V VI VII VIII

Step (i):

A solution of Compound I (5 g) in ethyl formate (8 mL) was stirred under reflux for 16 hours. The solution was concentrated *in vacuo* to give Compound II (quantitative). Repetitions of Step (i) gave enough amounts of Compound II for Step (ii).

Step (ii):

To an ice-cooled solution of Compound II (49.1 g) in THF (500 mL) was added dropwise borane-dimethyl sulfide complex (171 mL). After the completion of dropwise, the mixture was stirred at room temperature. After ceasing of gas generation, the mixture was stirred at 50°C for 3 hours, and then stirred at room temperature overnight. To the ice-cooled reaction solution was added dropwise methanol (200 mL), and then the mixture was stirred at room temperature for 30 minutes and concentrated *in vacuo*. Then, to the residue was added water (100 mL), and the mixture was acidified with hydrochloric acid. The mixture was stirred for 2 hours, and then the resulting solid was filtered off. The filtrate was extracted with toluene twice. To the aqueous layer was added sodium hydroxide, and the mixture was adjusted to pH>12 and extracted with dichloromethane three times. The organic layer was washed with brine, and then dried over sodium sulfate and concentrated *in vacuo* to give Compound III (40.94 g).

Step (iii):

To an ice-cooled mixture of Compound III (40.94 g), sodium bicarbonate (42.17 g), ethyl acetate (200 mL) and water (200 mL) was added dropwise a solution of $Boc_2O$ (73.1 g) in ethyl acetate (200 mL). After 4 hours, the organic layer was separated. The aqueous layer was extracted with ethyl acetate. The combined organic layer was washed with brine, and then dried over sodium sulfate and concentrated *in vacuo*. The residue was purified by silica gel chromatography (eluent: hexane/ethyl acetate = 1/1 to 1/2) to give Compound IV.

Step (iv):

To Compound IV obtained in Step (iii) were added acetic acid (200 mL) and platinum oxide ($PtO_2$, 5 g), and the mixture was stirred under 3-4 $kgf/cm^2$ of hydrogen atmosphere overnight. The reaction mixture was filtered through Celite® and washed with methanol. The filtrate was concentrated *in vacuo*, and then the residue was extracted with

sodium hydroxide solution and dichloromethane. The organic layer was dried over sodium sulfate and concentrated *in vacuo* to give Compound V (66.93 g).

Step (v):

To an ice-cooled mixture of Compound V (66.93 g), sodium carbonate (62.1 g), toluene (200 mL) and water (300 mL) was added dropwise a solution of benzyloxycarbonyl chloride (55 g) in toluene (200 mL). The mixture was stirred overnight, and then a toluene layer was separated. The aqueous layer was extracted with ethyl acetate. The combined organic layer was dried over sodium sulfate and concentrated *in vacuo*. To the residue was added 4N hydrochloric acid-dioxane (80 mL), and the mixture was stirred at room temperature and concentrated *in vacuo*. Then, thereto were added diisopropylether and hexane, and the mixture was allowed to stand overnight at room temperature. The resulting solid was filtered and washed with diisopropylether and hexane, and then dried *in vacuo* to give Compound VI (68.56 g).

Step (vi):

To Compound VI (25 g) was added sodium hydroxide solution, and the mixture was stirred and then extracted with dichloromethane. The organic layer was dried over sodium sulfate and concentrated *in vacuo*. After obtaining the free form of Compound VI, thereto was added THF (100 mL). The solution was added dropwise to an ice-cooled solution of 1,1'-thiocarbonyl diimidazole (17.3 g) in THF (300 mL). After completion of dropping, the mixture was stirred at room temperature for 1.5 hours. The reaction solution was ice-cooled, and thereto was added hydrazine monohydrate (12.6 mL) and the mixture was stirred at room temperature overnight and concentrated *in vacuo*. Then, the residue was extracted with ethyl acetate and brine. The organic layer was washed with brine, and then dried over sodium sulfate and concentrated *in vacuo*. To the residue were added diisopropylether and hexane, and the mixture was stirred for 30 minutes, and then filtered and dried *in vacuo* to give Compound VII (27.43 g).

Step (vii):

To an ice-cooled mixed solution of Compound VII (27.43 g), sodium bicarbonate (13.7 g), 95% ethanol (400 mL) and THF (250 m) was added ethyl bromopyruvate (12.8 mL). The mixture was stirred for 20 minutes and then stirred at 90˚C. After 1 hour, thereto was added acetic acid (250 mL), and the mixture was stirred at 125˚C while removing solvents with a Dean-Stark apparatus. The mixture was stirred overnight, and then cooled to room temperature and concentrated *in vacuo*. To the residue was added saturated sodium bicarbonate water, and the mixture was extracted with ethyl acetate. The organic layer was dried over sodium sulfate and concentrated *in vacuo*. The residue was purified by silica gel chromatography (eluent: hexane/ethyl acetate = 1/1) to give the titled Compound VIII (19.07 g).
$^1$H-NMR ($CDCl_3$) δ 1.19 (m, 2H), 1.40 (t, J=8Hz, 3H), 1.72 (m, 2H), 1.92 (m, 1H), 2.78 (m, 2H), 2.94 (s, 3H), 3.13 (d, J=12Hz, 2H), 4.22 (m, 2H), 4.37 (q, J=8Hz, 2H), 5.14 (s, 2H), 6.09 (s, 1H), 7.32-7.42 (m, 5H), 9.80 (br, 1H)

Reference Example 15

Benzyl 4-{2-[[3-(ethoxycarbonyl-1*H*-pyrazol-5-yl](methyl)amino]ethyl}piperidine-1-carboxylate

4-(2-Aminoethyl)pyridine was treated in the similar manner to Reference Example 14 to give the following compounds.

[Chemical Formula 41]

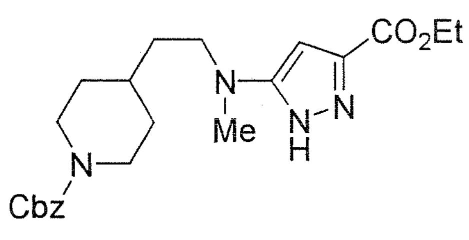

$^1$H-NMR ($CDCl_3$) δ 1.18 (m, 2H), 1.38 (t, J=8Hz, 3H), 1.48-1.56 (m, 3H), 1.69-1.75 (m, 2H), 2.77 (m, 2H), 2.87 (s, 3H), 3.28 (m, 2H), 4.17 (m, 2H), 4.37 (q, J=8Hz, 2H), 5.13 (s, 2H), 6.11 (s, 1H), 7.30-7.37 (m, 5H), 9.73 (br, 1H)

Reference Example 16

Ethyl 5-[{(3*R*)-1-[(benzyloxy)carbonyl]pyrrolidin-3-yl}(methyl)amino]-1*H*-pyrazole-3-carboxylate

[Chemical Formula 42]

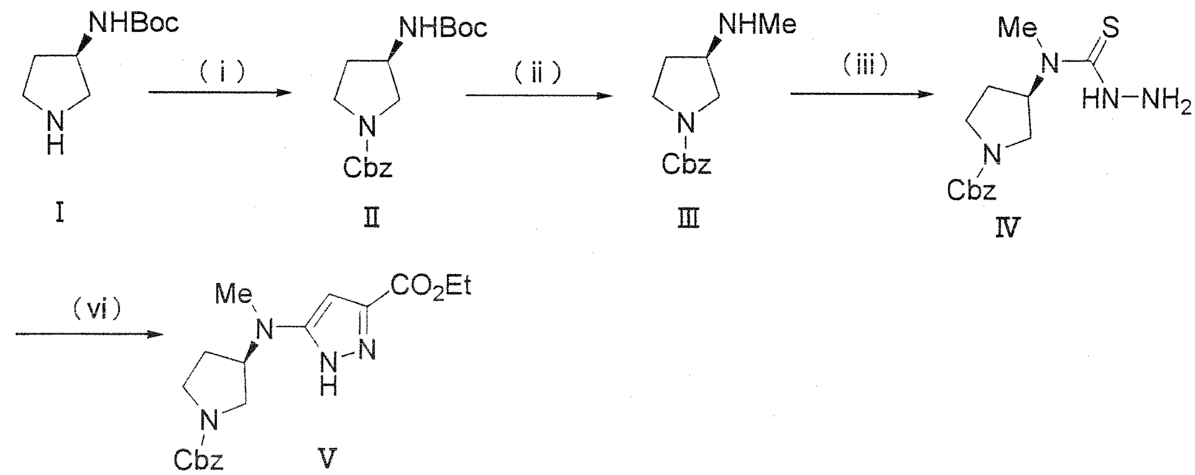


I II III IV V

Step (i):

To an ice-cooled mixed solution of Compound I (25.6 g), toluene (200 mL), sodium carbonate (32.0 g) and water (300 mL) was added dropwise a solution of benzyloxycarbonyl chloride (25.8 g) in toluene (100 mL). The mixture was stirred overnight, and then thereto was added ethyl acetate and the mixture was stirred. Then, the reaction solution was filtered and the organic layer of the filtrate was separated. The aqueous layer was extracted with ethyl acetate. The combined organic layer was washed with water, and then dried over sodium sulfate and concentrated *in vacuo*. To the residue were added diisopropylether and hexane, and the mixture was stirred for 20 minutes and then the resulting solid was filtered and dried *in vacuo* to give Compound II (41.48 g).

Step (ii):

To an ice-cooled solution of Compound II (41.48 g) in DMF (300 mL) was added sodium hydride (5.7 g) in small portions. The mixture was stirred for 1.5 hours at room temperature, and then the reaction solution was ice-cooled. Thereto was added dropwise methyl iodide (9.8 mL), and then the mixture was stirred at room temperature overnight. The reaction solution was poured into citric acid solution, and the mixture was extracted with ethyl acetate. The organic layer was washed with brine, and then dried over sodium sulfate and concentrated *in vacuo*. The residue was purified by silica gel chromatography (eluent: hexane/ethyl acetate = 1/1). Thereto was added 15% hydrochloric acid-ethanol (100 mL), and the mixture was allowed to stand for 3 days at room temperature. The mixture was concentrated *in vacuo*, and then thereto was added hydrochloric acid solution and the mixture was extracted with toluene. The toluene layer was extracted with 1N hydrochloric acid. The combined acidic aqueous layer was adjusted to pH>14 with sodium hydroxide. The alkaline aqueous layer was extracted with dichloromethane, and the organic layer was dried over sodium sulfate and concentrated *in vacuo* to give Compound III (28.45 g).

Step (iii):

To an ice-cooled mixed solution of Compound III obtained in Step (ii), sodium bicarbonate (22.4 g), THF (100 mL) and water (100 mL) was added dropwise a solution of 4-chlorophenyl chlorothioformate (20 mL) in THF (100 mL), and the mixture was stirred at room temperature overnight. The organic layer was separated, and then the aqueous layer was extracted with ethyl acetate. The organic layer was combined, dried over sodium sulfate and concentrated *in vacuo*. Thereto was added DMF (200 mL), and the mixture was ice-cooled. Then, thereto was added hydrazine monohydrate (14.2 mL), and the mixture was stirred at room temperature for 2 hours. Thereto was added brine, and then the mixture was extracted with ethyl acetate. The organic layer was washed with brine, and then dried over sodium sulfate

and concentrated *in vacuo*. The residue was purified by silica gel chromatography (eluent: hexane/ethyl acetate = 1/1 to chloroform/methanol = 10/1) to give Compound IV (34.74 g).

Step (iv):

To an ice-cooled mixed solution of Compound IV (34.74 g), sodium bicarbonate (18.9 g) and 95% ethanol (300 mL) was added ethyl bromopyruvate (17.7 mL). The mixture was stirred at room temperature for 20 minutes, and then stirred at 90˚C. After 1.5 hours, thereto was added acetic acid (200 mL), and the mixture was stirred at 125˚C while removing solvents with a Dean-Stark apparatus. The mixture was stirred overnight, and then the mixture was cooled back to room temperature and concentrated *in vacuo*. To the residue was added saturated sodium bicarbonate water, and the mixture was extracted with ethyl acetate. The organic layer was dried over sodium sulfate and concentrated *in vacua*. The residue was purified by silica gel chromatography (eluent: hexane/ethyl acetate = 1/1 to chloroform/methanol = 10/1) to give the titled Compound V (20.9 g) (49.8% yields).
$^1$H-NMR ($CDCl_3$) δ 1.39 (t, J=8Hz, 3H), 2.09 (m, 2H), 2.82 (s, 3H), 3.34-3.48 (m, 2H), 3.56-3.75 (m, 2H), 4.37 (q, J=8Hz, 2H), 4.45 (m, 1H), 5.15 (s, 2H), 6.20 (s, 1H), 7.31-7.41 (m, 5H), 9.85 (br, 1H)

Reference Example 17

Ethyl 5-[{(3*S*)-1-[(benzyloxy)carbonyl]pyrrolidin-3-yl}(methyl)amino]-1*H*-pyrazole-3-carboxylate

The titled compound was prepared in the similar manner to Reference Example 16.
[Chemical Formula 43]

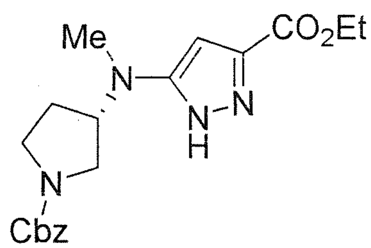

$^1$H-NMR ($CDCl_3$) δ 1.39 (t, J=8Hz, 3H), 2.09 (m, 2H), 2.82 (s, 3H), 3.33-3.49 (m, 2H), 3.56-3.76 (m, 2H), 4.37 (q, J=8Hz, 2H), 4.45 (m, 1H), 5.15 (s, 2H), 6.20 (s, 1H), 7.31-7.41 (m, 5H), 9.85 (br, 1H)

Reference Example 18

Benzyl (3*R*)-3-[[3-(ethoxycarbonyl)-1*H*-pyrazol-5-yl](methyl)amino]piperidine-1-carboxylate

The titled compound was prepared in the similar manner to Reference Example 16.
[Chemical Formula 44]

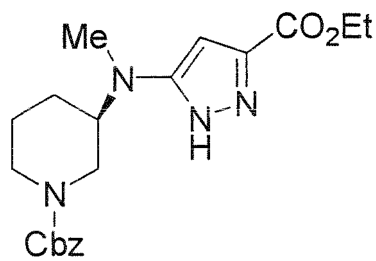

$^1$H-NMR ($CDCl_3$) δ 1.35-1.39 (m, 3H), 1.51-1.74 (m, 4H), 1.78-1.81 (m, 1H), 1.92-1.95 (m, 1H), 2.65-2.73 (m, 1H), 2.82

(s, 3H), 3.57-3.64 (m, 1H), 4.12-4.27 (m, 2H), 4.33-4.38 (m, 2H), 5.13 (s, 2H), 6.17 (s, 1H), 7.29-7.39 (m, 5H)

Reference Example 19

Benzyl (3*S*)-3-[[3-(ethoxycarbonyl)-1*H*-pyrazol-5-yl](methyl)amino]piperidine-1-carboxylate

The titled compound was prepared in the similar manner to Reference Example 16.

[Chemical Formula 45]

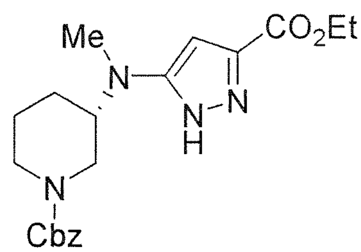

$^1$H-NMR ($CDCl_3$) δ 1.35-1.39 (m, 3H), 1.51-1.74 (m, 4H), 1.78-1.81 (m, 1H), 1.92-1.95 (m, 1H), 2.65-2.73 (m, 1H), 2.82 (s, 3H), 3.57-3.64 (m, 1H), 4.12-4.27 (m, 2H), 4.33-4.38 (m, 2H), 5.13 (s, 2H), 6.17 (s, 1H), 7.29-7.39 (m, 5H)

Example 1

*N*-[(*E*)-5-(aminocarbonyl)-2-adamantyl]-5-(dimethylamino)-1-methyl-1H-pyrazole-3-carboxamide and *N*-[(*Z*)-5-(aminocarbonyl)-2-adamantyl]-5-(dimethylamino)-1-methyl-1H-pyrazole-3-carboxamide

[Chemical Formula 46]

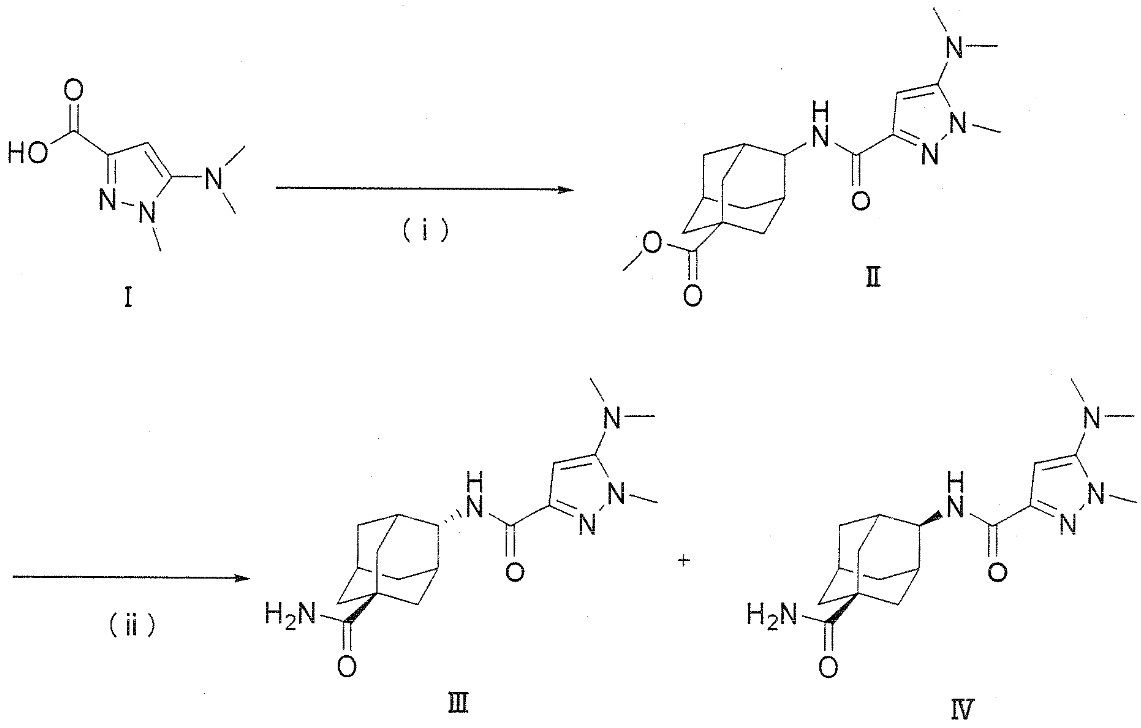


I II III + IV

Step (i):

Compound I (153 mg) was dissolved in DMF (5 mL), and then thereto were added methyl 4-aminoadamantane-1-carboxylate hydrochloride (200 mg), WSCI•HCl (217 mg), HOBt•$H_2O$ (146 mg) and triethylamine (158 μL), and the mixture was stirred at room temperature for 6 hours. Thereto was added water, and the mixture was extracted with ethyl acetate. The organic layer was dried over sodium sulfate and concentrated *in vacua*. The residue was purified by preparative TLC (eluent: chloroform/methanol = 10/1) to give Compound II (82 mg).

Step (ii):

Compound II (72 mg) was dissolved in methanol (3 ml), and then thereto was added 2N sodium hydroxide solution (1 mL) and the mixture was stirred at room temperature overnight and concentrated *in vacuo*. Then, the mixture was acidified by diluted hydrochloric acid, and then extracted with ethyl acetate. The organic layer was dried over sodium sulfate and concentrated *in vacuo*. The residue was dissolved in DMF (5 ml), and thereto were added ammonium chloride (13 mg), WSCI•HCl (45 mg), HOBt•$H_2O$ (32 mg) and triethylamine (55 μL) and the mixture was stirred at room temperature overnight. Thereto was added water, and then the mixture was extracted with ethyl acetate. The organic layer was dried over sodium sulfate and concentrated *in vacuo*. The residue was purified by preparative TLC (eluent: chloroform/methanol = 10/1) to give Compound III (16 mg) as a high-polar ingredient and Compound IV (4.9 mg) as a low-polar one. The structures were determined by X-ray crystallographic analysis.

*N*-[(*E*)-5-(Aminocarbonyl)-2-adamantyl]-5-(dimethylamino)-1-methyl-1H-pyrazole-3-carboxamide A high-polar ingredient

$^1$H-NMR ($CDCl_3$) δ 1.63-1.68 (m, 3H), 1.93-1.97 (m, 4H), 2.00-2.08 (m, 4H), 2.08-2.16 (m, 2H), 2.69 (s, 6H), 3.75 (s, 3H), 4.19-4.21 (m, 1H), 5.27 (s, 1H), 5.62 (s, 1H), 6.31 (s, 1H), 7.17 (m, 1H) *N*-[(*Z*)-5-(Aminocarbonyl)-2-adamantyl]-5-(dimethylamino)-1-methyl-1H-pyrazole-3-carboxamide

A low-polar ingredient

$^1$H-NMR ($CDCl_3$) δ 1.55-1.68 (m, 4H), 1.77-1.84 (m, 3H), 1.93-1.97 (m, 3H), 2.08-2.10 (m, 2H), 2.20 (m, 1H), 2.70 (s, 6H), 3.75 (s, 3H), 4.15 (m, 1H), 5.24 (s, 1H), 5.65 (s, 1H), 6.32 (m, 1H), 7.13 (m, 1H)

Example 2

5-[Cyclopropyl(3-methoxybenzyl)amino]-*N*-[(*E*)-5-hydroxy-2-adamantyl]-1-methyl-1H-pyrazole-3-carboxamide

[Chemical Formula 47]

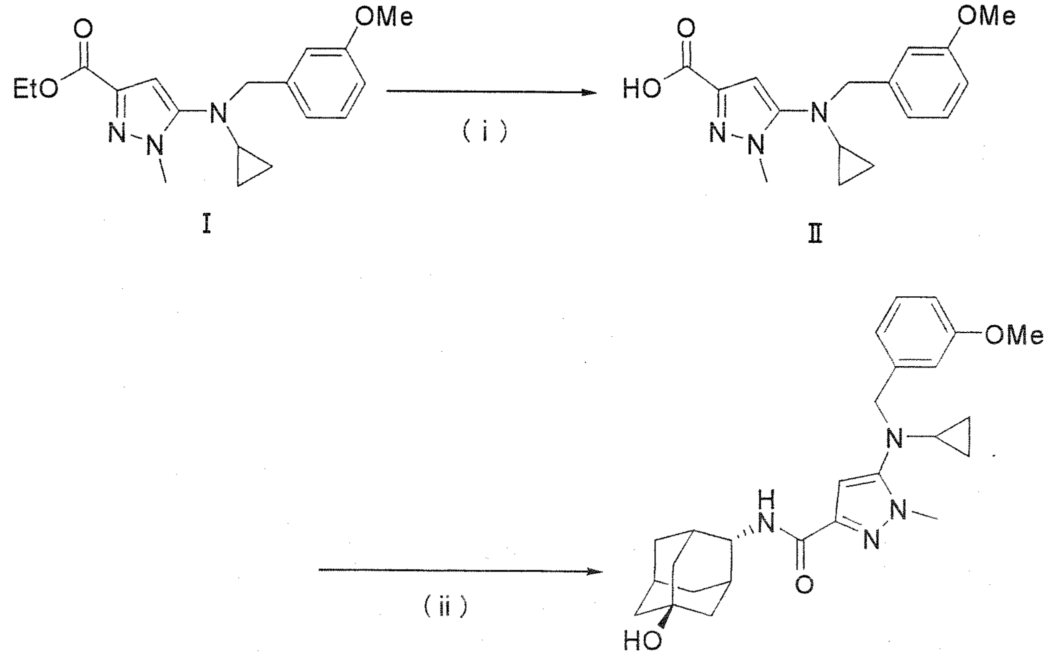


I II III

Step (i):

Compound I (24 mg) was dissolved in methanol (5 mL), and then thereto was added 2N sodium hydroxide solution (500 μL) and the mixture was stirred at room temperature overnight. The reaction solution was concentrated *in vacuo*, and then acidified by 1N hydrochloric acid and extracted with ethyl acetate. The organic layer was dried over sodium sulfate and concentrated *in vacuo* to give Compound II (14 mg).

Step (ii):

Compound II (14 mg) was dissolved in DMF (2 mL), and then thereto were added (*E*)-4-aminoadamantan-1-ol hydrochloride (14 mg), WSCI•HCl (86 mg), HOBt•$H_2O$ (60 mg) and triethylamine (19 μL), and the mixture was stirred at room temperature overnight. Thereto was added water, and then the mixture was extracted with ethyl acetate. The organic layer was dried over sodium sulfate and concentrated *in vacua*. The residue was purified by preparative TLC (eluent: chloroform/methanol = 10/1) to give Compound III (21 mg).
$^1$H-NMR ($CDCl_3$) δ 0.44-0.46 (m, 2H), 0.56-0.57 (m, 2H), 1.52-1.55 (m, 2H), 1.64 (m, 1H), 1.78-1.80 (m, 4H), 1.85-1.95 (m, 4H), 2.19-2.22 (m, 3H), 2.44 (m, 1H), 3.55 (s, 3H), 3.75 (s, 3H), 4.10 (s, 2H), 4.17 (m, 1H), 6.67 (s, 1H), 6.76 (m, 1H), 6.80 (m, 1H), 7.10 (m, 1H), 7.19 (m, 1H)
The following compounds were synthesized in the similar manner to Example 2.

Example 3

5-[Cyclopropyl(4-methoxybenzyl)amino]-*N*-[(*E*)-5-hydroxy-2-adamantyl]-1-methyl-1H-pyrazole-3-carboxamide

[Chemical Formula 48]

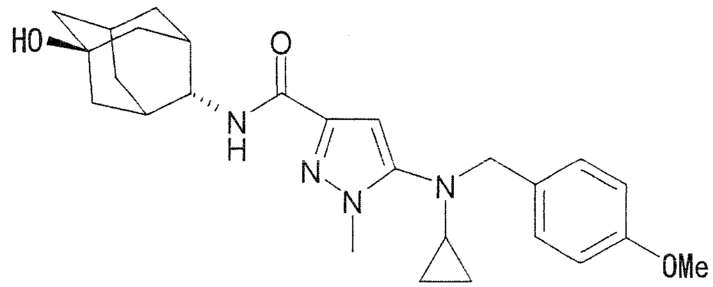

$^1$H-NMR ($CDCl_3$) δ 0.42-0.43 (m, 2H), 0.55-0.57 (m, 2H), 1.52-1.60 (m, 3H), 1.78-1.80 (m, 4H), 1.86-1.96 (m, 4H), 2.19-2.22 (m, 3H), 2.39 (m, 1H), 3.52 (s, 3H), 3.79 (s, 3H), 4.06 (s, 2H), 4.18 (m, 1H), 6.45 (s, 1H), 6.78-6.81 (m, 2H), 7.01-7.05 (m, 2H), 7.11 (m, 1H)

Example 4

N-[(*E*)-5-Hydroxy-2-adamantyl]-1-methyl-5-[methyl(2-phenoxyethyl)amino]-1H-pyrazole-3-carboxamide

[Chemical Formula 49]

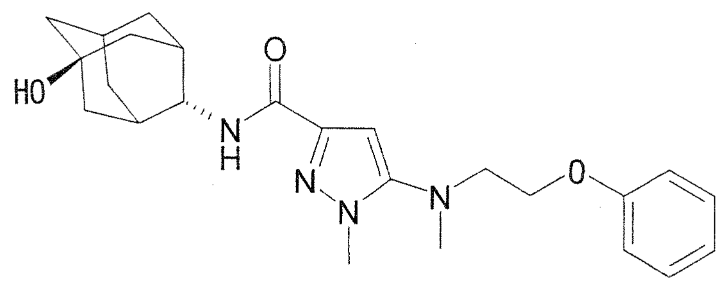

$^1$H-NMR ($CDCl_3$) δ 1.52-1.54 (m, 3H), 1.78-1.80 (m, 4H), 1.85-1.88 (m, 2H), 1.92-1.95 (m, 2H), 2.18-2.22 (m, 3H), 2.78 (s, 3H), 3.28 (t, 5.4Hz, 2H), 3.75 (s, 3H), 4.04 (t, 5.4Hz, 2H), 4.16-4.18 (m, 1H), 6.44 (s, 1H), 6.85-6.87 (m, 2H), 6.93-6.97 (m, 1H), 7.09-7.11 (m, 1H), 7.27-7.29 (m, 2H)

Example 5

5-[[4-(4-Fluorophenoxy)benzyl](methyl)amino]-N-[(E)-5-hydroxy-2-adamantyl]-1-methyl-1H-pyrazole-3-carboxamide

[Chemical Formula 50]

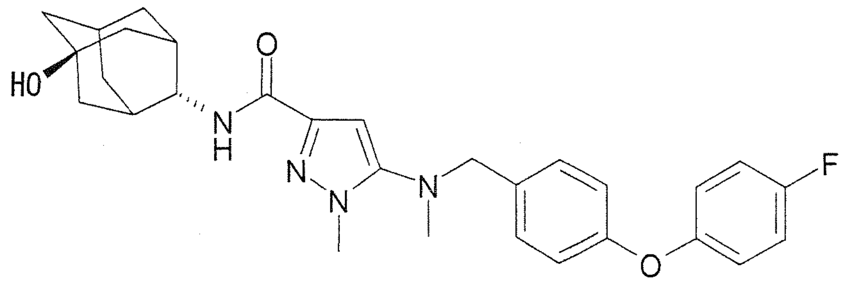

$^1$H-NMR ($CDCl_3$) δ 1.52-1.55 (m, 3H), 1.78-1.80 (m, 4H), 1.84-1.88 (m, 2H), 1.92-1.95 (m, 2H), 2.19-2.21 (m, 3H), 2.61 (s, 3H), 3.74 (s, 3H), 3.96 (s, 2H), 4.16-4.18 (m, 1H), 6.40 (s, 1H), 6.90-6.92 (m, 2H), 6.96-7.06 (m, 4H), 7.09-7.11 (m, 1H), 7.22-7.24 (m, 2H)

Example 6

N-[(*E*)-5-Hydroxy-2-adamantyl]-1-methyl-5-{methyl[3-(methylsulfonyl)benzyl]amino}-1H-pyrazole-3-carboxamide

[Chemical Formula 51]

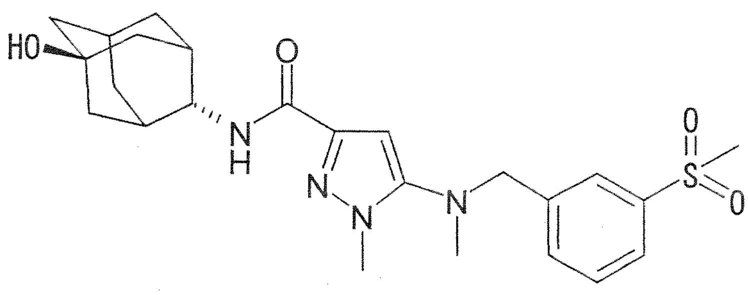

$^1$H-NMR ($CDCl_3$) δ 1.50-1.58 (m, 2H), 1.75-1.94 (m, 9H), 2.20 (m, 3H), 2.64 (s, 3H), 3.06 (s, 3H), 3.73 (s, 3H), 4.12 (s, 2H), 4.13-4.19 (m, 1H), 6.39 (s, 1H), 7.08-7.15 (m, 1H), 7.51-7.63 (m, 2H), 7.74-7.90 (m, 2H)

Example 7

*N*-[(*E*)-5-Carbamoyladamantan-2-yl]-1-methyl-5-[methyl(propyl)amino]-1*H*-pyrazole-3-carboxamide

[Chemical Formula 52]

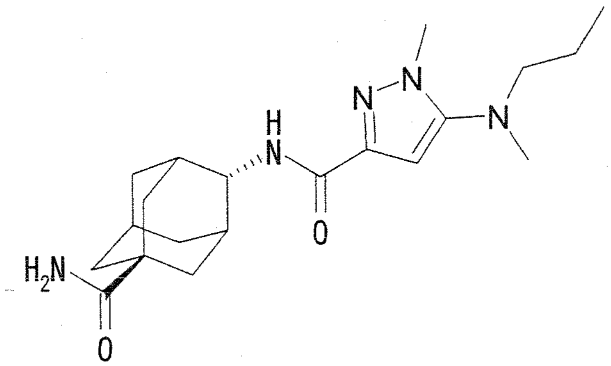

$^1$H-NMR ($CDCl_3$) δ 0.82 (t, J=7.1Hz, 3H), 1.39-1.47 (m, 2H), 1.54-1.57 (m, 2H), 1.70 (m, 1H), 1.85-1.89 (m, 4H), 1.93-1.97 (m, 4H), 1.99-2.10 (m, 2H), 2.57 (s, 3H), 2.71-2.78 (m, 2H), 3.66 (s, 3H), 4.12 (m, 1H), 5.37(bs, 1H), 5.60(bs, 1H), 6.28 (s, 1H), 7.10 (m, 1H)

Example 8

*N*-[(*E*)-5-Carbamoyladamantan-2-yl]-5-[isopropyl(methyl)amino]-1-methyl-1*H*-pyrazole-3-carboxamide

[Chemical Formula 53]

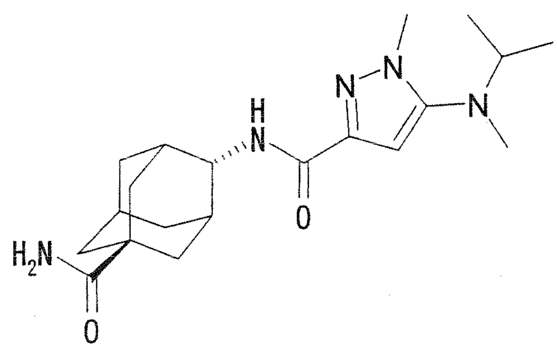

$^1$H-NMR ($CDCl_3$) δ 1.10 (d, J=6.5Hz, 6H), 1.54 (m, 1H), 1.63-1.66 (m, 2H), 1.94-1.99 (m, 4H), 2.02-2.08 (m, 4H), 2.18-2.20 (m, 2H), 2.58 (s, 3H), 3.17 (sept, J=6.5Hz, 1H), 3.73 (s, 3H), 4.21 (m, 1H), 5.20 (bs, 1H), 5.59 (bs, 1H), 6.42 (s, 1H), 7.01 (m, 1H)

Example 9

*N*-[(E)-5-Carbamoyladamantan-2-yl]-5-[ethyl(methyl)amino]-1-methyl-1*H*-pyrazole-3-carboxamide

[Chemical Formula 54]

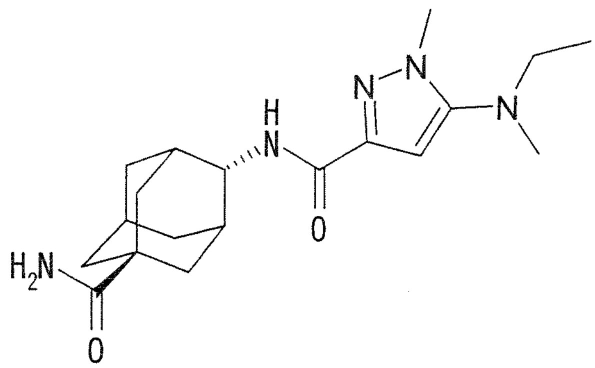

$^1$H-NMR ($CDCl_3$) δ 1.09 (t, J=7.2Hz, 3H), 1.62-1.67 (m, 3H), 1.93-1.98 (m, 4H), 2.02-2.08 (m, 4H), 2.18-2.20 (m, 2H), 2.65 (s, 3H), 2.92 (q, J=7.2Hz, 2H), 3.73 (s, 3H), 4.20 (m, 1H), 5.33 (bs, 1H), 5.63 (bs, 1H), 6.36 (s, 1H), 7.16 (m, 1H)

Example 10

*N*-[(E)-5-Carbalnoyladamantan-2-yl]-5-[isobutyl(methyl)amino]-1-methyl-1*H*-pyrazole-3-carboxamide

[Chemical Formula 55]

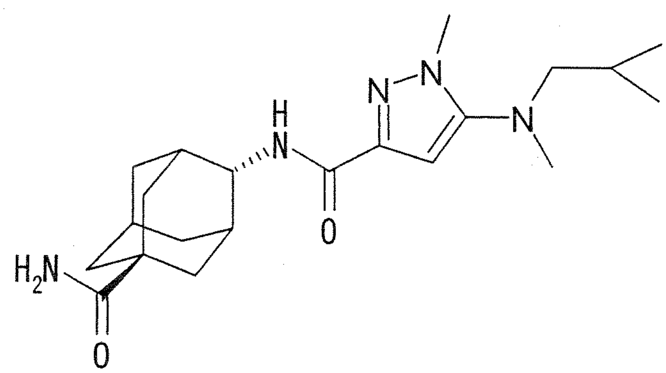

$^1$H-NMR ($CDCl_3$) δ 0.90 (m, 6H), 1.57-1.68 (m, 2H), 1.78 (m, 1H), 1.90-2.09 (m, 9H), 2.13-2.21 (m, 2H), 2.60-2.64 (m, 5H), 3.73 (s, 3H), 4.17-4.23 (m, 1H), 5.32 (bs, 1H), 5.63 (bs, 1H), 6.35 (s, 1H), 7.14-7.20 (m, 1H)

Example 11

*N*-[(*E*)-5-Carbamoyladamantan-2-yl]-4-chloro-5-(dimethylamino)-1-methyl-1*H*-pyrazole-3-carboxamide

[Chemical Formula 56]

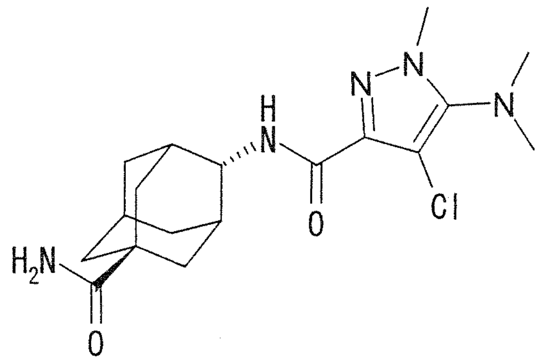

$^1$H-NMR ($CDCl_3$) δ 1.53-1.56 (m, 2H), 1.63-1.66 (m, 2H), 1.90-1.94 (m, 3H), 2.03-2.07 (m, 4H), 2.18-2.22 (m, 2H), 2.86 (s, 6H), 3.73 (s, 3H), 4.22 (m, 1H), 5.19 (bs, 1H), 5.59 (bs, 1H), 7.08 (m, 1H)

Examples 12

*N*-[(E)-5-Hydroxyadamantan-2-yl]-1-methyl-5-{methyl[4-(methylsulfonyl)benzyl]amino}-1*H*-pyrazole-3-carboxamide

[Chemical Formula 57]

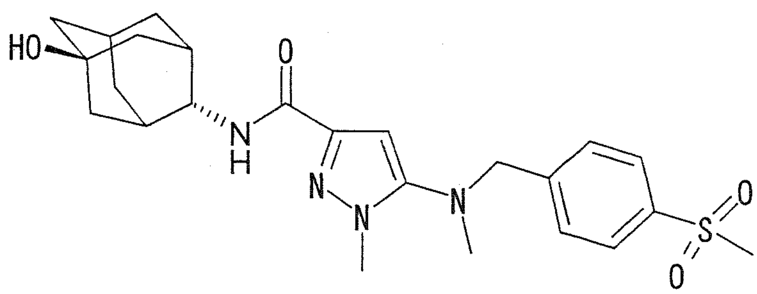

$^1$H-NMR ($CDCl_3$) δ 1.47-1.58 (m, 2H), 1.73-1.96 (m, 9H), 2.13-2.25 (m, 3H), 2.65 (s, 3H), 3.05 (s, 3H), 3.75 (s, 3H), 4.11 (s, 2H), 4.13-4.18 (m, 1H), 6.41 (s, 1H), 7.08-7.13 (m, 1H), 7.49-7.53 (m, 2H), 7.88-7.91 (m, 2H)

Example 13

N-[(E)-5-Carbamoyladamantan-2-yl]-5-[(cyclopropylmethyl)(propyl)amino]-1-methyl-1H-pyrazole-3-carboxamide

[Chemical Formula 58]

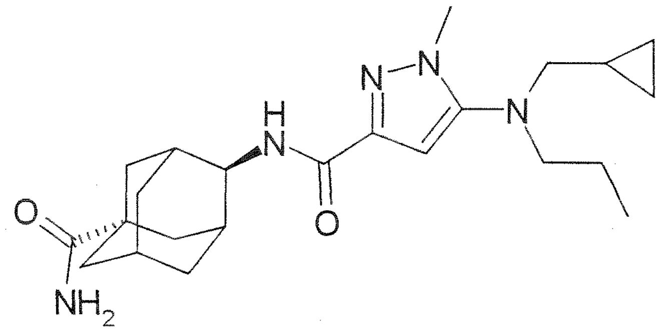

$^1$H-NMR ($CDCl_3$) δ 0.02-0.05 (m, 2H), 0.40-0.45 (m, 2H), 0.81-0.88 (m, 1H), 0.86 (t, J=7.4Hz, 3H), 1.36-1.43 (m, 2H), 1.60-1.65 (m, 2H), 1.93-2.18 (m, 11H), 2.73 (d, J=6.8Hz, 2H), 2.91 (t, J=7.4Hz, 2H), 3.76 (s, 3H), 4.19-4.21 (m, 1H), 5.16 (bs, 1H), 5.58 (bs, 1H), 6.47 (s, 1H), 7.19-7.21 (m, 1H)

Example 14

*N*-[(E)-5-Carbamoyladamantan-2-yl]-5-[(2-methoxyethyl)(methyl)amino]-1-methyl-1*H*-pyrazole-3-carboxamide

[Chemical Formula 59]

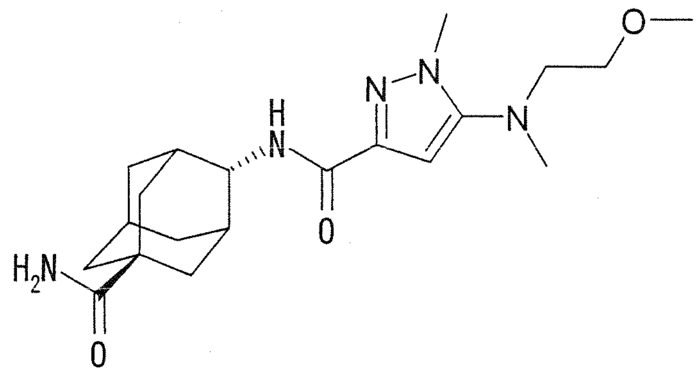

$^1$H-NMR ($CDCl_3$) δ 1.58-1.66 (m, 2H), 1.90-2.09 (m, 9H), 2.13-2.20 (m, 2H), 2.72 (s, 3H), 3.05 (m, 2H), 3.34 (s, 3H), 3.46 (m, 2H), 3.75 (s, 3H), 4.16-4.23 (m, 1H), 5.29 (bs, 1H), 5.62 (bs, 1H), 6.38 (s, 1H), 7.14-7.20 (m, 1H)

Example 15

*N*-[(E)-5-Carbamoyladamantan-2-yl]-5-[(cyclopropylmethyl)(methyl)amino]-1-methyl-1*H*-pyrazole-3-carboxamide

[Chemical Formula 60]

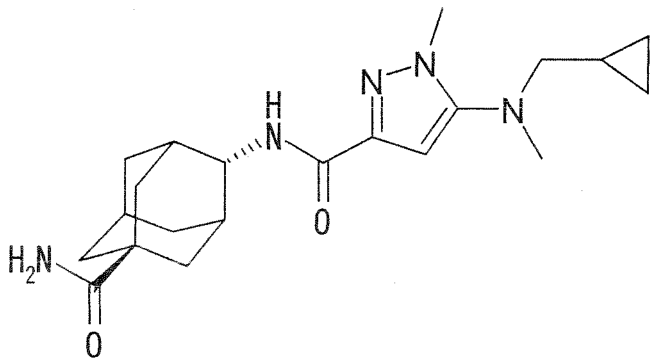

$^1$H-NMR ($CDCl_3$) δ 0.08-0.11 (m, 2H), 0.43-0.53 (m, 2H), 0,85-0.95 (m, 1H), 1.58-1.68 (m, 2H), 1.90-2.09 (m, 9H), 2.13-2.20 (m, 2H), 2.68-2.74 (m, 5H), 3.74 (s, 3H), 4.15-4.23 (m, 1H), 5.25 (bs, 1H), 5.60 (bs, 1H), 6.37 (s, 1H), 7.14-7.20 (m, 1H)

Example 16

5-(Cyclopropyl{[1-(3,3,3-trifluoropropyl)piperidin-4-yl]methyl}amino)-N-[(E)-5-hydroxyadamantan-2-yl]-1-methyl-1*H*-pyrazole-3-carboxamide

[Chemical Formula 61]

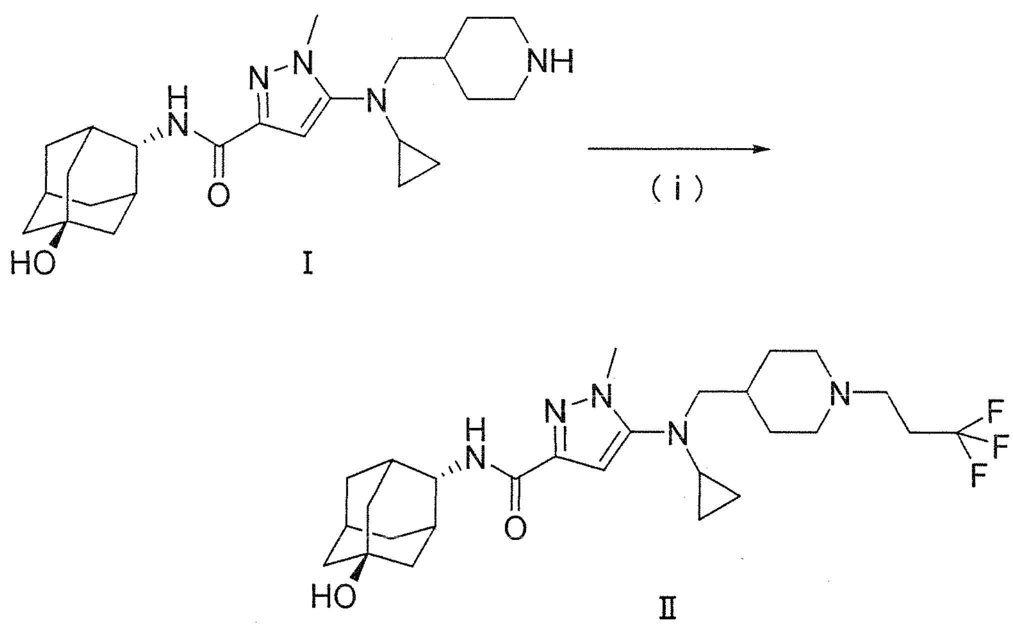


I

II

Step (i):

Compound I (50 mg) was dissolved in dichloromethane (1 mL), and then thereto were added 3,3,3-trifluoro propionaldehyde (23 mg) and acetic acid (100 μL). The mixture was stirred at room temperature for 1.5 hours, and then thereto was added $NaBH(OAc)_3$ (60 mg) and the mixture was stirred at room temperature overnight. Thereto was added water, and the mixture was extracted with chloroform. The organic layer was washed with saturated sodium bicarbonate water. The organic layer was dried over sodium sulfate and concentrated *in vacuo*. The residue was purified by preparative TLC (eluent: chloroform/methanol = 10/1) to give the titled Compound II (48 mg).

$^1$H-NMR ($CDCl_3$) δ 0.35-0.44 (m, 2H), 0.53-0.61 (m, 2H), 1.13-1.28 (m, 2H), 1.46-1.98 (m, 16H), 2.16-2.35 (m, 5H), 2.45 (m, 1H), 2.52-2.59 (m, 2H), 2.81-2.92 (m, 4H), 3.68 (s, 3H), 4.13-4.20 (m, 1H), 6.43 (s, 1H), 7.09-7.14 (m, 1H)

Example 17

5-(Cyclopropyl{[1-(2-methoxyethyl)piperidin-4-yl]methyl}amino)-*N*-[(E)-5-hydroxyadamantan-2-yl]-1-methyl-1*H*-pyrazole-3-carboxamide

[Chemical Formula 62]

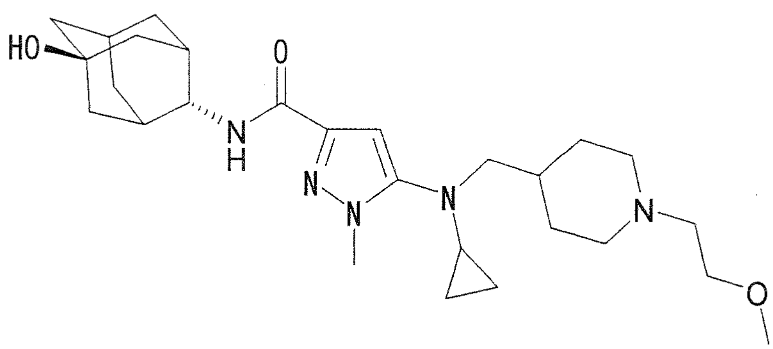

$^1$H-NMR ($CDCl_3$) δ 0.36-0.45 (m, 2H), 0.53-0.61 (m, 2H), 1.20-1.33 (m, 2H), 1.46-1.72 (m, 6H), 1.74-1.98 (m, 10H), 2.15-2.26 (m, 3H), 2.45 (m, 1H), 2.50-2.58 (m, 2H), 2.85-2.97 (m, 4H), 3.34 (s, 3H), 3.49 (m, 2H), 3.67 (s, 3H), 4.14-4.20 (m, 1H), 6.43 (s, 1H), 7.09-7.14 (m, 1H)

Example 18

*N*-[(E)-5-Carbamoyladamantan-2-yl]-5-(diethylarmino)-1-methyl-1*H*-pyrazole-3-carboxamide

[Chemical Formula 63]

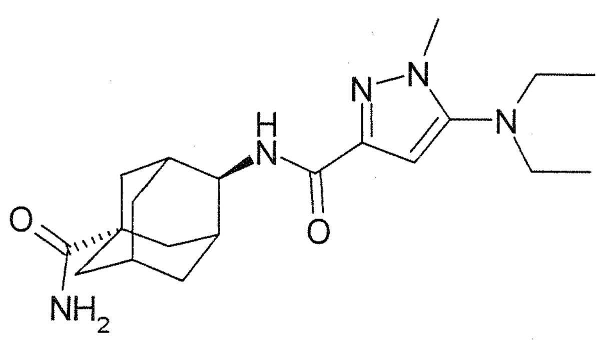

$^1$H-NMR ($CDCl_3$) δ 1.00 (t, J=7.1Hz, 6H), 1.62-1.65 (m, 2H), 1.93-2.18 (m, 11H), 2.93 (q, J=7.1Hz, 4H), 3.73 (s, 3H), 4.19-4.21 (m, 1H), 5.17 (bs, 1H), 5.58 (bs, 1H), 6.44 (s, 1H), 7.19-7.21 (m, 1H)

Example 19

*N*-[(E)-5-Carbamoyladamantan-2-yl]-5-[cyclobutyl(methyl)amino]-1-methyl-1*H*-pyrazole-3-carboxamide

[Chemical Formula 64]

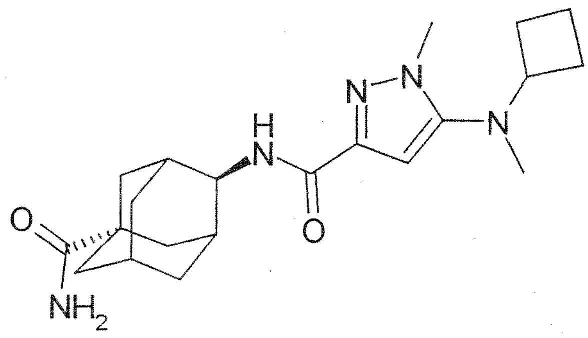

$^1$H-NMR ($CDCl_3$) δ 1.40-1.70 (m, 4H), 1.77-1.87 (m, 2H), 1.93-2.11 (m, 11H), 2.17-2.18 (m, 2H), 2.52 (s, 3H), 3.48-3.56 (m, 1H), 3.73 (s, 3H), 4.19-4.21 (m, 1H), 5.19 (bs, 1H), 5.58 (bs, 1H), 6.33 (s, 1H), 7.17-7.19 (m, 1H)

Example 20

5-[Cyclopropyl(piperidin-4-ylmethyl)amino]-N-[(E)-5-hydroxyadamantan-2-yl]-1-methyl-1*H*-pyrazole-3-carboxamide

[Chemical Formula 65]

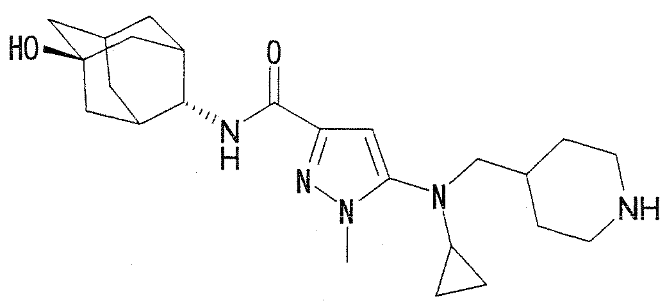

$^1$H-NMR ($CDCl_3$) δ 0.40-0.45 (m, 2H), 0.55-0.61 (m, 2H), 1.50-1.67 (m, 4H), 1.68-1.97 (m, 13H), 2.15-2.26 (m, 3H), 2.48 (m, 1H), 2.72-2.83 (m, 2H), 2.94-2.99 (m, 2H), 3.41-3.46 (m, 2H), 3.69 (s, 3H), 4.13-4.19 (m, 1H), 6.43 (s, 1H), 7.09-7.15 (m, 1H)

Example 21

5-{[(1-Acetylpiperidin-4-yl)methyl](cyclopropyl)amino}-N-[(E)-5-hydroxyadamantan-2-yl]-1-methyl-1*H*-pyrazole-3-carboxamide

[Chemical Formula 66]

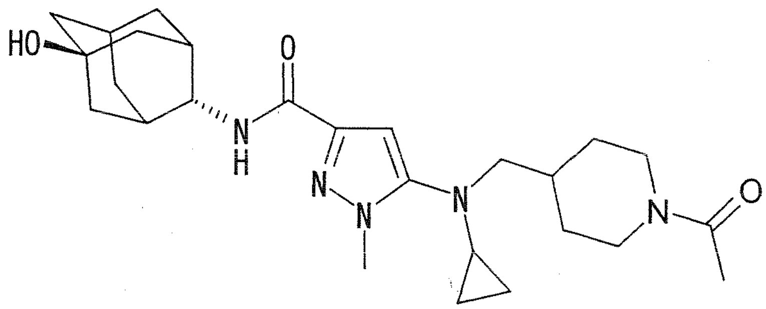

$^1$H-NMR ($CDCl_3$) δ 0.38-0.45 (m, 2H), 0.54-0.61 (m, 2H), 1.01-1.15 (m, 2H), 1.41-1.65 (m, 5H), 1.67-1.82 (m, 5H), 1.83-1.97 (m, 4H), 2.06 (s, 3H), 2.15-2.28 (m, 3H), 2.42-2.53 (m, 2H), 2.86-2.31 (m, 3H), 3.69 (s, 3H), 3.73-3.81 (m, 1H), 4.13-4.21 (m, 1H), 4.53-4.62 (m, 1H), 6.44 (s, 1H), 7.09-7.15 (m, 1H)

Example 22

N-[(E)-5-(Aminocarbonyl)-2-adamantyl]-4-chloro-5-[[1-(4-methoxyphenyl)cyclopropyl](methyl)amino]-1-methyl-1H-pyrazole-3-carboxamide

[Chemical Formula 67]

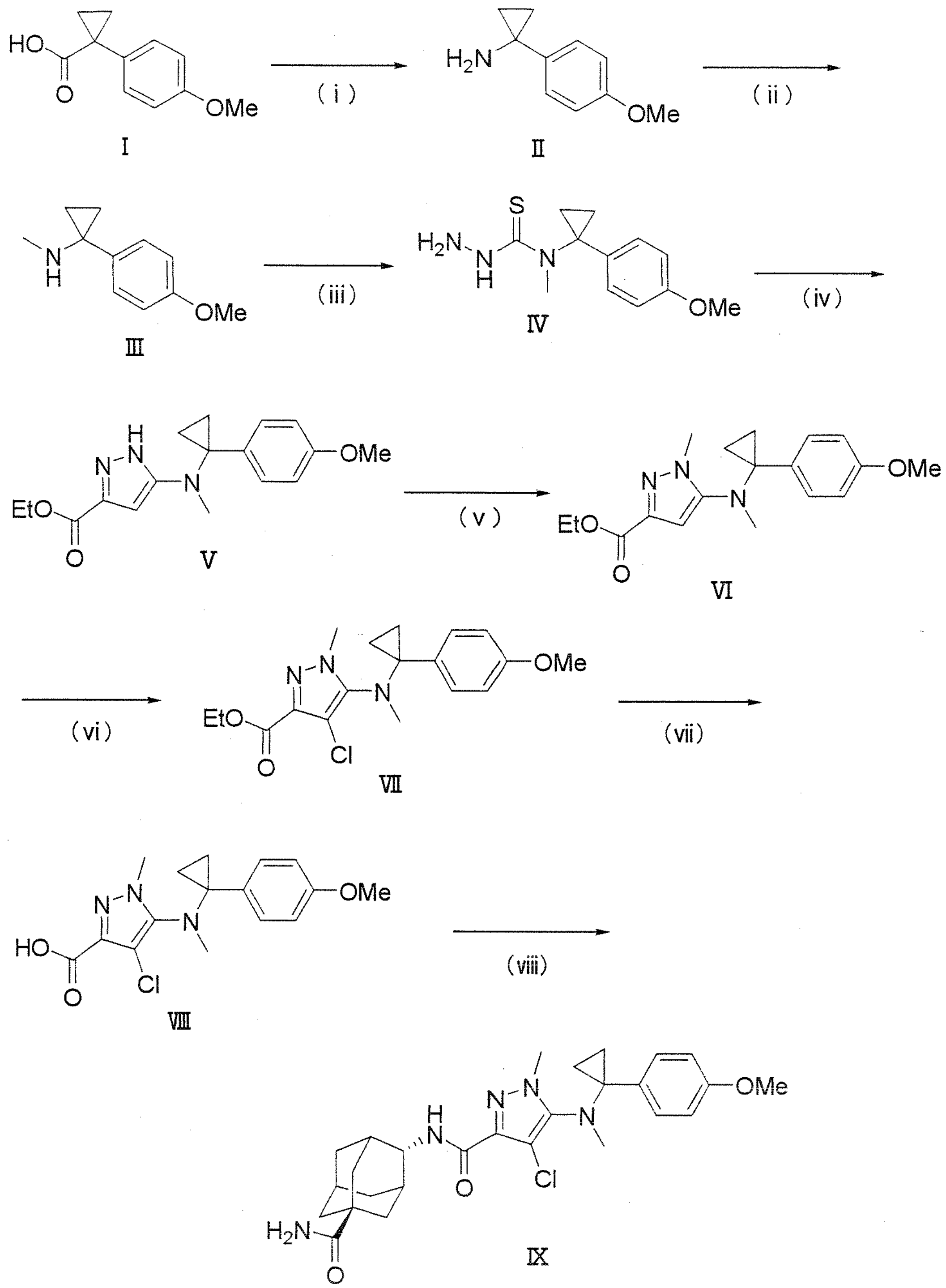


Step (i):

1-(4-Methoxyphenyl)-cyclopropanecarboxylic acid (5.0 g) was dissolved in toluene (80 mL), and then thereto were added triethylamine (3. mL) and diphenyl phosphoryl azide (5.9 mL), and the mixture was stirred at 100˚C for 5 hours. The reaction solvent was concentrated *in vacuo,* and then dissolved in THE (80 ml). Then, thereto was added 2N sodium hydroxide solution (30 any the mixture was stirred at room temperature for 2 hours. The mixture was concentrated *in vacuo,* and then thereto was added 1N hydrochloric acid and the mixture was extracted with ethyl acetate. The aqueous layer was basified by sodium hydroxide solution and extracted with chloroform. The organic layer was dried over sodium sulfate and concentrated *in vacuo* to give Compound II (2.9 g).

Step (ii):

Compound II (2.9 g) was dissolved in ethyl formate (30 mL) and stirred in a sealed tube at 90˚C for 3 days, and the reaction solvent was concentrated *in vacuo.* The residue was dissolved in THF (10 mL) and added dropwise to a solution of lithium aluminum hydride (2.7 g) in THF (80 mL). The mixture was stirred at 80˚C for 3 hours, and then thereto were added water (3 mL), 15% sodium hydroxide solution (3 mL) and water (9 mL) at 0˚C in sequence. The reaction solution was filtered through Elite®. The filtrate was concentrated *in vacuo* to give Compound III (2.5 g).

Step (iii):

4-Chlorophenyl chlorothioformate (2.9 g) was dissolved in THF (30 mL), and thereto were added triethylamine (2.1 mL) and Compound III (2.5 g) at 0˚C and the mixture was stirred at room temperature for 2 hours. The reaction solution was concentrated *in vacuo,* and then thereto was added water and the mixture was extracted with ethyl acetate. The organic layer was washed with brine, dried over sodium sulfate, and then concentrated *in vacuo.* The residue was dissolved in NMP (30 mL), and then thereto was added hydrazine monohydrate (3.4 mL) and the mixture was stirred at 70˚C for 6 hours. Thereto was added water, and the mixture was extracted with ethyl acetate. The organic layer was washed with brine, and then dried over sodium sulfate and concentrated *in vacuo.* The residue was purified by silica gel chromatography (eluent: hexane/ethyl acetate = 7/3) to give Compound IV (2.1 g).

Step (iv):

Compound IV (2.1 g) was dissolved in a mixed solvent of ethanol (25 mL) and THE (25 mL), and thereto were added sodium bicarbonate (690 mg) and ethyl bromopyruvate (1.2 mL) and the mixture was stirred at 70˚C for 4 hours. The reaction solution was concentrated *in vacuo,* and then thereto was added acetic acid (50 mL) and the mixture was stirred at 80˚C for 5 hours and concentrated *in vacuo.* Then, thereto was added saturated sodium bicarbonate water, and the mixture was extracted with ethyl acetate. The organic layer was dried over sodium sulfate and concentrated *in vacuo.* The residue was purified by silica gel chromatography (eluent: hexane/ethyl acetate = 2/1) to give Compound V (1.6 g).

Step (v):

To a solution of sodium hydride (250 mg) in THF (20 mL) was added dropwise a solution of Compound V (1.6 g) in THF (5 mL) at 0˚C, and the mixture was stirred at room temperature for 1 hour. Then, thereto was slowly added methyl iodide (380 μL) at 0˚C, and the mixture was stirred at room temperature overnight. Then, thereto was added water, and then the mixture was concentrated *in vacuo.* Thereto was added saturated sodium bicarbonate water, and the mixture was extracted with chloroform. The organic layer was dried over sodium sulfate and concentrated *in vacuo.* The residue was purified by silica gel chromatography (eluent: hexane/ethyl acetate = 2/1) to give Compound VI (1.2 g).

Step (vi):

Compound VI (599 mg) was dissolved in DMF (10 mL), and then thereto was added N-chlorosuccinimide (267 mg) in small portions, and the mixture was stirred at room temperature overnight. Then, thereto was added water, and then the mixture was extracted with ethyl acetate. The organic layer was sequentially washed with saturated sodium bicarbonate water and brine. The organic layer was dried over sodium sulfate and concentrated *in vacuo.* The residue was purified by silica gel chromatography (eluent: hexane/ethyl acetate = 3/1) to give Compound VII (530 mg).

Step (vii):

Compound VII (530 mg) was dissolved in ethanol (15 mL), and then thereto was added 2N lithium hydroxide solution (2.2 mL), and the mixture was stirred at room temperature overnight. The reaction solution was concentrated *in vacuo,* and then acidified by 1N hydrochloric acid and extracted with chloroform. The organic layer was dried over sodium sulfate and concentrated *in vacuo* to give Compound VIII (490 mg).

Step (viii):

Compound VIII (80 mg) was dissolved in DMF (1.5 mL), and then thereto were added methyl (E)-4-aminoadamantane-1-carboxylate (50 mg), WSCI•HCl (69 mg), HOBt•$H_2O$ (49 mg) and triethylamine (100 μL), and the mixture was stirred at room temperature overnight. Then, thereto was added water, and then the mixture was extracted with

ethyl acetate. The organic layer was sequentially washed with saturated sodium bicarbonate water, 1N hydrochloric acid and brine. The organic layer was dried over sodium sulfate and concentrated *in vacuo.* The residue was dissolved in methanol (1.5 mL), and then thereto was added 2N lithium hydroxide solution (400 μL) and the mixture was stirred at room temperature overnight. The reaction solution was concentrated *in vacuo,* and then acidified by 1N hydrochloric acid and extracted with ethyl acetate. The organic layer was dried over sodium sulfate and concentrated *in vacuo.* The residue was dissolved in DMF (1.5 mL), and then thereto were added ammonium chloride (134 mg), WSCI•HCl (69 mg), HOBt•$H_2O$ (49 mg) and triethylamine (460 μL) and the mixture was stirred at room temperature overnight. Then, thereto was added water, and then the mixture was extracted with ethyl acetate. The organic layer was sequentially washed with saturated sodium bicarbonate water, 1N hydrochloric acid and brine. The organic layer was dried over sodium sulfate and concentrated *in vacuo.* The residue was purified by preparative TLC (eluent: chloroform/methanol = 10/1) to give the titled Compound IX (101 mg) as a white solid.
$^1$H-NMR ($CDCl_3$) δ 0.98-1.01 (m, 2H), 1.10-1.13 (m, 2H), 1.63-1.66 (m, 2H), 1.89-2.19 (m, 11H), 2.96 (s, 3H), 3.61 (s, 3H), 3.81 (s, 3H), 4.23-4.25 (m, 1H), 6.11 (bs,1H), 6.64 (bs,1H), 6.85-6.90 (m, 2H), 7.13-7.15 (m, 1H), 7.22-7.25 (m, 2H)

The following compounds were obtained in the similar manner.

[Chemical Formula 68]

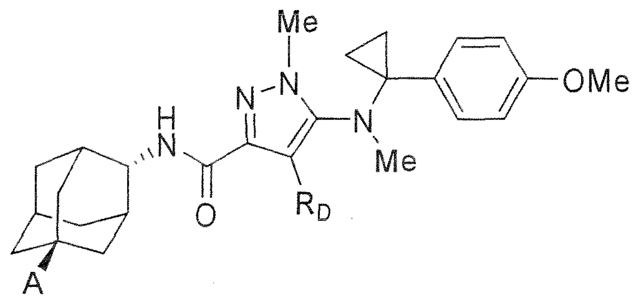

| Example No. | $R_D$ | A |
|---|---|---|
| 23 | H | $CONH_2$ |
| 24 | Cl | OH |
| 25 | H | OH |

Example 23

$^1$H-NMR (CDCh) δ 1.03-1,11 (m, 4H), 1.61-2.18 (m, 13H), 2.82 (s, 3H), 3.71 (s, 3H), 3.81 (s, 3H), 4.19-4.21 (m, 1H), 5.24 (bs,1H), 5.61 (bs,1H), 6.24 (s, 1H), 6.87-6.91 (m, 2H), 7.17-7.20 (m, 2H), 7.45-7.47 (m, 1H)

Example 24

$^1$H-NMR ($CDCl_3$) δ 0.98-1.01 (m, 2H), 1.11-1.14 (m, 2H), 1.52-1.55 (m, 2H), 1.77-1.85 (m, 7H). 1.91-1.94 (m, 2H), 2.18-222 (m, 3H), 2.97 (s, 3H), 3.59 (s, 3H), 3.81 (s, 3H), 4.18-4.20 (m, 1H), 6.87-6.90 (m, 2H), 7.05-7.06 (m, 1H), 7.23-7.25 (m, 2H)

Example 25

$^1$H-NMR ($CDCl_3$) δ 0.97-1.04 (m, 4H), 1.61-1.64 (m, 3H), 1.69-1.81 (m, 6H), 1.93-1.96 (m, 2H), 2.15-2.16 (m, 1H), 2.27 (bs,2H), 2.75 (s, 3H), 3.65 (s, 3H), 3.81 (s, 3H), 4.08-4.10 (m, 1H), 6.21 (s, 1H), 6.87-6.91 (m, 2H), 7.18-7.25 (m, 3H)

Example 26

N-[(E)-5-(Aminocarbonyl)-2-adamantyl]-4-fluoro-5-[isopropyl(methyl)amino]-1-methyl-1H-pyrazole-3-carboxamide

[Chemical Formula 69]

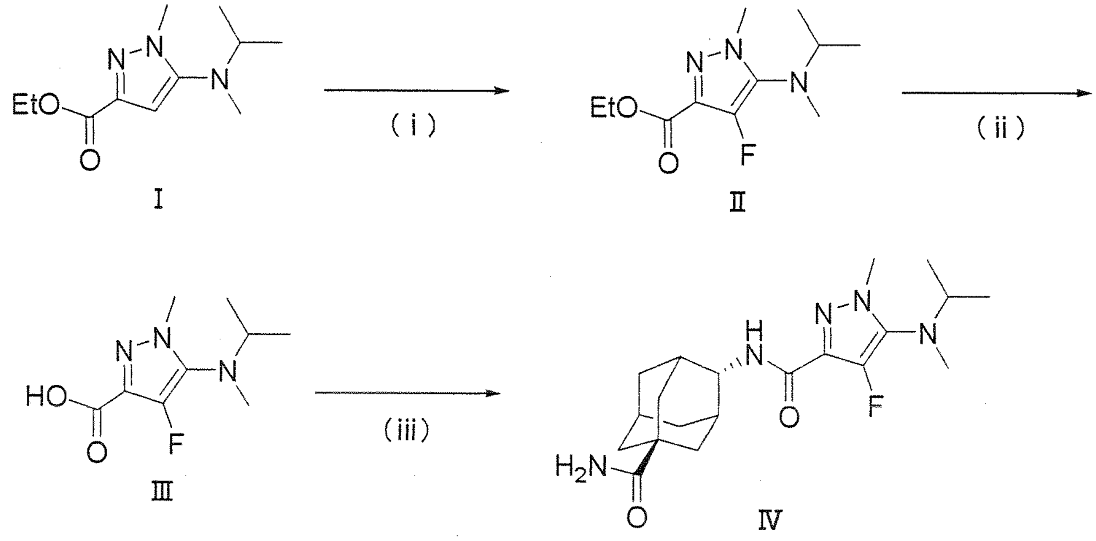


Step (i):

To a solution of Compound I (200 mg) in DMF (15 mL) was added saturated sodium bicarbonate water (3 mL), and then thereto was added 1-chloromethyl-4-fluoro-1,4-diazoniabicyclo[2.2.2.]octane bis(tetrafluoroborate) (638 mg) in small portions and the mixture was stirred at room temperature for 3 hours. Then, thereto was added water, and then the mixture was extracted with ethyl acetate. The organic layer was dried over sodium sulfate and concentrated *in vacuo.* The residue was purified by silica gel chromatography (eluent: hexane/ethyl acetate = 3/1) to give Compound II (64 mg).

Step (ii):

Compound II (65 mg) was dissolved in ethanol (1.5 mL), and then thereto was added 2N lithium hydroxide solution (380 μL) and the mixture was stirred at room temperature overnight. The reaction solution was concentrated *in vacuo,* and then acidified by 1N hydrochloric acid and extracted with ethyl acetate. The organic layer was dried over sodium sulfate and concentrated *in vacuo* to give Compound III (45 mg).

Step (iii):

Compound III (23 mg) was dissolved in DMF (0.5 mL), and then thereto were added methyl (E)-4-aminoadamantane-1-carboxylate (31 mg), WSCI•HCl (29 mg), HOBt•$H_2O$ (20 mg) and triethylamine (40 μL), and the mixture was stirred at room temperature overnight. Then, thereto was added water, and then the mixture was extracted with ethyl acetate. The organic layer was sequentially washed with saturated sodium bicarbonate water, 1N hydrochloric acid and brine. The organic layer was dried over sodium sulfate and concentrated *in vacuo.* The residue was dissolved in methanol (0.5 mL), and then thereto was added 2N lithium hydroxide solution (140 μL) and the mixture was stirred at room temperature overnight. The reaction solution was concentrated *in vacuo,* and then acidified by 1N hydrochloric acid and extracted with ethyl acetate. The organic layer was dried over sodium sulfate and concentrated *in vacuo.* The residue was dissolved in DMF (0.5 mL), and then thereto were added ammonium chloride (53 mg), WSCI•HCl (28 mg), HOBt•$H_2O$ (20 mg) and triethylamine (130 μL) and the mixture was stirred at room temperature overnight. Then, thereto was added water, and then the mixture was extracted with ethyl acetate. The organic layer was sequentially washed with saturated sodium bicarbonate water, 1N hydrochloric acid and brine. The organic layer was dried over sodium sulfate and concentrated *in vacuo.* The residue was purified by preparative TLC (eluent: chloroform/methanol = 10/1) to give the titled Compound IV (22 mg) as a white solid.
$^1$H-NMR ($CDCl_3$) δ 1.10-1.12 (m, 6H), 1.63-1.66 (m, 2H), 1.89-2.18 (m, 11H), 2.74 (s, 3H), 3.18-3.27 (m, 1H), 3.71 (s, 3H), 4.20-4.25 (m, 1H), 6.00 (bs, 1H), 6.41 (bs, 1H), 6.93-6.95 (m, 1H)

Example 27

5-[[(1-Acetylpiperidin-4-yl)methyl](methyl)amino]-N-[(E)-5-hydroxy-2-adamantyl]-1-methyl-1H-pyrazole-3-carboxam-

ide

[Chemical Formula 70]

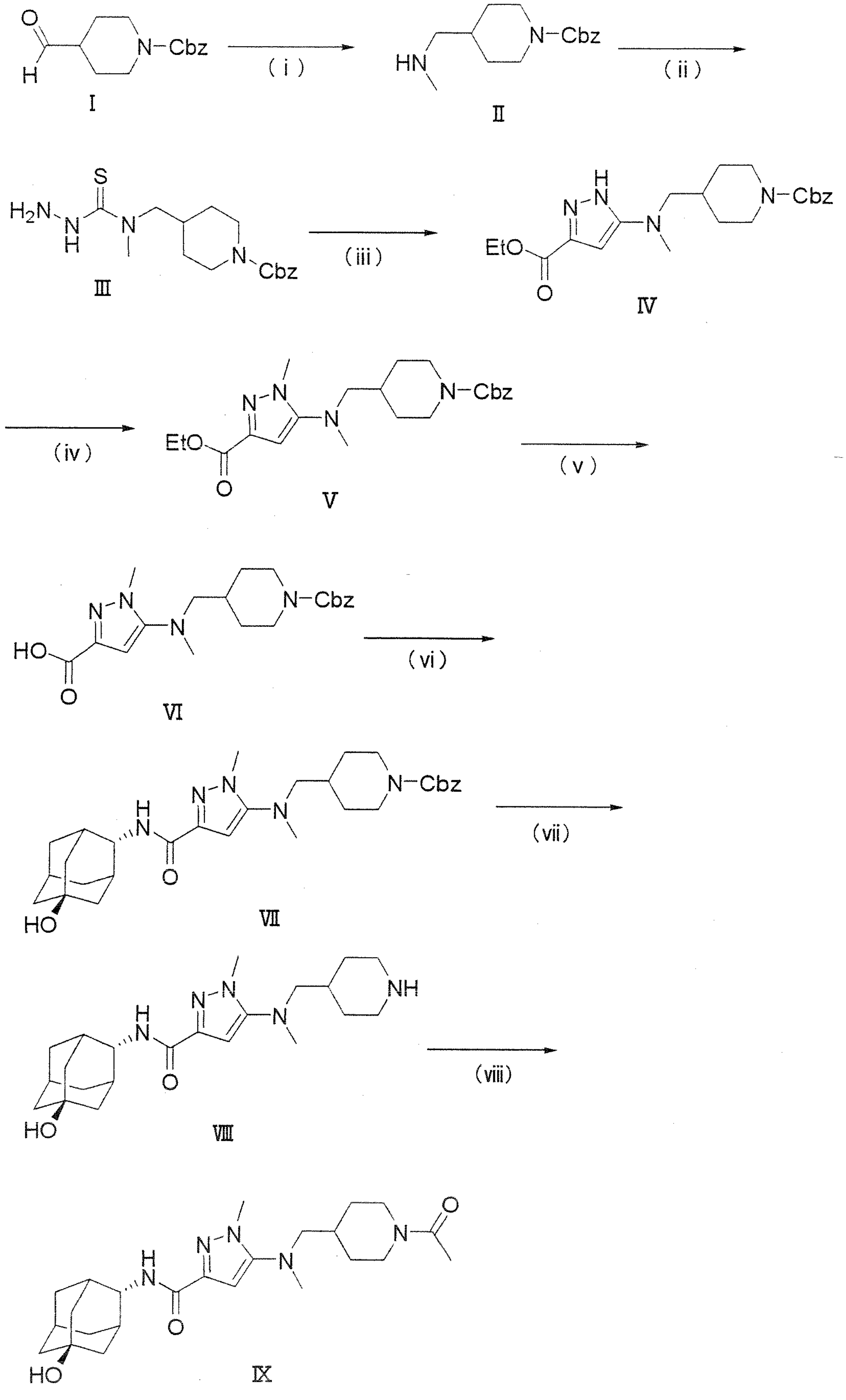


I II III IV V VI VII VIII IX

Step (i):

Compound I (6.2 g) was dissolved in dichloromethane (70 mL), and then thereto were added 2M methylamine-THF solution (40 mL) and acetic acid (2 mL), and the mixture was stirred at room temperature for 1 hour. Then, thereto was added $NaBH(OAc)_3$ (8.0 g) and the mixture was stirred at room temperature overnight. Then, thereto was added water, and then the mixture was concentrated *in vacuo.* Then, thereto was added saturated sodium bicarbonate water, and the mixture was extracted with chloroform. The organic layer was dried over sodium sulfate and concentrated *in vacuo.* The residue was purified by silica gel chromatography (eluent: chloroform/methanol = 10/1) to give Compound II (2.3 g).

Step (ii):

To a solution of 1,1'-thiocarbonyldiimidazole (1.1 g) in THF (25 mL) was added a solution of Compound II (2.3 g) in THF (5 mL), and the mixture was stirred at room temperature overnight. Then, thereto was added hydrazine monohydrate (1.7 mL), and the mixture was stirred at 70˚C for 6 hours and concentrated *in vacuo.* Then, thereto was added brine, and the mixture was extracted with chloroform. The organic layer was dried over sodium sulfate, and then concentrated *in vacuo.* The residue was purified by silica gel chromatography (eluent: chloroform/methanol = 5/1) to give Compound III (2.5 g).

Step (iii):

Compound III (2.5 g) was dissolved in a mixed solvent of ethanol (25 mL) and THF (25 mL), and then thereto were added sodium bicarbonate (680 mg) and ethyl bromopyruvate (1.1 mL) and the mixture was stirred at 80˚C for 4 hours. Then, thereto was added acetic acid (25 mL), and the mixture was stirred at 70˚C for 3 hours and concentrated *in vacuo.* Then, thereto was added saturated sodium bicarbonate water, and the mixture was extracted with ethyl acetate. The organic layer was dried over sodium sulfate and concentrated *in vacuo.* The residue was purified by silica gel chromatography (eluent: hexane/ethyl acetate = 1/1) to give Compound IV (1.3 g).

Step (iv):

To a solution of sodium hydride (160 mg) in THF (10 mL) was added dropwise a solution of Compound IV (1.3 g) in THF (6 mL) at 0˚C, and the mixture was stirred at room temperature for 1 four. Then, thereto was slowly added methyl iodide (310 $\mu$L) at 0˚C, and the mixture was stirred at room temperature overnight. Then, thereto was added water, and the mixture was extracted with ethyl acetate. The organic layer was dried over sodium sulfate and concentrated *in vacuo.* The residue was purified by silica gel chromatography (eluent: hexane/ethyl acetate = 2/1) to give Compound V (851 mg).

Step (v):

Compound V (845 mg) was dissolved in ethanol (7 mL), and then thereto was added 6N lithium hydroxide solution (1 mL) and the mixture was stirred at 40˚C for 3 hours. The reaction solution was concentrated *in vacuo,* and then acidified by 1N hydrochloric acid and extracted with chloroform. The organic layer was dried over sodium sulfate and concentrated *in vacuo* to give Compound VI (750 mg).

Step (vi):

Compound VI (390 mg) was dissolved in DMF (5 mL), and then thereto were added (E)-4-aminoadamantan-1-ol hydrochloride (305 mg), WSCI•HCl (382 mg), HOBt•$H_2O$ (270 mg) and triethylamine (340 $\mu$L), and the mixture was stirred at room temperature overnight. Then, thereto was added saturated sodium bicarbonate water, and then the mixture was extracted with ethyl acetate and the organic layer was washed with brine. The organic layer was dried over sodium sulfate and concentrated *in vacuo.* The residue was purified by preparative TLC (eluent: chloroform/methanol = 10/1) to give Compound VII (450 mg).

Step (vii):

Compound VII (450 mg) was dissolved in methanol (4 mL), and then thereto was added palladium-carbon (50 mg) and the mixture was stirred under hydrogen atmosphere (3 atm) for 4.5 hours. The resulting solid was filtered through Celite®, and then the filtrate was concentrated to give Compound VIII (340 mg).

Step (viii):

Compound VIII (80 mg) was dissolved in dichloromethane (1 mL), and then thereto were added triethylamine (86 μL) and acetyl chloride (30 μL) and the mixture was stirred at room temperature for 3 hours. Then, thereto was added 2N sodium hydroxide solution, and the mixture was extracted with chloroform. The organic layer was dried over sodium sulfate and concentrated *in vacuo.* The residue was purified by preparative TLC (eluent: chlorofiorm/methanol = 10/1) to give the titled Compound IX (38 mg).

$^1$H-NMR ($CDCl_3$) δ 1.05-1.18 (m, 2H), 1.45-1.96 (m, 14H), 2.07 (s, 3H), 2.16-2.21 (m, 3H), 2.42-2.52 (m, 1H), 2.63 (s, 3H), 2.68-2.78 (m, 2H), 2.92-3.05 (m, 1H), 3.72 (s, 3H), 3.73-3.83 (m, 1H), 4.10-4.19 (m, 1H), 4.55-4.65 (m, 1H), 6.38 (s, 1H), 7.08-7.14 (m, 1H)

Example 28

5-[[(1-Acetylpiperidin-4-yl)methyl](methyl)amino]-4-chloro-N-[(2s,5r)-5-hydroxy-2-adamantyl]-1-methyl-1H-pyrazole-3-carboxamide

[Chemical Formula 71]

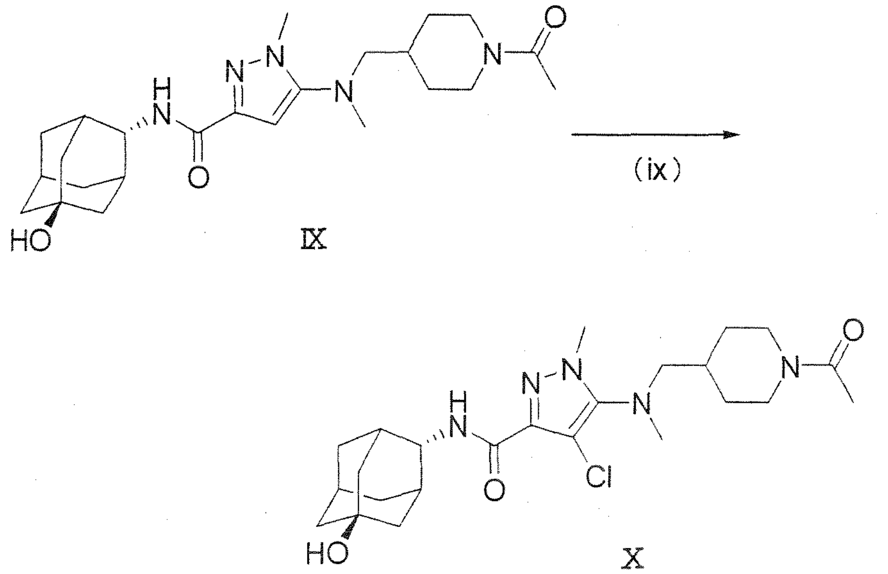


IX

X

Step (ix):

Compound (16 mg) of Example 27 was dissolved in DMF (200 μL), and then thereto was added N-chlorosuccinimide (6 mg) and the mixture was stirred at 65°C for 3 hours. Then, thereto was added water, and then the mixture was extracted with ethyl acetate. The organic layer was washed with brine, dried over sodium sulfate and concentrated *in vacuo.* The residue was purified by preparative TLC (eluent: chloroform/methanol = 10/1) to give the titled Compound X (7 mg) as a white solid.

$^1$H-NMR ($CDCl_3$) δ 1.05-1.18 (m, 2H), 1.44-1.60 (m, 3H), 1.72-1.97 (m, 9H), 2.08 (s, 3H), 2.15-2.26 (m, 3H), 2.45-2.56 (m, 1H), 2.72-2.85 (m, 5H), 2.91-3.12 (m, 3H), 3.73 (s, 3H), 3.75-3.85 (m, 1H), 4.15-4.25 (m, 1H), 4.55-4.68 (m, 1H), 6.96-7.08 (m, 1H),

Example 29

4-Chloro-5-[cyclobutyl(2,2.2-trifluoroethyl)amino]-N-[(E)-5-hydroxy-2-adamantyl]-1-metlayl-1H-pyrazole-3-carboxamide

[Chemical Formula 72]

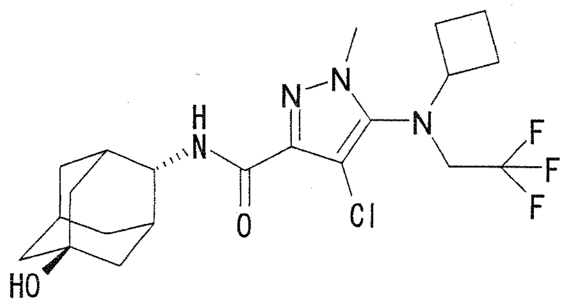

Compound (100 mg) of Reference Example 7 was dissolved in DMF (1.5 mL), and then thereto were added (E)-4-aminoadamantan-1-ol hydrochloride (78 mg), WSCI•HCl (122 mg), HOBt•$H_2O$ (86 mg) and triethylamine (150 μL), and the mixture was stirred at room temperature overnight. Then, thereto was added water, and then the mixture was extracted with ethyl acetate. The organic layer was washed with brine. The organic layer was dried over sodium sulfate and concentrated *in vacuo.* The residue was purified by preparative TLC (eluent: chloroform/methanol = 10/1) to give the titled Compound (128 mg) as a white solid.

$^1$H-NMR ($CDCl_3$) δ 1.42-1.68 (m, 5H), 1.71-2.00 (m, 10H), 2.02-2.29 (m, 5H), 3.45-3.80 (m, 5H), 3.80-3.95 (m, 1H), 4.16-4.25 (m, 1H), 6.99-7.09 (m, 1H)

Compounds of Examples 30-131 were prepared in the similar manner.

[Chemical Formula 73]

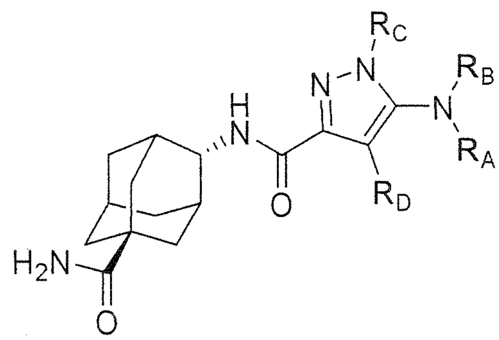

| Example No. | $R_A$ | $R_B$ | $R_C$ | $R_D$ |
|---|---|---|---|---|
| 30 | $CH_3$ | $CH_2CH_3$ | $CH_3$ | Cl |
| 31 | $CH_3$ | 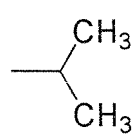 | $CH_3$ | Cl |
| 32 | $CH_3$ | 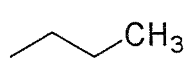 | $CH_3$ | Cl |
| 33 | $CH_3$ | 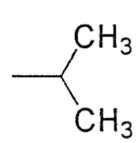 | $CH_2CH_3$ | H |

(continued)

| Example No. | $R_A$ | $R_B$ | $R_C$ | $R_D$ |
|---|---|---|---|---|
| 34 | $CH_3$ | 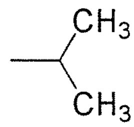 | $CH_2CH_3$ | Cl |
| 35 | $CH_3$ | $CH_3$ | $CH_2CH_3$ | Cl |
| 36 | $CH_2CH_3$ | 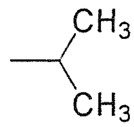 | $CH_3$ | Cl |
| 37 | $CH_2CH_3$ | $CH_2CH_3$ | $CH_3$ | Cl |
| 38 | $CH_2CH_3$ | 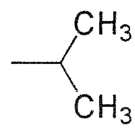 | $CH_3$ | H |
| 39 | $CH_3$ | 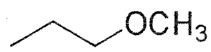 | $CH_3$ | Cl |
| 40 | $CH_3$ | 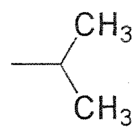 | $CH_3$ | Cl |
| 41 | $CH_3$ | $CH_2$-$CF_3$ | $CH_3$ | Cl |
| 42 | $CH_3$ | $CH_2$-$CF_3$ | $CH_3$ | H |
| 43 | $CH_3$ | 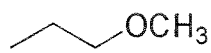 | $CH_3$ | H |

Example 30

$^1$H-NMR ($CDCl_3$) δ 1.02 (t, J=8.0Hz, 3H), 1.61-1.63 (m, 4H), 1.88-1.90 (m, 2H), 1.97-2.04 (m, 4H), 2.12-2.16 (m, 2H), 2.30 (m, 1H), 2.80 (s, 3H), 3.10 (q, J=8.0Hz, 2H), 3.70 (s, 3H), 4.18 (m, 1H), 5.21 (bs, 1H), 5.59 (bs, 1H), 7.08 (m, 1H)

Example 31

$^1$H-NMR ($CDCl_3$) δ 1.08 (d, J=4.0Hz, 6H), 1.86-1.88 (m, 4H), 1.97-2.04 (m, 6H), 2.15-2.17 (m, 3H), 2.77 (s, 3H), 3.38 (m, 1H), 3.70 (s, 3H), 4.20 (m, 1H), 5.25 (bs, 1H), 5.60 (bs, 1H), 7.10 (m, 1H)

Example 32

$^1$H-NMR ($CDCl_3$) δ 0.89 (t, J=8.0Hz, 3H), 1.39 (q, J=8.0Hz, 2H), 1.61-1.65 (m, 2H), 1.89-1.92 (m, 3H), 1.97-2.06 (m, 4H), 2.15-2.17 (m, 2H), 2.80 (s, 3H), 3.06 (t, J=8.0Hz, 3H), 3.72 (s, 3H), 4.02 (m, 1H), 4.22 (m, 1H), 5.34 (bs, 1H); 5.66 (bs, 1H), 7.08 (m, 1H)

Example 33

$^1$H-NMR ($CDCl_3$) δ 1.10 (d, J=8.0Hz, 6H), 1.40-1.47 (m, 3H), 1.50-1.67 (m, 2H), 1.88-1.95 (m, 2H), 1.98-2.12 (m, 6H), 2.17-2.23 (m, 2H), 2.60 (s, 3H), 3.13-3.18 (m, 2H), 4.07-4.14 (m, 2H), 4.21 (m, 1H), 5.35 (bs, 1H), 5.64 (bs, 1H), 6.45 (s, 1H), 7.34 (m, 1H)

Example 34

$^1$H-NMR ($CDCl_3$) δ 1.10 (d, J=8.0Hz, 6H), 1.38-1.43 (m, 3H), 1.62-1.69 (m, 2H), 1.89-1.96 (m, 4H), 2.00-2.13 (m, 5H), 2.16-2.23 (m, 2H), 2.79 (s, 3H), 3.39 (m, 1H), 4.11-4.15 (m, 2H), 4.24 (m, 1H), 6.01 (bs, 1H), 6.50 (bs, 1H), 7.18 (m, 1H)

Example 35

$^1$H-NMR ($CDCl_3$) δ 1.40 (t, J=8.0Hz, 3H), 1.59-1.68 (m, 2H), 1.89-1.93 (m, 4H), 1.99-2.06 (m, 5H), 2.19-2.23 (m, 2H), 2.84 (s, 6H), 4.09 (q, J=8.0Hz, 2H), 4.20 (m, 1H), 5.21 (bs, 1H), 5.60 (bs, 1H); 7.10

(m, 1H)

Example 36

$^1$H-NMR ($CDCl_3$) δ 0.90-0.93 (m, 3H), 1.08-1.09 (m, 6H), 1.62-1.65 (m, 2H), 1.92-1.94 (m, 4H), 1.99-2.07 (m, 5H), 2.19 (bs,2H), 3.17-3.19 (m, 2H), 3.39-3.45 (m, 1H), 3.72 (s, 3H), 4.21-4.23 (m, 1H), 5.44 (bs,1H), 5.65 (bs,1H), 7.12-7.14 (m, 1H)

Example 37

$^1$H-NMR ($CDCl_3$) δ 0.98-1.01 (m, 6H), 1.60-1.66 (m, 2H), 1.91-1.93 (m, 4H), 1.99-2.09 (m, 5H), 2.19 (bs,2H), 3.13-3.19 (m, 4H), 3.74 (s, 3H), 4.21-4.23 (m, 1H), 5.34 (bs, 1H), 5.70 (bs, 1H), 7.11-7.13 (m, 1H)

Example 38

$^1$H-NMR ($CDCl_3$) δ 0.88-0.92 (m, 4H), 1.04-1.05 (m, 6H), 1.61-1.65 (m, 2H), 1.93-2.08 (m, 9H), 2.18 (bs, 1H), 2.88-2.94 (m, 2H), 3.11-3.18 (m, 1H), 3.73 (s, 3H), 4.20-4.22 (m, 1H), 5.33 (bs, 1H), 5.61 (bs, 1H), 6.49 (s, 1H), 7.21-7.23 (m, 1H)

Example 39

$^1$H-NMR ($CDCl_3$) δ 1.59-1.66 (m, 2H), 1.85-2.09 (m, 9H), 2.15-2.24 (m, 2H), 2.88 (s, 3H), 3.26-3.33 (m, 5H), 3.35-3.41 (m, 2H), 3.75 (s, 3H), 4.15-4.24 (m, 1H), 5.62 (bs, 1H), 5.78 (bs, 1H), 7.06-7.14 (m, 1H)

Example 40

$^1$H-NMR ($CDCl_3$) δ 0.89-0.93 (m, 6H), 1.59-1.78 (m, 3H), 1.85-2.09 (m, 9H), 2.15-2.21 (m. 2H), 2.77 (s, 3H), 2.88-2.93 (m, 2H), 3.74 (s, 3H), 4.17-4.25 (m, 1H), 5.45 (bs, 1H), 5.71 (bs, 1H), 7.07-7.14 (m, 1H)

Example 41

$^1$H-NMR ($CDCl_3$) δ 1.58-1.70 (m, 2H), 1.86-2.10 (m, 9H), 2.15-2.22 (m, 2H), 2.95 (s, 3H), 3.59-3.71 (m, 2H), 3.78 (s, 3H), 4.18-4.25 (m, 1H), 5.58 (bs, 1H), 5.73 (bs, 1H), 7.07-7.13 (m, 1H)

Example 42

$^1$H-NMR ($CDCl_3$) δ 1.58-1.70 (m, 2H), 1.88-2.12 (m, 9H), 2.14-2.21 (m, 2H), 2.86 (s, 3H), 3.42-3.51 (m, 2H), 3.78 (s, 3H), 4.15-4.23 (m, 1H), 5.34 (bs, 1H), 5.63 (bs, 1H), 6.48 (s, 1H), 7.13-7.23 (m, 1H)

Example 43

$^1$H-NMR ($CDCl_3$) δ C1.95-1.04 (m, 3H), 1.58-1.69 (m, 2H), 1.88-2.12 (m, 9H), 2.13-2.22 (m, 2H), 2.95-3.04 (m, 2H), 3.05-3.13 (m, 2H), 3.30 (s, 3H), 3.32-3.40 (m, 2H), 3.75 (s, 3H), 4.17-4.24 (m, 1H), 5.38 (bs, 1H), 5.65 (bs, 1H), 6.47 (s, 1H), 7.15-7.24 (m, 1H)

[Chemical Formula 74]

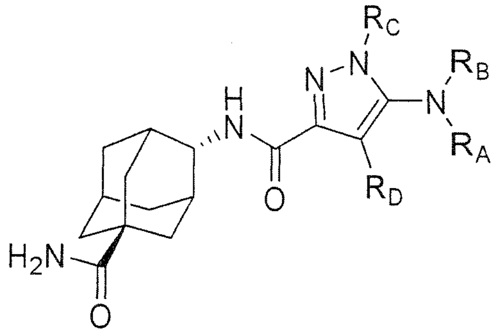

| Example No. | $R_A$ | $R_B$ | $R_C$ | $R_D$ |
|---|---|---|---|---|
| 44 | 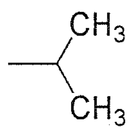 | 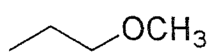 | $CH_3$ | H |
| 45 | $CH_2CH_3$ | 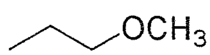 | $CH_3$ | Cl |
| 46 | 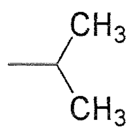 | 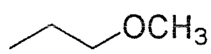 | $CH_3$ | Cl |
| 47 | $CH_3$ | $CH_2$-$CF_3$ | $CH_2CH_3$ | H |
| 48 | $CH_3$ | $CH_2$-$CF_3$ | $CH_3$ | $CH_3$ |
| 49 | $CH_3$ | $CH_2$-$CF_3$ | $CH_2CH_3$ | Cl |
| 50 | $CH_2$-$CF_3$ | 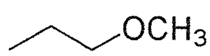 | $CH_3$ | Cl |
| 51 | $CH_2$-$CF_3$ | 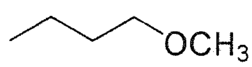 | $CH_3$ | Cl |
| 52 | $CH_2$-$CF_3$ | 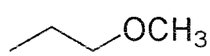 | $CH_3$ | H |
| 53 | $GH_2$-$GF_3$ | 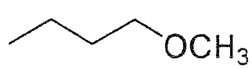 | $CH_3$ | H |
| 54 | $CH_3$ | $CH_2$-$CF_3$ | $CH_2CH_3$ | Cl |
| 55 | $CH_2$-$CF_3$ | 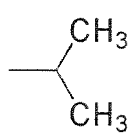 | $CH_3$ | H |

(continued)

| Example No. | $R_A$ | $R_B$ | $R_C$ | $R_D$ |
|---|---|---|---|---|
| 56 | $CH_2$-$CF_3$ | 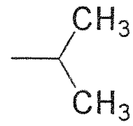 | $CH_3$ | Cl |

Example 44

$^1$H-NMR ($CDCl_3$) δ 1.00-1.12 (m, 6H), 1.58-1.69 (m, 2H), 1.89-2.12 (m, 9H), 2.13-2.23 (m, 2H), 3.02-3.12 (m, 2H), 3.13-3.31 (m, 6H), 3.75 (s, 3H), 4.18-4.25 (m, 1H), 5.35 (bs, 1H), 5.64 (bs, 1H), 6.52 (s, 1H), 7.18-7.27 (m, 1H)

Example 45

$^1$H-NMR ($CDCl_3$) δ 0.95-1.04 (m, 3H), 1.58-1.69 (m, 2H), 1.85-2.10 (m, 9H), 2.15-2.22 (m, 2H), 3.15-3.22 (m, 2H), 3.28-3.37 (m, 7H), 3.75 (s, 3H), 4.18-4.25 (m, 1H), 5.30 (bs, 1H), 5.64 (bs, 1H), 7.15-7.27 (m, 1H)

Example 46

$^1$H-NMR ($CDCl_3$) δ 1.05-1.16 (m, 6H), 1.58-1.68 (m, 2H), 1.88-2.10 (m, 9H), 2.18-2.24 (m, 2H), 3.22-3.28 (m, 5H), 3.29-3.48 (m, 3H), 3.75 (s, 3H), 4.18-4.25 (m, 1H), 5.32 (bs, 1H), 5.65 (bs, 1H), 7.05-7.16(m, 1H)

Example 47

$^1$H-NMR ($CDCl_3$) δ 1.38-1.48 (m, 3H), 1.59-1.70 (m, 2H), 1.88-2.11 (m, 9H), 2.14-2.21 (m, 2H), 2.84 (s, 3H), 3.40-3.52 (m, 2H), 4.07-4.24 (m, 3H), 5.32 (bs, 1H), 5.63 (bs, 1H), 6.50 (s, 1H), 7.18-7.25 (m, 1H)

Example 48

$^1$H-NMR ($CDCl_3$) δ 1.56-1.75 (m, 2H), 1.89-2.10 (m, 9H), 2.13-2.19 (m, 2H), 2.28 (s, 3H), 2.92 (s, 3H), 3.50-3.62 (m, 2H), 3.74 (s, 3H), 4.14-4.22 (m, 1H), 5.32 (bs, 1H), 5.62 (bs, 1H), 7.18-7.29 (m, 1H)

Example 49

$^1$H-NMR ($CDCl_3$) δ 1.38-1.50 (m, 3H), 1.58-1.72 (m, 2H), 1.86-2.11 (m, 9H), 2.15-2.25 (m, 2H), 2.95 (s, 3H), 3.60-3.72 (m, 2H), 4.08-4.26 (m, 3H), 5.33 (bs, 1H), 5.64 (bs, 1H), 7.05-7.14 (m, 1H)

Example 50

$^1$H-NMR ($CDCl_3$) δ 1.62-1.69 (m, 2H), 1.88-2.11 (m, 9H), 2.15-2.24 (m, 2H), 3.27-3.45 (m, 7H), 3.72-3.87 (m, 5H), 4.16-4.27 (m, 1H), 5.32 (bs, 1H), 5.64 (bs, 1H), 7.06-7.14 (m, 1H)

Example 51

$^1$H-NMR ($CDCl_3$) δ 1.58-1.72 (m, 4H), 1.85-2.12 (m, 9H), 2.13-2.22 (m, 2H), 3.23-3.36 (m, 5H), 3.36-3.46 (m, 2H), 3.63-3.72 (m, 2H), 3.77 (s, 3H), 4.16-4.25 (m, 1H), 5.29 (bs, 1H), 5.62 (bs, 1H), 7.05-7.14(m, 1H)

Example 52

$^1$H-NMR ($CDCl_3$) δ 1.59-1.70 (m, 2H), 1.88-2.11 (m, 9H), 2.14-2.21 (m, 2H), 3.18-3.25 (m, 2H), 3.32 (s, 3H), 3.36-3.43 (m, 2H), 3.61-3.72 (m, 2H), 3.77 (s, 3H), 4.17-4.23 (m, 1H), 5.33 (bs, 1H), 5.63 (bs, 1H), 6.55 (s, 1H), 7.16-7.25 (m, 1H)

Example 53

$^1$H-NMR ($CDCl_3$) δ 1.59-1.72 (m, 4H), 1.89-2.11 (m, 9H), 2.12-2.22 (m, 2H), 3.09-3.11 (m, 2H), 3.30 (s, 3H),

3.33-3.41 (m, 2H), 3.45-3.66 (m, 2H), 3.77 (s, 3H), 4.16-4.25 (m, 1H), 5.35 (bs, 1H), 5.63 (bs, 1H), 6.54 (s, 1H), 7.16-7.23 (m, 1H)

Example 54

$^1$H-NMR ($CDCl_3$) δ 1.39-1.46 (m, 3H), 1.59-1.68 (m, 2H), 1.85-2.20 (m, 12H), 2.95 (s, 3H), 3.61-3.71 (m, 2H), 4.08-4.17 (m, 2H), 4.18-4.25 (m, 1H), 7.08-7.14 (m, 1H)

Example 55

$^1$H-NMR ($CDCl_3$) δ 1.05-1.14 (m, 6H), 1.61-1.68 (m, 2H), 1.90-2.10 (m, 9H), 2.15-2.21 (m, 2H), 3.18-3.28 (m, 1H), 3.41-3.52 (m, 2H), 3.77 (s, 3H), 4.17-4.24 (m, 1H), 5.31 (bs. 1H), 5.62 (bs, 1H), 6.54 (s, 1H), 7.15-7.27 (m, 1H)

Example 56

$^1$H-NMR ($CDCl_3$) δ 1.08-1.21 (m, 6H), 1.60-1.68 (m, 2H), 1.83-2.10 (m, 9H), 2.18-2.22 (m, 2H), 3.38-3.48 (m, 1H), 3.50-3.82 (m, 5H), 4.18-4.24 (m, 1H), 5.30 (bs, 1H), 5.63 (bs, 1H), 7.08-7.17 (m. 1H)

[Chemical Formula 75]

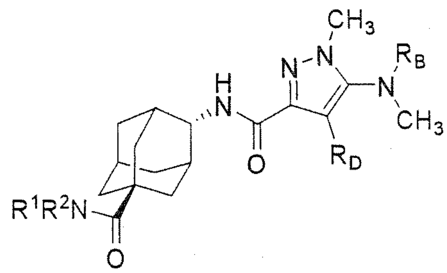

| Example No. | $R_B$ | $R_D$ | $R^1$ | $R^2$ |
|---|---|---|---|---|
| 57 | $CH_3$ | Cl | H | 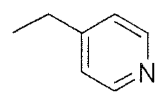 |
| 58 | $CH_3$ | Cl | H | 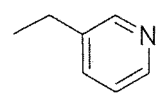 |
| 59 | 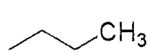 | Cl | H | 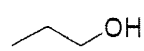 |
| 60 | 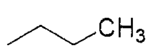 | Cl | H | 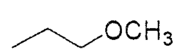 |
| 61 | 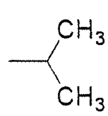 | Cl | H | 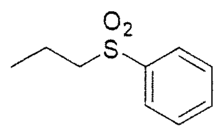 |
| 62 | $CH_2$-$CF_3$ | $CH_3$ | $CH_2CH_3$ | $CH_2CH_3$ |

Example 57

$^1$H-NMR ($CDCl_3$) δ 1.62-1.65 (m, 2H), 1.75 (m, 1H), 1.89-1.95 (m, 4H), 2.05-2.10 (m, 4H), 2.15-2.28 (m, 2H), 2.84 (s, 6H), 3.71 (s, 3H), 4.21 (m, 1H), 4.45 (d, J=4.0Hz, 2H), 6.24 (m, 1H), 7.07 (m, 1H), 7.14 (d, J=8.0Hz, 2H), 8.53 (d, J=8.0Hz, 2H)

Example 58

$^1$H-NMR ($CDCl_3$) δ 1.60-1.63 (m, 2H), 1.80-1.87 (m, 5H), 2.01-2.05 (m, 4H), 2.13-2.15 (m, 2H), 2.84 (s, 6H), 3.71 (s, 3H), 4.18 (m, 1H), 4.45 (d, J=4.0Hz, 2H), 6.21 (m, 1H), 7.07 (d, J=8.0Hz, 1H), 7.25 (d, J=8.0Hz, 1H), 7.59 (d, J=8.0Hz, 1H), 8.49-8.51 (m, 2H)

Example 59

$^1$H-NMR ($CDCl_3$) δ 0.90 (t, J=8.0Hz, 3H), 1.46 (q, J=8.0Hz, 2H), 1.62-1.65 (m, 2H), 1.88-1.91 (m, 5H), 2.01-2.07 (m, 5H), 2.15-2.17 (m, 2H), 2.81 (s, 3H), 3.04 (t, J=8.0Hz, 2H), 3.46-3.50 (m, 2H), 3.73 (s, 3H), 3.76-3.80 (m, 2H), 4.20 (m, 1H), 6.34 (m, 1H), 7.17 (m, 1H)

Example 60

$^1$H-NMR ($CDCl_3$) δ 0.90 (t, J=8.0Hz, 3H), 1.44 (q, J=8.0Hz, 2H), 1.62-1.65 (m, 2H), 1.86-1.93 (m, 4H), 1.98-2.08 (m, 4H), 2.15-2.20 (m, 3H), 2.81 (s, 3H), 3.04 (t, J=8.0Hz, 2H), 3.37 (s, 3H), 3.42-3.50 (m, 4H), 3.72 (s, 3H), 4.21 (m, 1H), 6.12 (m, 1H), 7.15 (m, 1H)

Example 61

$^1$H-NMR ($CDCl_3$) δ 1.10 (d, J=4.0Hz, 6H), 1.60-1.63 (m, 2H), 1.86-1.95 (m, 8H), 2.05 (m, 1H), 2.13-2.16 (m, 2H), 2.79 (s, 3H), 3.28-3.31 (m, 2H), 3.38 (m, 1H), 3.68-3.72 (m, 5H), 4.18 (m, 1H), 6.51 (m, 1H), 7.10 (m, 1H), 7.57-7.62 (m, 2H), 7.70 (m, 1H), 7.90-7.92 (m, 2H)

Example 62

$^1$H-NMR ($CDCl_3$) δ 1.08-1.17 (m, 6H), 1.58-1.70 (m, 2H), 1.86-1.95 (m, 2H), 2.01-2.25 (m, 9H), 2.28 (s, 3H), 2.92 (s, 3H), 3.36-3.50 (m, 4H), 3.52-3.61 (m, 2H), 3.74 (s, 3H), 4.18-4.25 (m, 1H), 7.22-7.29 (m, 1H)

[Chemical Formula 76]

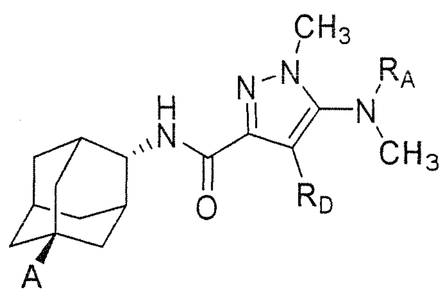

| Example No. | $R_A$ | $R_B$ | $R_D$ | A |
|---|---|---|---|---|
| 63 | 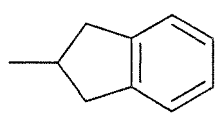 | $CH_3$ | H | $CONH_2$ |
| 64 | 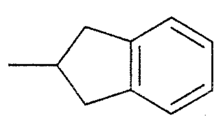 | $CH_3$ | Cl | OH |
| 65 | 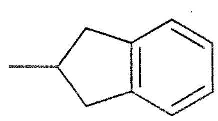 | $CH_3$ | Cl | $CONH_2$ |

(continued)

| Example No. | $R_A$ | $R_B$ | $R_D$ | A |
|---|---|---|---|---|
| 66 | 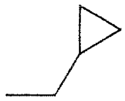 | $CH_3$ | Cl | $CONH_2$ |
| 67 | 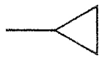 | CH2-CF3 | Cl | OH |
| 68 | 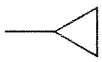 | 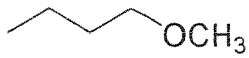 | H | $CONH_2$ |
| 69 | 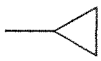 | 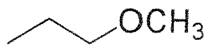 | H | $CONH_2$ |
| 70 | 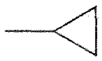 | 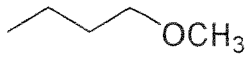 | Cl | OH |
| 71 | 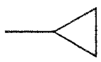 | 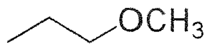 | Cl | OH |

Example 63

$^1$H-NMR ($CDCl_3$) δ 1.62-1.66 (m, 2H), 1.93-2.08 (m, 9H), 2.15-2.18 (m, 2H), 2.61 (s, 3H), 2.87-2.92 (m, 2H), 3.07-3.12 (m, 2H), 3.75 (s, 3H), 3.90 (m, 1H), 4.22 (m, 1H), 5.17 (bs, 1H), 5.58 (bs, 1H), 6.51 (s, 1H), 7.13-7.19 (m, 5H)

Example 64

$^1$H-NMR ($CDCl_3$) δ 1.53-1.62 (m, 4H), 1.78-1.95 (m, 7H), 2.17-2.23 (m, 3H), 2.77-2.79 (m, 2H), 2.84 (s, 3H), 3.06-3.12 (m, 2H), 3.64 (s, 3H), 4.19 (m, 1H), 4.30 (m, 1H), 7.04 (m, 1H), 7.15-7.20 (m, 4H)

Example 65

$^1$H-NMR ($CDCl_3$) δ 1.63-1.69 (m, 2H), 1.89-1.95 (m, 4H), 2.02-2.07 (m, 5H), 2.15-2.19 (m, 2H), 2.77-2.82 (m, 2H), 2.84 (s, 3H), 3.06-3.11 (m, 2H), 3.64 (s, 3H), 4.23 (m, 1H), 4.32 (m, 1H), 5.36 (bs, 1H), 5.66 (bs, 1H), 7.11-7.15 (m, 5H)

Example 66

$^1$H-NMR ($CDCl_3$) δ 0.05-0.10 (m, 2H), 0.39-0.48 (m, 2H), 0.79-0.90 (m, 1H), 1.58-1.68 (m, 2H), 1.88-2.09 (m, 9H), 2.16-2.23 (m, 2H), 2.86 (s, 3H), 2.86-2.93 (m, 2H), 3.75 (s, 3H), 4.17-4.23 (m, 1H), 5.46 (bs, 1H), 5.71 (bs, 1H), 7.07-7.14 (m, 1H)

Example 67

$^1$H-NMR ($CDCl_3$) δ 0.52-0.68 (m, 4H), 1.42-1.49 (m, 1H), 1.51-1.60 (m, 2H), 1.73-1.98 (m, 8H), 2.15-2.27 (m, 3H), 3.00-3.08 (m, 1H), 3.68-3.82 (m, 5H), 4.16-4.25 (m, 1H), 7.00-7.08 (m, 1H)

Example 68

$^1$H-NMR ($CDCl_3$) δ 0.44-0.45 (m, 2H), 0.63-0.64 (m, 2H), 1.61-1.66 (m, 2H), 1.75-1.82 (m, 2H), 1.89-2.18 (m, 11H), 2.49-2.54 (m, 1H), 3.14-3.18 (m, 2H), 3.28 (s, 3H), 3.34-3.37 (m, 2H), 3.75-3.76 (m, 3H), 4.20-4.21 (m, 1H), 5.27-5.29 (m, 1H), 5.61-5.66 (m, 1H), 6.48 (s, 1H), 7.44-7.52 (m, 1H)

Example 69

$^1$H-NMR ($CDCl_3$) δ 0.45-0.46 (m, 2H), 0.60-0.63 (m, 2H), 1.61-1.64 (m, 2H), 1.89-2.07 (m, 8H), 2.18 (bs, 3H),

2.58-2.59 (m, 1H), 3.22-3.26 (m, 2H), 3.29 (s, 3H), 3.44-3.47 (m, 2H), 3.74 (s, 3H), 4.20-4.22 (m, 1H), 5.28 (bs, 1H), 5.63 (bs, 1H), 6.51 (s, 1H), 7.40-7.42 (m, 1H)

Example 70

$^1$H-NMR ($CDCl_3$) δ 0.38-0.41 (m, 2H), 0.54-0.58 (m, 2H), 1.68-1.82 (m, 10H), 1.91-1.94 (m, 2H), 2.14-2.23 (m, 4H), 2.90-2.95 (m, 1H), 3.23-3.27 (m, 2H), 3.30 (s, 3H), 3.36-3.39 (m, 2H), 3.65 (s, 3H), 4.19-4.21 (m, 1H), 7.03-7.05 (m, 1H)

Example 71

$^1$H-NMR ($CDCl_3$) δ 0.41-0.44 (m, 2H), 0.54-0.58 (m, 2H), 1.50-1.61 (m, 2H), 1.73-1.86 (m, 7H), 1.89-1.94 (m, 2H), 2.17-2.25 (m, 3H), 2.95-3.00 (m, 1H), 3.28 (s, 3H), 3.35-3.39 (m, 4H), 3.69 (s, 3H), 4.17-4.22 (m, 1H), 7.05-7.08 (m, 1H)

[Chemical Formula 77]

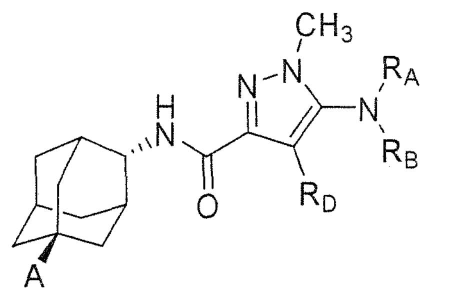

| Example No. | $R_A$ | $R_B$ | $R_D$ | A |
|---|---|---|---|---|
| 72 | 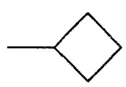 | 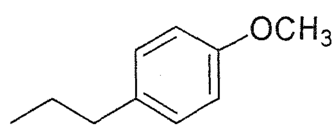 | H | $CONH_2$ |
| 73 | 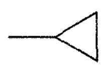 | 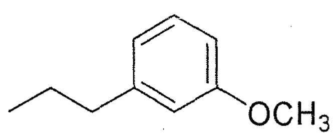 | Cl | OH |
| 74 | 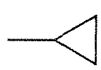 | 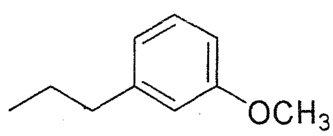 | Cl | $CONH_2$ |
| 75 | 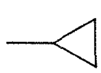 | 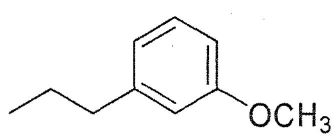 | H | $CONH_2$ |
| 76 | 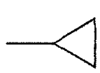 | 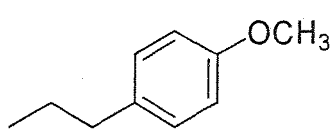 | Cl | OH |

(continued)

| Example No. | $R_A$ | $R_B$ | $R_D$ | A |
|---|---|---|---|---|
| 77 | 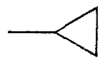 | 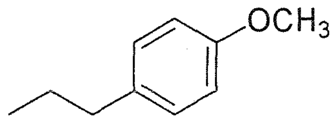 | Cl | $CONH_2$ |
| 78 | 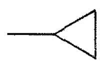 | 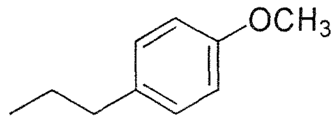 | H | $CONH_2$ |

Example 72

$^1$H-NMR ($CDCl_3$) δ 1.57-1.66 (m, 2H), 1.71-1.81 (m, 3H), 1.91-2.17 (m, 14H), 2.54-2.58 (m, 2H), 3.05-3.09 (m, 2H), 3.63-3.69 (m, 4H), 3.78 (s, 3H), 4.22-4.24 (m, 1H), 5.43 (bs, 1H), 5.67 (bs, 1H), 6.53 (s, 1H), 6.78-6.81 (m, 2H), 6.97-7.00 (m, 2H), 7.32-7.35 (m, 1H)

Example 73

$^1$H-NMR ($CDCl_3$) δ 0.39-0.43 (m, 2H), 0.55-0.59 (m, 2H), 1.52-1.56 (m, 2H), 1.78-1.94 (m, 9H), 2.19-2.23 (m, 3H), 2.71-2.75 (m, 2H), 2.92-2.96 (m, 1H), 3.43-3.48 (m, 5H), 3.78 (s, 3H), 4.19-4.21 (m. 1H), 6.65-6.66 (m, 1H), 6.70-6.75 (m, 2H), 7.04-7.06 (m, 1H), 7.16-7.20 (m, 1H)

Example 74

$^1$H-NMR ($CDCl_3$) δ 0.40-0.44 (m, 2H), 0.55-0.60 (m, 2H), 1.64-1.67 (m, 2H), 1.82-2.20 (m, 11H), 2.72-2.76 (m, 2H), 2.91-2.96 (m, 1H), 3.44-3.47 (m, 2H), 3.50 (s, 3H), 3.78 (s, 3H), 4.24-4.26 (m, 1H), 6.07 (bs,1H), 6.55 (bs, 1H), 6.66-6.68 (m, 1H), 6.71-6.75 (m, 2H), 7.14-7.23 (m, 2H)

Example 75

$^1$H-NMR ($CDCl_3$) δ 0.42-0.46 (m, 2H), 0.56-0.63 (m, 2H), 1.62-1.65 (m, 2H), 1.93-2.18 (m, 11H), 2.51-2.57 (m, 1H), 2.73-2.77 (m, 2H), 3.25-3.29 (m, 2H), 3.61 (s, 3H), 3.78 (s, 3H), 4.20-4.22 (m, 1H), 5.33 (bs, 1H), 5.65 (bs, 1H), 6.52 (s, 1H), 6.65-6.66 (m, 1H), 6.70-6.75 (m, 2H), 7.17-7.21 (m, 1H), 7.28-7.30 (m, 1H)Example 76

$^1$H-NMR ($CDCl_3$) δ 0.39-0.42 (m, 2H), 0.54-0.59 (m, 2H), 1.52-1.56 (m, 2H), 1.74-1.95 (m, 9H), 2.19-2.24 (m, 3H), 2.67-2.71 (m, 2H), 2.91-2.96 (m, 1H), 3.40-3.44 (m, 2H), 3.48 (s, 3H), 3.78 (s, 3H), 4.19-4.21 (m, 1H), 6.79-6.86 (m, 2H), 7.02-7.06 (m, 3H)

Example 77

$^1$H-NMR ($CDCl_3$) δ 0.39-0.43 (m, 2H), 0.55-0.59 (m, 2H), 1.64-1.67 (m, 2H), 1.89-2.20 (m, 11H), 2.67-2.71 (m, 2H), 2.90-2.95 (m, 1H), 3.40-3.44 (m, 2H), 3.50 (s, 3H), 3.78 (s, 3H), 4.24-4.25 (m, 1H), 5.88 (bs, 1H), 6.34 (bs, 1H), 6.79-6.83 (m, 2H), 7.02-7.05 (m, 2H), 7.13-7.15 (m, 1H)

Example 78

$^1$H-NMR ($CDCl_3$) δ 0.42-0.45 (m, 2H), 0.59-0.62 (m, 2H), 1.62-1.65 (m, 2H), 1.93-2.19 (m, 11H), 2.51-2.55 (m, 1H), 2.67-2.74 (m, 2H), 3.23-3.27 (m, 2H), 3.62 (s, 3H), 3.78 (s, 3H), 4.21-4.23 (m, 1H), 5.30 (bs, 1H), 5.63 (bs, 1H), 6.50 (s, 1H), 6.79-6.83 (m, 2H), 7.01-7.05 (m, 2H), 7.33-7.35 (m, 1H)

[Chemical Formula 78]

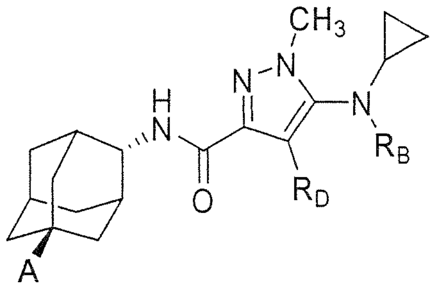

| Example No. | $R_B$ | $R_D$ | A |
|---|---|---|---|
| 79 | 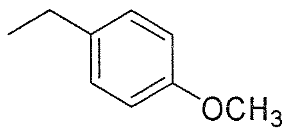 | Cl | $CONH_2$ |
| 80 | 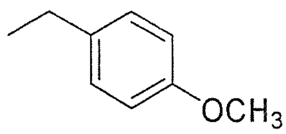 | Cl | OH |
| 81 | 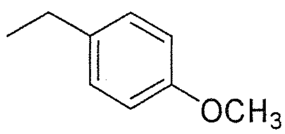 | H | $CONH_2$ |
| 82 | 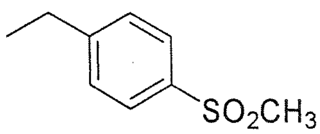 | Cl | OH |
| 83 | 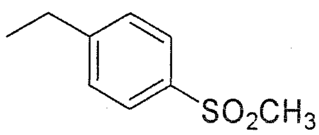 | Cl | $CONH_2$ |
| 84 | 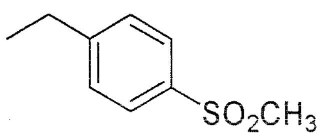 | H | OH |
| 85 | 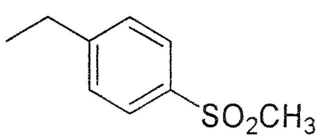 | H | $CONH_2$ |
| 86 | 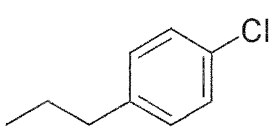 | H | $CONH_2$ |

Example 79

$^1$H-NMR ($CDCl_3$) δ 0.43-0.46 (m, 2H), 0.58-0.61 (m, 2H), 1.62-1.65 (m, 2H), 1.88-1.93 (m, 3H), 1.99-2.10 (m, 5H), 2.17-2.18 (m, 3H), 2.96-3.01 (m, 1H), 3.28 (s, 3H), 3.77 (s, 3H), 4.23 (m, 3H), 5.89 (bs, 1H), 6.35 (bs, 1H), 6.75-6.79 (m, 2H), 7.03-7.05 (m, 2H), 7.08-7.10 (m, 1H)

Example 80

$^1$H-NMR ($CDCl_3$) δ 0.43-0.46 (m, 2H), 0.56-0.61 (m, 2H), 1.51-1.54 (m, 2H), 1.72-1.83 (m, 7H), 1.91-1.94 (m, 2H), 2.17-2.22 (m, 3H), 2.97-3.02 (m, 1H), 3.26 (s, 3H), 3.76 (s, 3H), 4.17-4.19 (m, 1H), 4.23 (s, 2H), 6.74-6.78 (m, 2H), 6.99-7.01 (m, 1H), 7.02-7.06 (m, 2H)

Example 81

$^1$H-NMR ($CDCl_3$) δ 0.43-0.47 (m, 2H), 0.56-0.61 (m, 2H), 1.61-1.64 (m, 2H), 1.88-1.92 (m, 3H), 1.99-2.08 (m, 5H), 2.17-2.18 (m, 3H), 2.39-2.44 (m, 1H), 3.58 (s, 3H), 3.78 (s, 3H), 4.09 (s, 2H), 4.19-4.21 (m, 1H), 5.31 (bs, 1H), 5.64 (bs, 1H), 6.45 (s, 1H), 6.77-6.81 (m, 2H), 7.00-7.05 (m, 2H), 7.34-7.36 (m, 1H)

Example 82

$^1$H-NMR ($CDCl_3$) δ 0.47-0.51 (m, 2H), 0.60-0.65 (m, 2H), 1.52-1.56 (m, 3H), 1.73-1.83 (m, 6H), 1.91-1.94 (m, 2H), 2.18-2.22 (m, 3H), 2.97-3.02 (m, 1H), 3.04 (s, 3H), 3.35 (s, 3H), 4.16-4.18 (m, 1H), 4.40 (s, 2H), 6.99-7.00 (m, 1H), 7.38-7.39 (m, 2H), 7.84-7.86 (m, 2H)

Example 83

$^1$H-NMR ($CDCl_3$) δ 0.49-0.54 (m, 2H), 0.62-0.64 (m, 2H), 1.65-2.18 (m, 13H), 2.99-3.01 (m, 1H), 3.04 (s, 3H), 3.36 (s, 3H), 4.19-4.21 (m, 1H), 4.40 (s, 2H), 5.35 (bs, 1H), 5.69 (bs, 1H), 7.06-7.08 (m, 1H), 7.38-7.40 (m, 2H), 7.84-7.86 (m, 2H)

Example 84

$^1$H-NMR ($CDCl_3$) δ 0.46-0.50 (m, 2H), 0.58-0.62 (m, 2H), 1.52-1.55 (m, 2H), 1.78-1.94 (m, 9H), 2.19-2.21 (m, 3H), 2.42-2.45 (m, 1H), 3.05 (s, 3H), 3.58 (s, 3H), 4.15-4.17 (m, 1H), 4.21 (s, 2H), 6.45 (s, 1H), 7.09-7.11 (m, 1H), 7.34-7.36 (m, 2H), 7.84-7.86 (m, 2H)

Example 85

$^1$H-NMR ($CDCl_3$) δ 0.48-0.50 (m, 2H), 0.58-0.61 (m, 2H), 1.62-1.65 (m, 2H), 1.92-2.07 (m, 9H), 2.17 (bs, 2H), 2.43-2.46 (m, 1H), 3.05 (s, 3H), 3.59 (s, 3H), 4.18-4.20 (m, 1H), 4.22 (s, 2H), 5.66 (bs, 2H), 6.45 (s, 1H), 7.19-7.21 (m, 1H), 7.34-7.36 (m, 2H), 7.84-7.87 (m, 2H)

Example 86

$^1$H-NMR ($CDCl_3$) δ 0.40-0.44 (m, 2H), 0.56-0.61 (m, 2H), 1.62-1.65 (m, 2H), 1.93-2.18 (m, 11H), 2.48-2.53 (m, 1H), 2.71-2.75 (m, 2H), 3.21-3.26 (m, 2H), 3.58 (s, 3H), 4.20-4.22 (m, 1H), 5.44 (bs, 1H), 5.67 (bs, 1H), 6.51 (s, 1H), 7.03-7.05 (m, 2H), 7.22-7.24 (m, 3H)

[Chemical Formula 79]

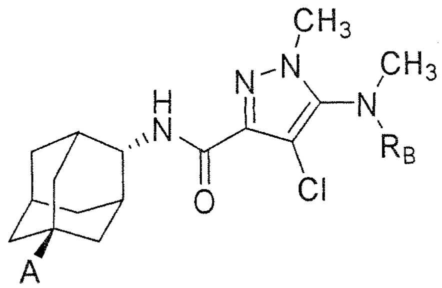

| Example No. | $R_B$ | A |
|---|---|---|
| 87 | 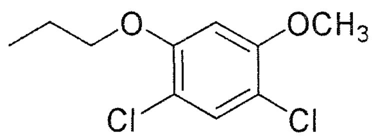 | $CONH_2$ |
| 88 | 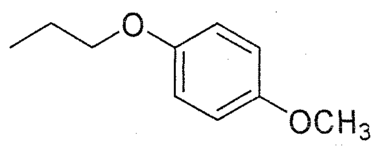 | $CONH_2$ |
| 89 | 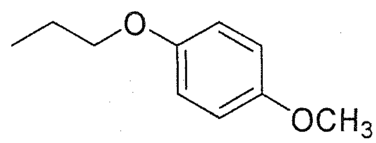 | OH |
| 90 | 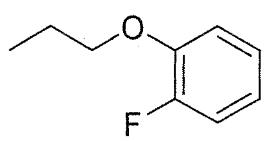 | OH |
| 91 | 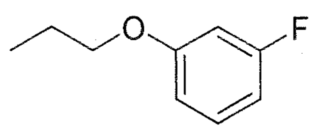 | OH |
| 92 | 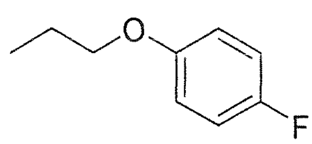 | OH |
| 93 | 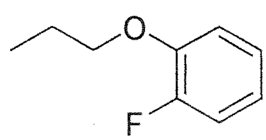 | $CONH_2$ |

(continued)

| Example No. | $R_B$ | A |
|---|---|---|
| 94 | 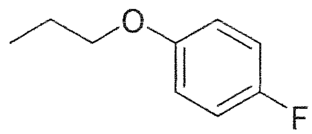 | $CONH_2$ |

Example 87

$^1$H-NMR ($CDCl_3$) δ 1.62-1.66 (m, 2H), 1.89-1.93 (m, 4H), 2.03-2.09 (m, 5H), 2.15-2.18 (m, 2H), 2.96 (s, 3H), 3.57-3.59 (m, 2H), 3.75 (s, 3H), 3.91 (s, 3H), 4.02-4.04 (m, 2H), 4.20 (m, 1H), 5.78 (bs, 1H), 6.20 (bs, 1H), 6.44 (s, 1H), 7.12 (m, 1H), 7.35 (s, 1H)

Example 88

$^1$H-NMR ($CDCl_3$) δ 1.62-1.66 (m, 2H), 1.89-1.93 (m, 4H), 2.03-2.10 (m, 5H), 2.17-2.19 (m, 2H), 2.93 (s, 3H), 3.47-3.50 (m, 2H), 3.73 (s, 3H), 3.76 (s, 3H), 3.91-3.93 (m, 2H), 4.21 (m, 1H), 5.94 (bs, 1H), 6.35 (bs, 1H), 6.76-6.83 (m, 4H), 7.13 (m, 1H)

Example 89

$^1$H-NMR ($CDCl_3$) δ 1.52-1.60 (m, 4H), 1.77-1.84 (m, 5H), 1.91-1.94 (m, 2H), 2.18-2.22 (m, 3H), 2.92 (s, 3H), 3.47-3.50 (m, 2H), 3.73 (s, 3H), 3.76 (s, 3H), 3.90-3.92 (m, 2H), 4.18 (m, 1H), 6.75-6.83 (m, 4H), 7.01 (m, 1H)

Example 90

$^1$H-NMR ($CDCl_3$) δ 1.51-1.54 (m, 2H), 1.77-1.84 (m, 6H), 1.90-1.93 (m, 2H), 2.17-2.22 (m, 4H), 2.92 (s, 3H), 3.52-3.55 (m, 2H), 3.76 (s, 3H), 4.01-4.04 (m, 2H), 4.18 (m, 1H), 6.87-6.92 (m, 2H), 7.02-7.09 (m, 3H)

Example 91

$^1$H-NMR ($CDCl_3$) δ 1.53-1.56 (m, 2H), 1.78-1.83 (m, 5H), 1.91-1.94 (m, 2H), 2.18-2.23 (m, 2H), 2.33-2.36 (m, 3H), 2.92 (s, 3H), 3.50-3.52 (m, 2H), 3.72 (s, 3H), 3.94-3.97 (m, 2H), 4.18 (m, 1H), 6.53-6.66 (m, 3H), 7.06 (m, 1H), 7.22 (m, 1H)

Example 92

$^1$H-NMR ($CDCl_3$) δ 1.52-1.55 (m, 2H), 1.77-1.84 (m, 7H), 1.91-1.94 (m, 2H), 2.18-2.22 (m, 3H), 2.92 (s, 3H), 3.48-3.51 (m, 2H), 3.72 (s, 3H), 3.91-3.95 (m, 2H), 4.39 (m, 1H), 6.75-6.78 (m, 2H), 6.93-6.98 (m, 3H)

Example 93

$^1$H-NMR ($CDCl_3$) δ 1.61-1.64 (m, 2H), 1.88-1.92 (m, 4H), 1.98-2.10 (m, 5H), 2.13-2.17 (m, 2H), 2.92 (s, 3H), 3.52-3.54 (m, 2H), 3.75 (s, 3H), 4.02-4.04 (m, 2H), 4.20 (m, 1H), 5.76 (bs, 1H), 6.12 (bs, 1H), 6.87-6.92 (m, 2H), 7.01-7.11 (m, 3H)

Example 94

$^1$H-NMR ($CDCl_3$) δ 1.62-1.66 (m, 2H), 1.85-1.93 (m, 4H), 2.02-2.10 (m, 5H), 2.15-2.18 (m, 2H), 2.93 (s, 3H), 3.47-3.51 (m, 2H), 3.78 (s, 3H), 3.92-3.98 (m, 2H), 4.21 (m, 1H), 5.87 (bs, 1H), 6.30 (bs, 1H), 6.75-6.78 (m, 2H), 6.93-6.98 (m, 2H), 7.12 (m, 1H).

[Chemical Formula 80]

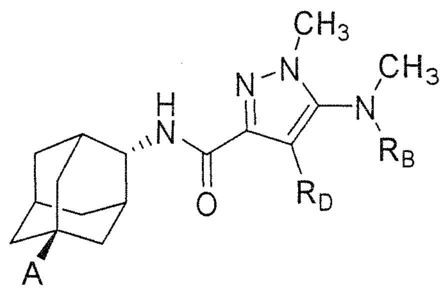

| Example No. | $R_B$ | $R_D$ | A |
|---|---|---|---|
| 95 | 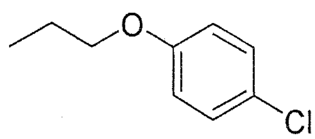 | H | $CONH_2$ |
| 96 | 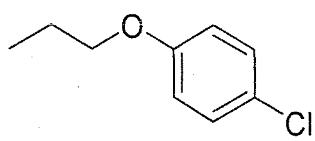 | Cl | OH |
| 97 | 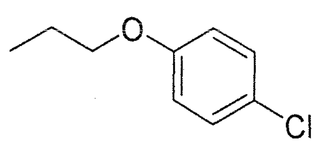 | Cl | $CONH_2$ |
| 98 | 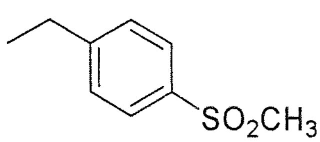 | Cl | OH |
| 99 | 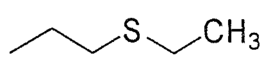 | H | $CONH_2$ |
| 100 | 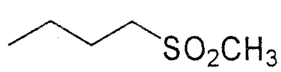 | H | $CONH_2$ |
| 101 | 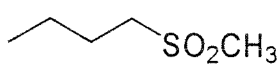 | Cl | $CONH_2$ |

Example 95

$^1$H-NMR ($CDCl_3$) δ 1.61-1.64 (m, 2H), 1.92-2.07 (m, 9H), 2.15-2.18 (m, 2H), 2.79 (s, 3H), 3.30 (t, J=4.0Hz, 2H), 3.77 (s, 3H), 4.01 (t, J=4.0Hz, 2H), 4.20 (m, 1H), 5.53 (bs, 1H), 5.77 (bs, 1H), 6.44 (s, 1H), 6.76-6.79 (m, 2H), 7.21-7.24 (m, 2H), 7.28 (m, 1H)

Example 96

$^1$H-NMR ($CDCl_3$) δ 1.46-1.56 (m, 2H), 1.60-1.71 (m, 2H), 1.75-1.89 (m, 6H), 1.91-1.95 (m, 2H), 2.15-2.26 (m, 2H), 2.92 (s, 3H), 3.49-3.51 (t, J=4.OHz, 2H), 3.71 (s, 3H), 3.92-3.94 (t, J=4.0Hz, 2H), 4.20 (m, 1H), 6.75 (d, J=8.0Hz, 2H), 7.02 (m, 1H), 7.21 (d, J=8.0Hz, 2H)

Example 97

$^1$H-NMR ($CDCl_3$) δ 1.62-1.65 (m, 2H), 1.89-1.99 (m, 4H), 2.02-2.07 (m, 5H), 2,15-2.18 (m, 2H), 2.92 (s, 3H), 3.47-3.52 (m, 2H), 3.71 (s, 3H), 3.92-3.95 (m, 2H), 4.20 (m, 1H), 5.57 (bs, 1H), 5.93 (bs, 1H), 6.74-6.76 (m, 2H), 7.09 (m, 1H), 7.20-7.23 (m, 2H)

Example 98

$^1$H-NMR ($CDCl_3$) δ 1.48-1.96 (m, 11H), 2.16-2.28 (m, 3H), 2.85 (s, 3H), 3.06 (s, 3H), 3.65 (s, 3H), 4.15-4.22 (m, 1H), 4.37 (s, 2H), 6.95-7.03 (m, 1H), 7.48-7.56 (m, 2H), 7.88-7.94 (m, 2H)

Example 99

$^1$H-NMR ($CDCl_3$) δ 1.21-1.29 (m, 3H), 1.59-1.68 (m, 2H), 1.90-2.11 (m, 9H), 2.16-2.22 (m, 2H), 2.49-2.58 (m, 2H), 2.60-2.68 (m, 2H), 2.70 (s, 3H), 3.05-3.14 (m, 2H), 3.77 (s, 3H), 4.15-4.24 (m, 1H), 5.34 (bs, 1H), 5.63 (bs, 1H), 6.39 (s, 1H), 7.15-7.23 (m, 1H)

Example 100

$^1$H-NMR ($CDCl_3$) δ 1.60-1.64 (m, 2H), 1.90-2.11 (m, 11H), 2.15-2.20 (m, 2H), 2.67 (s, 3H), 2.90 (s, 3H), 3.02-3.11 (m, 4H), 3.75 (s, 3H), 4.15-4.24 (m, 1H), 5.31 (bs, 1H), 5.62 (bs, 1H), 6.40 (s, 1H), 7.15-7.22 (m, 1H)

Example 101

$^1$H-NMR ($CDCl_3$) δ 1.58-1.68 (m, 2H), 1.85-2.11 (m, 11H), 2.15-2.22 (m, 2H), 2.83 (s, 3H), 2.91 (s, 3H), 3.02-3.11 (m, 2H), 3.26-3.35 (m, 2H), 3.75 (s, 3H), 4.16-4.25 (m, 1H), 5.27 (bs, 1H), 5.62 (bs, 1H), 7.05-7.14 (m, 1H)

Example 102

4-Chloro-5-[cyclopropyl(tetrahydro-2H-4-pyranyl)amino]-N-[(E)-5-hydroxy-2-adamantyl]-1-methyl-1H-pyrazole-3-carboxamide

[Chemical Formula 81]

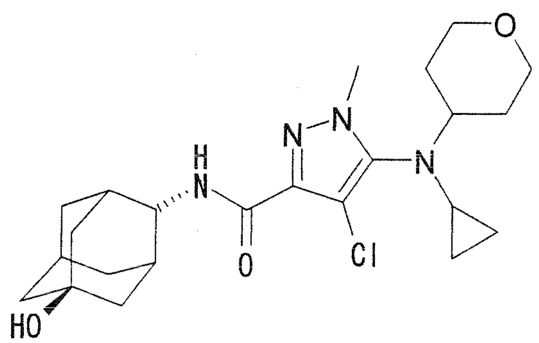

$^1$H-NMR ($CDCl_3$) δ 0.12-0.19 (m, 1H), 0.43-0.54 (m, 2H), 0.67-0.74 (m, 1H), 1.25-1.45 (m, 2H), 1.52-1.69 (m, 4H), 1.73-1.94 (m, 8H), 2.09-2.28 (m, 4H), 2.83-2.89 (m, 1H), 3.31-3.46 (m, 3H), 3.64 (s, 3H), 3.91-4.02 (m, 2H), 4.18-4.21 (m, 1H), 7.03-7.04 (m, 1H)

[Chemical Formula 82]

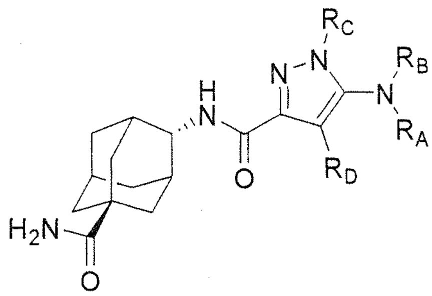

| Example No. | $R_A$ | $R_B$ | $R_C$ | $R_D$ |
|---|---|---|---|---|
| 103 | 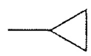 | 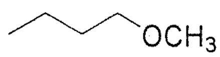 | $CH_3$ | Cl |
| 104 | 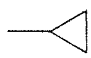 | 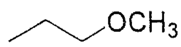 | CH2 | Cl |
| 105 | 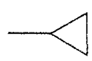 | 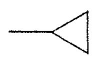 | $CH_3$ | H |
| 106 | 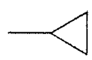 | 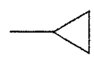 | $CH_3$ | Cl |
| 107 | 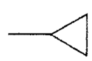 | 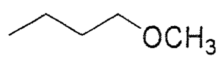 | $CH_3$ | F |
| 108 | 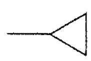 | 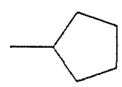 | $CH_3$ | Cl |
| 109 | 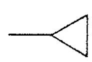 | 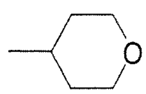 | $CH_3$ | Cl |
| 110 | $CH_3$ | $CH_2CH_3$ | $CH_3$ | F |
| 111 | $CH_3$ | $CH_2$-$CH_2$-$CF_3$ | $CH_3$ | Cl |
| 112 | $CH_3$ | $CH_2$-$CH_2$-$CF_3$ | $CH_3$ | F |

[Chemical Formula 83]

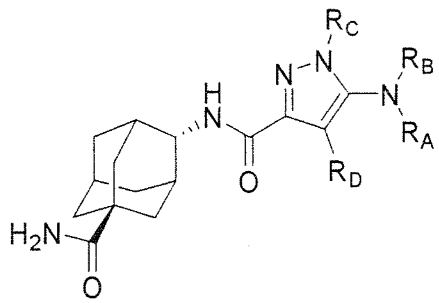

| Example No. | $R_A$ | $R_B$ | $R_C$ | $R_D$ |
|---|---|---|---|---|
| 113 | $CH_3$ | $CH_2$-$CF_2$-$CF_3$ | $CH_3$ | F |
| 114 | $CH_3$ | 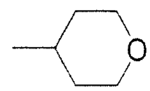 | $CH_3$ | H |
| 115 | $CH_3$ | 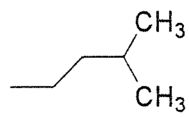 | $CH_3$ | F |
| 116 | $CH_3$ | 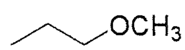 | $CH_3$ | F |
| 117 | $CH_3$ | $CH_2$-$CF_3$ | $CH_3$ | F |
| 118 | 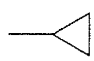 | 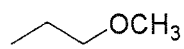 | $CH_3$ | F |
| 119 | $CH_3$ | $CH_2$-$CH_2$-$CH_3$ | $CH_2$ | F |
| 120 | $CH_3$ | $CH_2$-$CHF_2$ | $CH_3$ | F |
| 121 | 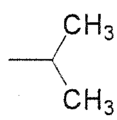 | 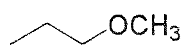 | $CH_3$ | F |
| 122 | $CH_2CH_3$ | 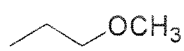 | $CH_3$ | F |

[Chemical Formula 84]

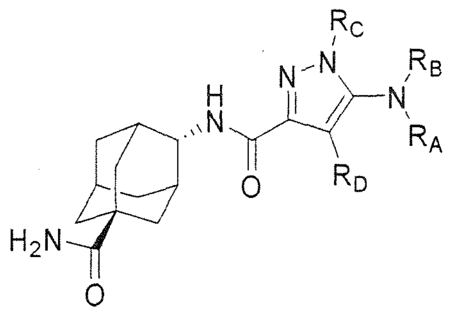

| Example No. | $R_A$ | $R_B$ | $R_C$ | $R_D$ |
|---|---|---|---|---|
| 123 | $CH_3$ | $CH_2$-$CHF_2$ | $CH_3$ | Cl |
| 124 | $CH_3$ | 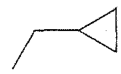 | $CH_3$ | F |

Example 103

$^1$H-NMR ($CDCl_3$) δ 0.36-0.39 (m, 2H), 0.52-0.56 (m, 2H), 1.60-1.63 (m, 2H), 1.66-1.73 (m, 2H), 1.88-2.16 (m, 11H), 2.87-2.92 (m, 1H), 3.21-3.25 (m, 2H), 3.28 (s, 3H), 3.35-3.38 (m, 2H), 3.64 (s, 3H), 4.18-4.20 (m, 1H), 5.94-6.02 (m, 2H), 7.09-7.11 (m, 1H)

Example 104

$^1$H-NMR ($CDCl_3$) δ 0.39-0.43 (m, 2H), 0.52-0.57 (m, 2H), 1.89-2.16 (m, 12H), 2.93-2.98 (m, 1H), 3.26 (s, 3H), 3.33-3.38 (m, 4H), 3.67 (s, 3H), 4.19-4.21 (m, 2H), 5.76-5.97 (m, 2H), 7.10-7.12 (m, 1H)

Example 105

$^1$H-NMR ($CDCl_3$) δ 0.42-0.46 (m, 4H), 0.55-0.60 (m, 4H), 1.61-1.64 (m, 2H), 1.92-2.16 (m, 11H), 2.53-2.58 (m, 2H), 3.68 (s, 3H), 4.18-4.20 (m, 1H), 5.44-5.68 (m, 2H), 6.42 (s, 1H), 7.24-7.28 (m, 1H)

Example 106

$^1$H-NMR ($CDCl_3$) δ 0.36-0.53 (m, 8H), 1.63-1.67 (m, 2H), 1.90-1.94 (m, 2H), 2.00-2.19 (m, 9H), 2.88-2.93 (m, 2H), 3.64 (s, 3H), 4.24-4.26 (m, 1H), 6.18 (bs, 1H), 6.74 (bs, 1H), 7.15-7.17 (m, 1H)

Example 107

$^1$H-NMR ($CDCl_3$) δ 0.39-0.43 (m, 2H), 0.55-0.59 (m, 2H), 1.63-1.66 (m, 2H), 1.71-2.11 (m, 10H), 2.14-2.18 (m, 3H), 2.73-2.75 (m, 1H), 3.14-3.18 (m, 2H), 3.30 (s, 3H), 3.37-3.40 (m, 2H), 3.63 (s, 3H), 4.21-4.25 (m, 1H), 5.67-5.74 (m, 1H), 6.13-6.18 (m, 1H), 6.92-6.94 (m, 1H)

Example 108

$^1$H-NMR ($CDCl_3$) δ 0.15-0.24 (m, 1H), 0.41-0.56 (m, 2H), 0.61-0.70 (m, 1H), 1.31-2.20 (m, 21H), 2.82-2.87 (m, 1H), 3.67 (s, 3H), 3.74-3.80 (m, 1H), 4.25-4.26 (m, 1H), 6.13-6.27 (m, 1H), 6.68-6.86 (m, 1H), 7.16-7.18 (m, 1H).

Example 109

$^1$H-NMR ($CDCl_3$) δ 0.13-0.20 (m, 1H), 0.45-0.55 (m, 2H), 0.67-0.75 (m, 1H), 1.51-1.71 (m, 6H), 1.73-2.20 (m, 11H), 2.84-2.89 (m, 1H), 3.32-3.45 (m, 3H), 3.68 (s, 3H), 3.92-4.03 (m, 2H), 4.23-4.25 (m, 1H), 6.11-6.27 (m, 1H), 6.57-6.70 (m, 1H), 7.15-7.17 (m, 1H)

Example 110

$^1$H-NMR ($CDCl_3$) δ 1.05-1.09 (m, 3H), 1.62-1.65 (m, 2H), 1.83-2.07 (m, 9H), 2.18 (brs, 2H), 2.78 (s, 3H), 3.00-3.05 (m, 2H), 3.69 (m, 3H), 4.19-4.23 (m, 1H), 5.23 (s, 1H), 5.62 (s, 1H), 6.90-6.93 (m, 1H)

Example 111

$^1$H-NMR ($CDCl_3$) δ 1.58-1.70 (m, 2H), 1.85-2.22 (m, 9H), 2.16-2.22 (m, 2H), 2.22-2.34 (m, 2H), 2.84 (s, 3H), 3.39-3.43 (m, 2H), 4.19-4.23 (m, 1H), 5.29 (s, 1H), 5.62 (s, 1H), 7.07-7.11 (m, 1H)

Example 112

$^1$H-NMR ($CDCl_3$) δ 1.55-1.67 (m, 2H), 1.83-2.10 (m, 12H), 2.11-2.19 (m, 2H), 2.20-2.36 (m, 2H), 2.77 (s, 3H), 3.24-3.29 (m, 2H), 3.67 (s, 3H), 4.16-4.23 (m, 1H), 5.42 (s, 1H), 5.80 (s, 1H), 6.87-6.95 (m, 1H)

Example 113

$^1$H-NMR ($CDCl_3$) δ 1.57-1.70 (m, 2H), 1.73-2.10 (m, 10H), 2.11-2.29 (m, 2H), 2.91 (s, 3H), 3.60-3.78 (m, 2H), 3.72 (s, 3H), 4.15-4.23 (m, 1H), 5.43 (s, 1H), 5.82 (s, 1H), 6.87-6.97 (m, 1H)

Example 114

$^1$H-NMR ($CDCl_3$) δ 1.55-1.78 (m, 6H), 1.93-2.07 (m, 9H), 2.17-2.18 (m, 2H), 2.62 (s, 3H), 2.87-2.95 (m, 1H), 3.31-3.37 (m, 2H), 3.75 (s, 3H), 3.97-4.00 (m, 2H), 4.20-4.21 (m, 1H), 5.26 (bs, 1H), 5.62 (bs, 1H), 6.48 (s, 1H), 7.22-7.24 (m, 1H)

Example 115

$^1$H-NMR (DMSO-$d_6$) δ 0.84-0.85 (m, 6H), 1.27-1.32 (m, 2H), 1.46-1.49 (m, 2H), 1.53-1.60 (m, 1H), 1.75-1.98 (m, 11H), 2.71 (s, 3H), 2.94-2.98 (m, 2H), 3.65 (s, 3H), 3.92-3.94 (m, 1H), 6.74 (s, 1H), 7.01 (s, 1H), 7.28-7.30 (m, 1H)

Example 116

$^1$H-NMR ($CDCl_3$) δ 1.62-1.66 (m, 2H), 1.89-2.08 (m, 10H), 2.18 (s, 2H), 2.84 (s, 3H), 3.17-3.19 (m, 2H), 3.33 (s, 3H), 3.42-3.44 (m, 2H), 3.72 (s, 3H), 4.21-4.25 (m, 1H), 5.43 (s, 1H), 5.75 (s, 1H), 6.92-6.94 (m, 1H)

Example 117

$^1$H-NMR ($CDCl_3$) δ 1.63-1.66 (m, 2H), 1.80-1.96 (m, 4H), 1.99-2.08 (m, 5H), 2.17-2.18 (m, 2H), 2.94 (s, 3H), 3.54-3.61 (m, 2H), 3.74 (s, 3H), 4.20-4.22 (m, 1H), 5.47 (bs, 1H), 5.85 (bs, 1H), 6.92-6.94 (m, 1H)

Example 118

$^1$H-NMR ($CDCl_3$) δ 0.41-0.45 (m, 2H), 0.54-0.59 (m, 2H), 1.62-1.66 (m, 2H), 1.90-1.93 (m, 4H), 1.99-2.07 (m, 5H), 2.18-2.19 (m, 2H), 2.79-2.83 (m, 1H), 3.25-3.28 (m, 2H), 3.29 (s, 3H), 3.40-3.43 (m, 2H), 3.64 (s, 3H), 4.22-4.24 (m, 1H), 5.23 (bs, 1H), 5.62 (bs, 1H), 6.92-6.94 (m, 1H)

Example 119

$^1$H-NMR ($CDCl_3$) δ 0.88-0.92 (m, 3H), 1.44-1.53 (m, 2H), 1.62-1.65 (m, 2H), 1.89-1.92 (m, 4H), 1.98-2.07 (m, 5H), 2.17-2.18 (m, 2H), 2.76 (s, 3H), 2.92-2.95 (m, 2H), 3.69 (s, 3H), 4.21-4.23 (m, 1H), 5.23 (bs, 1H), 5.62 (bs, 1H), 6.90-6.92 (m, 1H)

Example 120

$^1$H-NMR ($CDCl_3$) δ 1.62-1.66 (m, 2H), 1.88-1.92 (m, 4H), 1.98-2.07 (m, 5H), 2.17-2.18 (m, 2H), 2.89 (s, 3H), 3.33-3.47 (m, 2H), 3.73 (s, 3H), 4.20-4.22 (m, 1H), 5.19 (bs, 1H), 5.59 (bs, 1H), 5.69-5.99 (m, 1H), 6.91-6.93 (m, 1H)

Example 121

$^1$H-NMR ($CDCl_3$) δ 1.09-1.10 (m, 6H), 1.63-1.66 (m, 2H), 1.91-1.93 (m, 4H), 1.99-2.07 (m, 5H), 2.19 (bs, 2H), 3.21-3.28 (m, 8H), 3.71 (s, 3H), 4.22-4.24 (m, 1H), 5.23 (bs, 1H), 5.63 (bs, 1H), 6.94-6.96 (m, 1H)

Example 122

$^1$H-NMR ($CDCl_3$) δ 1.01-1.05 (m, 3H), 1.63-1.65 (m, 2H), 1.90-1.93 (m, 4H), 1.99-2.07 (m, 5H), 2.18 (bs, 2H), 3.07-3.13 (m, 2H), 3.19-3.23 (m, 2H), 3.29 (s, 3H), 3.35-3.38 (m, 2H), 3.71 (s, 3H), 4.22-4.23 (m, 1H), 5.32 (bs, 1H), 5.68 (bs, 1H), 6.93-6.95 (m, 1H)

Example 123

$^1$H-NMR ($CDCl_3$) δ 1.63-1.66 (m, 2H), 1.86-1.95 (m, 4H), 1.99-2.07 (m, 5H), 2.18 (bs, 2H), 2.92 (s, 3H), 3.43-3.50 (m, 2H), 3.77 (s, 3H), 4.20-4.22 (m, 1H), 5.22 (bs, 1H), 5.60 (bs, 1H), 5.67-5.97 (m, 1H), 7.08-7.10 (m, 1H)

Example 124

$^1$H-NMR ($CDCl_3$) δ 0.07-0.11 (m, 2H), 0.45-0.50 (m, 2H), 0.84-0.89 (m, 1H), 1.62-1.66 (m, 2H), 1.89-1.93 (m, 4H), 1.99-2.07 (m, 5H), 2.18 (bs, 2H), 2.81-2.83 (m, 5H), 3.72 (s, 3H), 4.22-4.23 (m, 1H), 5.24 (bs, 1H), 5.63 (bs, 1H), 6.91-6.93 (m, 1H)

[Chemical Formula 85]

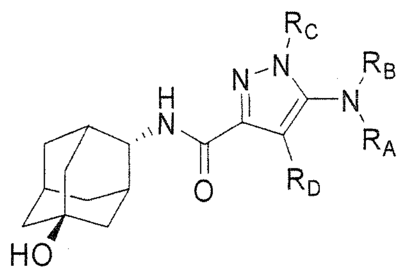

| Example No. | $R_A$ | $R_B$ | $R_C$ | $R_D$ |
|---|---|---|---|---|
| 125 | 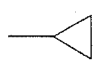 | 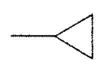 | $CH_3$ | Cl |
| 126 | 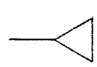 | 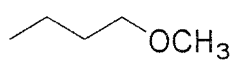 | $CH_3$ | F |
| 127 | $CH_3$ | 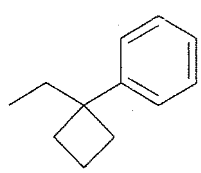 | $CH_3$ | Cl |
| 128 | $CH_3$ | 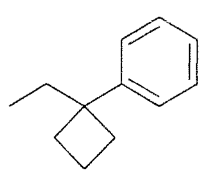 | $CH_3$ | F |

(continued)

| Example No. | $R_A$ | $R_B$ | $R_C$ | $R_D$ |
|---|---|---|---|---|
| 129 | $CH_3$ | 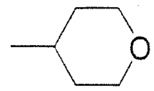 | $CH_3$ | Cl |
| 130 | $CH_3$ | 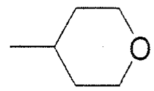 | $CH_3$ | F |
| 131 | $CH_3$ | 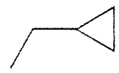 | $CH_3$ | F |

Example 125

$^1$H-NMR ($CDCl_3$) δ 1.00-1.12 (m, 6H), 1.58-1.69 (m, 2H), 1.89-2.12 (m, 9H), 2.13-2.23 (m, 2H), 3.02-3.12 (m, 2H), 3.13-3.31 (m, 6H), 3.75 (s, 3H), 4.18-4.25 (m, 1H), 5.35 (bs, 1H), 5.64 (bs, 1H), 6.52 (s, 1H), 7.18-7.27 (m, 1H)

Example 126

$^1$H-NMR ($CDCl_3$) δ 0.39-0.42 (m, 2H), 0.54-0.58 (m, 2H), 1.52-1.55 (m, 2H), 1.71-1.84 (m, 9H), 1.92-1.95 (m, 2H), 2.18-2.22 (m, 3H), 2.72-2.75 (m, 1H), 3.14-3.18 (m, 2H), 3.33 (s, 3H), 3.36-3.39 (m, 2H), 3.61 (s, 3H), 4.18-4.20 (m, 1H), 6.84-6.86 (m, 1H)

Example 127

$^1$H-NMR (DMSO-$d_6$) δ 1.36-1.39 (m, 2H), 1.60-1.78 (m, 9H), 1.93-2.00 (m, 4H), 2.10-2.16 (m, 2H), 2.22-2.29 (m, 2H), 2.40 (s, 3H), 3.33 (s, 3H), 3.48 (s, 2H), 3.84-3.86 (m, 1H), 4.45 (s, 1H), 7.13-7.16 (m, 1H), 7.21-7.31 (m, 5H)

Example 128

$^1$H-NMR (DMSO-$d_6$) δ 1.36-1.40 (m, 2H), 1.60-1.63 (m, 4H), 1.68-1.79 (m, 5H), 1.92-2.00 (m, 4H), 2.11-2.17 (m, 2H), 2.20-2.27 (m, 2H), 2.43 (s, 3H), 3.34 (s, 3H), 3.39 (s, 2H), 3.84-3.86 (m, 1H), 4.44 (bs, 1H), 7.04-7.06 (m, 1H), 7.09-7.13 (m, 1H), 7.16-7.18 (m, 2H), 7.24-7.28 (m, 2H)

Example 129

$^1$H-NMR ($CDCl_3$) δ 1.25-1.57 (m, 5H), 1.74-1.84 (m, 8H), 1.91-1.94 (m, 2H), 2.18-2.22 (m, 3H), 2.80 (s, 3H), 3.28-3.40 (m, 3H), 3.73 (s, 3H), 3.96-3.98 (m, 2H), 4.18-4.19 (m, 1H), 7.01-7.03 (m, 1H)

Example 130

$^1$H-NMR ($CDCl_3$) δ 1.53-1.56 (m, 4H), 1.78-1.84 (m, 9H), 1.92-1.95 (m, 2H), 2.18-2.22 (m, 3H), 2.75 (s, 3H), 3.04-.311 (m, 1H), 3.34-3.40 (m, 2H), 3.69 (s, 3H), 3.97-4.00 (m, 2H), 4.18-4.20 (m, 1H), 6.85-6.87 (m, 1H)

Example 131

$^1$H-NMR ($CDCl_3$) δ 0.07-0.11 (m, 2H), 0.44-0.49 (m, 2H), 0.83-0.90 (m, 1H), 1.39(bs,1H), 1.52-1.55 (m, 2H), 1.77-1.84 (m, 6H), 1.92-1.94 (m, 2H), 2.18-2.22 (m, 3H), 2.81-2.86 (m, 5H), 3.71 (s, 3H), 4.18-4.20 (m, 1H), 6.83-6.85 (m, 1H)

Example 132

4-Chloro-*N*-[(*E*)-5-hydroxy-2-adamantyl]-1-methyl-5-{methyl[(3*S*)-piperidin-3-yl]amino}-1*H*-pyrazole-3-carboxamide

[Chemical Formula 86]

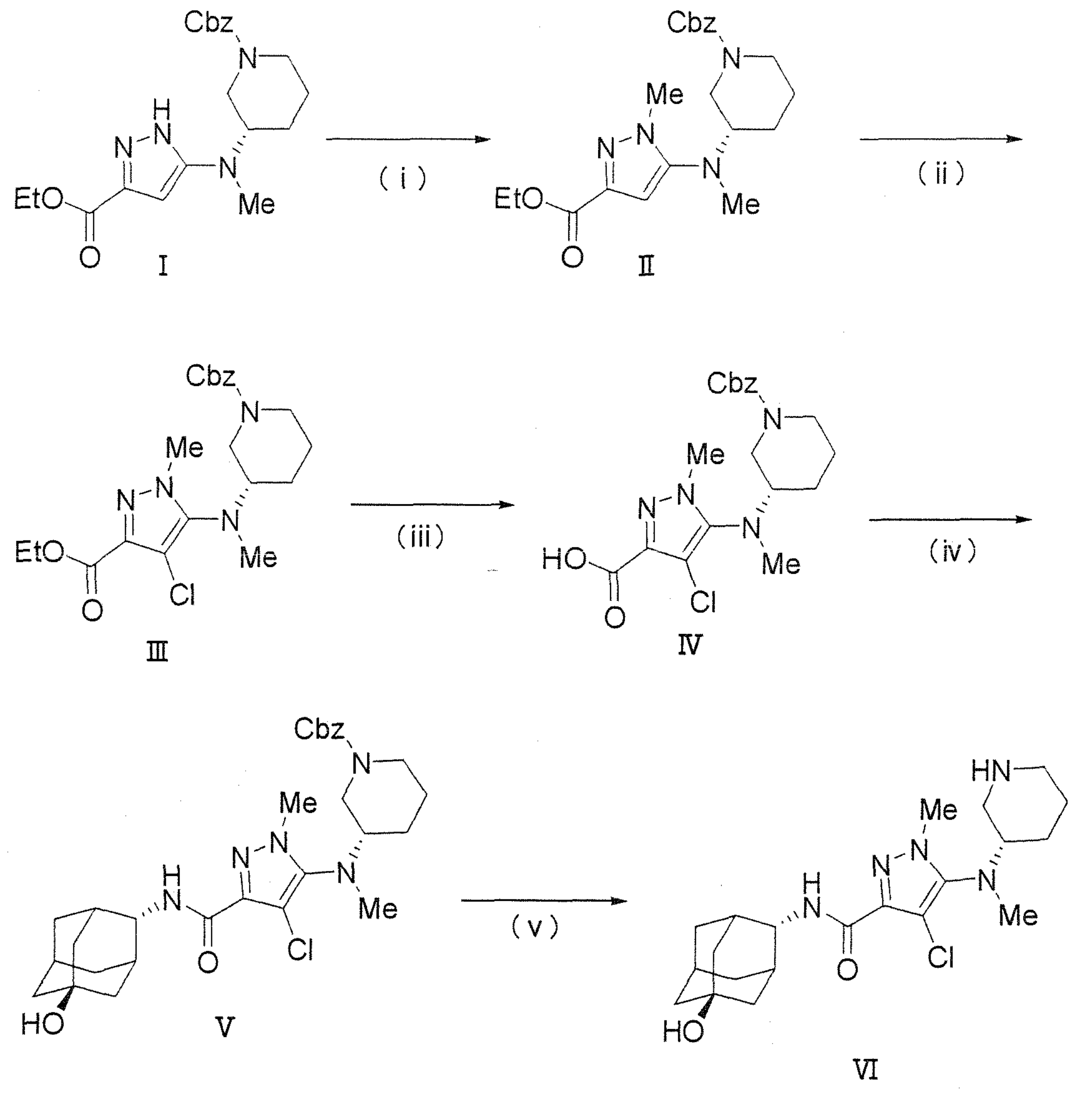


I II III IV V VI

Step (i):

To an ice-cooled solution of Compound (14.6 g) in THF (180 mL) was added sodium tertiary-butoxide (3.82 g), and the mixture was stirred for 1 hour. Then, thereto was slowly added methyl iodide (2.59 mL) at 0˚C, and the mixture was stirred for 6 hours. Then, thereto was added saturated aqueous ammonium chloride solution, and then the mixture was extracted with ethyl acetate. The organic layer was washed with brine, and then dried over magnesium sulfate and concentrated *in vacuo*. The residue was purified by silica gel chromatography (eluent: hexane/ethyl acetate = 1/1) to give Compound II (8.11 g).

Step (ii):

To a solution of Compound II (4.00 g) in DMF (30.0 mL) was added N-chlorosuccinimide (1.47 g), and the

mixture was stirred at 60˚C for 3 hours. Then, thereto was added water, and then the mixture was extracted with ethyl acetate. The organic layer was washed with water and brine, and then dried over magnesium sulfate and concentrated *in vacuo*. The residue was purified by silica gel chromatography (eluent: hexane/ethyl acetate = 1/1) to give Compound III (4.19 g).

Step (iii):

To a solution of Compound III (4.19 g) in ethanol (48.0 mL) was added 2N lithium hydroxide solution (14.4 mL), and the mixture was stirred at 50˚C for 2 hours. The reaction solution was concentrated *in vacuo*, and was adjusted to weak acidity by 1N hydrochloric acid and extracted with ethyl acetate. The organic layer was washed with brine, and then dried over magnesium sulfate and concentrated *in vacuo* to give Compound IV (3.61 g).

Step (iv):

To a solution of Compound IV (3.45 g) in DMF (80.0 mL) were added (*E*)-4-aminoadamantan-1-ol (1.70 g), WSCI•HCl (2.43 g), HOBt•$H_2O$ (1.72 g) and triethylamine (3.54 mL) at room temperature, and the mixture was stirred overnight. Then, thereto was added saturated aqueous ammonium chloride solution, and then the mixture was extracted with ethyl acetate. The organic layer was washed with water and brine, and then dried over magnesium sulfate and concentrated *in vacuo*. The residue was purified by silica gel chromatography (eluent: chloroform/methanol = 20/1) to give Compound V (3.81 g).

Step (v):

To a solution of Compound V (3.80 g) in methanol (35.0 mL) was added 10% palladium-carbon (380 mg), and the mixture was stirred at ambient temperature and normal pressure under hydrogen atmosphere for 6 hours. The reaction solution was filtered, and then the filtrate was concentrated *in vacuo* to give the titled Compound VI (3.00 g) as a white solid.
$^1$H-NMR ($CDCl_3$) δ 1.22-1.36 (m, 1H), 1.43-1.58 (m, 1H), 1.49-1.59 (m, 2H), 1.66-1.88 (m, 7H), 1.88-1.98 (m, 3H), 2.14-2.27 (m, 3H), 2.46-2.58 (m, 2H), 2.81 (s, 3H), 2.94-3.03 (m, 1H), 3.13-3.27 (m, 2H), 3.74 (s, 3H), 4.16-4.22 (m, 1H), 7.03 (d, J=7Hz, 1H)
Compounds of Examples 133-138 were prepared in the similar manner to Example 132.

Example 133

4-Chloro-*N*-[(*E*)-5hydroxy-2-adamantyl]-1-methyl-5-[methyl(piperidin-4-yl)amino]-1*H*-pyrazole-3-carboxamide

[Chemical Formula 87]

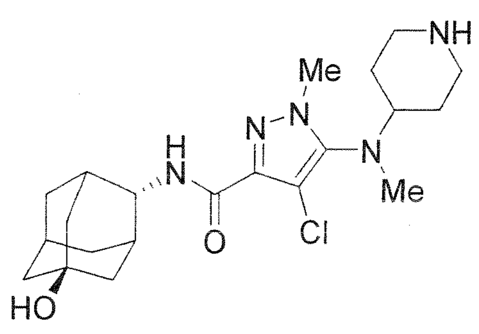

$^1$H-NMR ($CDCl_3$) δ 1.35-1.58 (m, 4H), 1.61-2.10 (m, 12H), 2.10-2.27 (m, 3H), 2.58-2.65 (m, 2H), 2.78 (s, 3H), 3.11-3.15 (m, 2H), 3.70 (s, 3H), 4.15-4.19 (m, 1H), 6.99-7.02 (m, 1H)

Example 134

4-Chloro-*N*-[(*E*)-5-hydroxy-2-adamantyl]-1-methyl-5-[methyl(piperidin-4-ylmethyl)amino]-1*H*-pyrazole-3-car-

boxamide

[Chemical Formula 88]

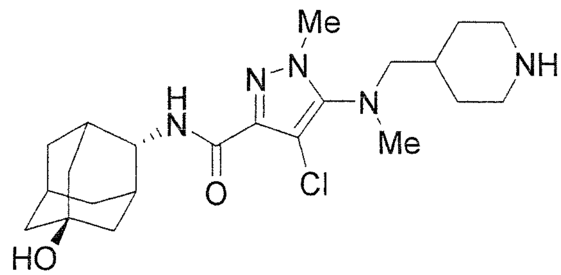

$^1$H-NMR ($CDCl_3$) δ 1.05-1.35 (m, 3H), 1.45-1.60 (m, 3H), 1.63-2.07 (m, 11H), 2.10-2.30 (m, 3H), 2.75 (m, 2H), 2.68-2.90 (s, 3H), 2.91-3.03 (m, 1H), 3.05-3.16 (m, 2H), 3.71 (s, 3H), 4.10-4.21 (m, 1H), 6.95-7.06 (m, 1H)

Example 135

4-Chloro-*N*-[(*E*)-5-hydroxy-2-adamantyl]-1-methyl-5-[methyl(2-piperidin-4-ylethyl)amino]-1*H*-pyrazole-3-carboxamide

[Chemical Formula 89]

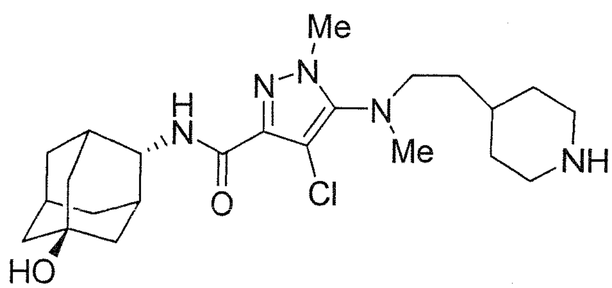

$^1$H-NMR ($CDCl_3$) δ 1.08-1.56 (m, 11H), 1.78-1.94 (m, 10H), 2.18-2.23 (m, 3H), 2.786-2.792 (m, 3H), 2.88-2.97 (m, 2H), 3.10-3.13 (m, 2H), 3.70-3.71 (m, 3H), 4.19-4.20 (m, 1H), 7.01-7.03 (m, 1H)

Example 136

4-Chloro-*N*-[(*E*)-5-hydroxy-2-adamantyl]-1-methyl-5-{methyl[(3*R*)-pyrrolidin-3-yl]amino}-1*H*-pyrazole-3-carboxamide

[Chemical Formula 90]

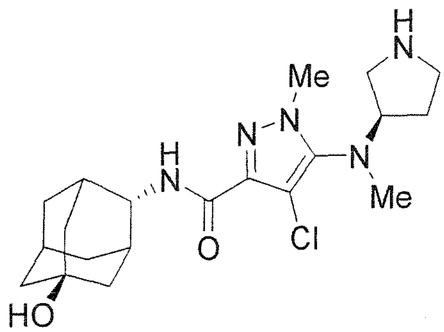

$^1$H-NMR ($CDCl_3$) δ 1.52-1.56 (m, 2H), 1.78-1.85 (m, 8H), 1.92-1.94 (m, 2H), 2.06-2.11 (m, 1H), 2.21 (bs, 3H), 2.63-2.75 (m, 4H), 3.03-3.28 (m, 4H), 3.74 (s, 3H), 3.92-3.95 (m, 1H), 4.15-4.17 (m, 1H), 6.87-6.89 (m, 1H)

Example 137

4-Chloro-*N*-[(*E*)-5-hydroxy-2-adamantyl]-1-methyl-5-{methyl[(3*S*)-pyrrolidin-3-yl]ammo}-1*H*-pyrazole-3-carboxamide

[Chemical Formula 91]

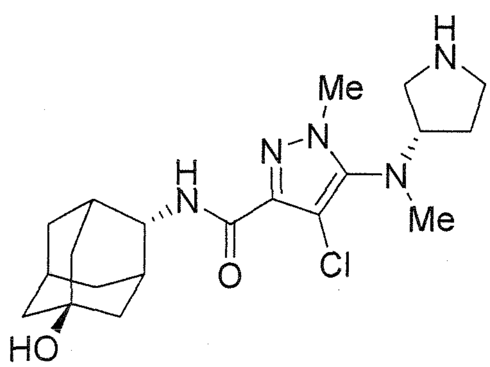

$^1$H-NMR ($CDCl_3$) δ 1.53-1.97 (m, 13H), 2.12-2.21 (m, 4H), 2.81 (s, 3H), 3.34-3.43 (m, 4H), 3.83 (s, 3H), 4.15-4.17 (m, 1H), 4.32-4.37 (m, 1H), 7.03-7.05 (m, 1H)

Example 138

4-Chloro-*N*-[(*E*)-5-hydroxy-2-adamantyl]-1-methyl-5-{methyl[(3*R*)-piperidin-3-yl]amino}-1*H*-pyrazole-3-carboxamide

[Chemical Formula 92]

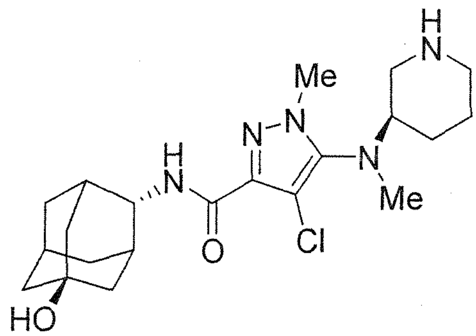

$^1$H-NMR ($CDCl_3$) δ 1.23-1.32 (m, 1H), 1.40-1.73 (m, 7H), 1.78-1.85 (m, 6H), 1.92-1.94 (m, 3H), 2.18-2.23 (m, 3H), 2.45-2.52 (m, 2H), 2.81 (s, 3H), 3.09-3.20 (m, 2H), 3.73 (s, 3H), 4.18-4.20 (m, 1H), 7.01-7.03 (m, 1H)

Example 139

4-Fluoro-*N*-[(*E*)-5-hydroxy-2-adamantyl]-1-methyl-5-{methyl[(3*S*)-piperidin-3-yl]amino}-1*H*-pyrazole-3-carboxamide

[Chemical Formula 93]

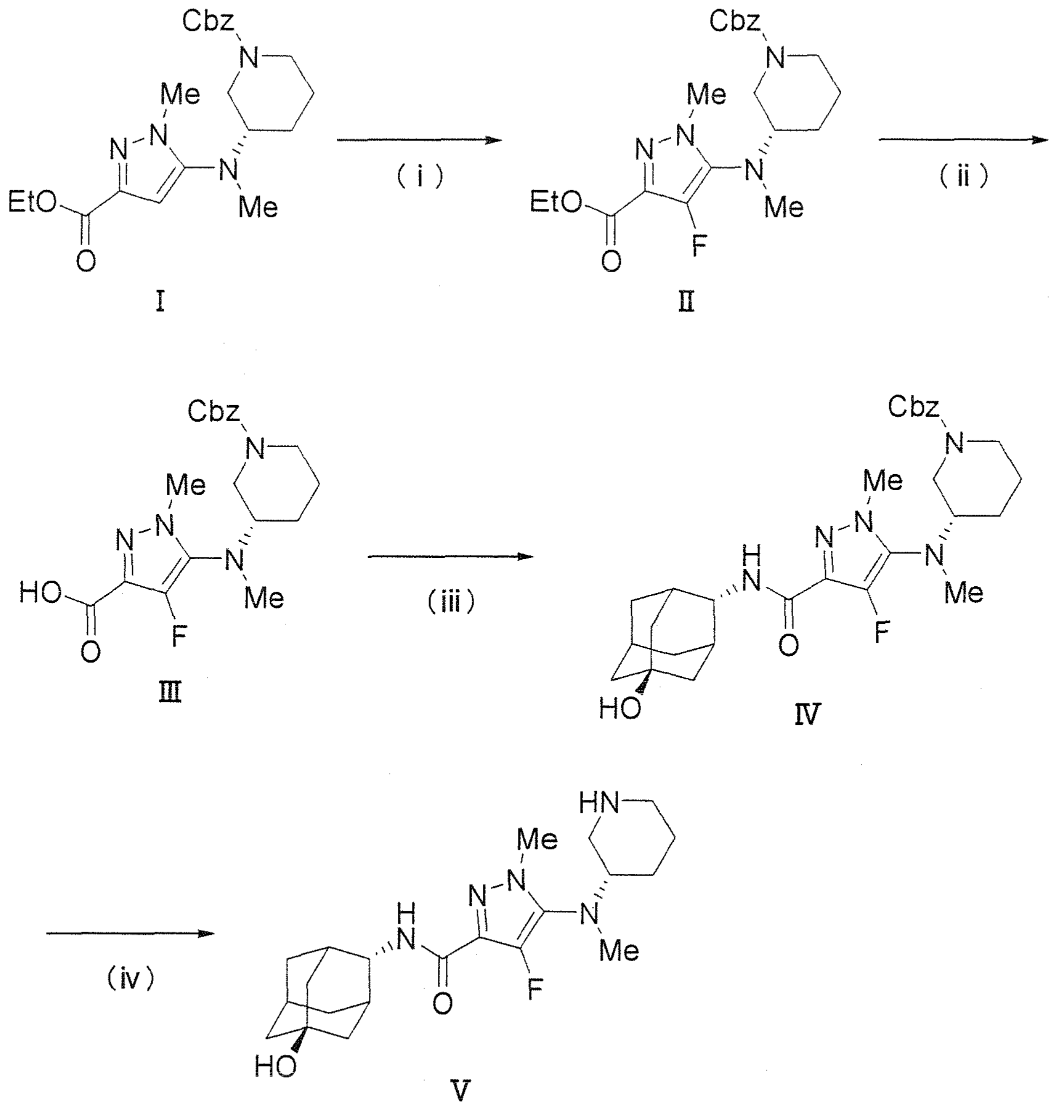

I II III IV V

Step (i):

To an ice-cooled solution of Compound I (4.14 g) in DMF (35.0 mL) was added dropwise an aqueous solution (35.0 mL) of 1-chloromethyl-4-fluoro-1,4-diazoniabicyclo[2.2.2.]octane-bis(tetrafluoroborate) (5.76 g), and the mixture was stirred at room temperature for 15 hours. Then, thereto was added water, and then the mixture was extracted with ethyl acetate. The organic layer was washed with water and brine, and then dried over magnesium sulfate and concentrated *in vacuo*. The residue was purified by silica gel chromatography (eluent: hexane/ethyl acetate = 1/1) to give Compound II (2.28 g).

Step (ii):

A mixed solution of Compound II (2.28 g), ethanol (27.3 mL) and 2N lithium hydroxide solution (8.15 mL) was stirred at 50°C for 2 hours. The reaction solution was concentrated *in vacuo*, and was adjusted to weak acidity by 1N hydrochloric acid and extracted with ethyl acetate. The organic layer was washed with brine, and then dried over magnesium sulfate and concentrated *in vacuo* to give Compound III (2.00 g).

Step (iii):

A mixed solution of Compound III (2.00 g), (*E*)-4-aminoadamantan-1-ol (1.03 g), WSCI•HCl (1.47 g), HOBt•$H_2O$ (1.04 g), triethylamine (2.14 mL) and DMF (45.0 mL) was stirred at room temperature overnight. Then, thereto was

added saturated aqueous ammonium chloride solution, and then the mixture was extracted with ethyl acetate. The organic layer was washed with water and brine, and then dried over magnesium sulfate and concentrated *in vacuo*. The residue was purified by silica gel chromatography (eluent: chloroform/methanol = 20/1) to give Compound IV (2.10 g).

Step (iv) :

Compound IV (2.10 g) was dissolved in methanol (30.0 mL), and then thereto was added 10% palladium-carbon (210 mg) and the mixture was stirred under hydrogen atmosphere for 3 hours. The reaction solution was filtered, and then the filtrate was concentrated *in vacuo* to give the titled Compound V (1.54 g) as a white solid.
$^1$H-NMR ($CDCl_3$) δ 1.24-1.35 (m, 1H), 1.42-1.55 (m, 1H), 1.50-1.58 (m, 2H), 1.70-1.86 (m, 7H), 1.90-2.00 (m, 3H), 2.18-2.22 (m, 3H), 2.42-2.53 (m, 2H), 2.76 (s, 3H), 2.92-3.02 (m, 2H), 3.20-3.26 (m, 1H), 3.68 (s, 3H), 4.15-4.25 (m, 1H), 6.85 (d, J=8Hz, 1H)
Compounds of Examples 140-146 were prepared in the similar manner to Example 139.

Example 140

4-Fluoro-*N*-[(*E*)-5-hydroxy-2-adamantyl-1-methyl-5-[methyl(piperidin-4-ylmethyl)amino]-1*H*-pyrazole-3-carboxamide

[Chemical Formula 94]

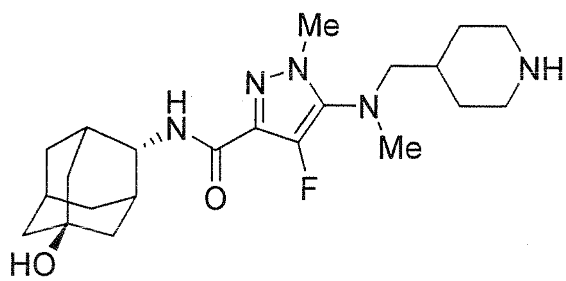

$^1$H-NMR ($CDCl_3$) δ 1.18-1.30 (m, 2H), 1.51-1.59 (m, 2H), 1.55-1.66 (m, 1H), 1.76-1.86 (m, 8H), 1.90-1.97 (m, 2H), 2.10-2.25 (m, 3H), 2.57-2.66 (m, 2H), 2.74 (s, 3H), 2.87-2.93 (m, 2H), 3.13-3.22 (m, 2H), 3.69 (s, 3H), 4.16-4.22 (m, 1H), 6.85 (d, J=8Hz, 1H)

Example 141

4-Fluoro-*N*-[(*E*)-5-hydroxy-2-adamantyl]-1-methyl-5-{methyl[(3*R*)-pyrrolidin-3-yl]amino}-1*H*-pyrazole-3-carboxamide

[Chemical Formula 95]

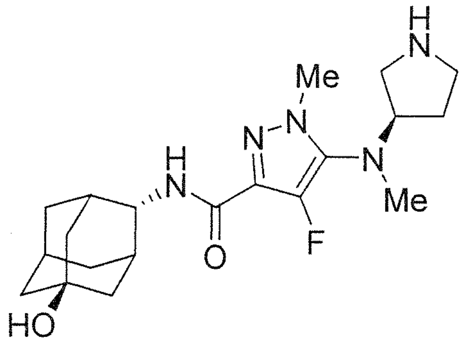

$^1H$-NMR ($CDCl_3$) δ 1.50-1.57 (m, 2H), 1.58-1.68 (m, 1H), 1.75-1.82 (m, 7H), 1.90-2.21 (m, 2H), 2.15-2.25 (m, 3H), 2.72 (s, 3H), 2.75-2.82 (m, 1H), 2.90-3.11 (m, 3H), 3.69 (s, 3H), 3.72-3.80 (m, 1H), 4.66-4.71 (m, 1H), 6.84 (d, J=8Hz, 1H)

Example 142

4-Fluoro-*N*-[(*E*)-5-hydroxy-2-adamantyl]-1-methyl-5-[methyl(piperidin-4-yl)amino]-1*H*-pyrazole-3-carboxamide

[Chemical Formula 96]

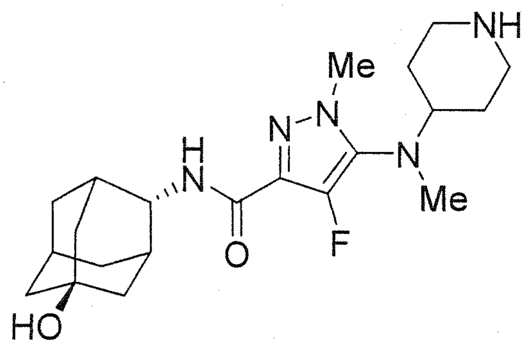

$^1H$-NMR ($CDCl_3$) δ 1.37-1.60 (m, 6H), 1.79-1.96 (m, 10H), 2.19-2.23 (m, 3H), 2.57-2.63 (m, 2H), 2.76 (s, 3H), 3.12-3.15 (m, 2H), 3.50 (s, 3H), 3.685-3.693 (m, 1H), 4.19-4.21 (m, 1H), 6.84-6.86 (m, 1H)

Example 143

4-Fluoro-*N*-[(*E*)-5-hydroxy-2-adamantyl]-1-methyl-5-{methyl[(3*S*)-pyrrolidin-3-yl]amino}-1*H*-pyrazole-3-carboxamide

[Chemical Formula 97]

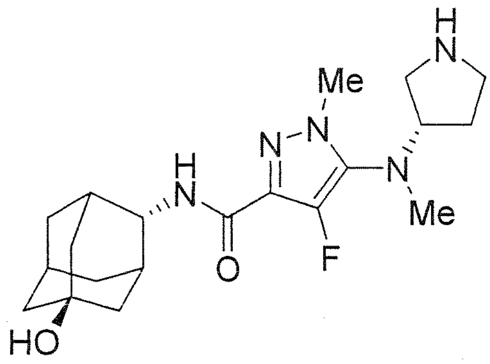

$^1$H-NMR ($CDCl_3$) δ 1.53-1.56 (m, 13H), 2.18-2.22 (m, 3H), 2.74-2.77 (m, 4H), 2.94-3.07 (m, 4H), 3.73-3.74 (m, 3H), 3.96-3.99 (m, 1H), 4.18-4.19 (m, 1H), 7.02-7.04 (m, 1H)

Example 144

4-Fluoro-*N*-[(*E*)-5-hydroxy-2-adamantyl]-1-methyl-5-{methyl[(3*R*)-piperidin-3-yl]amino}-1*H*-pyrazole-3-carboxamide

[Chemical Formula 98]

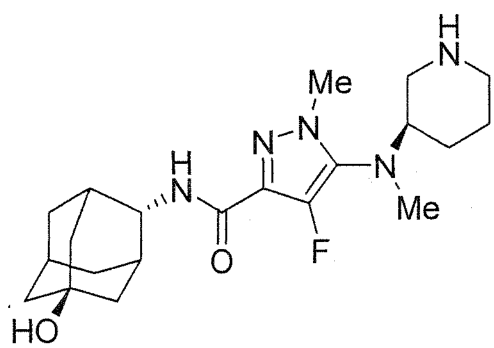

$^1$H-NMR ($CDCl_3$) δ 1.46-1.57 (m, 2H), 1.79-1.84 (m, 11H), 1.92-1.95 (m, 4H), 2.03-2.07 (m, 1H), 2.21(bs,3H), 2.74-2.81 (m, 4H), 3.28-3.31 (m, 1H), 3.48-3.51 (m, 1H), 3.74 (s, 3H), 4.16-4.17 (m, 1H), 6.89-6.91 (m, 1H)

Example 145

Benzyl 4-[[4-chloro-3-({[(*E*)-5-hydroxy-2-adamantyl]amino}carbonyl)-1-methyl-1*H*-pyrazol-5-yl]-(methyl)amino]piperidine-1-carboxylate

[Chemical Formula 99]

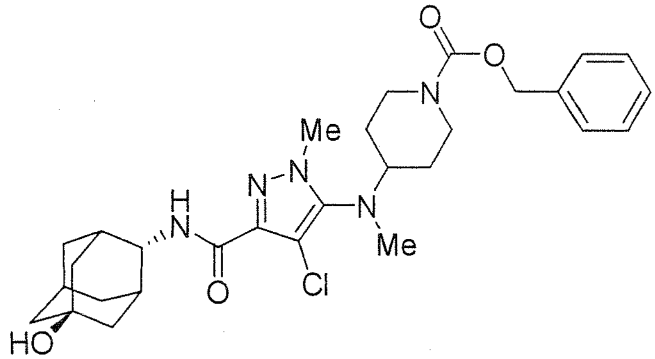

$^1$H-NMR ($CDCl_3$) δ 1.50-1.60 (m, 2H), 1.68-1.97 (m, 12H), 2.15-2.25 (m, 3H), 2.75-2.90 (m, 5H), 3.19-3.30 (m, 1H), 3.70 (s, 3H), 4.05-4.27 (m, 3H), 5.12 (s, 2H), 7.01-7.03 (m, 1H), 7.28-7.40 (m, 5H)

Example 146

Benzyl 4-{[[4-chloro-3-({[(*E*)-5-hydroxy-2-adamantyl]amino}carbonyl)-1-methyl-1*H*-pyrazol-5-yl](methyl)amino]methyl} piperidine-1-carboxylate

[Chemical Formula 100]

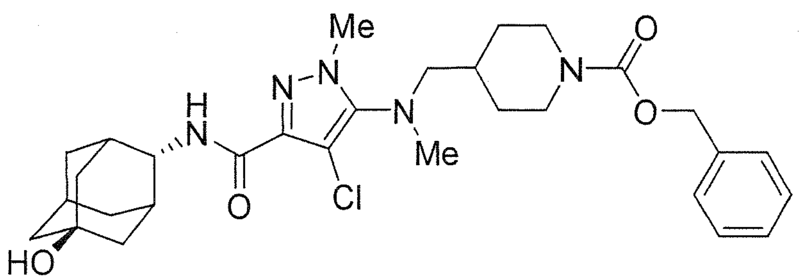

$^1$H-NMR ($CDCl_3$) δ 1.48-1.60 (m, 3H), 1.62-1.96 (m, 13H), 2.10-2.27 (m, 3H), 2.65-2.87 (m, 5H), 2.90-3.10 (m, 2H), 3.72 (s, 3H), 4.05-4.30 (m, 3H), 5.12 (s, 2H), 6.98-7.07 (m, 1H), 7.27-7.40 (m, 4H)

Example 147

4-Chloro-5-[[(3*S*)-1-(4-chlorobenzoyl)piperidin-3-yl](methyl)amino]-*N*-[(*E*)-5-hydroxy-2-adamantyl]-1-methyl-1*H*-pyrazole-3-carboxamide

[Chemical Formula 101]

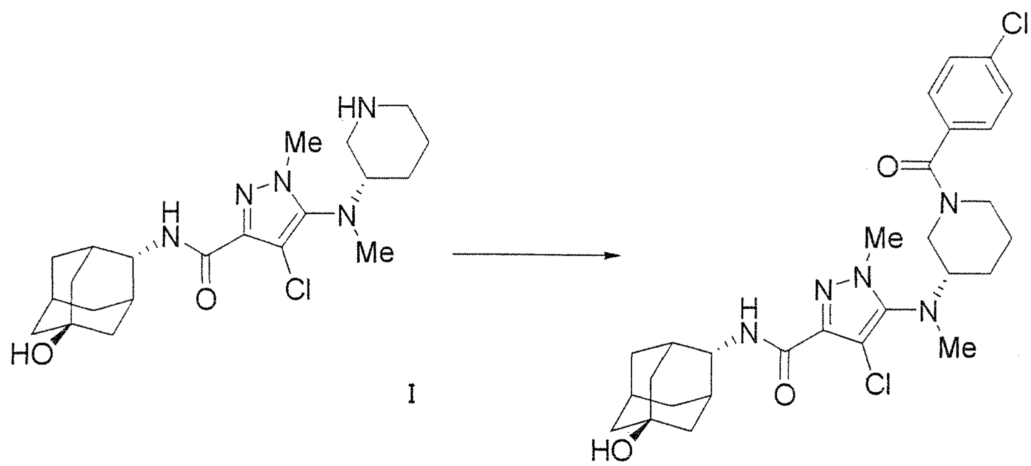

I

I I

Step (i):

To a solution of Compound I (20 mg) and triethylamine (20 μL) in THF (1 mL) was added 4-chlorobenzoyl chloride (10 mg), and the mixture was stirred at room temperature overnight. The reaction was quenched by methanol, and then filtered. The filtrate was concentrated *in vacuo*, and the residue was purified by a reverse phase HPLC (gradient condition 10%-) to give the titled Compound II (18.3 mg).

$^1$H-NMR ($CDCl_3$) δ 1.36-1.42 (m, 2H), 1.52-1.54 (m, 3H), 1.78-1.84 (m, 7H), 1.92-1.94 (m, 3H), 2.19-2.24 (m, 3H), 2.64-3.03 (m, 4H), 3.25-3.29 (m, 1H), 3.62-3.75 (m, 6H), 4.18-4.20 (m, 1H), 7.00-7.01 (m, 1H), 7.29-7.39 (m, 4H)

Example 148

4-Chloro-5-[{(3*S*)-1-[(3-fluorophenyl)sulfonyl]piperidin-3-yl}(methyl)amino]-*N*-[(*E*)-5-hydroxy-2-adamantyl]-1-methyl-1H-pyrazole-3-carboxamide

[Chemical Formula 102]

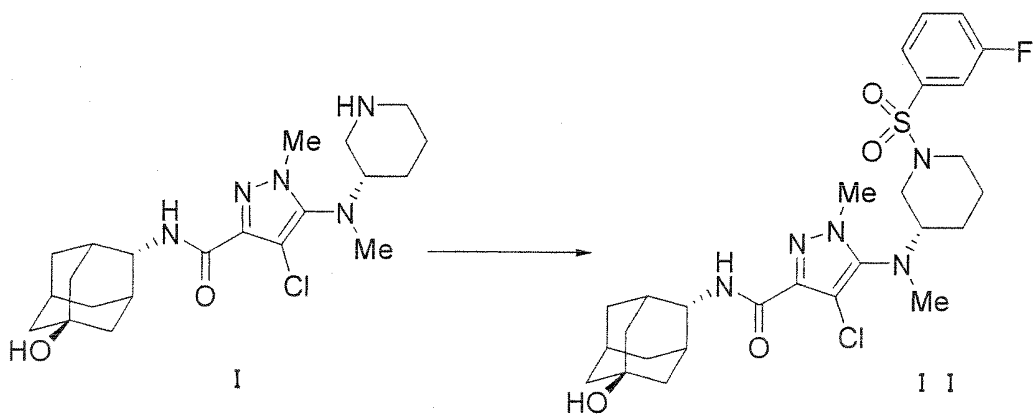

I

I I

To a solution of Compound I (20 mg) and triethylamine (20 μL) in THF (1 mL) was added 3-fluorobenzene sulfonyl chloride (11 mg), and the mixture was stirred at room temperature overnight. The reaction was quenched by methanol, and then filtered. The filtrate was concentrated *in vacuo*, and the residue was purified by a reverse phase HPLC (gradient condition 10%-) to give the titled Compound II (19.1 mg).

$^1$H-NMR ($CDCl_3$) δ 1.27-1.38 (m, 2H), 1.52-1.53 (m, 2H), 1.67-1.85 (m, 9H), 1.91-1.94 (m, 2H), 2.18-2.23 (m, 3H), 2.62-2.72 (m, 2H), 2.82 (s, 3H), 3.28-3.55 (m, 3H), 3.75 (s, 3H), 4.17-4.19 (m, 1H), 7.03-7.05 (m, 1H), 7.28-7.34 (m, 1H), 7.45-7.47 (m, 1H), 7.52-7.57 (m, 2H)

Example 149

4-[[4-Chloro-3-({[(*E*)-5-hydroxy-2-adamantyl]amino}carbonyl)-1-methyl-1H-pyrazol-5-yl](methyl)amino]-*N*-(2-methoxyphenyl)piperidine-1-carboxamide

[Chemical Formula 103]

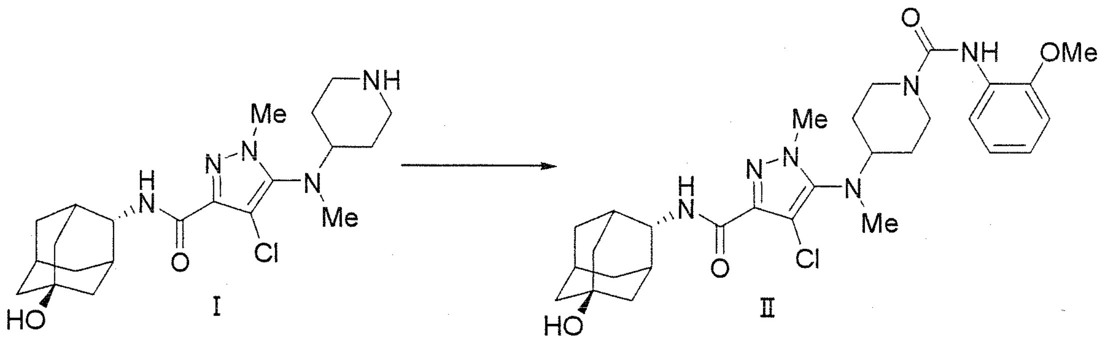

I

II

To an ice-cooled solution of Compound I (20 mg) and triethylamine (20 μL) in THF (3 mL) was added 2-methoxyphenyl isocyanate (9 μL), and the mixture was stirred at room temperature for 2 hours and concentrated *in vacuo*. Then, the residue was purified by silica gel column chromatography (chloroform/methanol = 20/1) and preparative thin-layer chromatography (ethyl acetate) to give the titled Compound II (18 mg).
$^1$H-NMR ($CDCl_3$) δ 1.17-1.60 (m, 5H), 1.66-1.96 (m, 11H), 2.08-2.20 (m, 3H), 2.75 (s, 3H), 2.83-2.91 (m, 2H), 3.20-3.28 (m, 1H), 3.65 (s, 3H), 3.81 (s, 3H), 3.94-4.20 (m, 3H), 6.80-6.95 (m, 4H), 8.00-8.10 (m, 1H)

Compounds of Examples 150-160 were prepared in the similar manner.

[Chemical Formula 104]

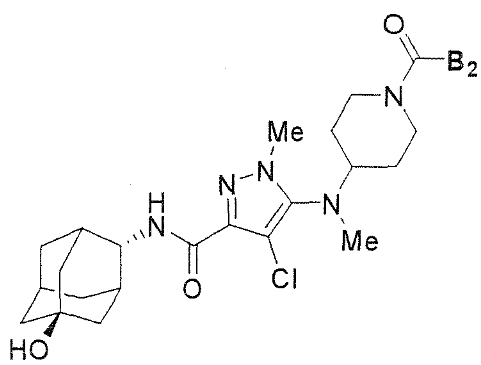

| Example No. | $B_2$ | Example No. | $B_2$ |
|---|---|---|---|
| 150 | 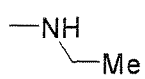 | 156 | 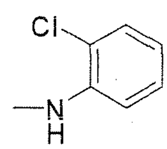 |

(continued)

| Example No. | **$B_2$** | Example No. | **$B_2$** |
|---|---|---|---|
| 151 | 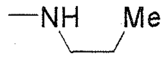 | 157 | 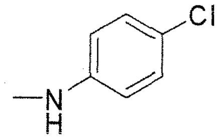 |
| 152 | 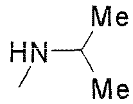 | 158 | 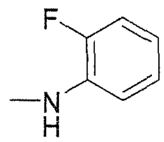 |
| 153 | 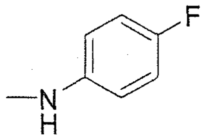 | 159 | 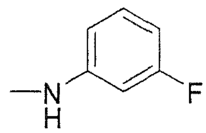 |
| 154 | 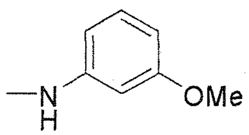 | 160 | 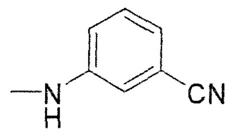 |
| 155 | 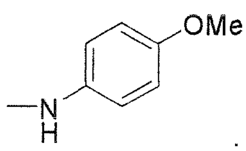 | | |

Example 150

$^1$H-NMR ($CDCl_3$) δ 1.05-1.18 (m, 3H), 1.25-1.45 (m, 3H), 1.45-1.60 (m, 3H), 1.70-1.96 (m, 12H), 2.72-2.80 (m, 5H), 3.17-3.28 (m, 3H), 3.68 (s, 3H), 3.87-3.91 (s, 2H), 4.15-4.17 (m, 1H), 4.38 (s, 1H), 6.99-7.02 (m, 1H)

Example 151

$^1$H-NMR ($CDCl_3$) δ 0.86-0.91 (m, 3H), 1.27-1.58 (m, 5H), 1.60-2.08 (m, 12H), 2.10-2.30 (m, 3H), 2.72-2.85 (m, 5H), 3.10-3.28 (m, 3H), 3.68 (s, 3H), 3.87-3.91 (s, 2H), 4.15-4.18 (m, 1H), 4.46 (s, 1H), 6.99-7.02 (m, 1H)

Example 152

$^1$H-NMR ($CDCl_3$) δ 1.00-1.20 (m, 6H), 1.25-1.45 (m, 2H), 1.45-1.96 (m, 13H), 2.10-2.27 (m, 3H), 2.78-2.90 (m, 5H), 3.12-3.27 (m, 1H), 3.68 (s, 3H), 3.80-4.28 (m, 3H), 4.10-4.28 (m, 1H), 6.99-7.02 (m, 1H)

Example 153

$^1$H-NMR ($CDCl_3$) δ 1.35-1.64 (m, 5H), 1.78-1.93 (m, 10H), 2.19-2.23 (m, 3H), 2.82 (s, 3H), 2.87-3.00 (m, 2H), 3.25-3.36 (m, 1H), 3.73 (s, 3H), 4.04-4.07 (m, 2H), 4.15-4.23 (m, 1H), 6.37 (s, 1H), 6.96-7.05 (m, 3H), 7.28-7.34 (m, 2H)

Example 154

$^1$H-NMR ($CDCl_3$) δ 1.17-1.60 (m, 5H), 1.70-2.10 (m, 11H), 2.14-2.30 (m, 3H), 2.80 (s, 3H), 2.86-3.10 (m, 2H), 3.25-3.40 (m, 1H), 3.71 (s, 3H), 3.78 (s, 3H), 3.92-4.11 (m, 2H), 4.12-4.22 (m, 1H), 6.50-6.70 (m, 1H), 6.77-6.85 (m, 1H), 6.98-7.08 (m, 1H), 7.10-7.20 (m, 2H)

Example 155

$^1H$-NMR ($CDCl_3$) δ 1.40-1.58 (m, 5H), 1.72-1.95 (m, 11H), 2.14-2.27 (m, 3H), 2.82 (s, 3H), 2.88-2.94 (m, 2H), 3.25-3.35 (m, 1H), 3.72 (s, 3H), 3.78 (s, 3H), 4.03-4.06 (m, 2H), 4.15-4.22 (m, 1H), 6.83-6.85 (m, 2H), 7.02-7.04 (m, 1H), 7.22-7.24 (m, 2H)

Example 156

$^1H$-NMR ($CDCl_3$) δ 1.40-1.60 (m, 5H), 1.70-1.84 (m, 7H), 1.85-2.00 (m, 4H), 2.12-2.23 (m, 3H), 2.81 (s, 3H), 2.93-3.00 (m, 2H), 3.29-3.38 (m, 1H), 3.71 (s, 3H), 4.06-4.09 (m, 2H), 4.13-4.20 (m, 1H), 6.91-6.96 (m, 1H), 6.98-7.05 (m, 1H), 7.21-7.27 (m, 1H), 7.30-7.32 (m, 1H), 8.14-8.16 (m, 1H)

Example 157

$^1H$-NMR ($CDCl_3$) δ 1.35-1.58 (m, 5H), 1.70-1.98 (m, 10H), 2.15-2.27 (m, 3H), 2.82 (s, 3H), 2.88-2.96 (m, 2H), 3.25-3.36 (m, 1H), 3.72 (s, 3H), 4.02-4.09 (m, 2H), 4.15-4.22 (m, 1H), 6.38 (s, 1H), 7.00-7.09 (m, 1H), 7.20-7.35 (m, 4H)

Example 158

$^1H$-NMR ($CDCl_3$) δ 1.30-1.60 (m, 5H), 1.65-2.00 (m, 10H), 2.09-2.23 (m, 3H), 2.75 (s, 3H), 2.85-2.93 (m, 2H), 3.23-3.30 (m, 1H), 3.66 (s, 3H), 3.98-4.13 (m, 3H), 6.54-6.56 (m, 1H), 6.86-7.05 (m, 4H), 7.96-8.02 (m, 1H)

Example 159

$^1H$-NMR ($CDCl_3$) δ 1.34-1.69 (m, 5H), 1.70-2.05 (m, 10H), 2.11-2.28 (m, 3H), 2.80 (s, 3H), 2.88-2.96 (m, 2H), 3.25-3.38 (m, 1H), 3.70 (s, 3H), 4.01-4.20 (m, 3H), 6.43-6.49 (m, 1H), 6.68-6.75 (m, 1H), 6.95-7.03 (m, 2H), 7.15-7.35 (m, 1H)

Example 160

$^1H$-NMR ($CDCl_3$) δ 1.27-1.69 (m, 5H), 1.72-1.97 (m, 10H), 2.15-2.25 (m, 3H), 2.83 (s, 3H), 2.92-3.02 (m, 2H), 3.30-3.38 (m, 1H), 3.73 (s, 3H), 4.03-4.20 (m, 3H), 7.00-7.05 (m, 1H), 7.29-7.32 (m, 1H), 7.35-7.39 (m, 1H), 7.52-7.57 (m, 1H), 7.73-7.76 (m, 1H)

Example 161

4-[[4-Chloro-3-({[(*E*)-5-hydroxy-2-adamantyl]amino}carbonyl)-1-methyl-1*H*-pyrazol-5-yl](methyl)amino]-*N*-(2,2,2-trifluoroethyl)piperidine-1-carboxamide

[Chemical Formula 105]

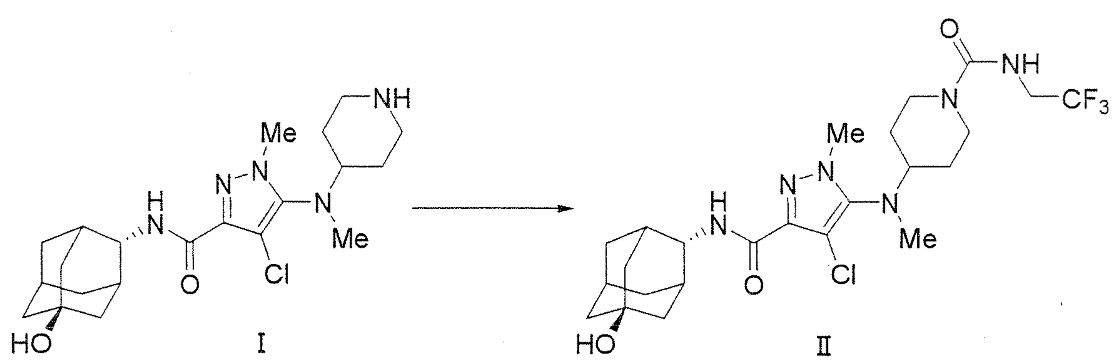

I II

To an ice-cooled solution of 1,1,1-trifluoroethylamine (4 μL) in THF (3 mL) was added chloro 4-nitrophenyl formate (10 mg), and the mixture was stirred at room temperature for 2 hours. The reaction solution was ice-cooled again, and thereto was added Compound I (20 mg) and the mixture was stirred for 2 hours. Then, thereto was added water, and the mixture was extracted with ethyl acetate. The organic layer was dried over sodium sulfate and concentrated *in vacuo*. The residue was purified by silica gel chromatography (chloroform/methanol = 20/1) to give the titled Compound II (12 mg). $^1$H-NMR ($CDCl_3$) δ 1.287-1.45 (m, 2H), 1.46-1.58 (m, 2H), 1.72-1.97 (m, 10H), 2.10-2.35 (m, 3H), 2.78-2.98 (m, 5H), 3.20-3.33 (m, 1H), 3.69 (s, 3H), 3.83-4.05 (m, 4H), 4.11-4.22 (m, 1H), 4.85-4.89 (m, 1H), 7.00-7.03 (m, 1H)

Compounds of Examples 162-164 were prepared in the similar manner.

[Chemical Formula 106]

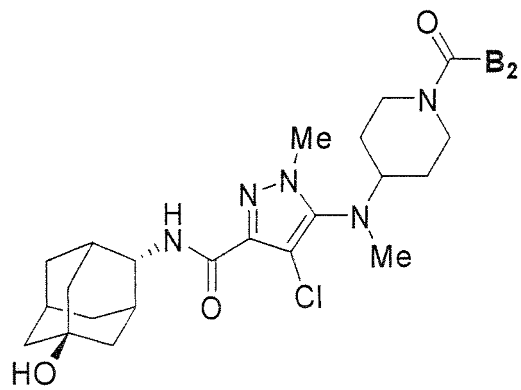

| Example No. | $B_2$ |
| --- | --- |
| **1 | 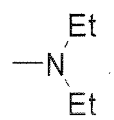 |
| **2 | 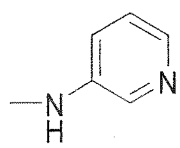 |
| **3 | 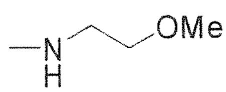 |

Example 162

$^1$H-NMR ($CDCl_3$) δ 1.00-1.18 (m, 6H), 1.35-1.70 (m, 2H), 1.72-2.02 (m, 10H), 2.15-2.30 (m, 3H), 2.68-2.90 (m, 6H), 3.00-3.10 (m, 1H), 3.11-3.30 (m, 6H), 3.53-3.70 (m, 2H), 3.72 (s, 3H), 4.15-4.25 (m, 1H), 7.01-7.05 (m, 1H)

Example 163

$^1$H-NMR ($CDCl_3$) δ 1.40-1.63 (m, 6H), 1.78-1.97 (m, 9H), 2.15-2.27 (m, 3H), 2.83 (s, 3H), 2.90-3.03 (m, 2H), 3.27-3.38 (m, 1H), 3.73 (s, 3H), 4.09-4.18 (m, 3H), 6.66 (s, 1H), 7.04-7.06 (m, 1H), 7.22-7.25 (m, 1H), 7.96-7.99 (m, 1H), 8.25-8.30 (m, 1H), 8.41-8.46 (m, 1H)

Example 164

$^1$H-NMR ($CDCl_3$) δ 1.25-1.43 (m, 2H), 1.48-1.53 (m, 2H), 1.65-2.00 (m, 12H), 2.10-2.22 (m, 3H), 2.70-2.85 (m, 5H), 3.14-3.28 (m, 1H), 3.32 (s, 3H), 3.34-3.48 (m, 3H), 3.67 (s, 3H), 3.88-3.92 (m, 2H), 4.11-4.19 (m, 1H), 6.99-7.02 (m, 1H)

Example 165

4-Chloro-5-[[1-(5-cyanopyridin-2-yl)piperidin-4-yl](methyl)amino]-*N*-[(*E*)-5-hydroxy-2-adamantyl]-1-methyl-1*H*-pyrazole-3-carboxamide

[Chemical Formula 107]

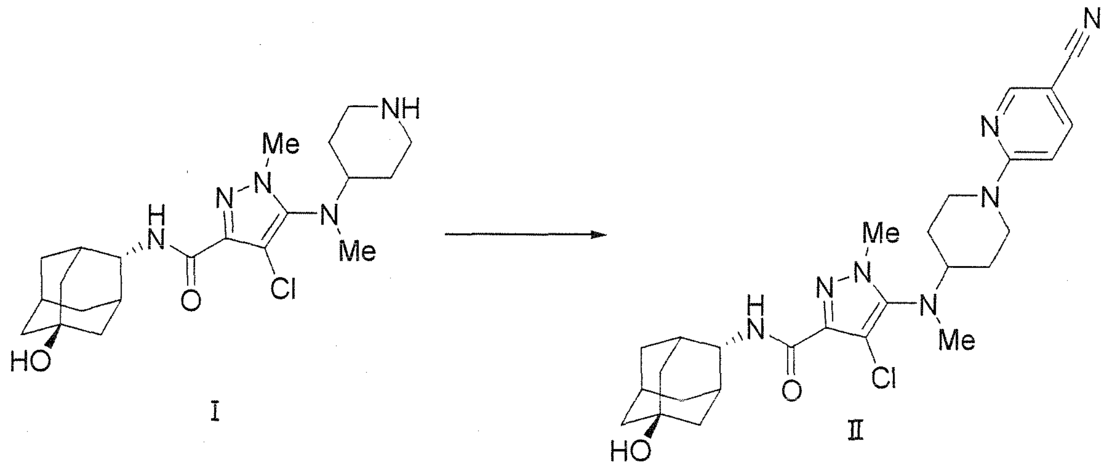

I

II

Compound I (20.0 mg) was dissolved in DMF (1.00 mL), and then thereto were added potassium carbonate (13.0 mg) and 6-chloro-3-pyridinecarbonitrile (10.0 mg) and the mixture was stirred at 100˚C for 12 hours. Then, thereto was added water, and then the mixture was extracted with ethyl acetate. The organic layer was washed with brine, and then dried over sodium sulfate and concentrated *in vacuo*. The residue was purified by silica gel chromatography (eluent: chloroform/methanol = 20/1) to give Compound II (11.0 mg).

$^1$H-NMR ($CDCl_3$) δ 1.34-1.54 (m, 4H), 1.72-2.00 (m, 11H), 2.10-2.30 (m, 3H), 2.79 (s, 3H), 2.90-3.07 (m, 2H), 3.31-3.48 (m, 1H), 3.68 (s, 3H), 4.13-4.17 (m, 1H), 4.36-4.40 (m, 2H), 6.56-6.59 (m, 1H), 7.00-7.03 (m, 1H), 7.55-7.58 (m, 1H), 8.30-8.45 (m, 1H)

Example 166

4-Chloro-5-[{[1-(5-cyanopyridin-2-yl)piperidin-4-yl]methyl}(methyl)amino]-*N*-[(*E*)-5-hydroxy-2-adamantyl]-1-methyl-1*H*-pyrazole-3-carboxamide

[Chemical Formula 108]

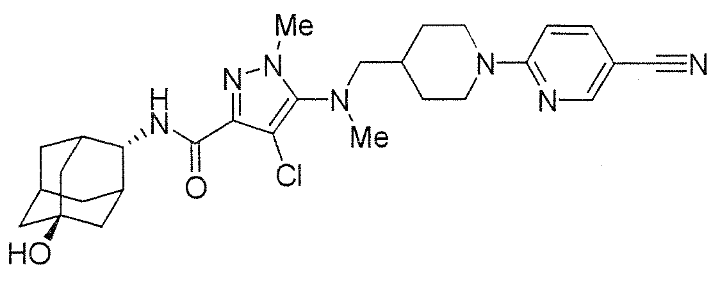

$^1$H-NMR ($CDCl_3$) δ 1.07-1.36 (m, 2H), 1.34-1.60 (m, 3H), 1.70-2.07 (m, 11H), 2.10-2.35 (m, 3H), 2.77 (s, 3H), 2.85-2.93 (m, 2H), 3.00-3.03 (m, 2H), 3.71 (s, 3H), 4.15-4.17 (m, 1H), 4.38-4.43 (m, 2H), 6.55-6.58 (m, 1H), 6.99-7.01 (m, 1H), 7.53-7.57 (m, 1H), 8.30-8.39 (m, 1H)

Example 167

4-Chloro-*N*-[(*E*)-5-hydroxy-2-adamantyl]-1-methyl-5-[methyl(1-pyridin-3-ylpiperidin-4-yl)amino]-1*H*-pyrazole-3-carboxamide

[Chemical Formula 109]

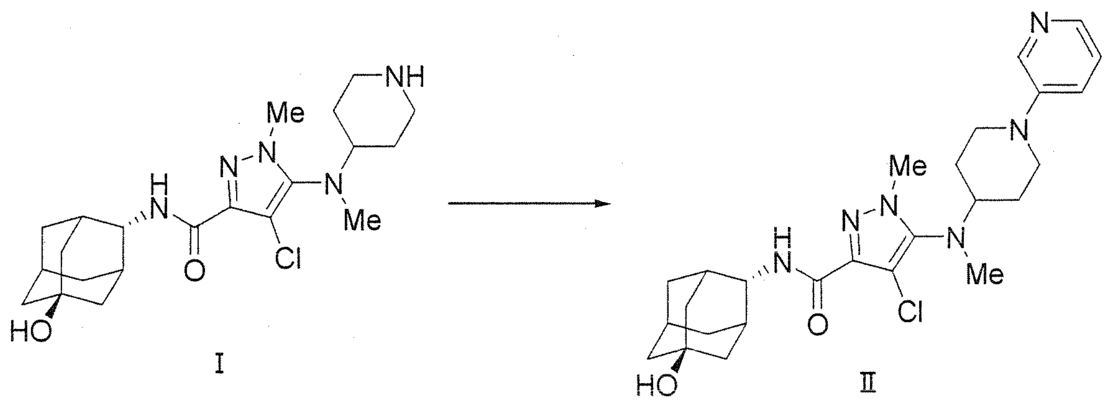

A solution of Compound I (50.0 mg), 3-bromopyridine (22.4 mg), sodium tertiary-butoxide (45.5 mg), 2.2'-bis(diphenylphosphino)-1,1'-binaphthyl (7.30 mg) and tris(dibenzylideneacetone)dipalladium (5.40 mg) in toluene (1.50 mL) was stirred under nitrogen at 100°C for 3 hours. Then, thereto was added water, and then the mixture was extracted with ethyl acetate. The organic layer was washed with brine, and then dried over magnesium sulfate and concentrated *in vacuo*. The residue was purified by a reverse phase HPLC (gradient condition 10%-) to give the titled Compound II (28.0 mg).

$^1$H-NMR ($CDCl_3$) δ 1.50-1.68 (m, 4H), 1.76-1.86 (m, 6H), 1.90-2.00 (m, 4H), 2.16-2.25 (m, 3H), 2.75-2.85 (m, 2H), 2.84 (s, 3H), 3.22-3.32 (m, 1H), 3.62-3.70 (m, 2H), 3.73 (s, 3H), 4.16-4.22 (m, 1H), 7.02 (d, J=8Hz, 1H), 7.12-7.19 (m, 2H), 8.08 (dd, J=2,4Hz, 1H), 8.29 (d, J=2Hz, 1H)

Compounds of Examples 168-180 were prepared in the similar manner to Example 167.

[Chemical Formula 110]

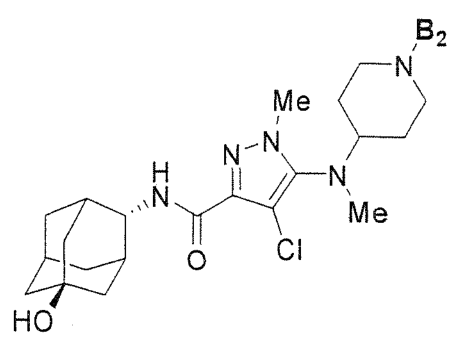

| Example No. | **$B_2$** | Example No. | **$B_2$** |
|---|---|---|---|
| 168 | 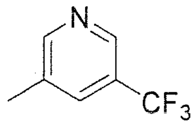 | 175 | 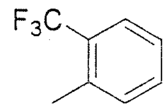 |
| 169 | 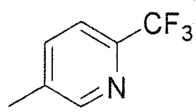 | 176 | 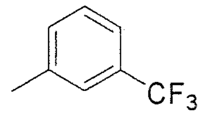 |
| 170 | 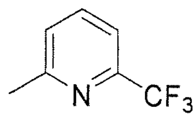 | 177 | 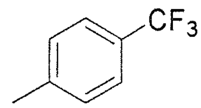 |
| 171 | 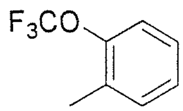 | 178 | 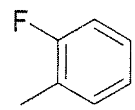 |
| 172 | 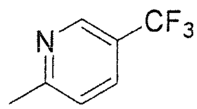 | 179 | 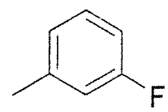 |
| 173 | 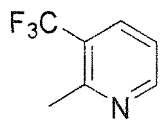 | 180 | 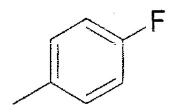 |
| 174 | 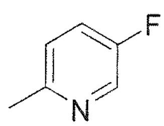 | 181 | 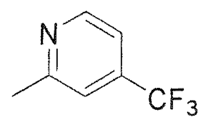 |

Example 168

$^1$H-NMR ($CDCl_3$) δ 1.45-1.60 (m, 4H), 1.70-1.84 (m, 6H), 1.85-2.10 (m, 5H), 2.10-2.27 (m, 3H), 2.74-2.93 (m, 5H), 3.25-3.35 (m, 1H), 3.65-3.78 (m, 5H), 4.13-4.22 (m, 1H), 7.00-7.03 (m, 1H), 7.29 (s, 1H), 8.27 (s, 1H), 8.41 (s, 1H)

Example 169

$^1$H-NMR ($CDCl_3$) δ 1.45-1.62 (m, 4H), 1.62-1.85 (m, 7H), 1.89-2.00 (m, 4H), 2.11-2.25 (m, 3H), 2.81 (s, 3H), 2.83-2.98 (m, 2H), 3.26-3.38 (m, 1H), 3.70 (s, 3H), 3.74-3.79 (m, 2H), 4.13-4.24 (m, 1H), 7.00-7.03 (m, 1H), 7.15-7.18 (m, 1H), 7.46-7.49 (m, 1H), 8.28-8.32 (m, 1H)

Example 170

$^1$H-NMR ($CDCl_3$) δ 1.35-1.58 (m, 4H), 1.72-2.00 (m, 11H), 2.11-2.27 (m, 3H), 2.80 (s, 3H), 2.84-2.93 (m, 2H), 3.26-3.38 (m, 1H), 3.69 (s, 3H), 4.12-4.21 (m, 1H), 4.29-4.34 (m, 2H), 6.74-6.77 (m, 1H), 6.88-6.90 (m, 1H), 7.00-7.03 (m, 1H), 7.50-7.56 (m, 1H)

Example 171

$^1$H-NMR ($CDCl_3$) δ 1.35-1.70 (m, 5H), 1.70-1.97 (m, 10H), 2.11-2.25 (m, 3H), 2.72-2.90 (m, 5H), 3.20-3.34 (m, 1H), 3.65-3.80 (m, 5H), 4.13-4.21 (m, 1H), 6.89-6.92 (m, 2H), 7.01-7.03 (m, 1H), 7.43-7.46 (m, 2H)

Example 172

$^1$H-NMR ($CDCl_3$) δ 1.36-1.57 (m, 5H), 1.70-2.00 (m, 10H), 2.11-2.25 (m, 3H), 2.80 (s, 3H), 2.89-2.97 (m, 2H), 3.30-3.43 (m, 1H), 3.69 (s, 3H), 4.11-4.22 (m, 1H), 4.34-4.38 (m, 2H), 6.61-6.64 (m, 1H), 7.00-7.03 (m, 1H), 7.57-7.61 (m, 1H), 8.35 (s, 1H)

Example 173

$^1$H-NMR ($CDCl_3$) δ 1.47-1.70 (m, 5H), 1.75-1.96 (m, 10H), 2.11-2.26 (m, 3H), 2.82 (s, 3H), 2.87-2.95 (m, 2H), 3.17-3.31 (m, 1H), 3.53-3.65 (m, 2H), 4.14-4.22 (m, 1H), 7.00-7.03 (m, 1H), 6.94-7.03 (m, 2H), 7.81-7.86 (m, 1H), 8.38-8.41 (m, 1H)

Example 174

$^1$H-NMR ($CDCl_3$) δ 1.39-1.59 (m, 5H), 1.75-2.00 (m, 10H), 2.11-2.25 (m, 3H), 2.75-2.90 (m, 5H), 3.22-3.33 (m, 1H), 3.69 (s, 3H), 4.08-4.22 (m, 3H), 6.58-6.62 (m, 1H), 7.00-7.03 (m, 1H), 7.19-7.25 (m, 1H), 8.00-8.10 (m, 1H)

Example 175

$^1$H-NMR ($CDCl_3$) δ 1.49-1.69 (m, 5H), 1.75-1.93 (m, 10H), 2.11-2.25 (m, 3H), 2.68-2.75 (m, 2H), 2.82 (s, 3H), 3.00-3.10 (m, 2H), 3.11-3.25 (m, 1H), 3.74 (s, 3H), 4.13-4.21 (m, 1H), 7.02-7.04 (m, 1H), 7.16-7.21 (m, 1H), 7.28-7.31 (m, 1H), 7.45-7.50 (m, 1H), 7.57-7.60 (m, 1H)

Example 176

$^1$H-NMR ($CDCl_3$) δ 1.47-1.69 (m, 5H), 1.72-2.00 (m, 10H), 2.11-2.27 (m, 3H), 2.70-2.90 (m, 5H), 3.20-3.32 (m, 1H), 3.64-3.68 (m, 2H), 3.71 (s, 3H), 4.13-4.21 (m, 1H), 7.01-7.09 (m, 3H), 7.29-7.34 (m, 1H)

Example 177

$^1$H-NMR ($CDCl_3$) δ 1.41-1.69 (m, 5H), 1.70-1.98 (m, 10H), 2.10-2.27 (m, 3H), 2.75-2.92 (m, 5H), 3.20-3.35 (m, 1H), 3.62-3.80 (m, 5H), 4.13-4.22 (m, 1H), 6.90-6.92 (m, 2H), 7.01-7.03 (m, 1H), 7.43-7.46 (m, 2H)

Example 178

$^1$H-NMR ($CDCl_3$) δ 1.35-1.70 (m, 5H), 1.75-2.02 (m, 10H), 2.12-2.29 (m, 3H), 2.59-2.78 (m, 2H), 2.82 (s, 3H), 3.13-3.28 (m, 1H), 3.38-3.50 (m, 2H), 3.73 (s, 3H), 4.14-4.25 (m, 1H), 6.82-7.14 (m, 5H)

Example 179

$^1$H-NMR ($CDCl_3$) δ 1.39-1.69 (m, 5H), 1.70-2.02 (m, 10H), 2.12-2.27 (m, 3H), 2.68-2.90 (m, 5H), 3.17-3.30 (m, 1H), 3.57-3.68 (m, 2H), 3.71 (s, 3H), 4.13-4.25 (m, 1H), 6.50-6.66 (m, 3H), 7.00-7.03 (m, 1H), 7.11-7.19 (m, 1H)

Example 180

$^1$H-NMR ($CDCl_3$) δ 1.31-1.62 (m, 5H), 1.70-2.07 (m, 10H), 2.12-2.27 (m, 3H), 2.54-2.80 (m, 2H), 2.82 (s, 3H), 3.11-3.30 (m, 1H), 3.44-3.56 (m, 2H), 3.72 (s, 3H), 4.12-4.24 (m, 1H), 6.70-7.12 (m, 4H)

Example 181

$^1$H-NMR ($CDCl_3$) δ 1.35-1.56 (m, 5H), 1.70-1.97 (m, 10H), 2.10-2.27 (m, 3H), 2.80 (s, 3H), 2.86-2.97 (m, 2H), 3.30-3.40 (m, 1H), 3.69 (s, 3H), 4.16-4.18 (m, 1H), 4.29-4.32 (m, 2H), 6.72-6.74 (m, 1H), 6.77 (s, 1H), 7.00-7.31 (m, 1H), 8.24-8.27 (m, 1H)

The following Example Compounds, Examples A1-AX9 were prepared in the similar manner to that used in the above Examples.

[Chemical formula 111]

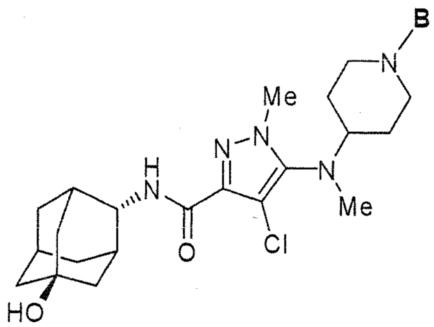

[Table 1]

| Ex. No. | - B | obs MS [M+1] | tR (min) | Measurement Method |
|---|---|---|---|---|
| A1 | 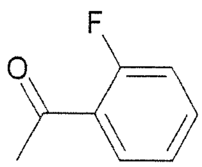 | 544.4 | 3.56 | SA |
| A2 | 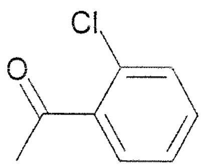 | 560.5 | 3.63 | SA |
| A3 | 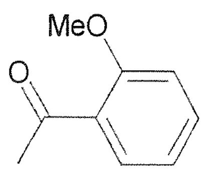 | 556.4 | 3.5 | SA |
| A4 | 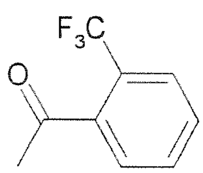 | 594.4 | 3.76 | SA |
| A5 | 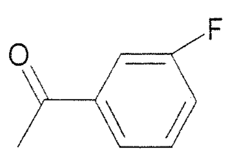 | 544.5 | 3.6 | SA |
| A6 | 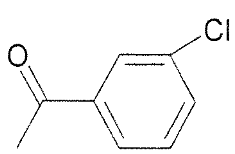 | 560.5 | 3.76 | SA |

(continued)

| Ex. No. | - B | obs MS [M+1] | tR (min) | Measurement Method |
|---|---|---|---|---|
| A7 | 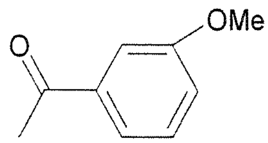 | 556.5 | 3.57 | SA |
| A8 | 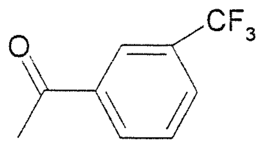 | 594.4 | 3.87 | SA |

[Table 2]

| Ex. No. | - B | obs MS [M+1] | tR (min) | Measurement Method |
|---|---|---|---|---|
| A9 | 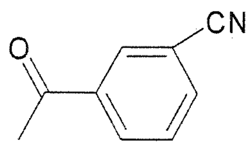 | 551.5 | 3.47 | SA |
| A10 | 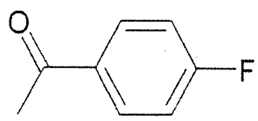 | 544.6 | 3.58 | SA |
| A11 | 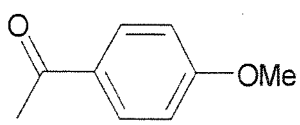 | 556.4 | 3.54 | SA |
| A12 | 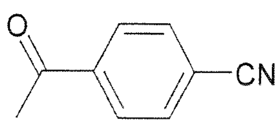 | 551.7 | 3.46 | SA |
| A13 | 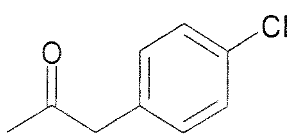 | 574.6 | 3.8 | SA |
| A14 | 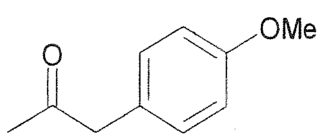 | 570.4 | 3.56 | SA |
| A15 | 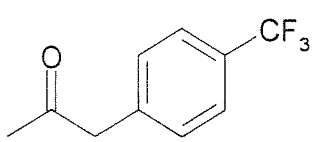 | 608.5 | 3.93 | SA |

(continued)

| Ex. No. | - B | obs MS [M+1] | tR (min) | Measurement Method |
|---|---|---|---|---|
| A16 | 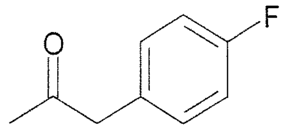 | 558.5 | 3.64 | SA |

[Table 3]

| Ex. No. | - B | obs MS [M+1] | tR (min) | Measurement Method |
|---|---|---|---|---|
| A17 | 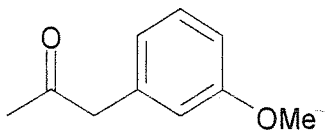 | 570.5 | 3.57 | SA |
| A18 | 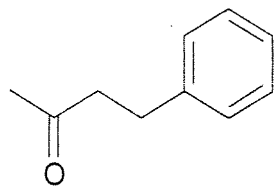 | 554.6 | 3.72 | SA |
| A19 | 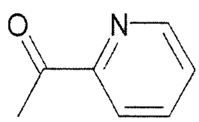 | 527.6 | 3.1 | SA |
| A20 | 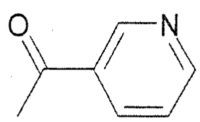 | 527.7 | 2.82 | SA |
| A21 | 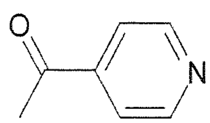 | 527.6 | 2.78 | SA |
| A22 | 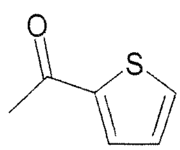 | 532.6 | 3.52 | SA |
| A23 | 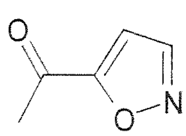 | 517.5 | 3.27 | SA |
| A24 | 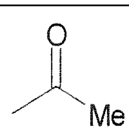 | 464.4 | 3.04 | SA |

[Table 4]

| Ex. No. | -B | obs MS [M+1] | tR (min) | Measurement Method |
|---|---|---|---|---|
| A25 | 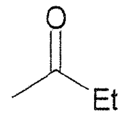 | 478.4 | 3.22 | SA |
| A26 | 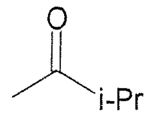 | 492.7 | 3.37 | SA |
| A27 | 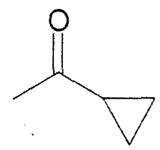 | 490.6 | 3.3 | SA |
| A28 | 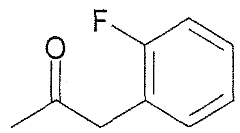 | 558.5 | 3.66 | SA |
| A29 | 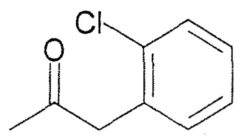 | 574.6 | 3.79 | SA |
| A30 | 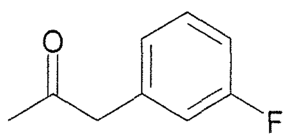 | 558.5 | 3.64 | SA |
| A31 | 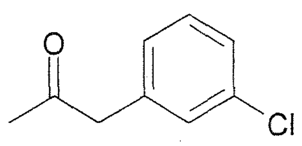 | 574.5 | 3.79 | SA |
| A32 | 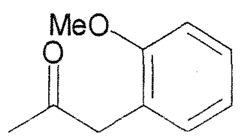 | 570.4 | 3.63 | SA |

[Table 5]

| Ex. No. | -B | obs MS [M+1] | tR (min) | Measurement Method |
|---|---|---|---|---|
| A33 | 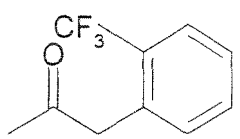 | 608.5 | 3.9 | SA |

(continued)

| Ex. No. | -B | obs MS [M+1] | tR (min) | Measurement Method |
|---|---|---|---|---|
| A34 | 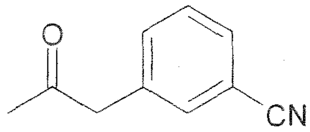 | 565.5 | 3.51 | SA |
| A35 | 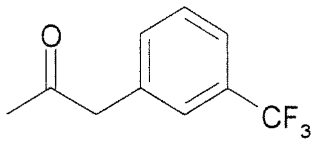 | 608.3 | 3.92 | SA |
| A36 | 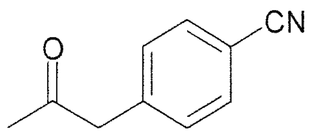 | 565.5 | 3.5 | SA |
| A37 | 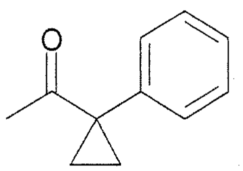 | 566.4 | 3.75 | SA |
| A38 | 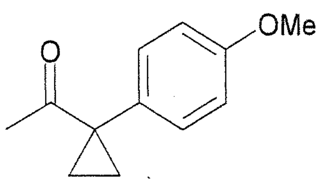 | 596.4 | 3.71 | SA |
| A39 | 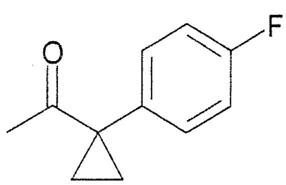 | 584.4 | 3.79 | SA |
| A40 | 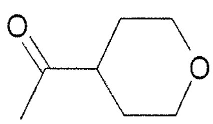 | 534.6 | 3.13 | SA |

[Table 6]

| Ex. No. | - B | obs MS [M+1] | tR (min) | Measurement Method |
|---|---|---|---|---|
| A41 | 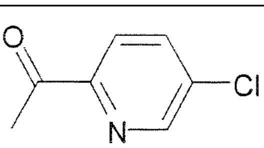 | 561.5 | 3.48 | SA |

(continued)

| Ex. No. | - B | obs MS [M+1] | tR (min) | Measurement Method |
|---|---|---|---|---|
| A42 | 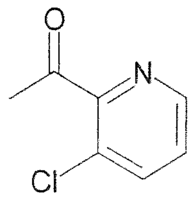 | 561.5 | 3.28 | SA |
| A43 | 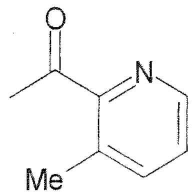 | 541.3 | 3.1 | SA |
| A44 | 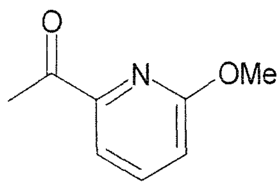 | 557.4 | 3.49 | SA |
| A45 | 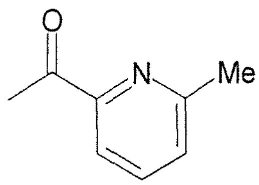 | 541.4 | 3.06 | SA |
| A46 | 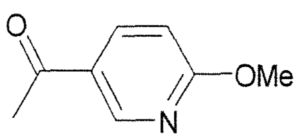 | 557.3 | 3.37 | SA |
| A47 | 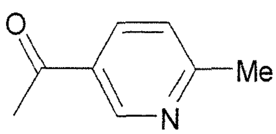 | 541.5 | 2.78 | SA |

[Table 7]

| Ex. No. | -B | obs MS [M+1] | tR (min) | Measurement Method |
|---|---|---|---|---|
| A49 | 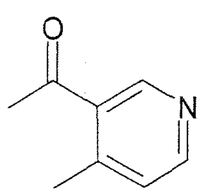 | 541.7 | 2.78 | SA |

(continued)

| Ex. No. | -B | obs MS [M+1] | tR (min) | Measurement Method |
|---|---|---|---|---|
| A50 | 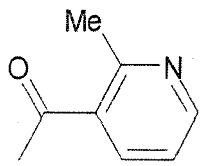 | 541.3 | 2.77 | SA |
| A51 | 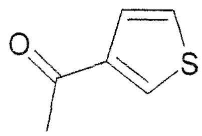 | 532.4 | 3.45 | SA |
| A52 | 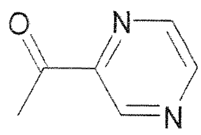 | 528 | 3.09 | SA |
| A53 | 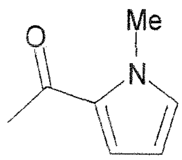 | 529.5 | 3.51 | SA |
| A54 | 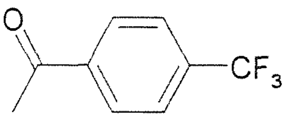 | 594.4 | 3.9 | SA |
| A55 | 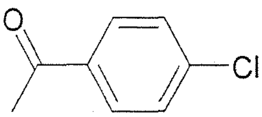 | 560.5 | 3.78 | SA |

[Table 8]

| Ex. No. | -B | obs MS [M+1] | tR (min) | Measurement Method |
|---|---|---|---|---|
| B1 | 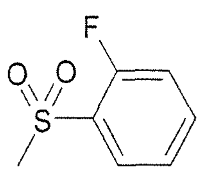 | 580.4 | 3.89 | SA |
| B2 | 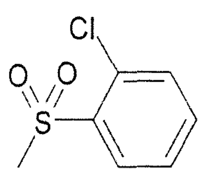 | 596.4 | 3.99 | SA |

(continued)

| Ex. No. | -B | obs MS [M+1] | tR (min) | Measurement Method |
|---|---|---|---|---|
| B3 | 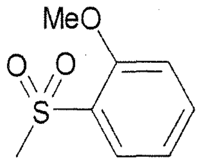 | 592.4 | 3.77 | SA |
| B4 | 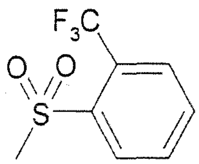 | 630.5 | 4.1 | SA |
| B5 | 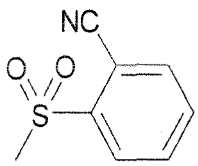 | 587.5 | 3.76 | SA |
| B6 | 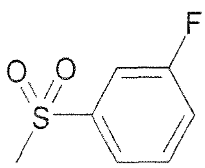 | 580.4 | 3.94 | SA |
| B7 | 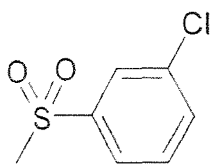 | 596.4 | 4.11 | SA |

[Table 9]

| Ex. No. | - B | obs MS [M+1] | tR (min) | Measurement Method |
|---|---|---|---|---|
| B9 | 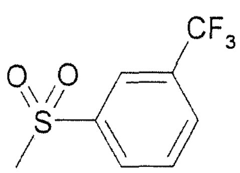 | 630.3 | 4.21 | SA |
| B10 | 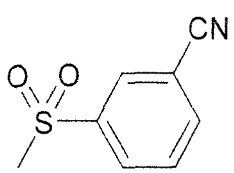 | 587.5 | 3.82 | SA |
| B11 | 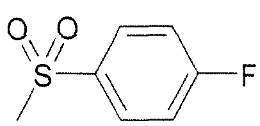 | 580.4 | 3.92 | SA |

(continued)

| Ex. No. | - B | obs MS [M+1] | tR (min) | Measurement Method |
|---|---|---|---|---|
| B12 | 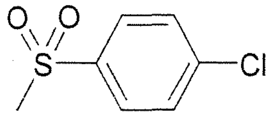 | 596.4 | 4.11 | SA |
| B13 | 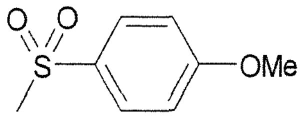 | 592.4 | 3.88 | SA |
| B14 | 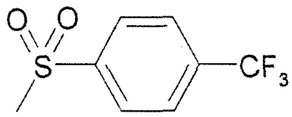 | 630.5 | 4.23 | SA |
| B15 | 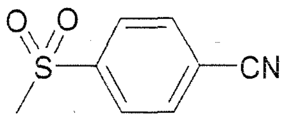 | 587.5 | 3.85 | SA |
| B16 | 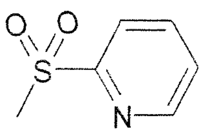 | 563.6 | 3.52 | SA |

[Table 10]

| Ex. No. | -B | obs MS [M+1] | tR (min) | Measurement Method |
|---|---|---|---|---|
| B17 | 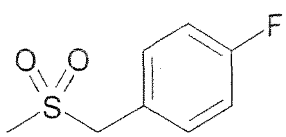 | 594.4 | 3.89 | SA |
| B18 | 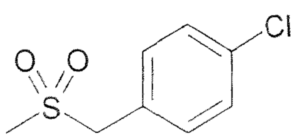 | 610.3 | 4.05 | SA |
| B19 | 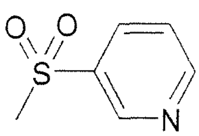 | 563.6 | 3.47 | SA |
| B20 | 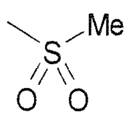 | 500.5 | 3.3 | SA |
| B21 | 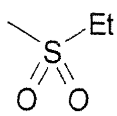 | 514.5 | 3.42 | SA |

(continued)

| Ex. No. | -B | obs MS [M+1] | tR (min) | Measurement Method |
|---|---|---|---|---|
| B22 | 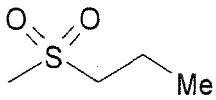 | 528.5 | 3.6 | SA |
| B23 | 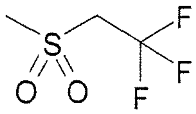 | 568.5 | 3.77 | SA |
| B24 | 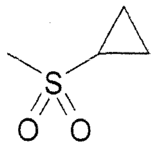 | 526.3 | 3.5 | SA |

[Table 11]

| Ex. No. | -B | obs MS [M+1] | tR (min) | Measurement Method |
|---|---|---|---|---|
| B25 | 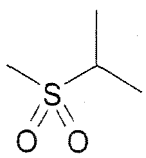 | 528.6 | 3.57 | SA |

[Table 12]

| Ex. No. | - B | obs MS [M+1] | tR (min) | Measurement Method |
|---|---|---|---|---|
| C1 | 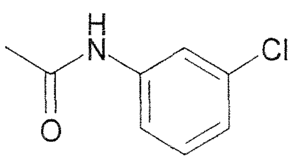 | 575.4 | 3.51 | SA |
| C2 | 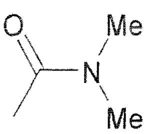 | 493.6 | 3.29 | SA |
| C3 | 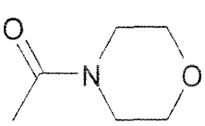 | 535.5 | 3.2 | SA |
| C4 | 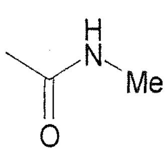 | 479.5 | 3.03 | SA |

(continued)

| Ex. No. | - B | obs MS [M+1] | tR (min) | Measurement Method |
|---|---|---|---|---|
| C5 | 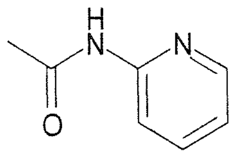 | 542.4 | 2.85 | SA |
| C6 | 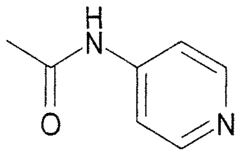 | 542.4 | 2.82 | SA |
| C7 | 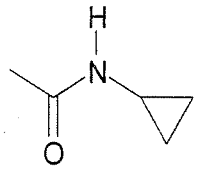 | 505.6 | 3.17 | SA |
| C8 | 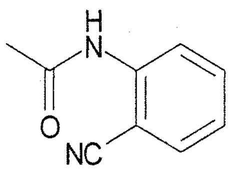 | 566.5 | 3.47 | SA |

[Table 13]

| Ex. No. | - B | obs MS [M+1] | tR (min) | Measurement Method |
|---|---|---|---|---|
| C9 | 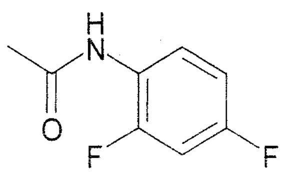 | 577.5 | 3.62 | SA |
| C10 | 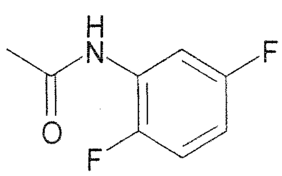 | 577.6 | 3.75 | SA |
| C11 | 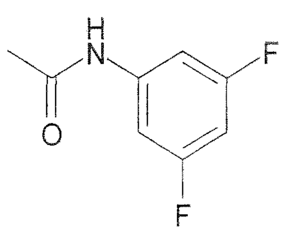 | 577.6 | 3.91 | SA |

(continued)

| Ex. No. | - B | obs MS [M+1] | tR (min) | Measurement Method |
|---|---|---|---|---|
| C12 | 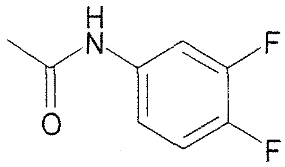 | 577.6 | 3.81 | SA |
| C13 | 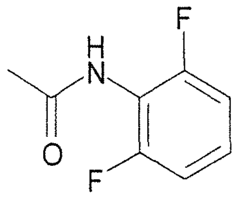 | 577.5 | 3.49 | SA |
| C14 | 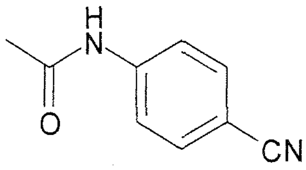 | 566.7 | 3.61 | SA |
| C15 | 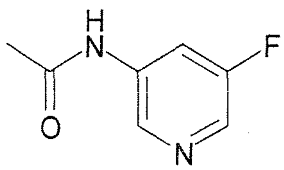 | 560.5 | 3.2 | SA |

[Table 14]

| Ex. No. | -B | obs MS [M+1] | tR (min) | Measurement Method |
|---|---|---|---|---|
| C17 | 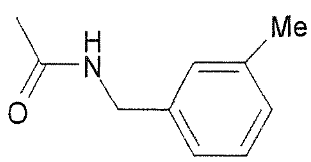 | 569.8 | 3.88 | SB |
| C18 | 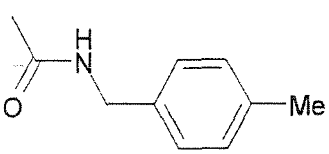 | 569.8 | 3.92 | SB |
| C19 | 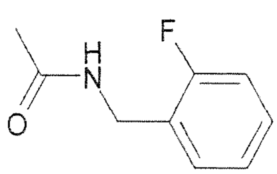 | 573.7 | 3.8 | SB |

(continued)

| Ex. No. | -B | obs MS [M+1] | tR (min) | Measurement Method |
|---|---|---|---|---|
| C20 | 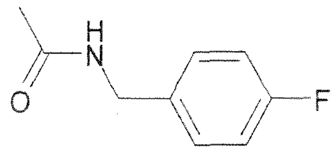 | 573.7 | 3.76 | SB |
| C21 | 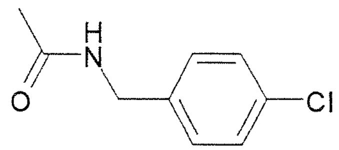 | 589.7 | 3.92 | SB |
| C22 | 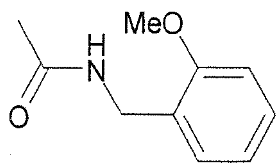 | 585.7 | 3.8 | SB |
| C23 | 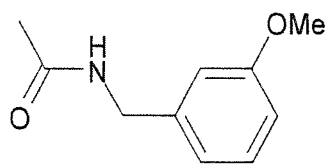 | 585.7 | 3.72 | SB |
| C24 | 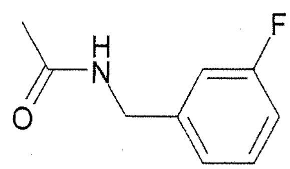 | 573.4 | 3.21 | SB |

[Table 15]

| Ex. No. | -B | obs MS [M+1] | tR (min) | Measurement Method |
|---|---|---|---|---|
| C25 | 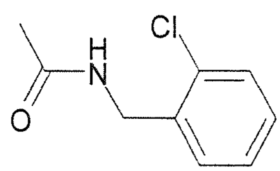 | 589.4 | 3.3 | SB |
| C26 | 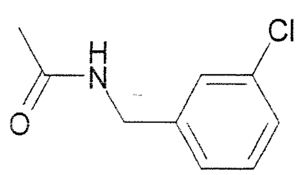 | 589.4 | 3.34 | SB |

(continued)

| Ex. No. | -B | obs MS [M+1] | tR (min) | Measurement Method |
|---|---|---|---|---|
| C27 | 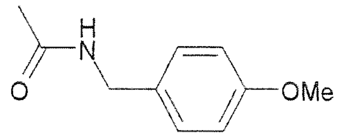 | 585.2 | 3.15 | SB |
| C28 | 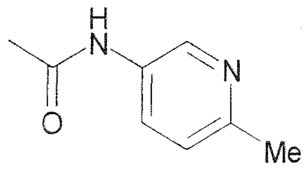 | 556.3 | 2.51 | SB |
| C29 | 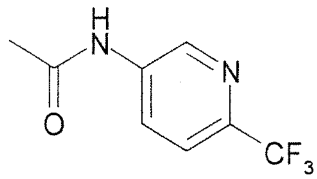 | 610.4 | 3.32 | SB |
| C30 | 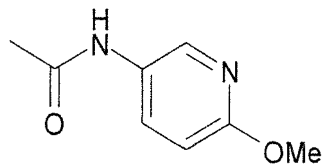 | 572.3 | 2.82 | SB |
| C31 | 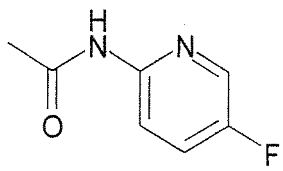 | 560.2 | 2.99 | SB |
| C32 | 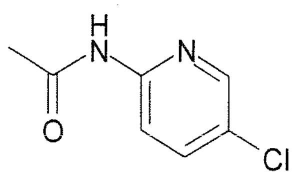 | 576.2 | 3.13 | SB |

[Table 16]

| Ex. No. | - B | obs MS [M+1] | tR (min) | Measurement Method |
|---|---|---|---|---|
| C33 | 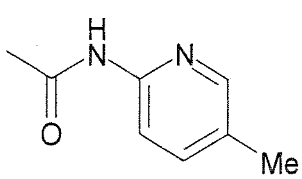 | 556.3 | 2.53 | SB |

(continued)

| Ex. No. | - B | obs MS [M+1] | tR (min) | Measurement Method |
|---|---|---|---|---|
| C34 | 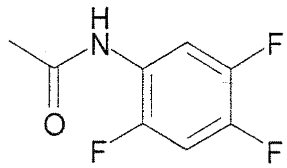 | 595.5 | 3.37 | SB |
| C35 | 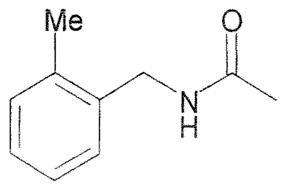 | 569.5 | 3.26 | SB |

[Table 17]

| Ex. No. | - B | obs MS [M+1] | tR (min) | Measurement Method |
|---|---|---|---|---|
| D1 | 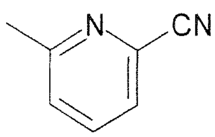 | 524.5 | 4.03 | SA |
| D2 | 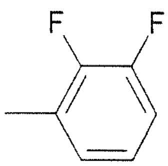 | 534.6 | 4.42 | SA |
| D3 | 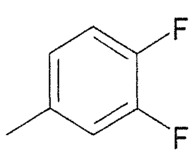 | 534.6 | 3.97 | SA |
| D4 | 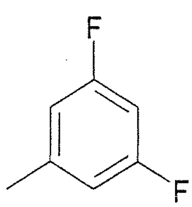 | 534.7 | 4.44 | SA |
| D5 | 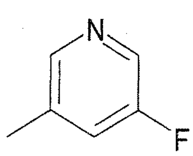 | 517.5 | 3.10 | SA |

(continued)

| Ex. No. | - B | obs MS [M+1] | tR (min) | Measurement Method |
|---|---|---|---|---|
| D6 | 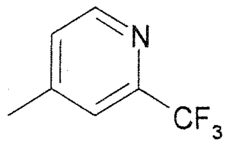 | 567.5 | 3.16 | SA |
| D7 | 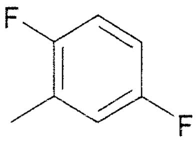 | 534.5 | 4.40 | SA |
| D8 | 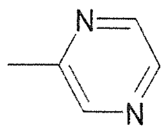 | 500.5 | 3.36 | SA |

[Table 18]

| Ex. No. | -B | obs MS [M+1] | tR (min) | Measurement Method |
|---|---|---|---|---|
| D9 | 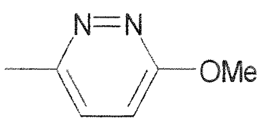 | 530.5 | 2.82 | SA |
| D10 | 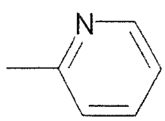 | 499.6 | 2.82 | SA |

[Chemical formula 112]

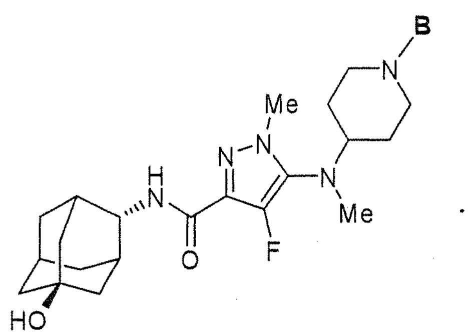

[Table 19]

| Ex. No. | -B | obs MS [M+1] | tR (min) | Measurement Method |
|---|---|---|---|---|
| E1 | 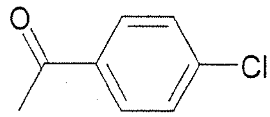 | 544.5 | 3.72 | SA |
| E2 | 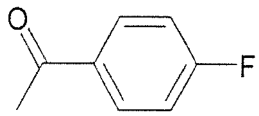 | 528.6 | 3.55 | SA |
| E3 | 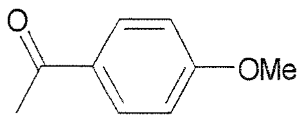 | 540.5 | 3.49 | SA |
| E4 | 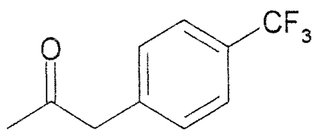 | 592.5 | 3.89 | SA |
| E5 | 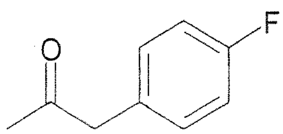 | 542.3 | 3.57 | SA |
| E6 | 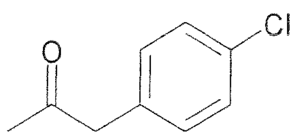 | 558.5 | 3.73 | SA |
| E7 | 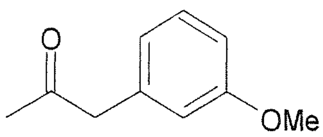 | 554.6 | 3.51 | SA |
| E8 | 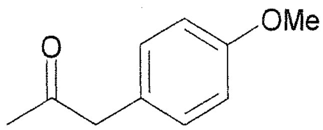 | 554.6 | 3.5 | SA |

[Table 20]

| Ex. No. | - B | obs MS [M+1] | tR (min) | Measurement Method |
|---|---|---|---|---|
| E9 | 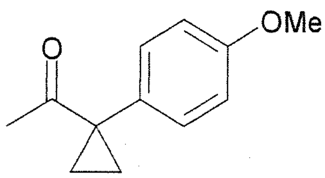 | 580.5 | 3.68 | SA |

(continued)

| Ex. No. | - B | obs MS [M+1] | tR (min) | Measurement Method |
|---|---|---|---|---|
| E10 | 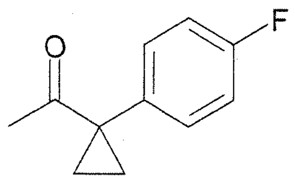 | 568.6 | 3.73 | SA |
| E11 | 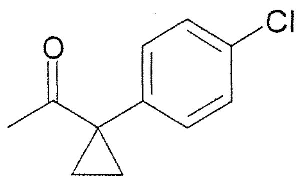 | 584.4 | 3.91 | SA |
| E12 | 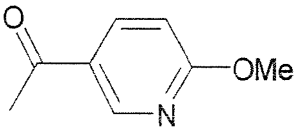 | 541.4 | 3.32 | SA |
| E13 | 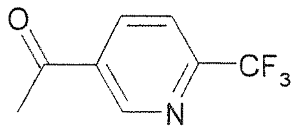 | 579.6 | 3.59 | SA |
| E14 | 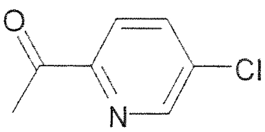 | 545.6 | 3.44 | SA |
| E15 | 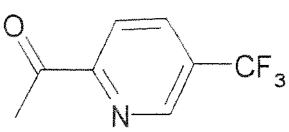 | 579.6 | 3.61 | SA |
| E16 | 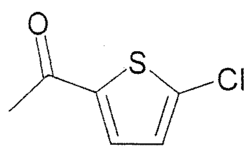 | 550.4 | 3.73 | SA |

[Table 21]

| Ex. No. | - B | obs MS [M+1] | tR (min) | Measurement Method |
|---|---|---|---|---|
| E17 | 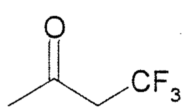 | 516.5 | 3.39 | SA |

[Table 22]

| Ex. No. | - B | obs MS [M+1] | tR (min) | Measurement Method |
|---|---|---|---|---|
| F1 | 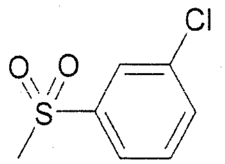 | 580.4 | 4.01 | SA |
| F2 | 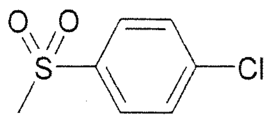 | 580.4 | 4.02 | SA |
| F3 | 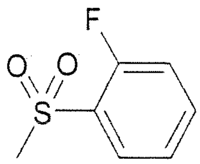 | 564.5 | 3.79 | SA |
| F4 | 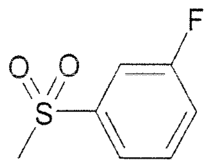 | 564.4 | 3.86 | SA |
| F5 | 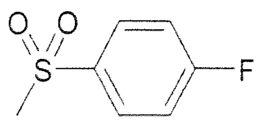 | 564.4 | 3.84 | SA |
| F6 | 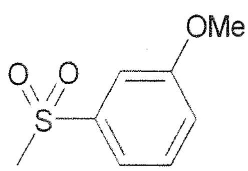 | 576.6 | 3.97 | SA |
| F7 | 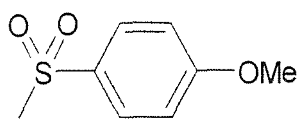 | 576.7 | 3.79 | SA |
| F8 | 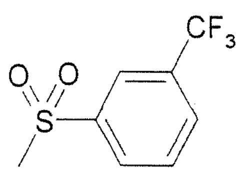 | 614.5 | 4.1 | SA |

[Table 23]

| Ex. No. | -B | obs MS [M+1] | tR (min) | Measurement Method |
|---|---|---|---|---|
| F9 | 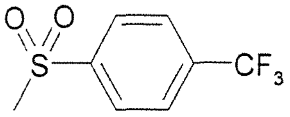 | 614.5 | 4.15 | SA |
| F10 | 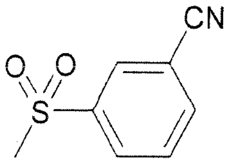 | 571.4 | 3.73 | SA |
| F11 | 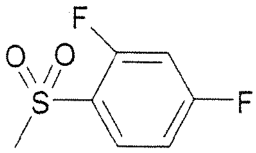 | 582.4 | 3.89 | SA |
| F12 | 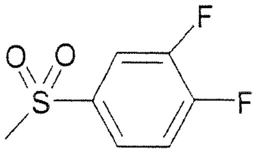 | 582.1 | 3.53 | SB |
| F13 | 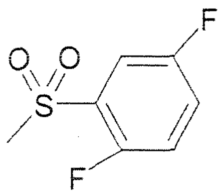 | 582.1 | 3.44 | SB |
| F14 | 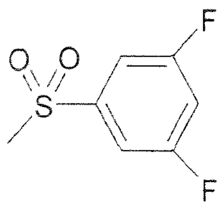 | 582.3 | 3.94 | SA |
| F15 | 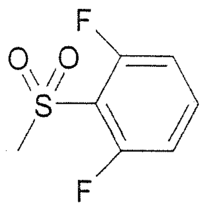 | 582.6 | 3.79 | SA |
| F16 | 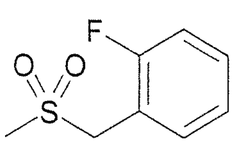 | 578.5 | 3.81 | SA |

[Table 24]

| Ex. No. | -B | obs MS [M+1] | tR (min) | Measurement Method |
|---|---|---|---|---|
| F17 | 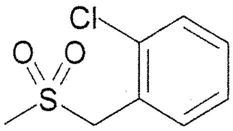 | 594.4 | 3.96 | SA |
| F18 | 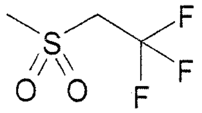 | 552.1 | 3.28 | SB |

[Table 25]

| Ex. No. | - B | obs MS [M+1] | tR (min) | Measurement Method |
|---|---|---|---|---|
| G1 | 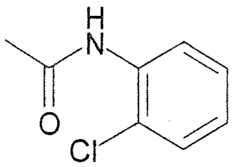 | 559.4 | 3.24 | SB |
| G2 | 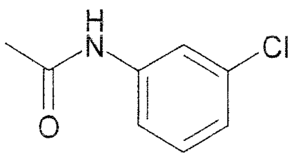 | 559.4 | 3.36 | SB |
| G3 | 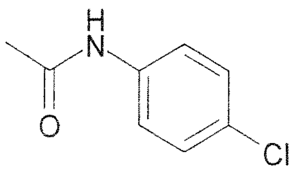 | 559.4 | 3.34 | SB |
| G4 | 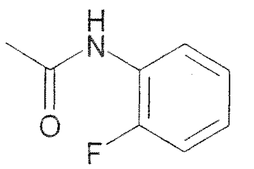 | 543.4 | 3.13 | SB |
| G5 | 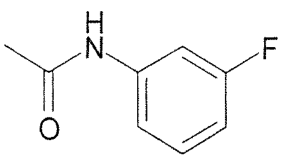 | 543.4 | 3.21 | SB |
| G6 | 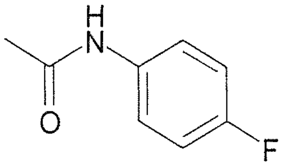 | 543.4 | 3.15 | SB |

(continued)

| Ex. No. | - B | obs MS [M+1] | tR (min) | Measurement Method |
|---|---|---|---|---|
| G7 | 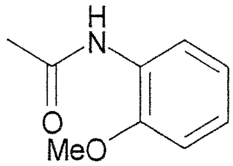 | 555.2 | 3.19 | SB |
| G8 | 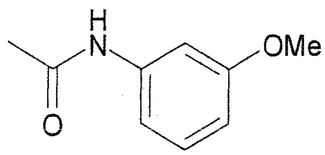 | 555.5 | 3.13 | SB |

[Table 26]

| Ex. No. | - B | obs MS [M+1] | tR (min) | Measurement Method |
|---|---|---|---|---|
| G9 | 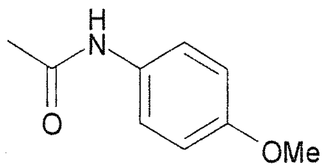 | 555.2 | 3.05 | SB |
| G10 | 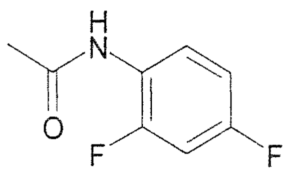 | 561.4 | 3.21 | SB |
| G11 | 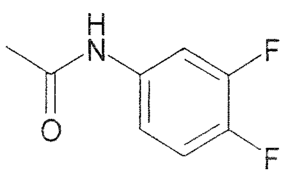 | 561.4 | 3.32 | SB |
| G12 | 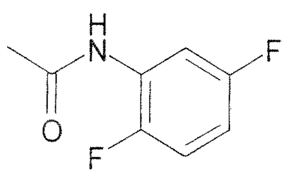 | 561.4 | 3.24 | SB |
| G13 | 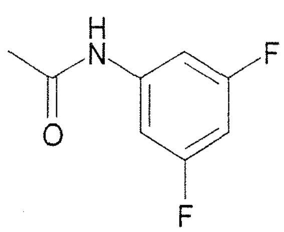 | 561.4 | 3.38 | SB |

(continued)

| Ex. No. | - B | obs MS [M+1] | tR (min) | Measurement Method |
|---|---|---|---|---|
| G14 | 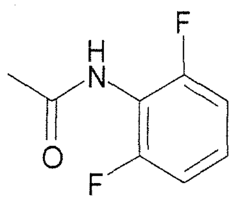 | 561.4 | 3.01 | SB |
| G15 | 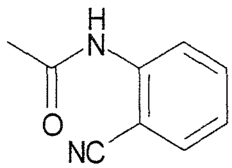 | 550.4 | 2.99 | SB |

[Table 27]

| Ex. No. | - B | obs MS [M+1] | tR (min) | Measurement Method |
|---|---|---|---|---|
| G17 | 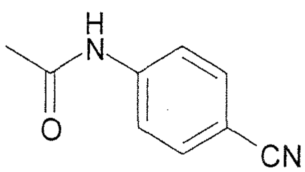 | 550.4 | 3.13 | SB |
| G18 | 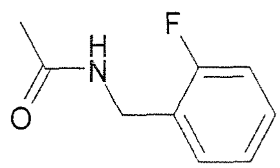 | 557.4 | 3.11 | SB |
| G19 | 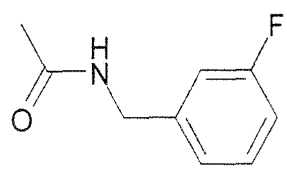 | 557.2 | 3.11 | SB |
| G20 | 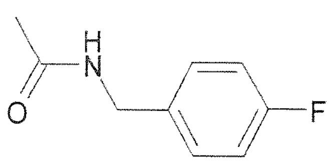 | 557.4 | 3.11 | SB |
| G21 | 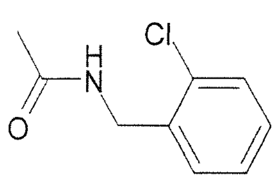 | 573.4 | 3.21 | SB |

(continued)

| Ex. No. | - B | obs MS [M+1] | tR (min) | Measurement Method |
|---|---|---|---|---|
| G22 | 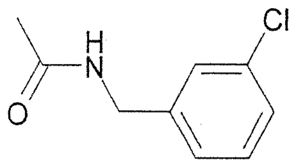 | 573.4 | 3.26 | SB |
| G23 | 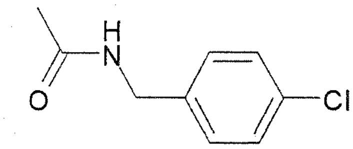 | 573.4 | 3.26 | SB |
| G24 | 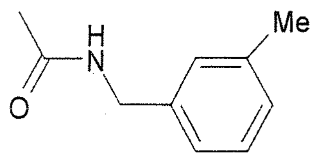 | 553.2 | 3.19 | SB |

[Table 28]

| Ex. No. | - B | obs MS [M+1] | tR (min) | Measurement Method |
|---|---|---|---|---|
| G25 | 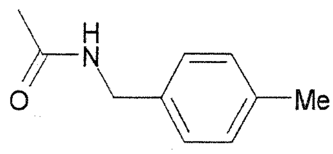 | 553.2 | 3.21 | SB |
| G26 | 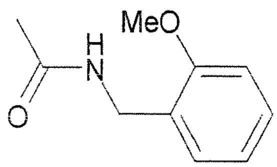 | 569.5 | 3.11 | SB |
| G27 | 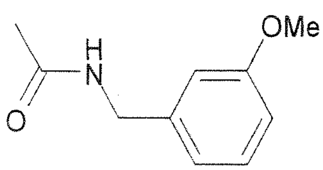 | 569.8 | 3.67 | SB |
| G28 | 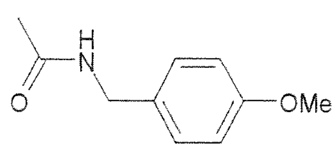 | 569.5 | 3.07 | SB |

(continued)

| Ex. No. | - B | obs MS [M+1] | tR (min) | Measurement Method |
|---|---|---|---|---|
| G29 | 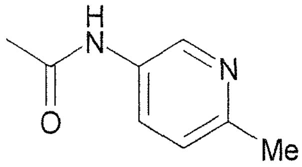 | 540.4 | 2.4 | SB |
| G30 | 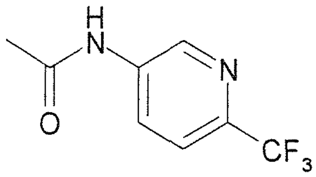 | 594.4 | 3.21 | SB |
| G31 | 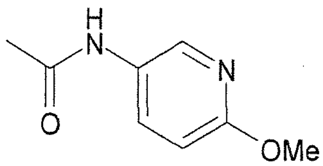 | 556.3 | 2.71 | SB |
| G32 | 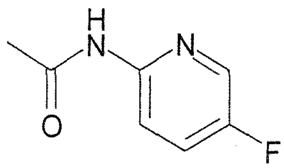 | 544.3 | 2.82 | SB |

[Table 29]

| Ex. No. | -B | obs MS [M+1] | tR (min) | Measurement Method |
|---|---|---|---|---|
| G33 | 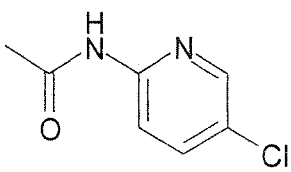 | 560.2 | 3.03 | SB |
| G34 | 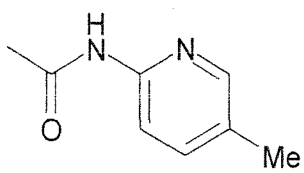 | 540.4 | 2.51 | SB |
| G35 | 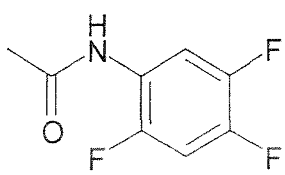 | 579.3 | 3.26 | SB |

(continued)

| Ex. No. | -B | obs MS [M+1] | tR (min) | Measurement Method |
|---|---|---|---|---|
| G36 | 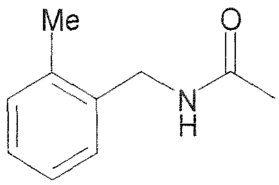 | 553.2 | 3.17 | SB |
| G37 | 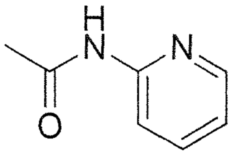 | 526.4 | 2.4 | SB |

[Table 30]

| Ex. No. | - B | obs MS [M+1] | tR (min) | Measurement Method |
|---|---|---|---|---|
| H1 | 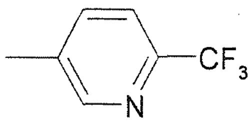 | 551.3 | 3.53 | SB |
| H2 | 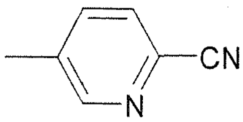 | 508.4 | 3.19 | SB |
| H3 | 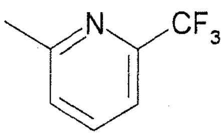 | 551.3 | 3.86 | SB |
| H4 | 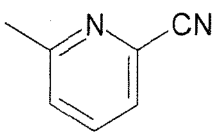 | 508.4 | 3.49 | SB |
| H5 | 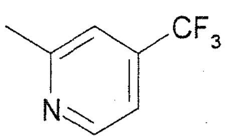 | 551.3 | 3.19 | SB |
| H6 | 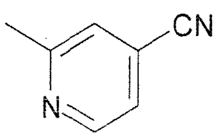 | 508.4 | 3.11 | SB |
| H7 | 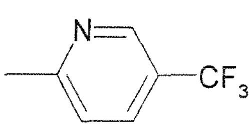 | 551.3 | 3.38 | SB |

(continued)

| Ex. No. | - B | obs MS [M+1] | tR (min) | Measurement Method |
|---|---|---|---|---|
| H8 | 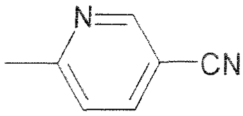 | 508.4 | 3.26 | SB |

[Table 31]

| Ex. No. | - B | obs MS [M+1] | tR (min) | Measurement Method |
|---|---|---|---|---|
| H9 | 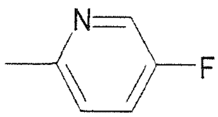 | 501.4 | 2.71 | SB |
| H10 | 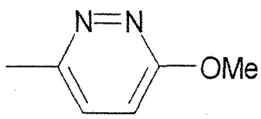 | 514.3 | 2.36 | SB |
| H11 | 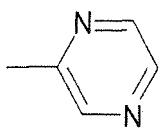 | 484.6 | 2.84 | SB |

[Chemical formula 113]

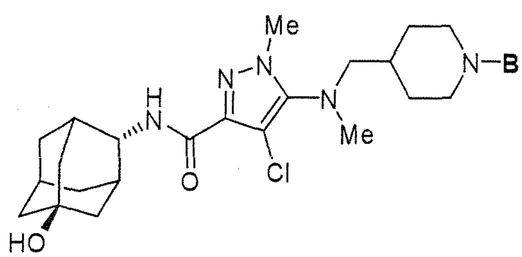

[Table 32]

| Ex. No. | - B | obs MS [M+1] | tR (min) | Measurement Method |
|---|---|---|---|---|
| I1 | 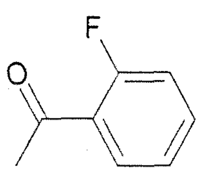 | 558.5 | 3.67 | SA |

(continued)

| Ex. No. | - B | obs MS [M+1] | tR (min) | Measurement Method |
|---|---|---|---|---|
| I2 | 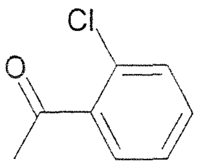 | 574.6 | 3.76 | SA |
| I3 | 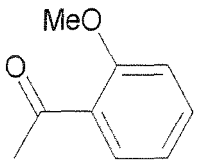 | 570.5 | 3.61 | SA |
| I4 | 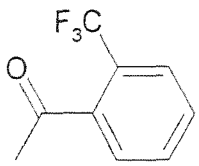 | 608.5 | 3.87 | SA |
| I5 | 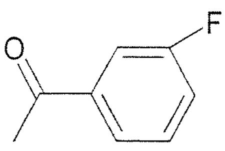 | 558.5 | 3.72 | SA |
| I6 | 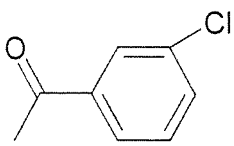 | 574.6 | 3.88 | SA |
| I7 | 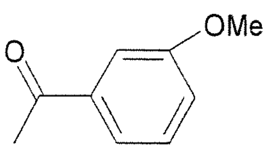 | 570.5 | 3.7 | SA |
| I8 | 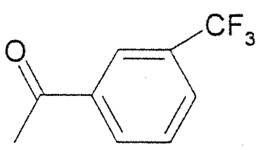 | 608.5 | 4 | SA |

[Table 33]

| Ex. No. | -B | obs MS [M+1] | tR (min) | Measurement Method |
|---|---|---|---|---|
| I9 | 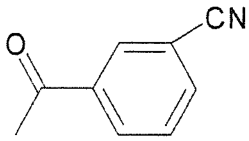 | 565.6 | 3.59 | SA |

(continued)

| Ex. No. | -B | obs MS [M+1] | tR (min) | Measurement Method |
|---|---|---|---|---|
| I10 | 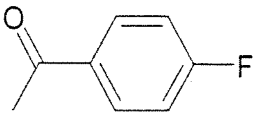 | 558.5 | 3.72 | SA |
| I11 | 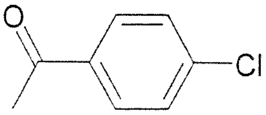 | 574.6 | 3.91 | SA |
| I12 | 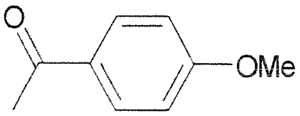 | 570.5 | 3.67 | SA |
| I13 | 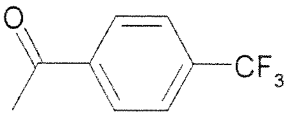 | 608.5 | 4.03 | SA |
| I14 | 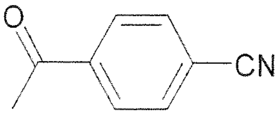 | 565.5 | 3.61 | SA |
| I15 | 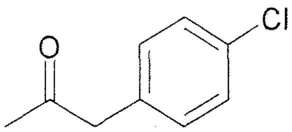 | 588.4 | 3.93 | SA |
| I16 | 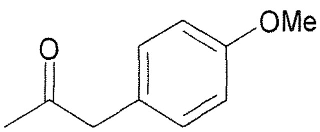 | 584.4 | 3.69 | SA |

[Table 34]

| Ex. No. | - B | obs MS [M+1] | tR (min) | Measurement Method |
|---|---|---|---|---|
| I17 | 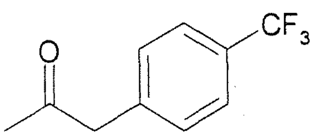 | 622.4 | 4.04 | SA |
| I18 | 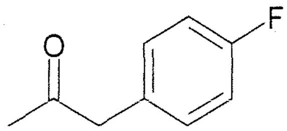 | 572.5 | 3.75 | SA |
| I19 | 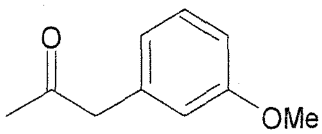 | 584.5 | 3.71 | SA |

(continued)

| Ex. No. | - B | obs MS [M+1] | tR (min) | Measurement Method |
|---|---|---|---|---|
| I20 | 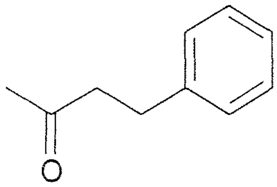 | 568.6 | 3.85 | SA |
| I21 | 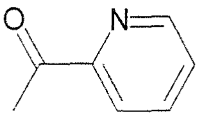 | 541.5 | 3.21 | SA |
| I22 | 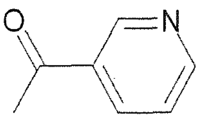 | 541.5 | 2.93 | SA |
| I23 | 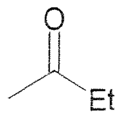 | 492.5 | 3.35 | SA |
| I24 | 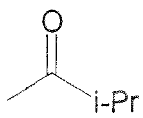 | 506.5 | 3.5 | SA |

[Table 35]

| Ex. No. | - B | obs MS [M+1] | tR (min) | Measurement Method |
|---|---|---|---|---|
| I25 | 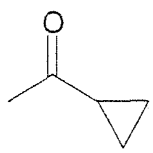 | 504.6 | 3.43 | SA |
| I26 | 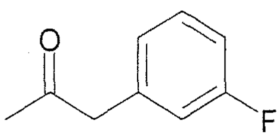 | 572.5 | 3.76 | SA |
| I27 | 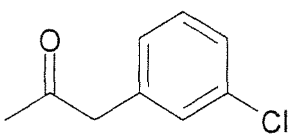 | 588.5 | 3.92 | SA |
| I28 | 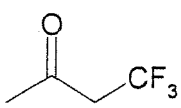 | 564.4 | 3.6 | SA |

(continued)

| Ex. No. | - B | obs MS [M+1] | tR (min) | Measurement Method |
|---|---|---|---|---|
| I29 | 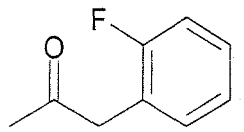 | 572.6 | 3.79 | SA |
| I30 | 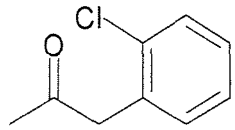 | 588.5 | 3.9 | SA |
| I31 | 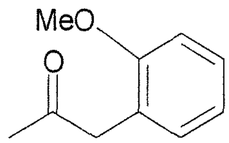 | 584.4 | 3.75 | SA |
| I32 | 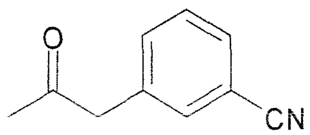 | 579.6 | 3.62 | SA |

[Table 36]

| Ex. No. | -B | obs MS [M+1] | tR (min) | Measurement Method |
|---|---|---|---|---|
| I33 | 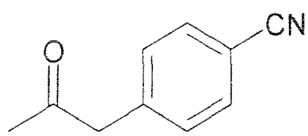 | 579.6 | 3.61 | SA |

[Table 37]

| Ex. No. | -B | obs MS [M+1] | tR (min) | Measurement Method |
|---|---|---|---|---|
| J1 | 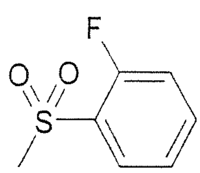 | 594.5 | 4.02 | SA |
| J2 | 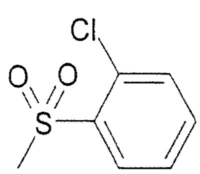 | 610.4 | 4.13 | SA |

(continued)

| Ex. No. | -B | obs MS [M+1] | tR (min) | Measurement Method |
|---|---|---|---|---|
| J3 | 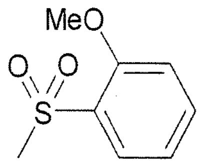 | 606.6 | 3.89 | SA |
| J4 | 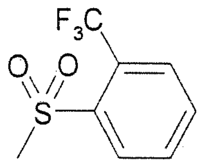 | 644.5 | 4.22 | SA |
| J5 | 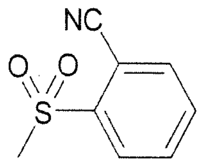 | 601.5 | 3.89 | SA |
| J6 | 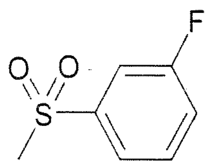 | 594.4 | 4.08 | SA |
| J7 | 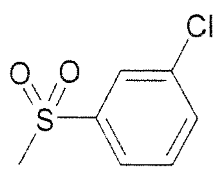 | 610.3 | 4.24 | SA |

[Table 38]

| Ex. No. | -B | obs MS [M+1] | tR (min) | Measurement Method |
|---|---|---|---|---|
| J9 | 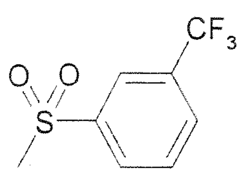 | 644.4 | 4.31 | SA |
| J10 | 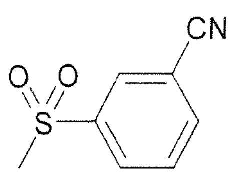 | 601.5 | 3.94 | SA |

(continued)

| Ex. No. | -B | obs MS [M+1] | tR (min) | Measurement Method |
|---|---|---|---|---|
| J11 | 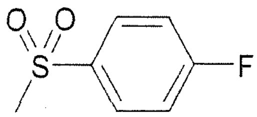 | 594.4 | 4.05 | SA |
| J12 | 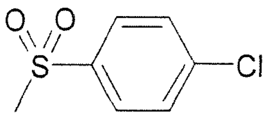 | 612.4 | 4.12 | SA |
| J13 | 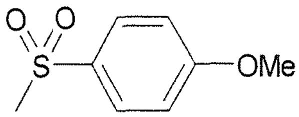 | 606.4 | 4 | SA |
| J14 | 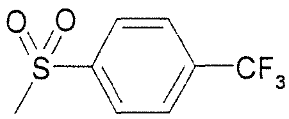 | 644.4 | 4.35 | SA |
| J15 | 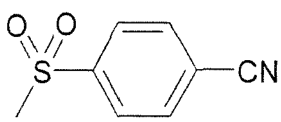 | 601.5 | 3.95 | SA |
| J16 | 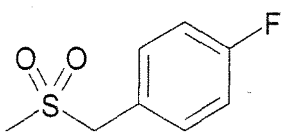 | 608.5 | 4.01 | SA |

[Table 39]

| Ex. No. | - B | obs MS [M+1] | tR (min) | Measurement Method |
|---|---|---|---|---|
| J17 | 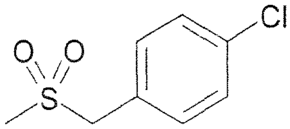 | 624.4 | 4.18 | SA |
| J18 | 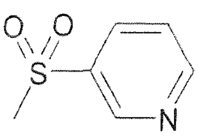 | 577.4 | 3.57 | SA |
| J19 | 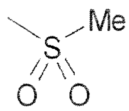 | 514.6 | 3.42 | SA |
| J20 | 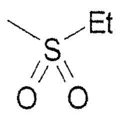 | 528.5 | 3.56 | SA |

(continued)

| Ex. No. | - B | obs MS [M+1] | tR (min) | Measurement Method |
|---|---|---|---|---|
| J21 | 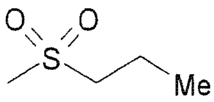 | 542.3 | 3.75 | SA |
| J22 | 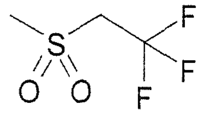 | 582.3 | 3.9 | SA |
| J23 | 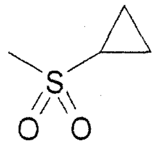 | 540.6 | 3.65 | SA |
| J24 | 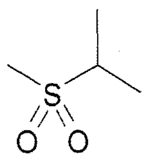 | 542.4 | 3.71 | SA |

[Table 40]

| Ex. No. | -B | obs MS [M+1] | tR (min) | Measurement Method |
|---|---|---|---|---|
| K1 | 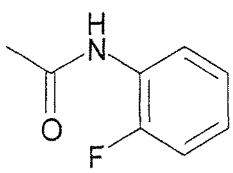 | 573.6 | 3.73 | SA |
| K2 | 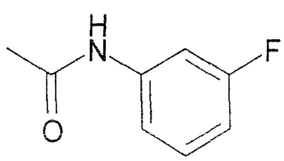 | 573.5 | 3.86 | SA |
| K3 | 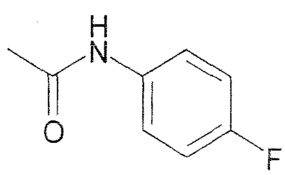 | 573.4 | 3.78 | SA |
| K4 | 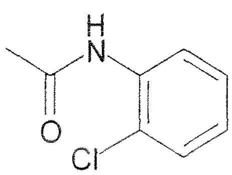 | 589.5 | 3.93 | SA |

(continued)

| Ex. No. | -B | obs MS [M+1] | tR (min) | Measurement Method |
|---|---|---|---|---|
| K5 | 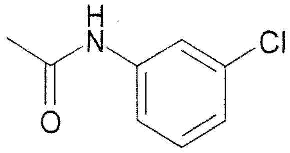 | 589.5 | 4.02 | SA |
| K6 | 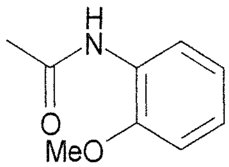 | 585.3 | 3.84 | SA |
| K7 | 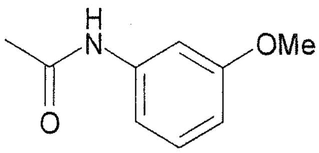 | 585.4 | 3.74 | SA |
| K8 | 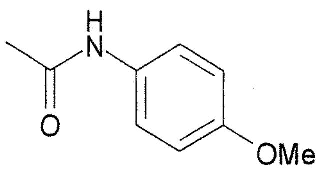 | 585.4 | 3.65 | SA |

[Table 41]

| Ex. No. | - B | obs MS [M+1] | tR (min) | Measurement Method |
|---|---|---|---|---|
| K9 | 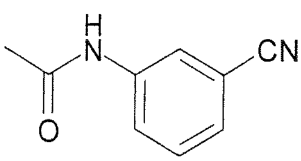 | 580.4 | 3.73 | SA |
| K10 | 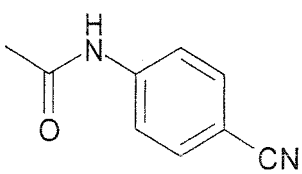 | 580.5 | 3.72 | SA |
| K11 | 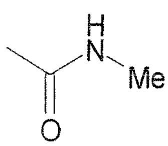 | 493.6 | 3.14 | SA |
| K12 | 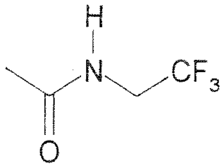 | 561.6 | 3.5 | SA |

(continued)

| Ex. No. | - B | obs MS [M+1] | tR (min) | Measurement Method |
|---|---|---|---|---|
| K13 | 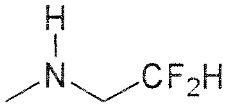 | 543.6 | 3.39 | SA |
| K14 | 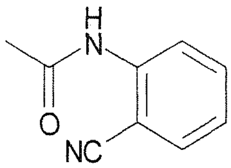 | 580.5 | 3.59 | SA |
| K15 | 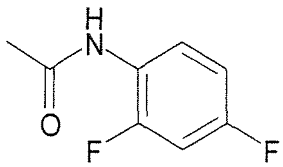 | 591.5 | 3.75 | SA |
| K16 | 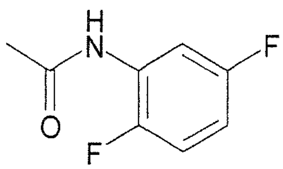 | 591.5 | 3.88 | SA |

[Table 42]

| Ex. No. | - B | obs MS [M+1] | tR (min) | Measurement Method |
|---|---|---|---|---|
| K17 | 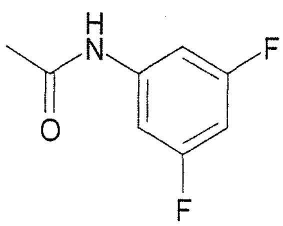 | 591.4 | 4.01 | SA |
| K18 | 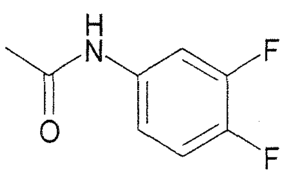 | 591.5 | 3.91 | SA |
| K19 | 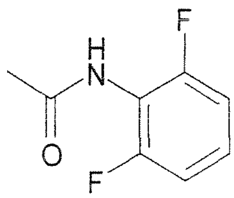 | 591.5 | 3.6 | SA |

(continued)

| Ex. No. | - B | obs MS [M+1] | tR (min) | Measurement Method |
|---|---|---|---|---|
| K20 | 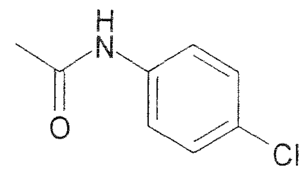 | 589.4 | 3.84 | SA |
| K21 | 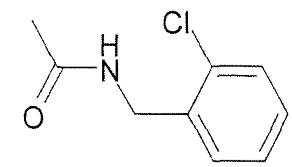 | 603.4 | 3.38 | SB |
| K22 | 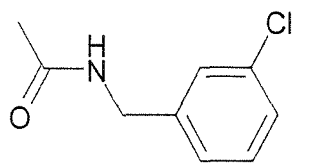 | 603.4 | 3.44 | SB |
| K23 | 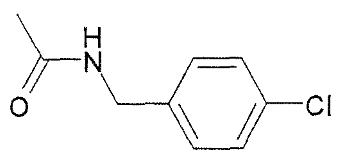 | 603.4 | 3.44 | SB |

[Table 43]

| Ex. No. | -B | obs MS [M+1] | tR (min) | Measurement Method |
|---|---|---|---|---|
| K25 | 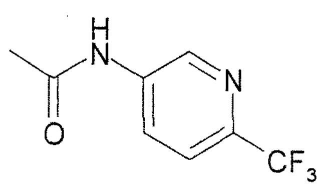 | 624.4 | 3.4 | SB |
| K26 | 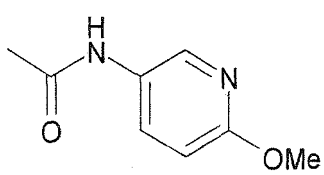 | 586.3 | 2.92 | SB |
| K27 | 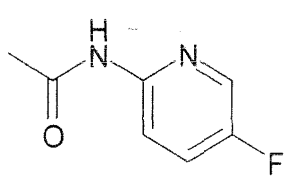 | 574.3 | 2.99 | SB |

(continued)

| Ex. No. | -B | obs MS [M+1] | tR (min) | Measurement Method |
|---|---|---|---|---|
| K28 | 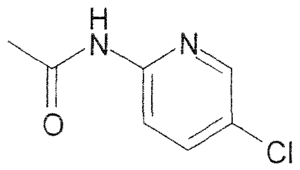 | 590.5 | 3.17 | SB |
| K29 | 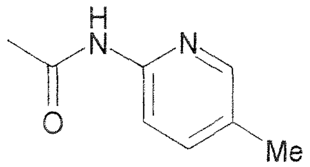 | 570.6 | 2.61 | SB |
| K30 | 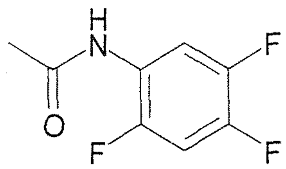 | 609.3 | 3.51 | SB |
| K31 | 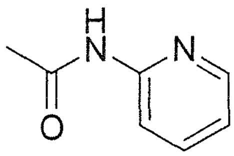 | 556.3 | 2.53 | SB |

[Table 44]

| Ex. No. | -B | obs MS [M+1] | tR (min) | Measurement Method |
|---|---|---|---|---|
| L1 | 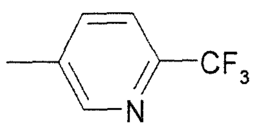 | 581.3 | 3.78 | SB |
| L2 | 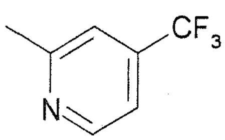 | 581.3 | 3.26 | SB |
| L3 | 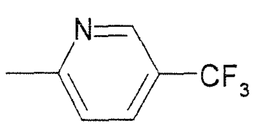 | 581.3 | 3.47 | SB |
| L4 | 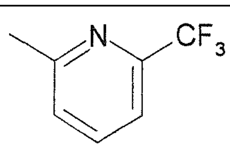 | 581.3 | 4.13 | SB |

(continued)

| Ex. No. | -B | obs MS [M+1] | tR (min) | Measurement Method |
|---|---|---|---|---|
| L5 | 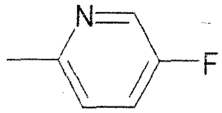 | 531.1 | 2.82 | SB |
| L6 | 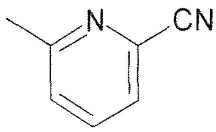 | 538.4 | 3.74 | SB |
| L7 | 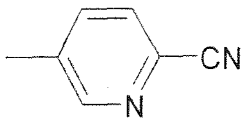 | 538.4 | 3.42 | SB |
| L8 | 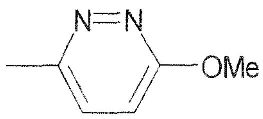 | 544.3 | 2.51 | SB |

[Table 45]

| Ex. No. | -B | obs MS [M+1] | tR (min) | Measurement Method |
|---|---|---|---|---|
| L9 | 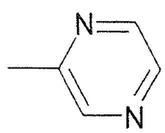 | 514.3 | 3.05 | SB |

[Chemical formula 114]

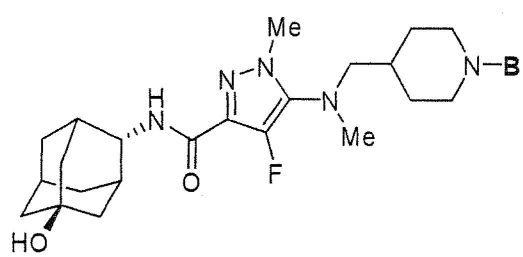

[Table 46]

| Ex. No. | - B | obs MS [M+1] | tR (min) | Measurement Method |
|---|---|---|---|---|
| M1 | 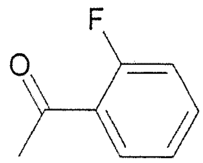 | 542.5 | 3.62 | SA |
| M2 | 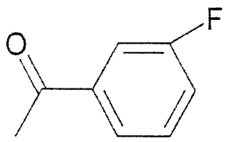 | 542.3 | 3.66 | SA |
| M3 | 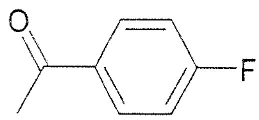 | 542.5 | 3.66 | SA |
| M4 | 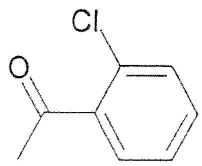 | 558.5 | 3.8 | SA |
| M5 | 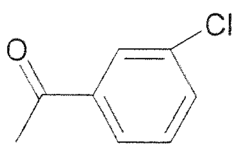 | 558.6 | 3.82 | SA |
| M6 | 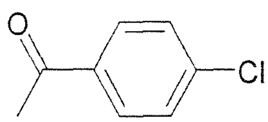 | 558.6 | 3.83 | SA |
| M7 | 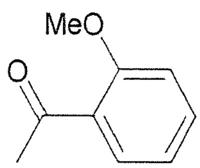 | 554.6 | 3.56 | SA |
| M8 | 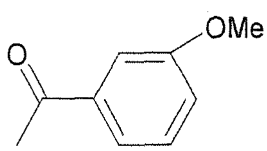 | 554.6 | 3.62 | SA |

[Table 47]

| Ex. No. | - B | obs MS [M+1] | tR (min) | Measurement Method |
|---|---|---|---|---|
| M9 | 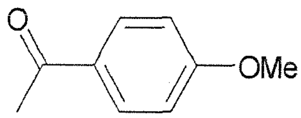 | 554.6 | 3.65 | SA |
| M10 | 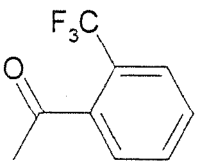 | 592.5 | 3.81 | SA |
| M11 | 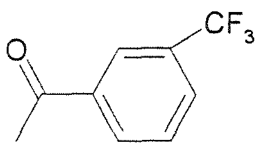 | 592.3 | 3.94 | SA |
| M12 | 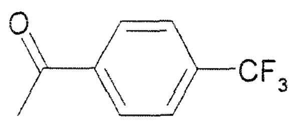 | 592.3 | 3.96 | SA |
| M13 | 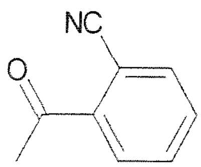 | 549.6 | 3.53 | SA |
| M14 | 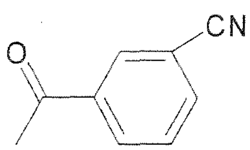 | 549.6 | 3.53 | SA |
| M15 | 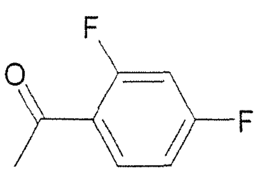 | 560.6 | 3.7 | SA |
| M16 | 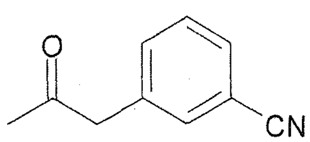 | 563.7 | 3.59 | SA |

[Table 48]

| Ex. No. | - B | obs MS [M+1] | tR (min) | Measurement Method |
|---|---|---|---|---|
| M17 | 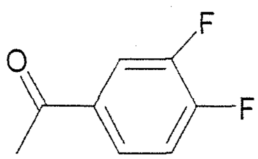 | 560.5 | 3.74 | SA |
| M18 | 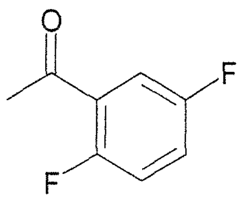 | 560.6 | 3.7 | SA |
| M19 | 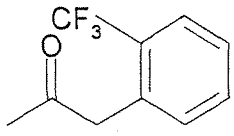 | 606.2 | 3.99 | SA |
| M20 | 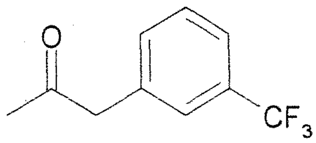 | 606.5 | 3.99 | SA |
| M21 | 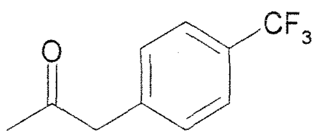 | 606.3 | 3.99 | SA |
| M22 | 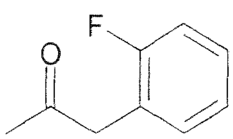 | 556.7 | 3.74 | SA |
| M23 | 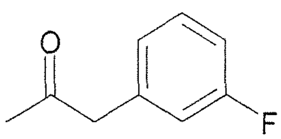 | 556.5 | 3.72 | SA |
| M24 | 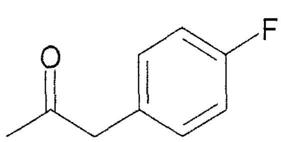 | 556.4 | 3.7 | SA |

[Table 49]

| Ex. No. | -B | obs MS [M+1] | tR (min) | Measurement Method |
|---|---|---|---|---|
| M25 | 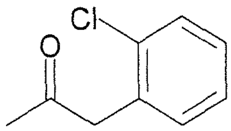 | 572.5 | 3.87 | SA |
| M26 | 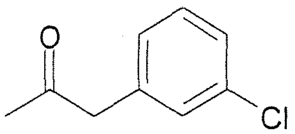 | 572.5 | 3.85 | SA |
| M27 | 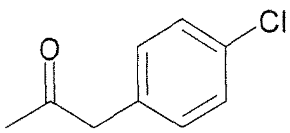 | 572.5 | 3.87 | SA |
| M28 | 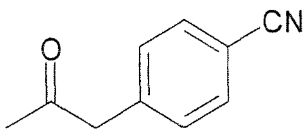 | 563.7 | 3.57 | SA |
| M29 | 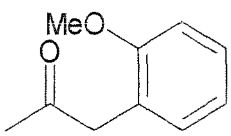 | 568.6 | 3.72 | SA |
| M30 | 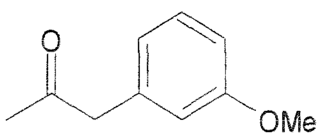 | 568.6 | 3.64 | SA |
| M31 | 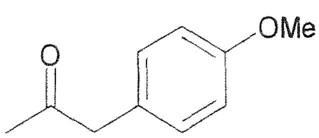 | 568.7 | 3.63 | SA |
| M32 | 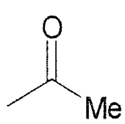 | 462.3 | 3.11 | SA |

[Table 50]

| Ex. No. | - B | obs MS [M+1] | tR (min) | Measurement Method |
|---|---|---|---|---|
| M33 | 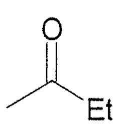 | 476.3 | 3.29 | SA |

(continued)

| Ex. No. | - B | obs MS [M+1] | tR (min) | Measurement Method |
|---|---|---|---|---|
| M34 | 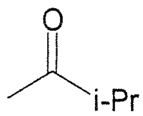 | 490.6 | 3.44 | SA |
| M35 | 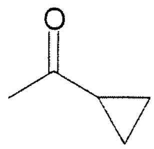 | 488.6 | 3.37 | SA |
| M36 | 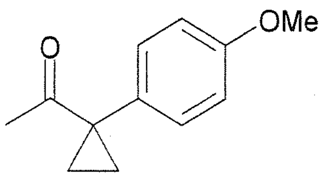 | 594.5 | 3.8 | SA |
| M37 | 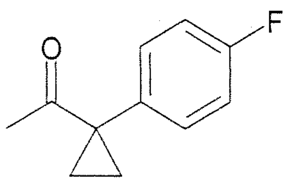 | 582.3 | 3.88 | SA |
| M38 | 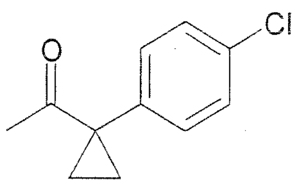 | 598.7 | 4.04 | SA |
| M39 | 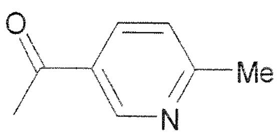 | 539.7 | 2.82 | SA |
| M40 | 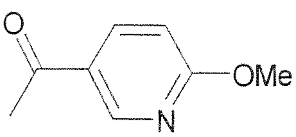 | 555.6 | 3.43 | SA |

[Table 51]

| Ex. No. | -B | obs MS [M+1] | tR (min) | Measurement Method |
|---|---|---|---|---|
| M41 | 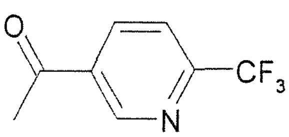 | 593.6 | 3.7 | SA |

(continued)

| Ex. No. | -B | obs MS [M+1] | tR (min) | Measurement Method |
|---|---|---|---|---|
| M42 | 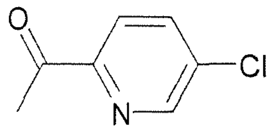 | 559.5 | 3.56 | SA |
| M43 | 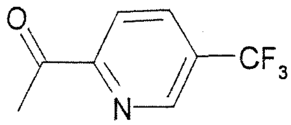 | 593.5 | 3.71 | SA |
| M44 | 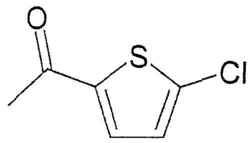 | 564.5 | 3.94 | SA |
| M45 | 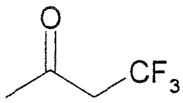 | 530.5 | 3.51 | SA |

[Table 52]

| Ex. No. | - B | obs MS [M+1] | tR (min) | Measurement Method |
|---|---|---|---|---|
| N1 | 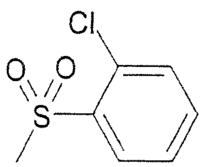 | 594.5 | 4.04 | SA |
| N2 | 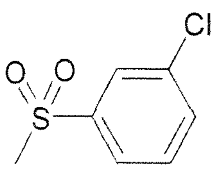 | 594.4 | 4.16 | SA |
| N3 | 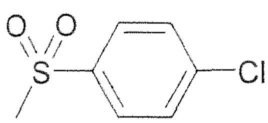 | 594.4 | 4.16 | SA |
| N4 | 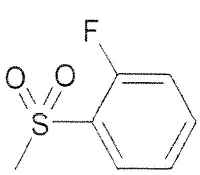 | 578.3 | 3.93 | SA |

(continued)

| Ex. No. | - B | obs MS [M+1] | tR (min) | Measurement Method |
|---|---|---|---|---|
| N5 | 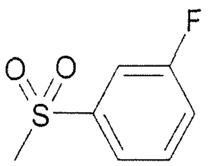 | 578.5 | 4.01 | SA |
| N6 | 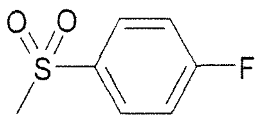 | 578.5 | 3.97 | SA |
| N7 | 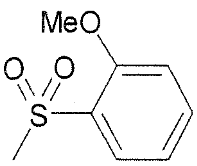 | 590.4 | 3.71 | SA |

[Table 53]

| Ex. No. | -B | obs MS [M+1] | tR (min) | Measurement Method |
|---|---|---|---|---|
| N9 | 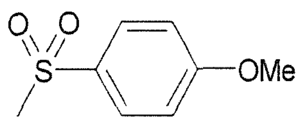 | 590.5 | 3.84 | SA |
| N10 | 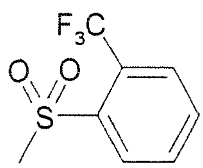 | 628.5 | 4.13 | SA |
| N11 | 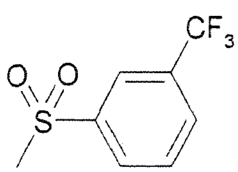 | 628.6 | 4.24 | SA |
| N12 | 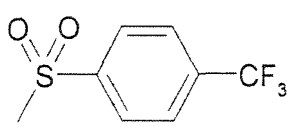 | 628.5 | 4.26 | SA |
| N13 | 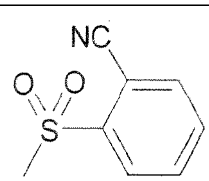 | 585.6 | 3.81 | SA |

(continued)

| Ex. No. | -B | obs MS [M+1] | tR (min) | Measurement Method |
|---|---|---|---|---|
| N14 | 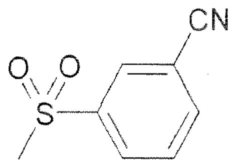 | 585.4 | 3.86 | SA |
| N15 | 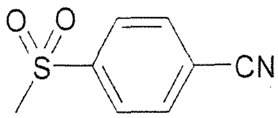 | 585.4 | 3.9 | SA |
| N16 | 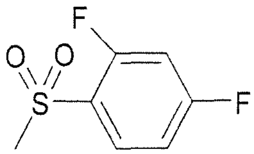 | 596.3 | 4.02 | SA |

[Table 54]

| Ex. No. | - B | obs MS [M+1] | tR (min) | Measurement Method |
|---|---|---|---|---|
| N17 | 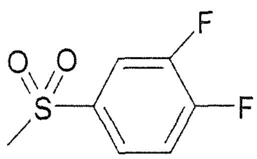 | 596.4 | 4.12 | SA |
| N18 | 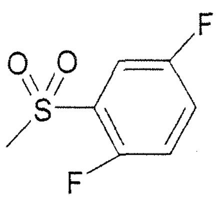 | 596.4 | 4.03 | SA |
| N19 | 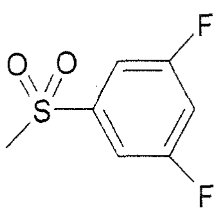 | 596.4 | 4.11 | SA |
| N20 | 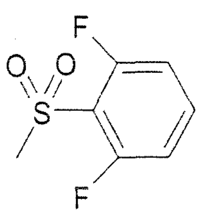 | 596.4 | 3.93 | SA |

(continued)

| Ex. No. | - B | obs MS [M+1] | tR (min) | Measurement Method |
|---|---|---|---|---|
| N21 | 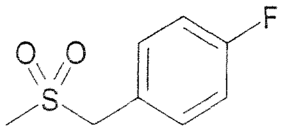 | 592.3 | 3.93 | SA |
| N22 | 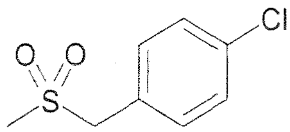 | 608.5 | 4.09 | SA |
| N23 | 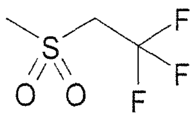 | 566.4 | 3.8 | SA |

[Table 55]

| Ex. No. | - B | obs MS [M+1] | tR (min) | Measurement Method |
|---|---|---|---|---|
| O1 | 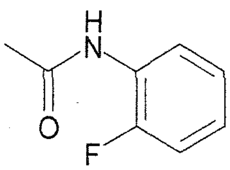 | 557.7 | 3.8 | SB |
| O2 | 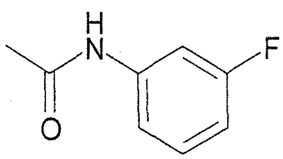 | 557.7 | 3.92 | SB |
| O3 | 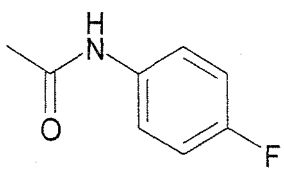 | 557.4 | 3.88 | SB |
| O4 | 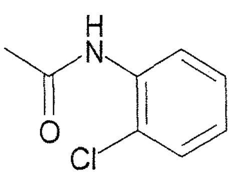 | 573.7 | 3.97 | SB |
| O5 | 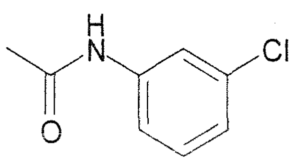 | 573.7 | 4.03 | SB |

(continued)

| Ex. No. | - B | obs MS [M+1] | tR (min) | Measurement Method |
|---|---|---|---|---|
| O6 | 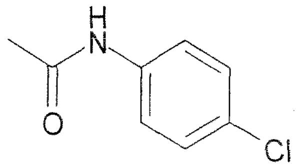 | 573.7 | 4.03 | SB |
| O7 | 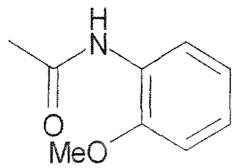 | 569.8 | 3.9 | SB |
| O8 | 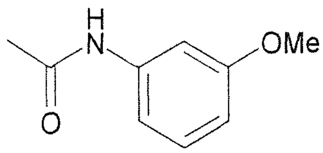 | 569.8 | 3.78 | SB |

[Table 56]

| Ex. No. | - B | obs MS [M+1] | tR (min) | Measurement Method |
|---|---|---|---|---|
| 09 | 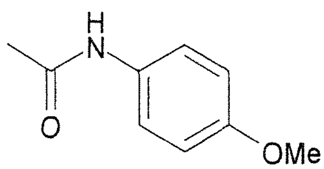 | 569.8 | 3.74 | SB |
| O10 | 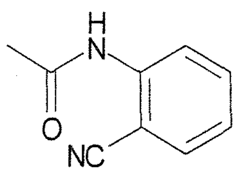 | 564.7 | 3.67 | SB |
| O11 | 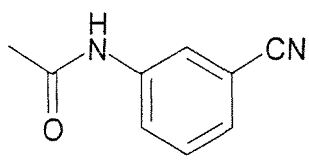 | 564.7 | 3.8 | SB |
| O12 | 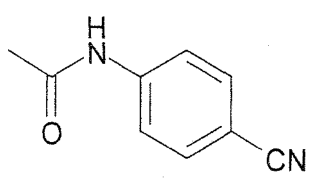 | 564.7 | 3.8 | SB |

(continued)

| Ex. No. | - B | obs MS [M+1] | tR (min) | Measurement Method |
|---|---|---|---|---|
| O13 | 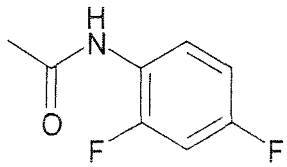 | 575.7 | 3.82 | SB |
| O14 | 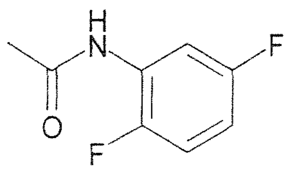 | 575.7 | 3.95 | SB |
| O15 | 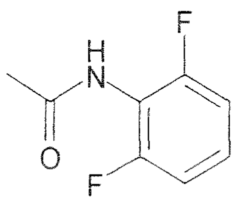 | 575.7 | 3.69 | SB |
| O16 | 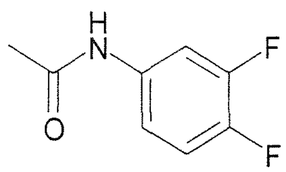 | 575.7 | 3.99 | SB |

[Table 57]

| Ex. No. | - B | obs MS [M+1] | tR (min) | Measurement Method |
|---|---|---|---|---|
| O17 | 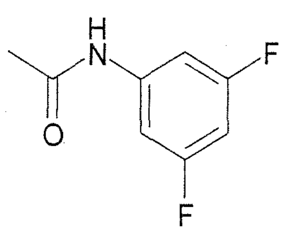 | 575.7 | 4.05 | SB |
| O18 | 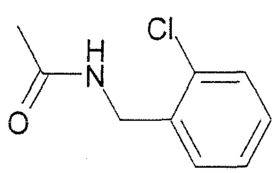 | 587.4 | 3.32 | SB |
| O19 | 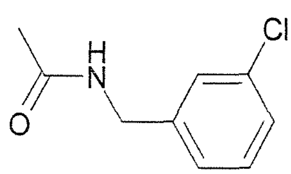 | 587.4 | 3.4 | SB |

(continued)

| Ex. No. | - B | obs MS [M+1] | tR (min) | Measurement Method |
|---|---|---|---|---|
| O20 | 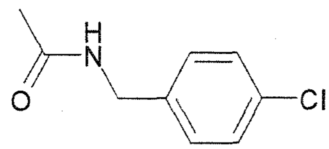 | 587.4 | 3.36 | SB |
| O21 | 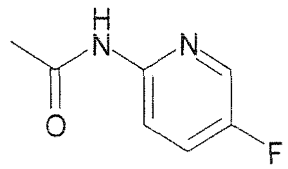 | 557.4 | 3.34 | SB |
| O22 | 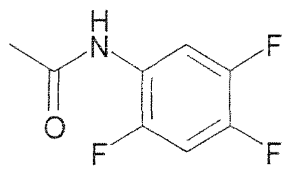 | 593.6 | 3.38 | SB |
| O23 | 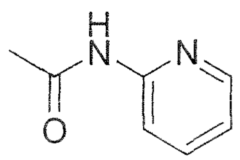 | 540.4 | 2.48 | SB |

[Table 58]

| Ex. No. | -B | obs MS [M+1] | tR (min) | Measurement Method |
|---|---|---|---|---|
| P1 | 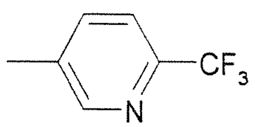 | 565.6 | 3.67 | SB |
| P2 | 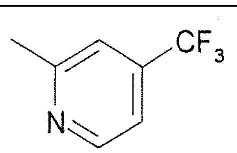 | 565.6 | 3.15 | SB |
| P3 | 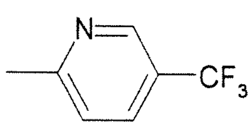 | 565.6 | 3.36 | SB |
| P4 | 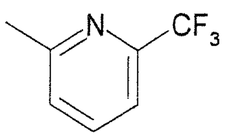 | 565.6 | 4.07 | SB |

(continued)

| Ex. No. | -B | obs MS [M+1] | tR (min) | Measurement Method |
|---|---|---|---|---|
| P5 | 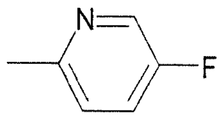 | 515.4 | 2.71 | SB |
| P6 | 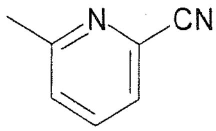 | 522.2 | 3.63 | SB |
| P7 | 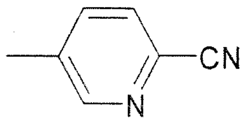 | 522.2 | 3.40 | SB |
| P8 | 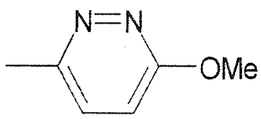 | 528.3 | 2.44 | SB |

[Table 59]

| Ex. No. | -B | obs MS [M+1] | tR (min) | Measurement Method |
|---|---|---|---|---|
| P9 | 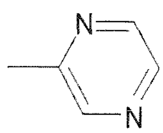 | 498.3 | 2.94 | SB |
| P10 | 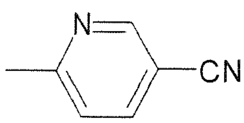 | 522.4 | 3.36 | SB |

[Chemical formula 115]

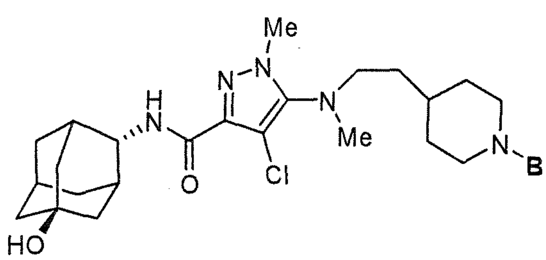

[Table 60]

| Ex. No. | - B | obs MS [M+1] | tR (min) | Measurement Method |
|---|---|---|---|---|
| Q1 | 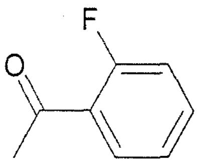 | 572.6 | 3.87 | SA |
| Q2 | 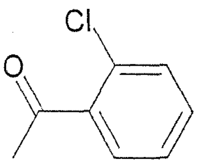 | 588.6 | 4.11 | SA |
| Q3 | 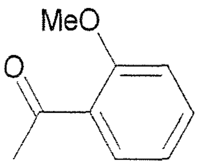 | 584.5 | 3.8 | SA |
| Q4 | 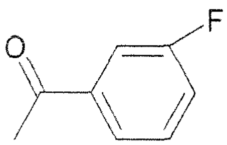 | 572.6 | 3.9 | SA |
| Q5 | 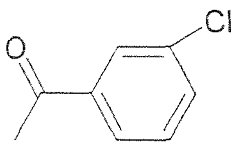 | 588.6 | 4.07 | SA |
| Q6 | 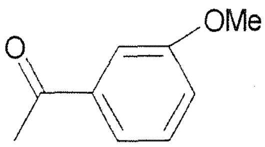 | 584.5 | 3.85 | SA |
| Q7 | 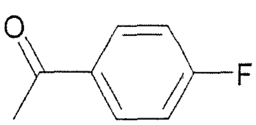 | 572.5 | 3.88 | SA |
| Q8 | 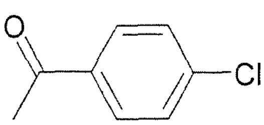 | 588.6 | 4.06 | SA |

[Table 61]

| Ex. No. | -B | obs MS [M+1] | tR (min) | Measurement Method |
|---|---|---|---|---|
| Q9 | 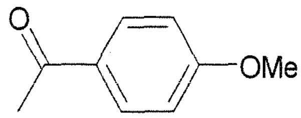 | 584.5 | 3.82 | SA |
| Q10 | 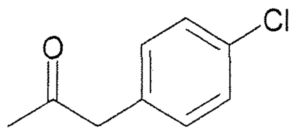 | 602.6 | 4.08 | SA |
| Q11 | 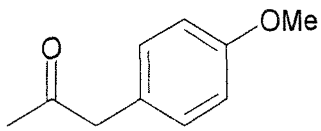 | 598.6 | 3.83 | SA |
| Q12 | 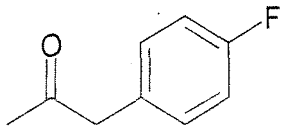 | 586.6 | 3.91 | SA |
| Q13 | 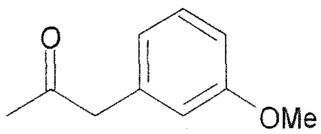 | 598.7 | 3.84 | SA |
| Q14 | 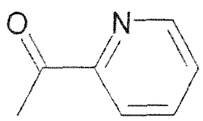 | 555.5 | 3.35 | SA |
| Q15 | 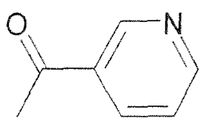 | 555.5 | 3.06 | SA |
| Q16 | 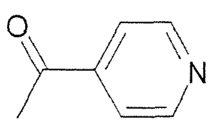 | 555.5 | 3 | SA |

[Table 62]

| Ex. No. | -B | obs MS [M+1] | tR (min) | Measurement Method |
|---|---|---|---|---|
| Q17 | 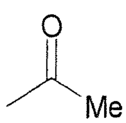 | 492.7 | 3.34 | SA |

(continued)

| Ex. No. | -B | obs MS [M+1] | tR (min) | Measurement Method |
|---|---|---|---|---|
| Q18 | 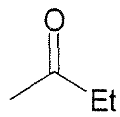 | 506.7 | 3.52 | SA |
| Q19 | 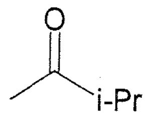 | 520.7 | 3.68 | SA |
| Q20 | 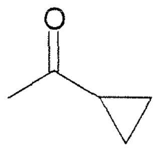 | 518.7 | 3.61 | SA |

[Table 63]

| Ex. No. | -B | obs MS [M+1] | tR (min) | Measurement Method |
|---|---|---|---|---|
| R1 | 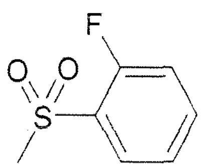 | 608.3 | 4.17 | SA |
| R2 | 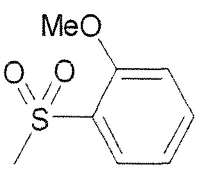 | 620.4 | 4.04 | SA |
| R3 | 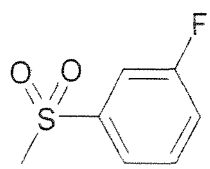 | 608.4 | 4.23 | SA |
| R4 | 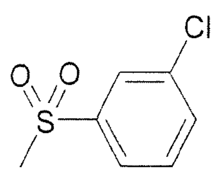 | 624.4 | 4.4 | SA |
| R5 | 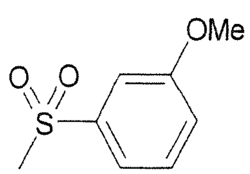 | 620.5 | 4.19 | SA |

(continued)

| Ex. No. | -B | obs MS [M+1] | tR (min) | Measurement Method |
|---|---|---|---|---|
| R6 | 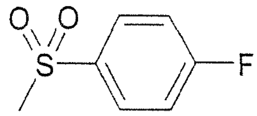 | 608.3 | 4.21 | SA |
| R7 | 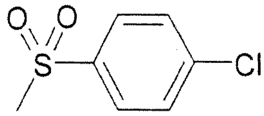 | 624.5 | 4.41 | SA |
| R8 | 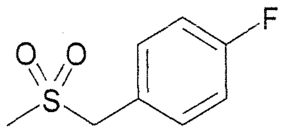 | 622.6 | 4.15 | SA |

[Table 64]

| Ex. No. | - B | obs MS [M+1] | tR (min) | Measurement Method |
|---|---|---|---|---|
| R9 | 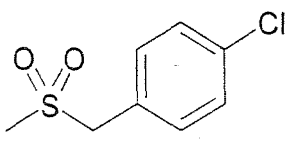 | 638.4 | 4.31 | SA |
| R10 | 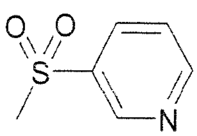 | 591.4 | 3.72 | SA |
| R11 | 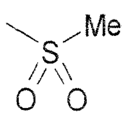 | 528.6 | 3.61 | SA |
| R12 | 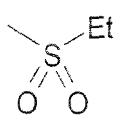 | 542.5 | 3.74 | SA |
| R13 | 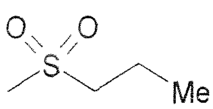 | 556.5 | 3.94 | SA |
| R14 | 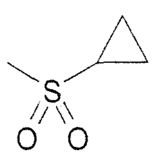 | 554.4 | 3.77 | SA |

(continued)

| Ex. No. | - B | obs MS [M+1] | tR (min) | Measurement Method |
|---|---|---|---|---|
| R15 | 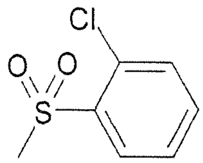 | 624.4 | 4.29 | SA |
| R16 | 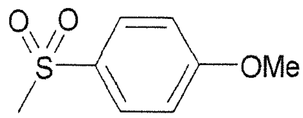 | 620.5 | 4.15 | SA |

[Table 65]

| Ex. No. | - B | obs MS [M+1] | tR (min) | Measurement Method |
|---|---|---|---|---|
| R17 | 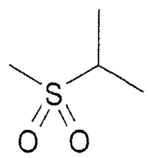 | 556.5 | 3.83 | SA |

[Table 66]

| Ex. No. | -B | obs MS [M+1] | tR (min) | Measurement Method |
|---|---|---|---|---|
| S1 | 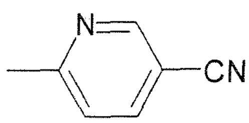 | 552.4 | 3.74 | SB |
| S2 | 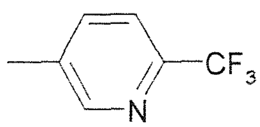 | 595.5 | 3.97 | SB |
| S3 | 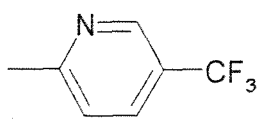 | 595.5 | 3.69 | SB |
| S4 | 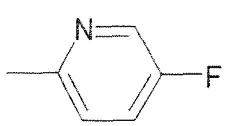 | 545.4 | 2.90 | SB |
| S5 | 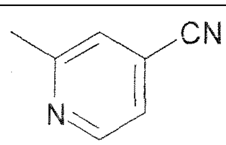 | 552.4 | 3.34 | SB |

(continued)

| Ex. No. | -B | obs MS [M+1] | tR (min) | Measurement Method |
|---|---|---|---|---|
| S6 | 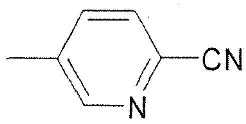 | 552.4 | 3.57 | SB |
| S7 | 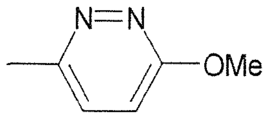 | 558.3 | 2.61 | SB |

[Chemical formula 116]

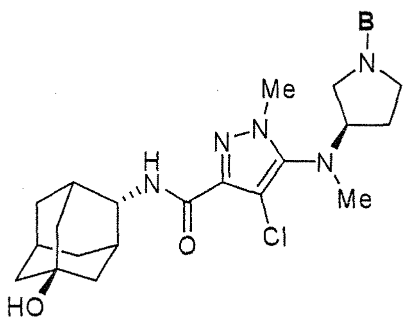

[Table 67]

| Ex. No. | - B | obs MS [M+1] | tR (min) | Measurement Method |
|---|---|---|---|---|
| U1 | 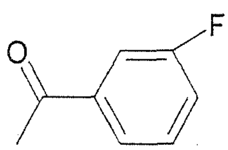 | 530.5 | 3.43 | SA |
| U2 | 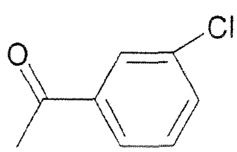 | 546.5 | 3.56 | SA |
| U3 | 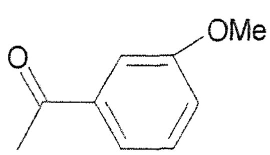 | 542.5 | 3.39 | SA |
| U4 | 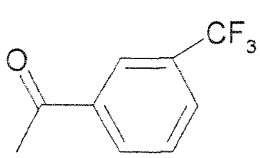 | 580.4 | 3.72 | SA |

(continued)

| Ex. No. | - B | obs MS [M+1] | tR (min) | Measurement Method |
|---|---|---|---|---|
| U5 | 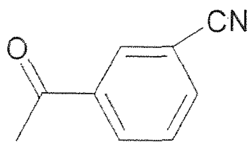 | 537.6 | 3.31 | SA |
| U6 | 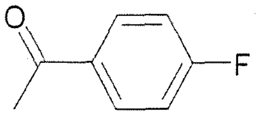 | 530.5 | 3.41 | SA |
| U7 | 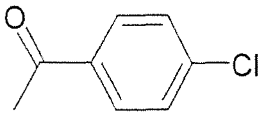 | 546.6 | 3.58 | SA |
| U8 | 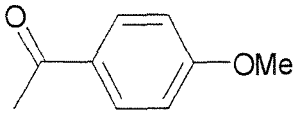 | 542.6 | 3.37 | SA |

[Table 68]

| Ex. No. | -B | obs MS [M+1] | tR (min) | Measurement Method |
|---|---|---|---|---|
| U9 | 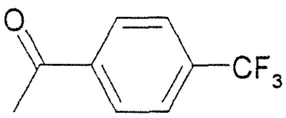 | 580.4 | 3.73 | SA |
| U10 | 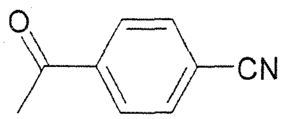 | 537.6 | 3.31 | SA |
| U11 | 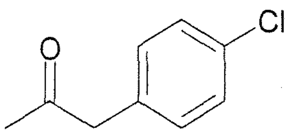 | 560.6 | 3.64 | SA |
| U12 | 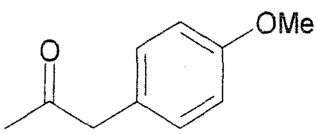 | 556.7 | 3.46 | SA |
| U13 | 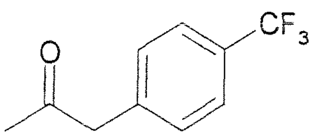 | 594.5 | 3.82 | SA |

(continued)

| Ex. No. | -B | obs MS [M+1] | tR (min) | Measurement Method |
|---|---|---|---|---|
| U14 | 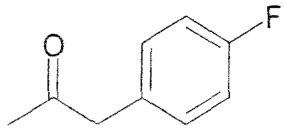 | 544.6 | 3.48 | SA |
| U15 | 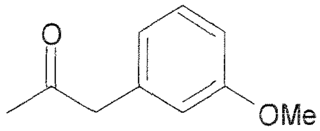 | 556.7 | 3.46 | SA |
| U16 | 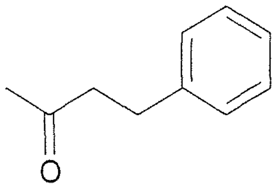 | 540.6 | 3.6 | SA |

[Table 69]

| Ex. No. | - B | obs MS [M+1] | tR (min) | Measurement Method |
|---|---|---|---|---|
| U17 | 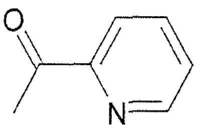 | 513.7 | 3.07 | SA |
| U18 | 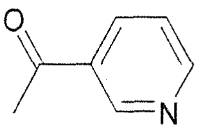 | 513.7 | 2.73 | SA |
| U19 | 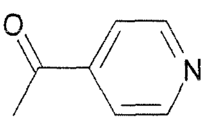 | 513.7 | 2.68 | SA |
| U20 | 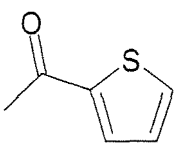 | 518.6 | 3.35 | SA |
| U21 | 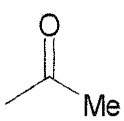 | 450.5 | 2.92 | SA |
| U22 | 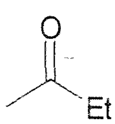 | 464.5 | 3.08 | SA |

(continued)

| Ex. No. | - B | obs MS [M+1] | tR (min) | Measurement Method |
|---|---|---|---|---|
| U23 | 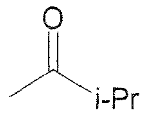 | 478.5 | 3.21 | SA |
| U24 | 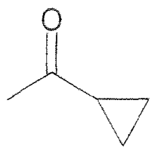 | 476.3 | 3.16 | SA |

[Table 70]

| Ex. No. | - B | obs MS [M+1] | tR (min) | Measurement Method |
|---|---|---|---|---|
| U25 | 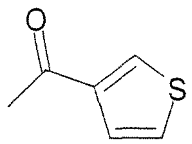 | 518.6 | 3.3 | SA |
| U26 | 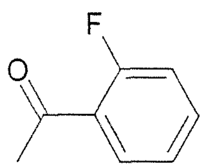 | 530.5 | 3.42 | SA |
| U27 | 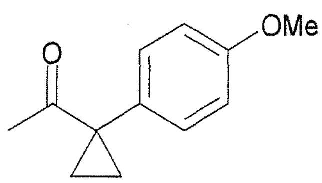 | 582.5 | 3.62 | SA |
| U28 | 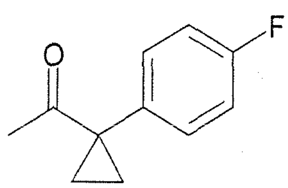 | 570.6 | 3.68 | SA |
| U29 | 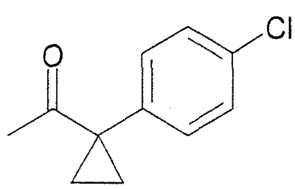 | 586.5 | 3.91 | SA |
| U30 | 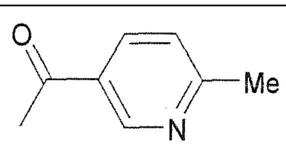 | 527.7 | 2.7 | SA |

(continued)

| Ex. No. | - B | obs MS [M+1] | tR (min) | Measurement Method |
|---|---|---|---|---|
| U31 | 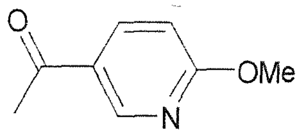 | 543.7 | 3.24 | SA |
| U32 | 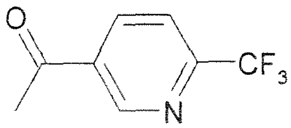 | 581.5 | 3.51 | SA |

[Table 71]

| Ex. No. | - B | obs MS [M+1] | tR (min) | Measurement Method |
|---|---|---|---|---|
| U33 | 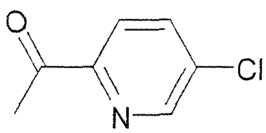 | 547.7 | 3.49 | SA |
| U34 | 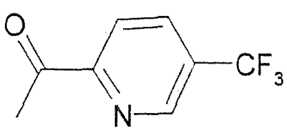 | 581.5 | 3.65 | SA |
| U35 | 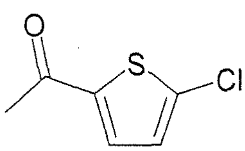 | 552.4 | 3.73 | SA |
| U36 | 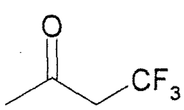 | 518.7 | 3.33 | SA |

[Table 72]

| Ex. No. | -B | obs MS [M+1] | tR (min) | Measurement Method |
|---|---|---|---|---|
| V1 | 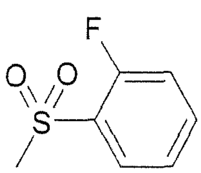 | 566.4 | 3.72 | SA |

(continued)

| Ex. No. | -B | obs MS [M+1] | tR (min) | Measurement Method |
|---|---|---|---|---|
| V2 | 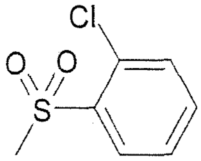 | 582.3 | 3.85 | SA |
| V3 | 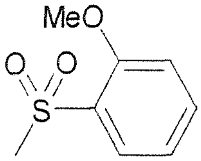 | 578.4 | 3.61 | SA |
| V4 | 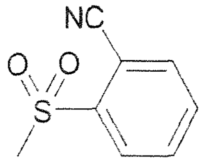 | 573.6 | 3.62 | SA |
| V5 | 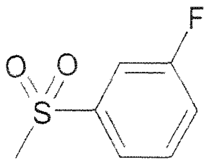 | 566.5 | 3.78 | SA |
| V6 | 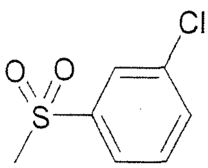 | 582.1 | 3.92 | SA |
| V7 | 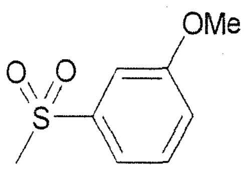 | 578.5 | 3.74 | SA |

[Table 73]

| Ex. No. | -B | obs MS [M+1] | tR (min) | Measurement Method |
|---|---|---|---|---|
| V9 | 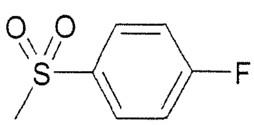 | 566.4 | 3.76 | SA |
| V10 | 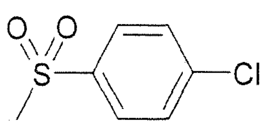 | 582.3 | 3.94 | SA |

(continued)

| Ex. No. | -B | obs MS [M+1] | tR (min) | Measurement Method |
|---|---|---|---|---|
| V11 | 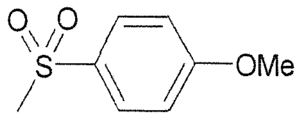 | 578.5 | 3.7 | SA |
| V12 | 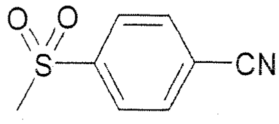 | 573.5 | 3.68 | SA |
| V13 | 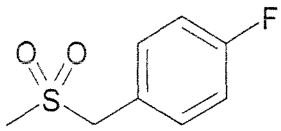 | 580.4 | 3.66 | SA |
| V14 | 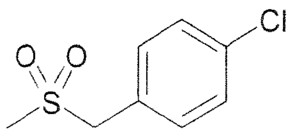 | 596.3 | 3.9 | SA |
| V15 | 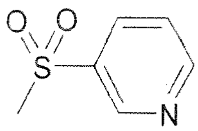 | 549.5 | 3.31 | SA |
| V16 | 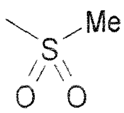 | 486.5 | 3.13 | SA |

[Table 74]

| Ex. No. | -B | obs MS [M+1] | tR (min) | Measurement Method |
|---|---|---|---|---|
| V17 | 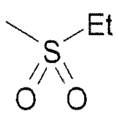 | 514.5 | 3.45 | SA |
| V18 | 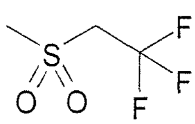 | 554.5 | 3.64 | SA |
| V19 | 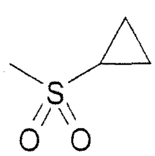 | 512.4 | 3.33 | SA |

(continued)

| Ex. No. | -B | obs MS [M+1] | tR (min) | Measurement Method |
|---|---|---|---|---|
| V20 | 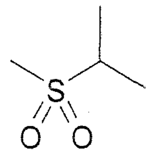 | 514.5 | 3.41 | SA |
| V21 | 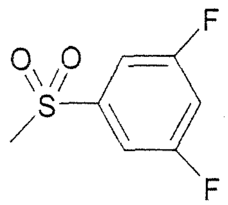 | 584.4 | 3.89 | SA |
| V22 | 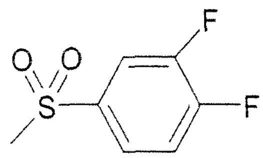 | 584.4 | 3.87 | SA |
| V23 | 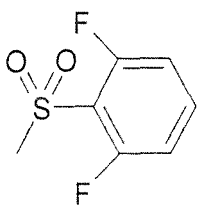 | 584.4 | 3.72 | SA |

[Table 75]

| Ex. No. | - B | obs MS [M+1] | tR (min) | Measurement Method |
|---|---|---|---|---|
| W1 | 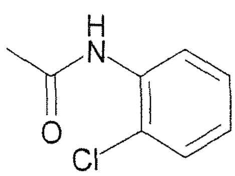 | 561.7 | 3.84 | SB |
| W2 | 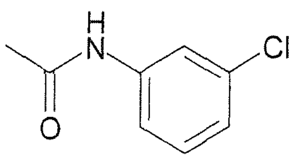 | 561.7 | 3.9 | SB |
| W3 | 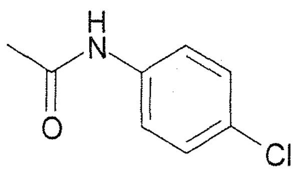 | 561.7 | 3.86 | SB |

(continued)

| Ex. No. | - B | obs MS [M+1] | tR (min) | Measurement Method |
|---|---|---|---|---|
| W4 | 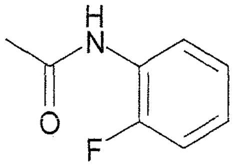 | 545.7 | 3.65 | SB |
| W5 | 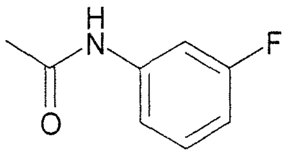 | 545.7 | 3.76 | SB |
| W6 | 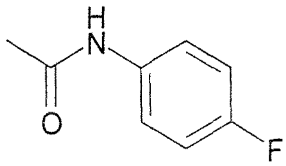 | 545.4 | 3.18 | SB |
| W7 | 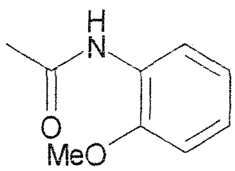 | 557.4 | 3.17 | SB |
| W8 | 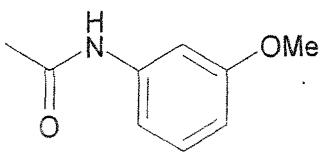 | 557.4 | 3.07 | SB |

[Table 76]

| Ex. No. | - B | obs MS [M+1] | tR (min) | Measurement Method |
|---|---|---|---|---|
| W9 | 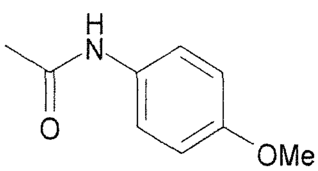 | 557.4 | 3.01 | SB |
| W10 | 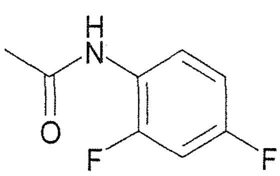 | 563.3 | 3.11 | SB |

(continued)

| Ex. No. | - B | obs MS [M+1] | tR (min) | Measurement Method |
|---|---|---|---|---|
| W11 | 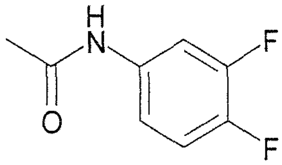 | 563.3 | 3.26 | SB |
| W12 | 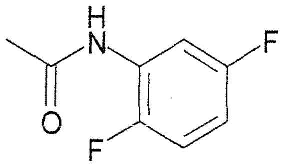 | 563.3 | 3.19 | SB |
| W13 | 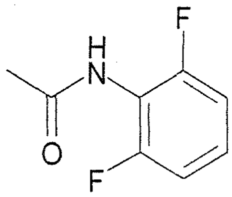 | 563.3 | 2.96 | SB |
| W14 | 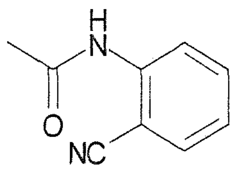 | 552.1 | 2.96 | SB |
| W15 | 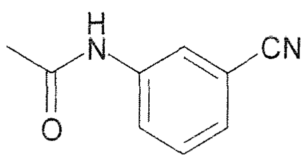 | 552.1 | 3.19 | SB |
| W16 | 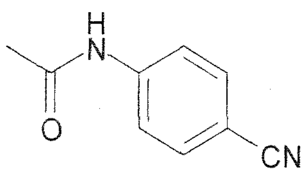 | 552.1 | 3.11 | SB |

[Table 77]

| Ex. No. | - B | obs MS [M+1] | tR (min) | Measurement Method |
|---|---|---|---|---|
| W17 | 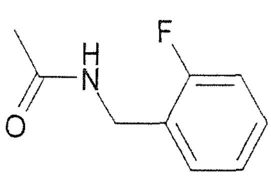 | 559.4 | 3.09 | SB |

(continued)

| Ex. No. | - B | obs MS [M+1] | tR (min) | Measurement Method |
|---|---|---|---|---|
| W18 | 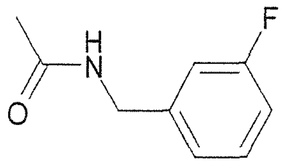 | 559.4 | 3.07 | SB |
| W19 | 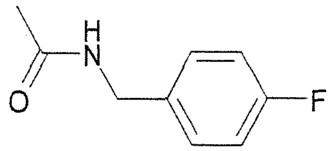 | 559.4 | 3.15 | SB |
| W20 | 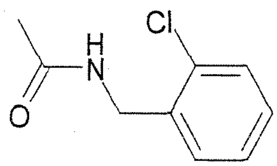 | 575.4 | 3.18 | SB |
| W21 | 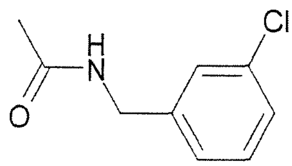 | 575.4 | 3.26 | SB |
| W22 | 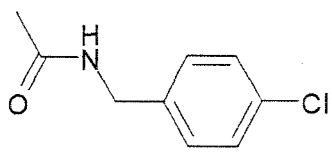 | 575.4 | 3.24 | SB |
| W23 | 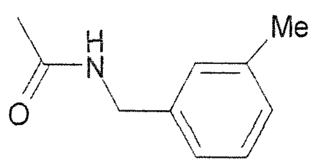 | 555.2 | 3.17 | SB |
| W24 | 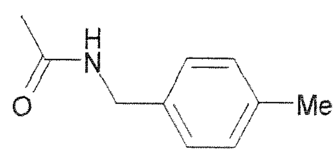 | 555.2 | 3.19 | SB |

[Table 78]

| Ex. No. | - B | obs MS [M+1] | tR (min) | Measurement Method |
|---|---|---|---|---|
| W25 | 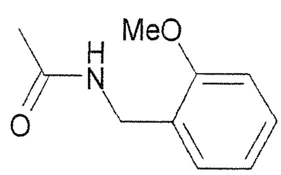 | 571.5 | 3.11 | SB |

(continued)

| Ex. No. | - B | obs MS [M+1] | tR (min) | Measurement Method |
|---|---|---|---|---|
| W26 | 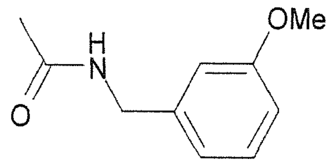 | 571.5 | 3.07 | SB |
| W27 | 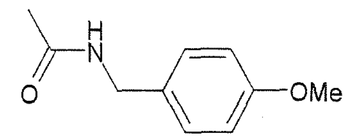 | 571.5 | 3.03 | SB |
| W28 | 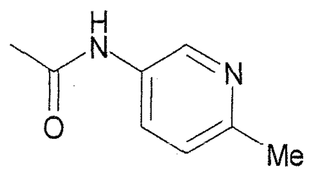 | 542.3 | 2.38 | SB |
| W29 | 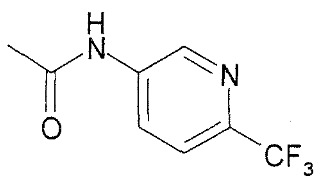 | 596.4 | 3.17 | SB |
| W30 | 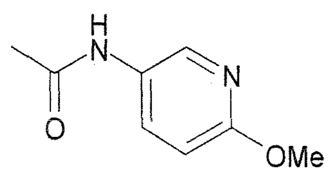 | 558.3 | 2.69 | SB |
| W31 | 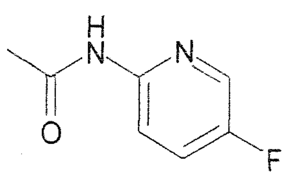 | 546.2 | 2.8 | SB |
| W32 | 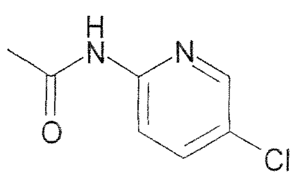 | 562.2 | 2.99 | SB |

[Table 79]

| Ex. No. | -B | obs MS [M+1] | tR (min) | Measurement Method |
|---|---|---|---|---|
| W33 | 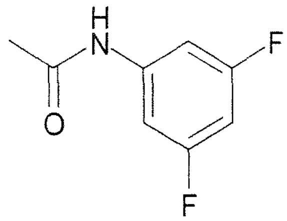 | 563.3 | 3.34 | SB |
| W34 | 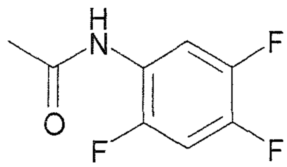 | 581.3 | 3.24 | SB |
| W35 | 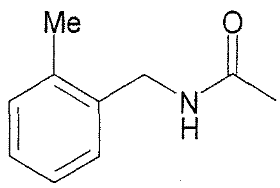 | 555.2 | 3.13 | SB |
| W37 | 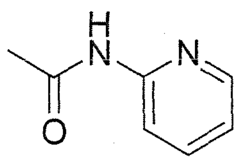 | 528.3 | 2.38 | SB |

[Table 80]

| Ex. No. | - B | obs MS [M+1] | tR (min) | Measurement Method |
|---|---|---|---|---|
| X1 | 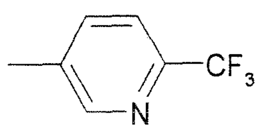 | 553.2 | 3.63 | SB |
| X2 | 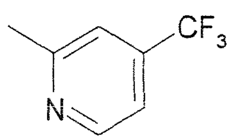 | 553.2 | 2.84 | SB |
| X3 | 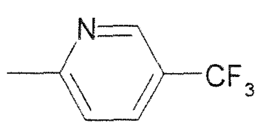 | 553.2 | 2.99 | SB |
| X4 | 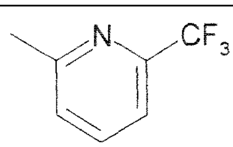 | 553.2 | 3.86 | SB |

(continued)

| Ex. No. | - B | obs MS [M+1] | tR (min) | Measurement Method |
|---|---|---|---|---|
| X5 | 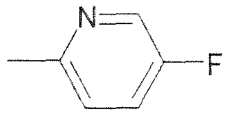 | 503.4 | 2.44 | SB |
| X6 | 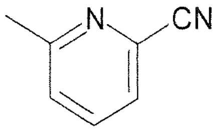 | 510.4 | 3.42 | SB |
| X7 | 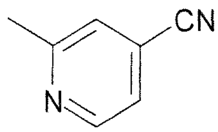 | 510.4 | 2.78 | SB |
| X8 | 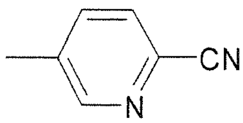 | 510.4 | 3.19 | SB |

[Table 81]

| Ex. No. | -B | obs MS [M+1] | tR (min) | Measurement Method |
|---|---|---|---|---|
| X9 | 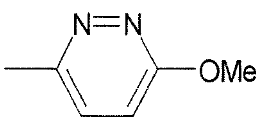 | 516.6 | 2.34 | SB |
| X10 | 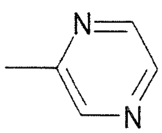 | 486.6 | 2.71 | SB |
| X11 | 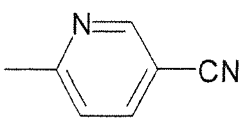 | 510.4 | 3.05 | SB |

[Chemical formula 117]

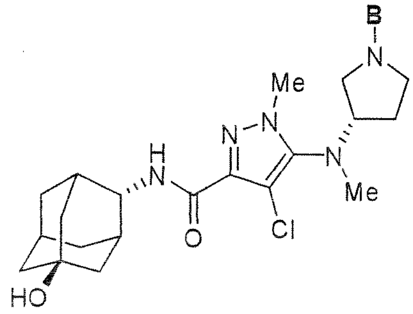

[Table 82]

| Ex. No. | - B | obs MS [M+1] | tR (min) | Measurement Method |
|---|---|---|---|---|
| Y1 | 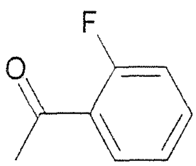 | 530.5 | 3.4 | SA |
| Y2 | 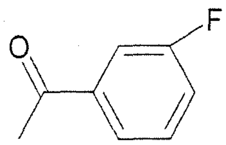 | 530.5 | 3.42 | SA |
| Y3 | 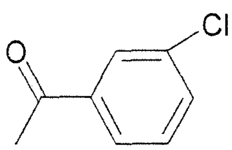 | 546.6 | 3.58 | SA |
| Y4 | 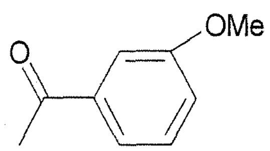 | 542.5 | 3.38 | SA |
| Y5 | 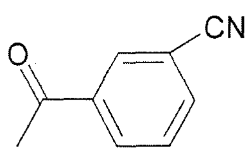 | 537.5 | 3.31 | SA |
| Y6 | 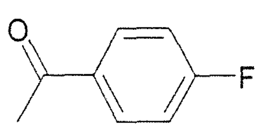 | 530.5 | 3.41 | SA |

(continued)

| Ex. No. | - B | obs MS [M+1] | tR (min) | Measurement Method |
|---|---|---|---|---|
| Y7 | 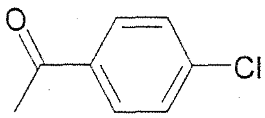 | 546.7 | 3.58 | SA |
| Y8 | 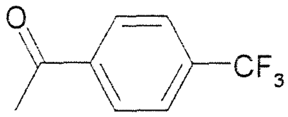 | 580.4 | 3.72 | SA |

[Table 83]

| Ex. No. | -B | obs MS [M+1] | tR (min) | Measurement Method |
|---|---|---|---|---|
| Y9 | 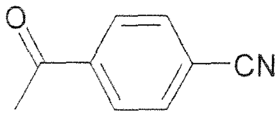 | 537.5 | 3.32 | SA |
| Y10 | 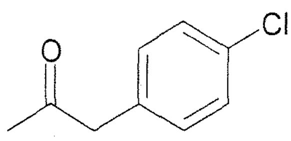 | 560.7 | 3.67 | SA |
| Y11 | 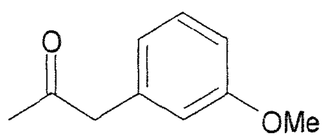 | 556.4 | 3.45 | SA |
| Y12 | 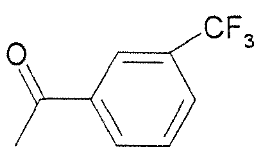 | 580.4 | 3.7 | SA |
| Y13 | 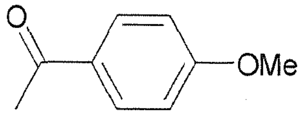 | 542.6 | 3.37 | SA |
| Y14 | 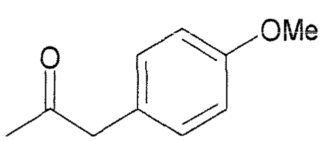 | 556.5 | 3.47 | SA |
| Y15 | 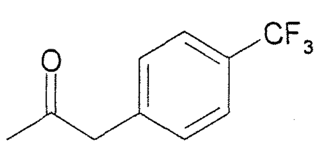 | 594.5 | 3.82 | SA |

(continued)

| Ex. No. | -B | obs MS [M+1] | tR (min) | Measurement Method |
|---|---|---|---|---|
| Y16 | 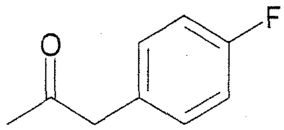 | 544.6 | 3.5 | SA |

[Table 84]

| Ex. No. | -B | obs MS [M+1] | tR (min) | Measurement Method |
|---|---|---|---|---|
| Y17 | 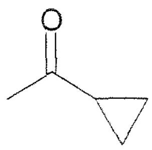 | 476.5 | 3.16 | SA |
| Y18 | 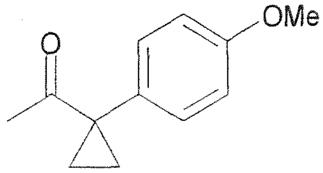 | 582.5 | 3.6 | SA |
| Y19 | 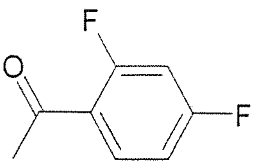 | 548.6 | 3.49 | SA |
| Y20 | 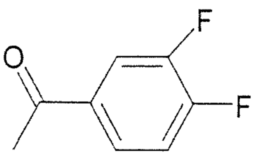 | 548.5 | 3.53 | SA |
| Y21 | 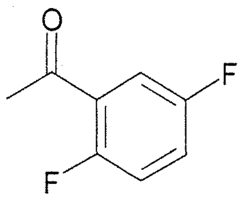 | 548.6 | 3.54 | SA |
| Y22 | 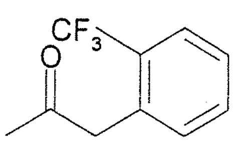 | 594.4 | 3.78 | SA |
| Y23 | 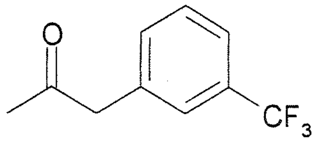 | 594.4 | 3.82 | SA |

[Table 85]

| Ex. No. | -B | obs MS [M+1] | tR (min) | Measurement Method |
|---|---|---|---|---|
| Y25 | 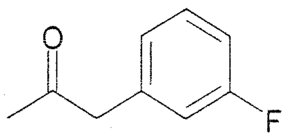 | 544.5 | 3.59 | SA |
| Y26 | 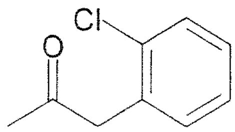 | 560.6 | 3.64 | SA |
| Y27 | 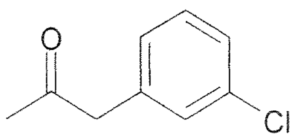 | 560.6 | 3.67 | SA |
| Y28 | 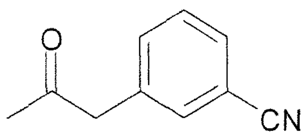 | 551.7 | 3.39 | SA |
| Y29 | 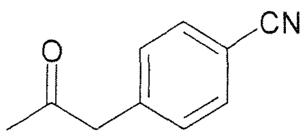 | 551.5 | 3.38 | SA |
| Y30 | 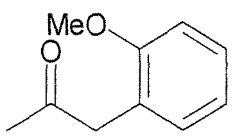 | 556.5 | 3.52 | SA |
| Y31 | 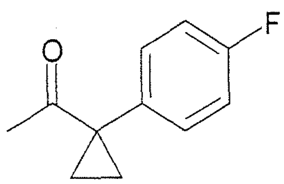 | 570.4 | 3.64 | SA |
| Y32 | 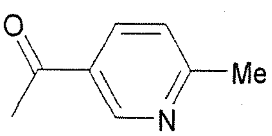 | 527.7 | 2.69 | SA |

[Table 86]

| Ex. No. | - B | obs MS [M+1] | tR (min) | Measurement Method |
|---|---|---|---|---|
| Y33 | 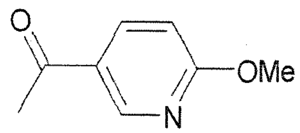 | 543.6 | 3.2 | SA |
| Y34 | 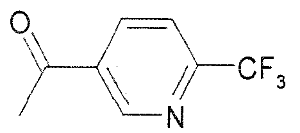 | 581.5 | 3.48 | SA |
| Y35 | 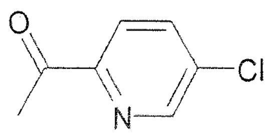 | 547.6 | 3.45 | SA |
| Y36 | 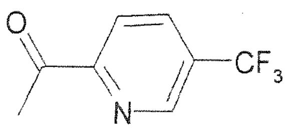 | 581.5 | 3.64 | SA |
| Y37 | 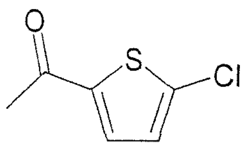 | 552.4 | 3.72 | SA |
| Y38 | 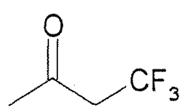 | 518.6 | 3.32 | SA |

[Table 87]

| Ex. No. | -B | obs MS [M+1] | tR (min) | Measurement Method |
|---|---|---|---|---|
| Z1 | 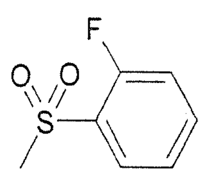 | 566.5 | 3.72 | SA |
| Z2 | 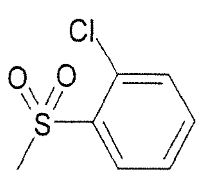 | 582.3 | 3.85 | SA |

(continued)

| Ex. No. | -B | obs MS [M+1] | tR (min) | Measurement Method |
|---|---|---|---|---|
| Z3 | 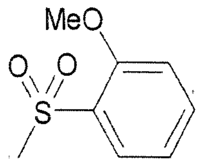 | 578.3 | 3.62 | SA |
| Z4 | 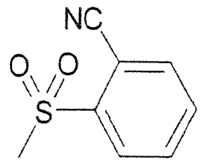 | 573.5 | 3.63 | SA |
| Z5 | 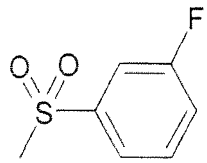 | 566.5 | 3.79 | SA |
| Z6 | 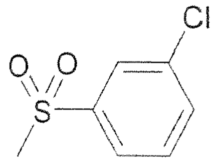 | 582.4 | 3.93 | SA |
| Z7 | 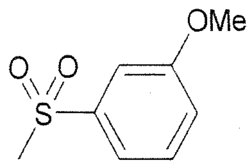 | 578.5 | 3.76 | SA |

[Table 88]

| Ex. No. | - B | obs MS [M+1] | tR (min) | Measurement Method |
|---|---|---|---|---|
| Z9 | 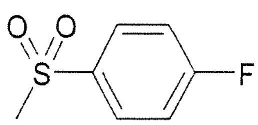 | 566.4 | 3.76 | SA |
| Z10 | 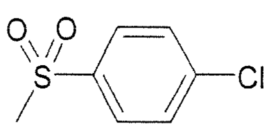 | 582.3 | 3.94 | SA |
| Z11 | 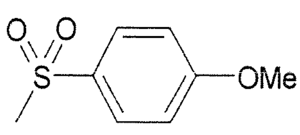 | 578.5 | 3.7 | SA |

(continued)

| Ex. No. | - B | obs MS [M+1] | tR (min) | Measurement Method |
|---|---|---|---|---|
| Z12 | 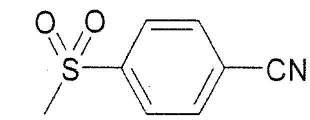 | 573.6 | 3.69 | SA |
| Z13 | 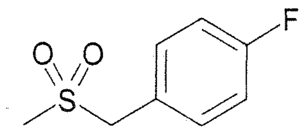 | 580.4 | 3.75 | SA |
| Z14 | 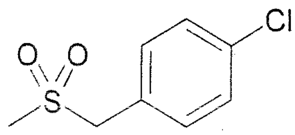 | 596.4 | 3.9 | SA |
| Z15 | 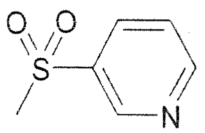 | 549.5 | 3.3 | SA |
| Z16 | 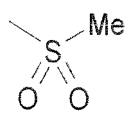 | 486.5 | 3.13 | SA |

[Table 89]

| Ex. No. | - B | obs MS [M+1] | tR (min) | Measurement Method |
|---|---|---|---|---|
| Z17 | 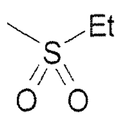 | 500.5 | 3.27 | SA |
| Z18 | 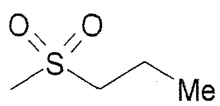 | 514.5 | 3.46 | SA |
| Z19 | 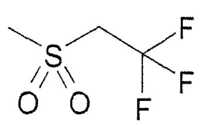 | 554.5 | 3.64 | SA |
| Z20 | 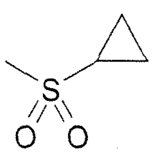 | 512.5 | 3.34 | SA |

(continued)

| Ex. No. | - B | obs MS [M+1] | tR (min) | Measurement Method |
|---|---|---|---|---|
| Z21 | 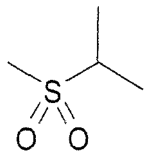 | 514.5 | 3.43 | SA |
| Z22 | 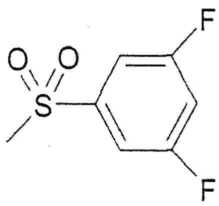 | 584.4 | 3.9 | SA |
| Z23 | 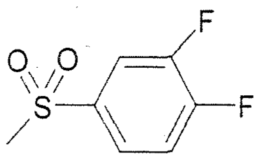 | 584.4 | 3.88 | SA |
| Z24 | 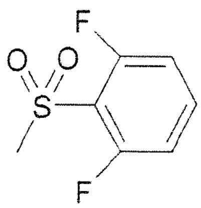 | 584.4 | 3.71 | SA |

[Table 90]

| Ex. No. | - B | obs MS [M+1] | tR (min) | Measurement Method |
|---|---|---|---|---|
| Z25 | 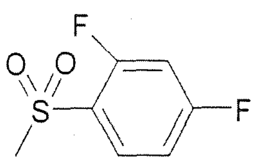 | 584.4 | 3.82 | SA |

[Table 91]

| Ex. No. | -B | obs MS [M+1] | tR (min) | Measurement Method |
|---|---|---|---|---|
| AA1 | 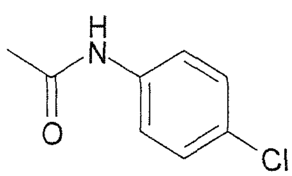 | 561.6 | 3.74 | SA |

(continued)

| Ex. No. | -B | obs MS [M+1] | tR (min) | Measurement Method |
|---|---|---|---|---|
| AA2 | 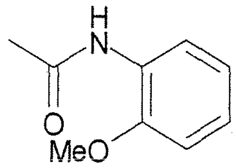 | 557.2 | 3.17 | SB |
| AA3 | 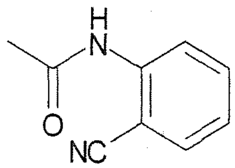 | 552.1 | 2.96 | SB |
| AA4 | 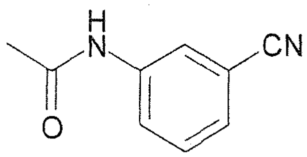 | 552.1 | 3.09 | SB |
| AA5 | 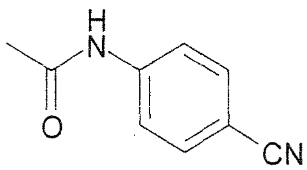 | 552.1 | 3.09 | SB |
| AA6 | 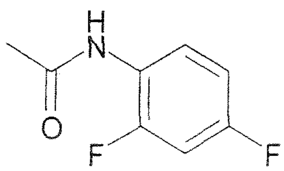 | 563.6 | 3.51 | SA |
| AA7 | 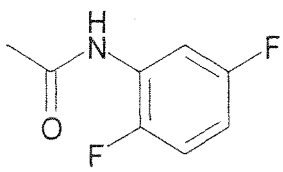 | 563.3 | 3.32 | SB |
| AA8 | 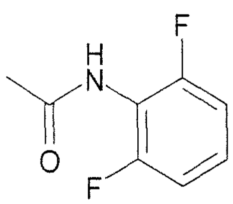 | 563.3 | 2.96 | SB |

[Table 92]

| Ex. No. | -B | obs MS [M+1] | tR (min) | Measurement Method |
|---|---|---|---|---|
| AA9 | 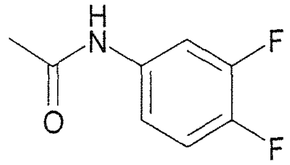 | 563.6 | 3.77 | SA |
| AA10 | 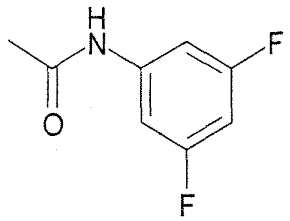 | 563.3 | 3.26 | SB |
| AA11 | 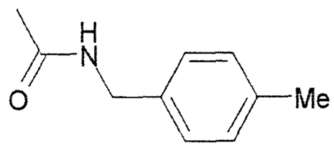 | 555.2 | 3.17 | SB |
| AA12 | 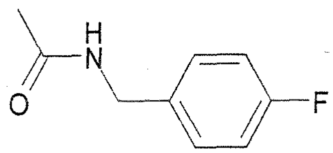 | 559.4 | 3.09 | SB |
| AA13 | 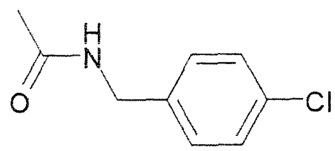 | 575.4 | 3.21 | SB |
| AA14 | 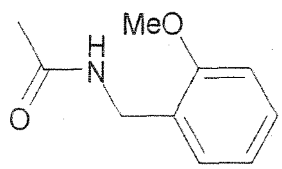 | 571.5 | 3.09 | SB |
| AA15 | 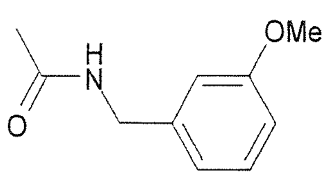 | 571.5 | 3.09 | SB |
| AA16 | 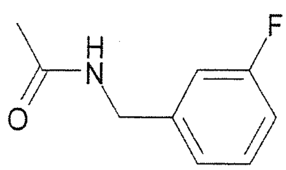 | 559.4 | 3.09 | SB |

[Table 93]

| Ex. No. | - B | obs MS [M+1] | tR (min) | Measurement Method |
|---|---|---|---|---|
| AA17 | 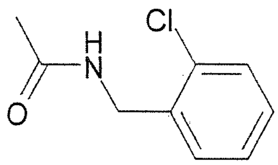 | 575.4 | 3.17 | SB |
| AA18 | 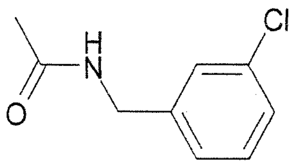 | 575.4 | 3.21 | SB |
| AA19 | 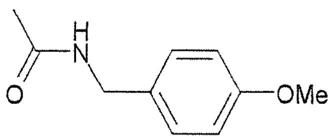 | 571.5 | 3.03 | SB |
| AA20 | 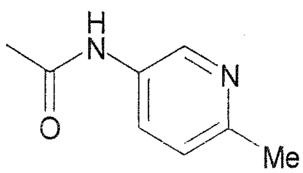 | 542.3 | 2.36 | SB |
| AA21 | 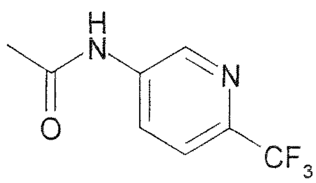 | 596.4 | 3.17 | SB |
| AA22 | 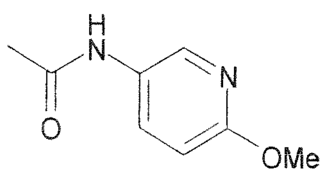 | 558.3 | 2.71 | SB |

[Table 94]

| Ex. No. | -B | obs MS [M+1] | tR (min) | Measurement Method |
|---|---|---|---|---|
| AB1 | 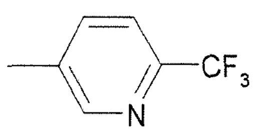 | 553.2 | 3.59 | SB |

(continued)

| Ex. No. | -B | obs MS [M+1] | tR (min) | Measurement Method |
|---|---|---|---|---|
| AB2 | 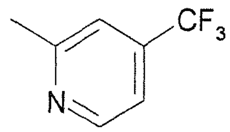 | 553.2 | 2.86 | SB |
| AB3 | 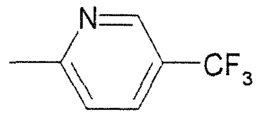 | 553.2 | 2.99 | SB |
| AB4 | 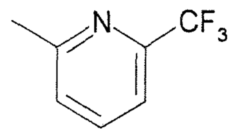 | 553.2 | 3.84 | SB |
| AB5 | 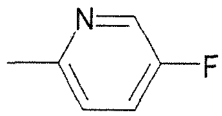 | 503.4 | 2.44 | SB |
| AB6 | 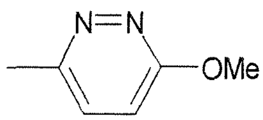 | 516.3 | 2.34 | SB |
| AB7 | 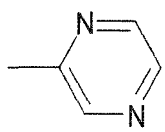 | 486.3 | 2.69 | SB |
| AB8 | 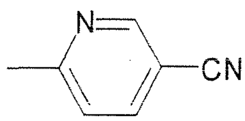 | 510.4 | 3.05 | SB |

[Chemical formula 118]

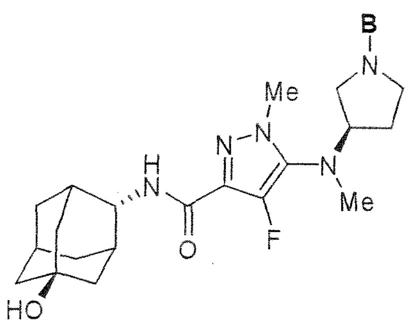

[Table 95]

| Ex. No. | -B | obs MS [M+1] | tR (min) | Measurement Method |
|---|---|---|---|---|
| AC1 | 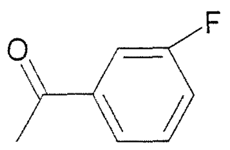 | 514.5 | 3.47 | SA |
| AC2 | 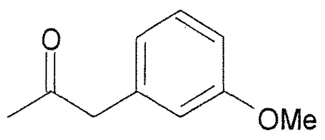 | 540.6 | 3.38 | SA |
| AC3 | 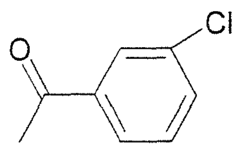 | 530.5 | 3.52 | SA |
| AC4 | 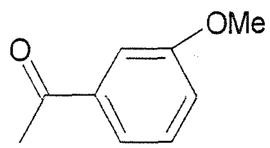 | 526.7 | 3.32 | SA |
| AC5 | 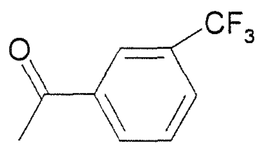 | 564.6 | 3.65 | SA |
| AC6 | 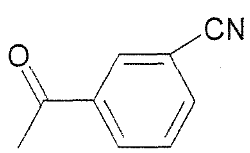 | 521.7 | 3.24 | SA |
| AC7 | 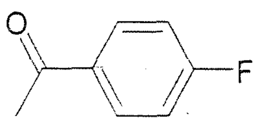 | 514.5 | 3.34 | SA |
| AC8 | 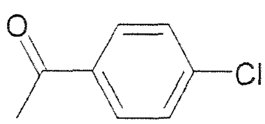 | 530.5 | 3.51 | SA |

[Table 96]

| Ex. No. | - B | obs MS [M+1] | tR (min) | Measurement Method |
|---|---|---|---|---|
| AC9 | 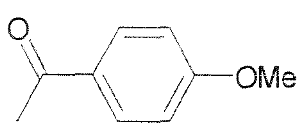 | 526.7 | 3.3 | SA |

(continued)

| Ex. No. | - B | obs MS [M+1] | tR (min) | Measurement Method |
|---|---|---|---|---|
| AC10 | 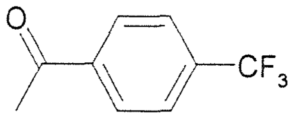 | 564.5 | 3.66 | SA |
| AC11 | 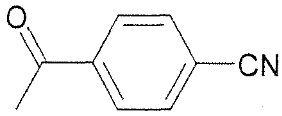 | 521.7 | 3.24 | SA |
| AC12 | 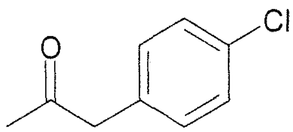 | 544.5 | 3.59 | SA |
| AC13 | 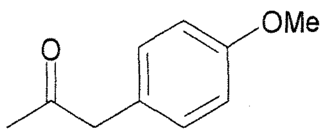 | 540.6 | 3.34 | SA |
| AC14 | 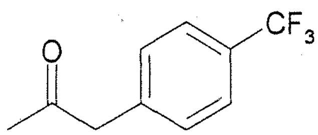 | 578.5 | 3.74 | SA |
| AC15 | 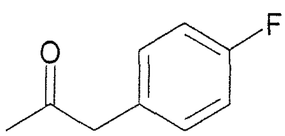 | 528.6 | 3.43 | SA |
| AC16 | 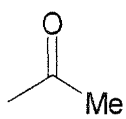 | 434.6 | 2.85 | SA |

[Table 97]

| Ex. No. | - B | obs MS [M+1] | tR (min) | Measurement Method |
|---|---|---|---|---|
| AC17 | 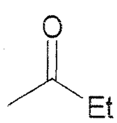 | 448.6 | 3 | SA |
| AC18 | 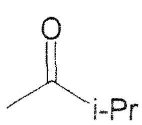 | 462.5 | 3.13 | SA |

(continued)

| Ex. No. | - B | obs MS [M+1] | tR (min) | Measurement Method |
|---|---|---|---|---|
| AC19 | 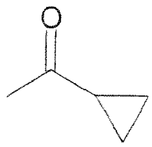 | 460.5 | 3.09 | SA |
| AC20 | 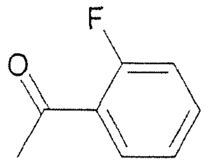 | 514.6 | 3.33 | SA |
| AC21 | 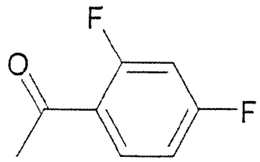 | 532.6 | 3.47 | SA |
| AC22 | 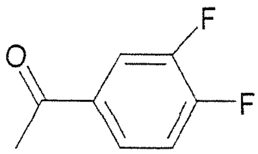 | 532.6 | 3.45 | SA |
| AC23 | 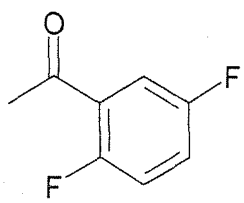 | 532.5 | 3.41 | SA |
| AC24 | 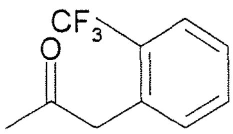 | 578.5 | 3.67 | SA |

[Table 98]

| Ex. No. | -B | obs MS [M+1] | tR (min) | Measurement Method |
|---|---|---|---|---|
| AC25 | 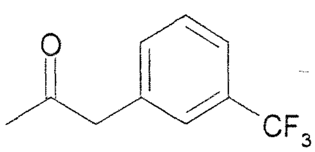 | 578.5 | 3.74 | SA |
| AC26 | 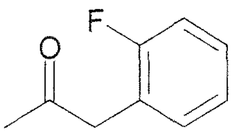 | 528.6 | 3.46 | SA |

(continued)

| Ex. No. | -B | obs MS [M+1] | tR (min) | Measurement Method |
|---|---|---|---|---|
| AC27 | 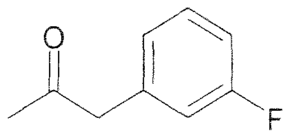 | 528.5 | 3.45 | SA |
| AC28 | 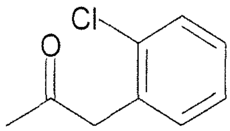 | 544.5 | 3.54 | SA |
| AC29 | 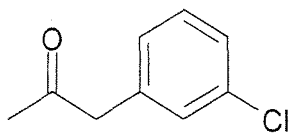 | 544.5 | 3.58 | SA |
| AC30 | 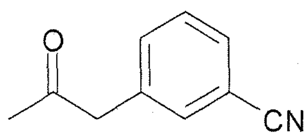 | 535.7 | 3.31 | SA |
| AC31 | 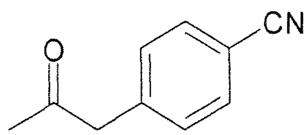 | 535.6 | 3.3 | SA |
| AC32 | 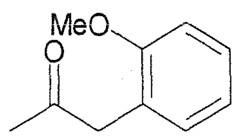 | 540.4 | 3.45 | SA |

[Table 99]

| Ex. No. | - B | obs MS [M+1] | tR (min) | Measurement Method |
|---|---|---|---|---|
| AC33 | 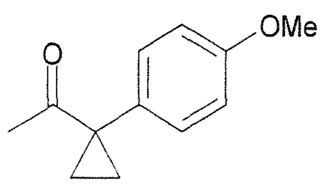 | 566.4 | 3.49 | SA |
| AC34 | 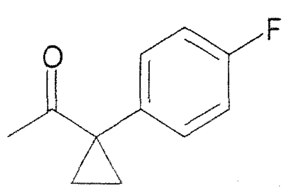 | 554.4 | 3.55 | SA |

(continued)

| Ex. No. | - B | obs MS [M+1] | tR (min) | Measurement Method |
|---|---|---|---|---|
| AC35 | 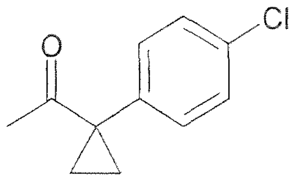 | 570.5 | 3.73 | SA |
| AC36 | 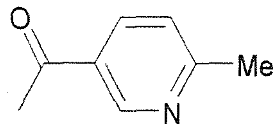 | 511.5 | 2.62 | SA |
| AC37 | 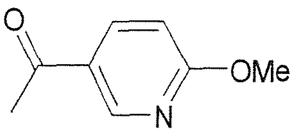 | 527.3 | 3.16 | SA |
| AC38 | 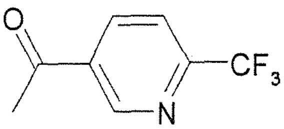 | 565.4 | 3.42 | SA |
| AC39 | 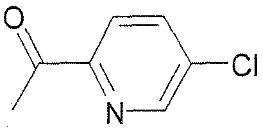 | 531.4 | 3.35 | SA |
| AC40 | 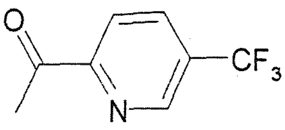 | 565.4 | 3.61 | SA |

[Table 100]

| Ex. No. | - B | obs MS [M+1] | tR (min) | Measurement Method |
|---|---|---|---|---|
| AC41 | 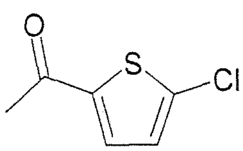 | 536.5 | 3.62 | SA |
| AC42 | 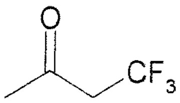 | 502.5 | 3.23 | SA |

[Table 101]

| Ex. No. | - B | obs MS [M+1] | tR (min) | Measurement Method |
|---|---|---|---|---|
| AD1 | 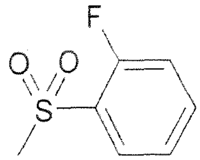 | 550.4 | 3.66 | SA |
| AD2 | 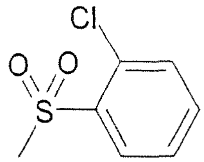 | 566.4 | 3.83 | SA |
| AD3 | 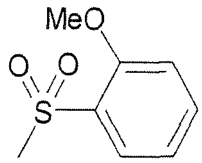 | 562.6 | 3.55 | SA |
| AD4 | 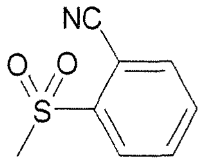 | 557.5 | 3.55 | SA |
| AD5 | 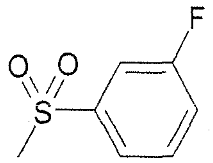 | 550.6 | 3.71 | SA |
| AD6 | 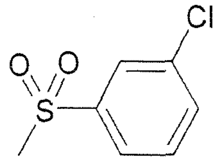 | 566.5 | 3.85 | SA |
| AD7 | 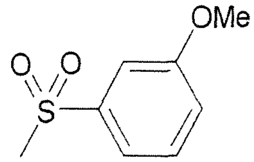 | 562.5 | 3.67 | SA |

[Table 102]

| Ex. No. | -B | obs MS [M+1] | tR (min) | Measurement Method |
|---|---|---|---|---|
| AD9 | 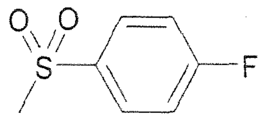 | 550.5 | 3.68 | SA |
| AD10 | 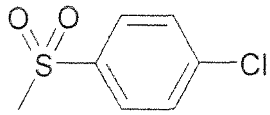 | 566.5 | 3.85 | SA |
| AD11 | 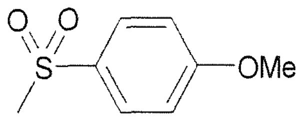 | 562.6 | 3.63 | SA |
| AD 12 | 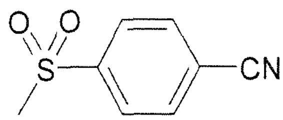 | 557.5 | 3.61 | SA |
| AD13 | 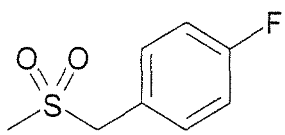 | 564.5 | 3.67 | SA |
| AD14 | 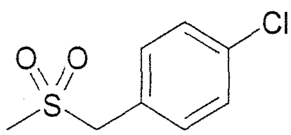 | 580.5 | 3.84 | SA |
| AD15 | 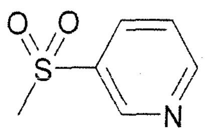 | 533.7 | 3.34 | SA |
| AD16 | 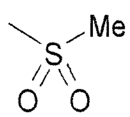 | 470.5 | 3.05 | SA |

[Table 103]

| Ex. No. | -B | obs MS [M+1] | tR (min) | Measurement Method |
|---|---|---|---|---|
| AD17 | 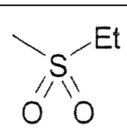 | 484.5 | 3.21 | SA |

(continued)

| Ex. No. | -B | obs MS [M+1] | tR (min) | Measurement Method |
|---|---|---|---|---|
| AD18 | 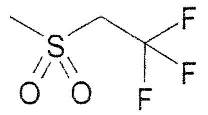 | 538.7 | 3.55 | SA |
| A19 | 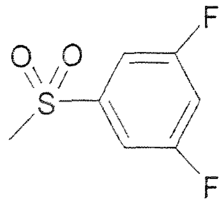 | 568.6 | 3.84 | SA |
| AD20 | 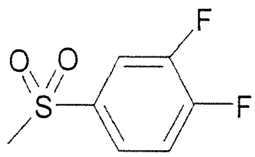 | 568.5 | 3.8 | SA |
| AD21 | 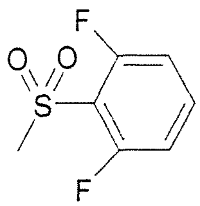 | 568.5 | 3.63 | SA |
| AD22 | 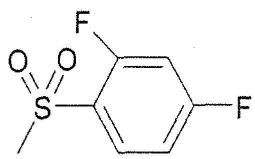 | 568.6 | 3.73 | SA |
| AD23 | 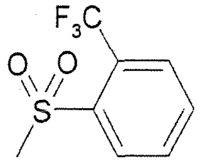 | 600.6 | 3.87 | SA |

[Table 104]

| Ex. No. | -B | obs MS [M+1] | tR (min) | Measurement Method |
|---|---|---|---|---|
| AD25 | 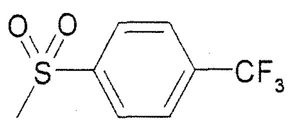 | 600.5 | 4 | SA |

(continued)

| Ex. No. | -B | obs MS [M+1] | tR (min) | Measurement Method |
|---|---|---|---|---|
| AD26 | 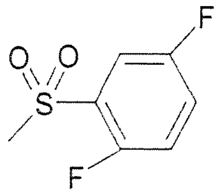 | 568.6 | 3.75 | SA |

[Table 105]

| Ex. No. | - B | obs MS [M+1] | tR (min) | Measurement Method |
|---|---|---|---|---|
| AE1 | 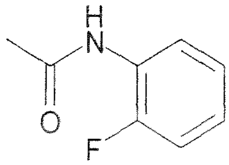 | 529.4 | 2.99 | SB |
| AE2 | 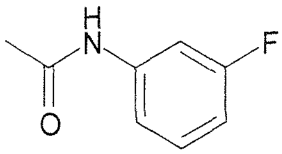 | 529.2 | 3.11 | SB |
| AE3 | 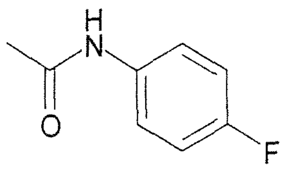 | 529.2 | 3.03 | SB |
| AE4 | 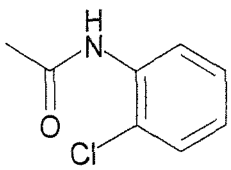 | 545.4 | 3.15 | SB |
| AE5 | 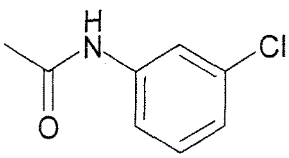 | 545.4 | 3.24 | SB |
| AE6 | 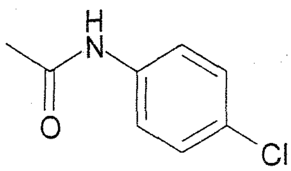 | 545.4 | 3.21 | SB |

(continued)

| Ex. No. | - B | obs MS [M+1] | tR (min) | Measurement Method |
|---|---|---|---|---|
| AE7 | 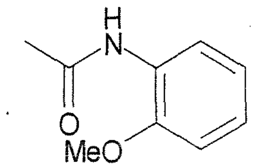 | 541.5 | 3.09 | SB |
| AE8 | 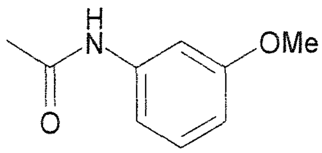 | 541.5 | 3.01 | SB |

[Table 106]

| Ex. No. | - B | obs MS [M+1] | tR (min) | Measurement Method |
|---|---|---|---|---|
| AE9 | 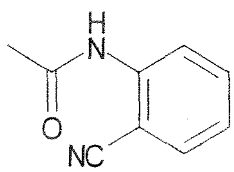 | 536.4 | 2.88 | SB |
| AE10 | 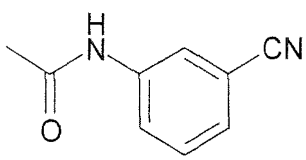 | 536.4 | 3.03 | SB |
| AE11 | 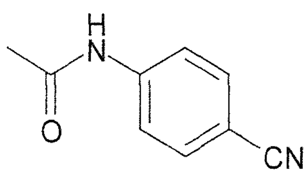 | 536.4 | 3.01 | SB |
| AE12 | 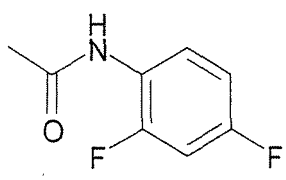 | 536.4 | 3.01 | SB |
| AE13 | 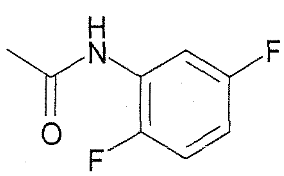 | 547.6 | 3.69 | SB |

(continued)

| Ex. No. | - B | obs MS [M+1] | tR (min) | Measurement Method |
|---|---|---|---|---|
| AE14 | 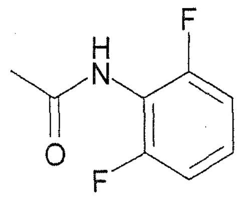 | 547.6 | 3.47 | SB |
| AE15 | 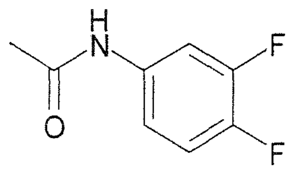 | 547.6 | 3.76 | SB |

[Table 107]

| Ex. No. | -B | obs MS [M+1] | tR (min) | Measurement Method |
|---|---|---|---|---|
| AE17 | 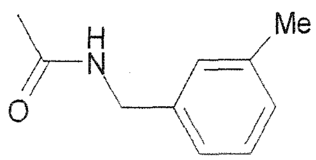 | 539.8 | 3.69 | SB |
| AE18 | 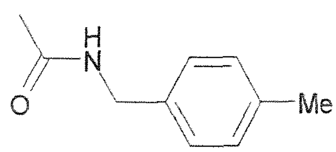 | 539.8 | 3.69 | SB |
| AE19 | 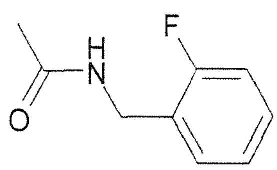 | 543.7 | 3.63 | SB |
| AE20 | 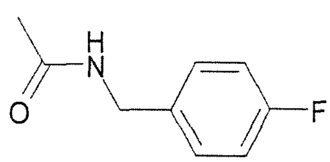 | 543.7 | 3.67 | SB |
| AE21 | 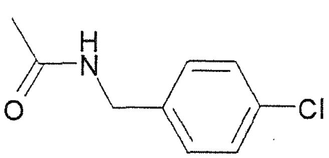 | 559.7 | 4.01 | SB |

(continued)

| Ex. No. | -B | obs MS [M+1] | tR (min) | Measurement Method |
|---|---|---|---|---|
| AE22 | 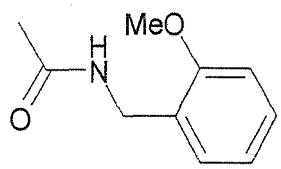 | 555.8 | 3.61 | SB |
| AE23 | 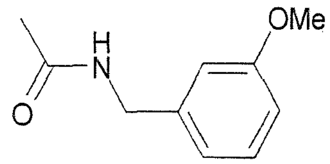 | 555.8 | 3.57 | SB |
| AE24 | 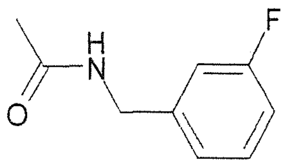 | 543.4 | 3.01 | SB |

[Table 108]

| Ex. No. | -B | obs MS [M+1] | tR (min) | Measurement Method |
|---|---|---|---|---|
| AE25 | 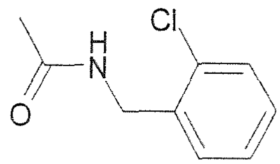 | 559.4 | 3.11 | SB |
| AE26 | 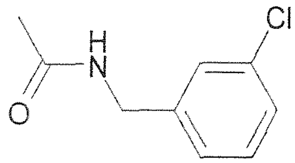 | 559.4 | 3.15 | SB |
| AE27 | 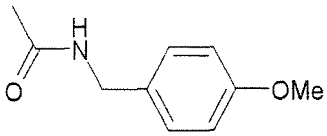 | 555.2 | 2.94 | SB |
| AE28 | 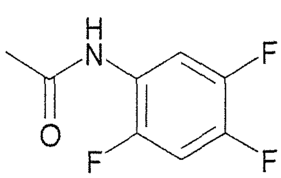 | 565.6 | 3.14 | SB |

(continued)

| Ex. No. | -B | obs MS [M+1] | tR (min) | Measurement Method |
|---|---|---|---|---|
| AE29 | 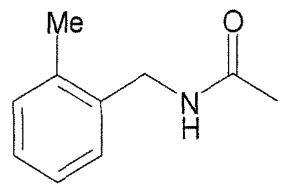 | 539.5 | 3.07 | SB |
| AE30 | 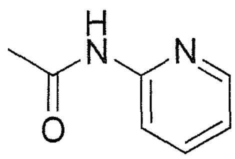 | 512.4 | 2.3 | SB |
| AE31 | 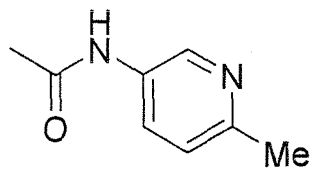 | 526.4 | 2.32 | SB |
| AE32 | 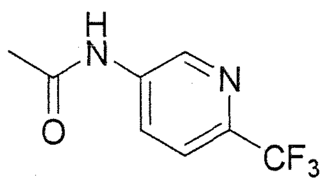 | 580.4 | 3.11 | SB |

[Table 109]

| Ex. No. | - B | obs MS [M+1] | tR (min) | Measurement Method |
|---|---|---|---|---|
| AE33 | 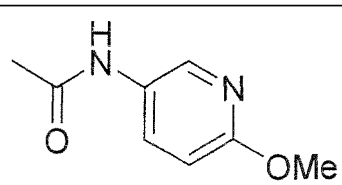 | 542.6 | 2.63 | SB |
| AE34 | 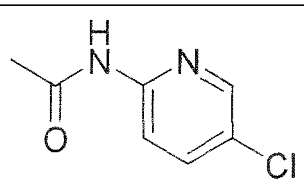 | 546.2 | 2.9 | SB |

[Table 110]

| Ex. No. | - B | obs MS [M+1] | tR (min) | Measurement Method |
|---|---|---|---|---|
| AF1 | 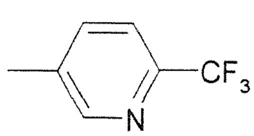 | 537.6 | 3.47 | SB |

(continued)

| Ex. No. | - B | obs MS [M+1] | tR (min) | Measurement Method |
|---|---|---|---|---|
| AF2 | 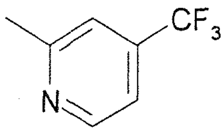 | 537.6 | 2.73 | SB |
| AF3 | 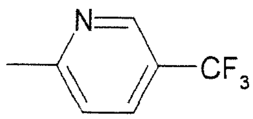 | 537.6 | 2.94 | SB |
| AF4 | 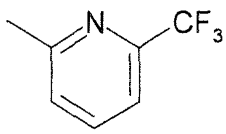 | 537.6 | 3.74 | SB |
| AF5 | 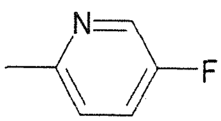 | 487.4 | 2.38 | SB |
| AF6 | 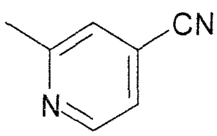 | 494.4 | 2.67 | SB |
| AF7 | 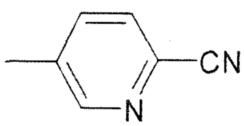 | 494.4 | 3.09 | SB |
| AF8 | 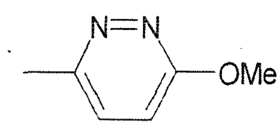 | 500.3 | 2.30 | SB |

[Table 111]

| Ex. No. | -B | obs MS [M+1] | tR (min) | Measurement Method |
|---|---|---|---|---|
| AF9 | 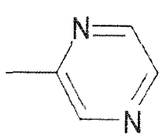 | 470.3 | 2.61 | SB |
| AF10 | 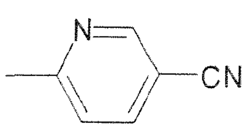 | 494.4 | 2.94 | SB |

[Chemical formula 119]

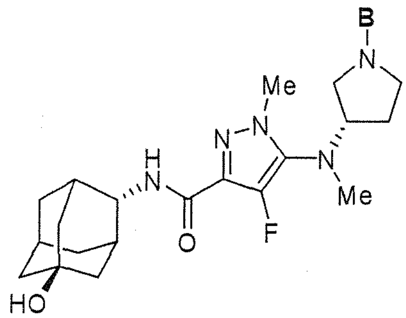

[Table 112]

| Ex. No. | -B | obs MS [M+1] | tR (min) | Measurement Method |
|---|---|---|---|---|
| AG1 | 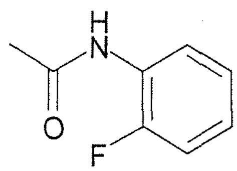 | 529.4 | 3.05 | SB |
| AG2 | 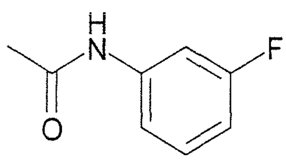 | 529.4 | 3.13 | SB |
| AG3 | 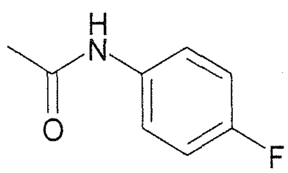 | 529.4 | 3.05 | SB |
| AG4 | 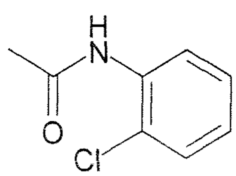 | 545.4 | 3.15 | SB |
| AG5 | 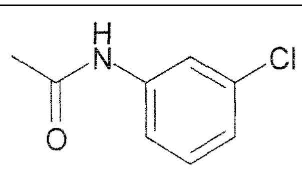 | 545.4 | 3.24 | SB |

(continued)

| Ex. No. | -B | obs MS [M+1] | tR (min) | Measurement Method |
|---|---|---|---|---|
| AG6 | 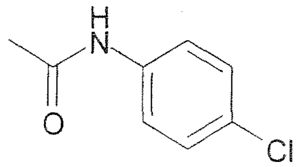 | 545.4 | 3.21 | SB |
| AG7 | 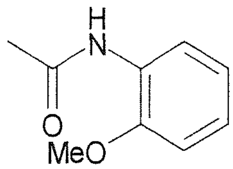 | 541.5 | 3.07 | SB |
| AG8 | 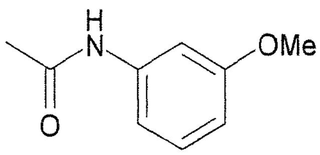 | 541.5 | 3.01 | SB |

[Table 113]

| Ex. No. | - B | obs MS [M+1] | tR (min) | Measurement Method |
|---|---|---|---|---|
| AG9 | 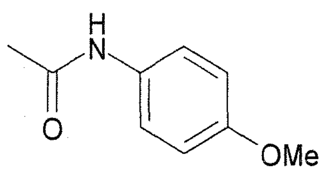 | 541.5 | 2.94 | SB |
| AG10 | 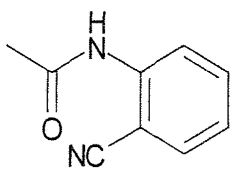 | 536.4 | 2.9 | SB |
| AG11 | 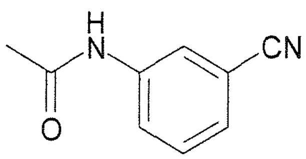 | 536.4 | 3.03 | SB |
| AG12 | 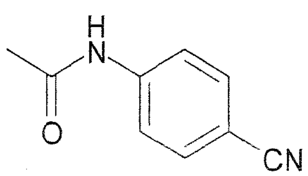 | 536.4 | 3.03 | SB |

(continued)

| Ex. No. | - B | obs MS [M+1] | tR (min) | Measurement Method |
|---|---|---|---|---|
| AG13 | 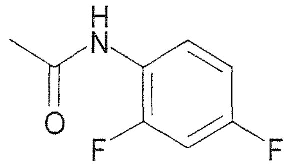 | 547.4 | 3.01 | SB |
| AG14 | 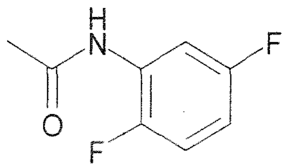 | 547.4 | 3.11 | SB |
| AG15 | 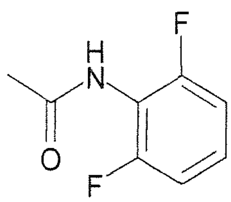 | 547.4 | 2.88 | SB |
| AG16 | 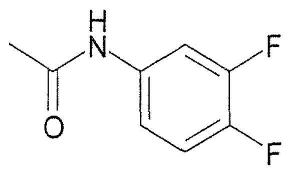 | 547.4 | 3.17 | SB |

[Table 114]

| Ex. No. | - B | obs MS [M+1] | tR (min) | Measurement Method |
|---|---|---|---|---|
| AG17 | 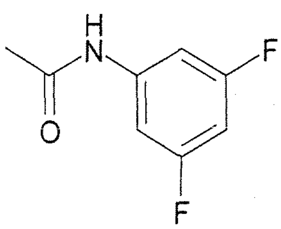 | 547.4 | 3.26 | SB |
| AG18 | 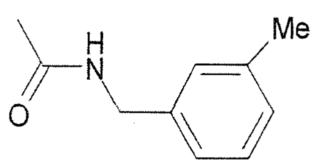 | 539.5 | 3.11 | SB |
| AG19 | 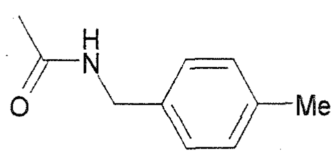 | 539.5 | 3.09 | SB |

(continued)

| Ex. No. | - B | obs MS [M+1] | tR (min) | Measurement Method |
|---|---|---|---|---|
| AG20 | 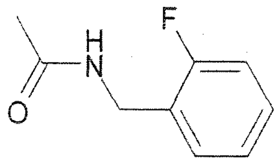 | 543.4 | 3.01 | SB |
| AG21 | 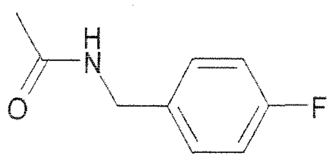 | 543.4 | 3.01 | SB |
| AG22 | 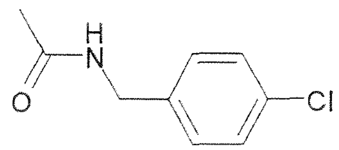 | 559.4 | 3.15 | SB |
| AG23 | 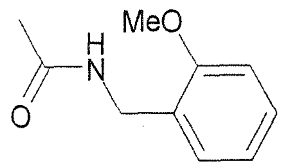 | 555.2 | 3.03 | SB |
| AG24 | 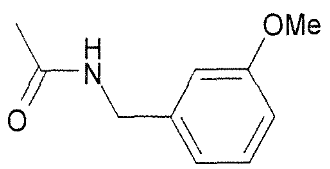 | 555.2 | 2.96 | SB |

[Table 115]

| Ex. No. | -B | obs MS [M+1] | tR (min) | Measurement Method |
|---|---|---|---|---|
| AH1 | 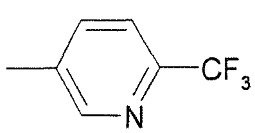 | 537.6 | 3.47 | SB |
| AH2 | 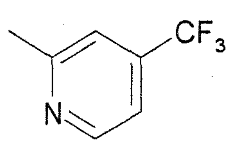 | 537.6 | 2.73 | SB |
| AH3 | 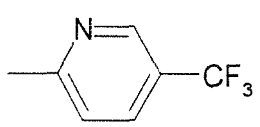 | 537.6 | 2.88 | SB |

(continued)

| Ex. No. | -B | obs MS [M+1] | tR (min) | Measurement Method |
|---|---|---|---|---|
| AH4 | 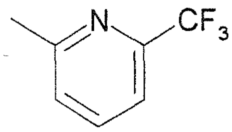 | 537.6 | 3.74 | SB |
| AH5 | 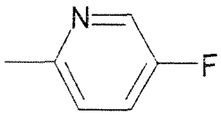 | 487.4 | 2.42 | SB |
| AH6 | 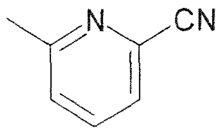 | 494.4 | 3.34 | SB |
| AH7 | 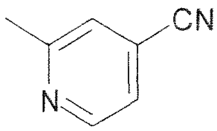 | 494.4 | 2.67 | SB |
| AH8 | 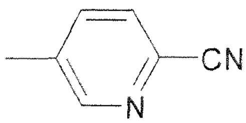 | 494.4 | 3.09 | SB |

[Table 116]

| Ex. No. | -B | obs MS [M+1] | tR (min) | Measurement Method |
|---|---|---|---|---|
| AH9 | 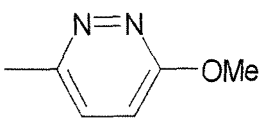 | 500.3 | 2.28 | SB |
| AH10 | 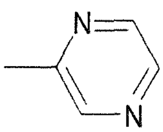 | 470.3 | 2.61 | SB |
| AH11 | 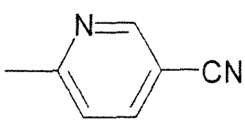 | 494.4 | 2.94 | SB |

[Chemical formula 120]

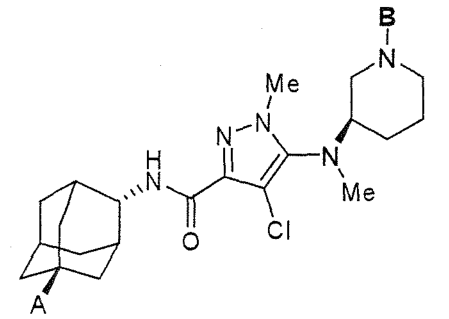

[Table 117]

| Ex. No. | -B | obs MS [M+1] | tR (min) | Measurement Method |
|---|---|---|---|---|
| AI1 | 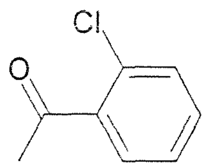 | 560.6 | 3.64 | SA |
| AI2 | 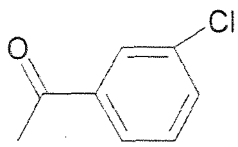 | 560.5 | 3.72 | SA |
| AI3 | 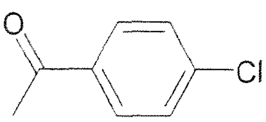 | 560.5 | 3.72 | SA |
| AI4 | 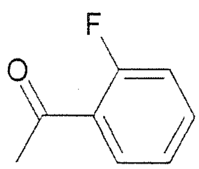 | 544.6 | 3.6 | SA |
| AI5 | 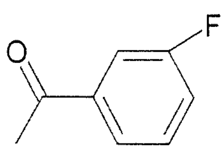 | 544.6 | 3.6 | SA |
| AI6 | 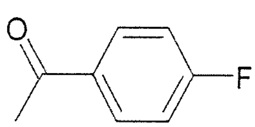 | 544.5 | 3.58 | SA |

(continued)

| Ex. No. | -B | obs MS [M+1] | tR (min) | Measurement Method |
|---|---|---|---|---|
| AI7 | 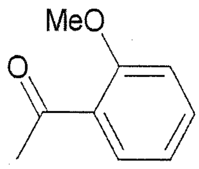 | 556.5 | 3.7 | SA |
| AI8 | 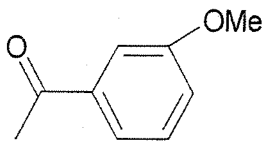 | 556.5 | 3.57 | SA |

[Table 118]

| Ex. No. | -B | obs MS [M+1] | tR (min) | Measurement Method |
|---|---|---|---|---|
| AI9 | 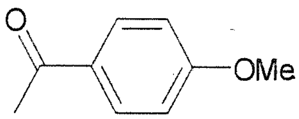 | 556.5 | 3.56 | SA |
| AI10 | 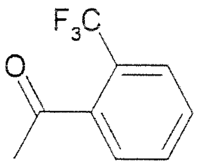 | 594.5 | 3.78 | SA |
| AI11 | 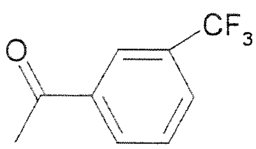 | 594.4 | 3.89 | SA |
| AI12 | 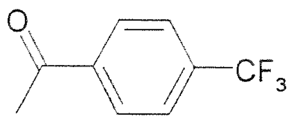 | 594.4 | 3.91 | SA |
| AI13 | 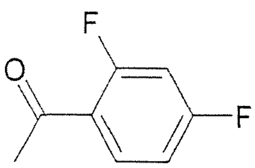 | 562.5 | 3.66 | SA |
| AI14 | 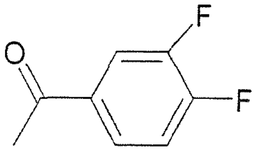 | 562.6 | 3.68 | SA |

(continued)

| Ex. No. | -B | obs MS [M+1] | tR (min) | Measurement Method |
|---|---|---|---|---|
| AI15 | 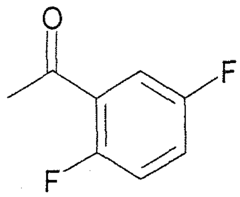 | 562.5 | 3.67 | SA |
| AI16 | 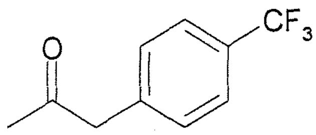 | 608.5 | 3.98 | SA |

[Table 119]

| Ex. No. | - B | obs MS [M+1] | tR (min) | Measurement Method |
|---|---|---|---|---|
| AI17 | 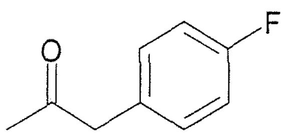 | 558.6 | 3.68 | SA |
| AI18 | 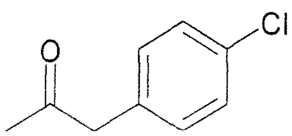 | 574.5 | 3.79 | SA |
| AI19 | 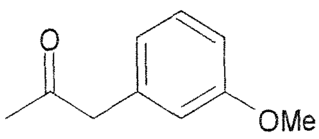 | 570.6 | 3.63 | SA |
| AI20 | 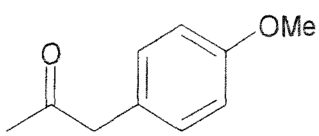 | 570.6 | 3.58 | SA |
| AI21 | 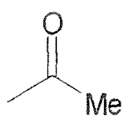 | 464.4 | 3.13 | SA |
| AI22 | 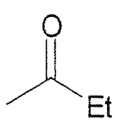 | 478.5 | 3.31 | SA |
| AI23 | 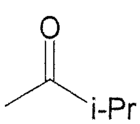 | 492.6 | 3.42 | SA |

(continued)

| Ex. No. | - B | obs MS [M+1] | tR (min) | Measurement Method |
|---|---|---|---|---|
| AI24 | 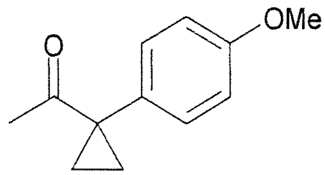 | 596.5 | 3.7 | SA |

[Table 120]

| Ex. No. | - B | obs MS [M+1] | tR (min) | Measurement Method |
|---|---|---|---|---|
| AI25 | 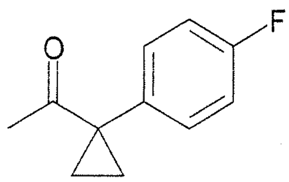 | 584.6 | 3.8 | SA |
| AI26 | 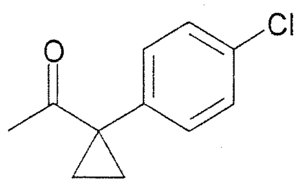 | 600.5 | 3.96 | SA |
| AI27 | 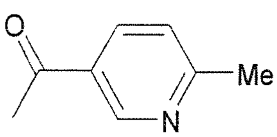 | 541.6 | 2.84 | SA |
| AI28 | 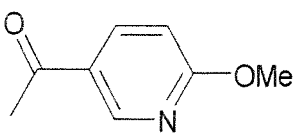 | 557.6 | 3.4 | SA |
| AI29 | 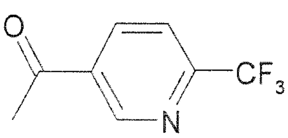 | 595.5 | 3.66 | SA |
| AI30 | 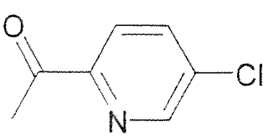 | 561.7 | 3.52 | SA |
| AI31 | 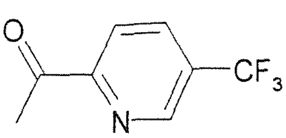 | 595.4 | 3.71 | SA |

(continued)

| Ex. No. | - B | obs MS [M+1] | tR (min) | Measurement Method |
|---|---|---|---|---|
| AI32 | 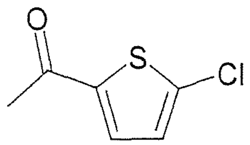 | 566.5 | 4 | SA |

[Table 121]

| Ex. No. | -B | obs MS [M+1] | tR (min) | Measurement Method |
|---|---|---|---|---|
| AI33 | 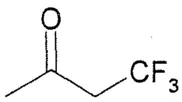 | 532.6 | 3.54 | SA |
| AI34 | 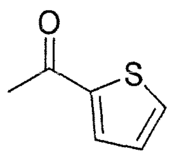 | 532.6 | 3.54 | SA |

[Table 122]

| Ex. No. | - B | obs MS [M+1] | tR (min) | Measurement Method |
|---|---|---|---|---|
| AJ1 | 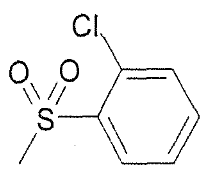 | 596.4 | 4.01 | SA |
| AJ2 | 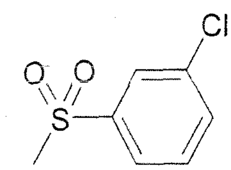 | 596.4 | 4.12 | SA |
| AJ3 | 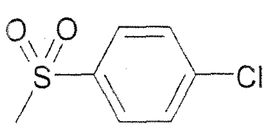 | 596.5 | 4.14 | SA |
| AJ4 | 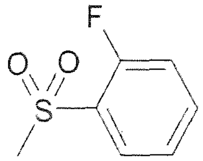 | 580.4 | 3.93 | SA |

(continued)

| Ex. No. | - B | obs MS [M+1] | tR (min) | Measurement Method |
|---|---|---|---|---|
| AJ5 | 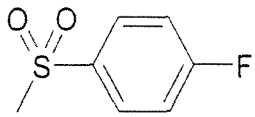 | 580.4 | 3.97 | SA |
| AJ6 | 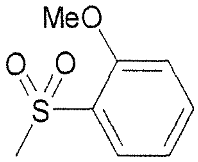 | 592.4 | 3.83 | SA |
| AJ7 | 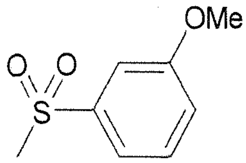 | 592.3 | 3.97 | SA |
| AJ8 | 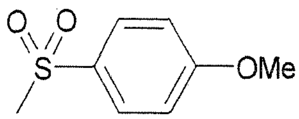 | 592.5 | 3.93 | SA |

[Table 123]

| Ex. No. | - B | obs MS [M+1] | tR (min) | Measurement Method |
|---|---|---|---|---|
| AJ9 | 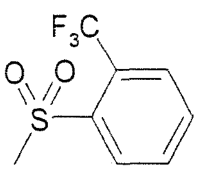 | 630.5 | 4.11 | SA |
| AJ10 | 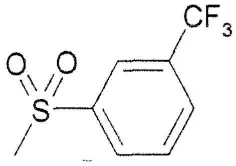 | 630.5 | 4.21 | SA |
| AJ11 | 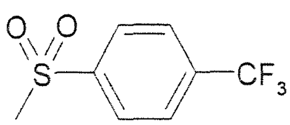 | 630.5 | 4.27 | SA |
| AJ12 | 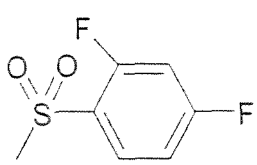 | 598.6 | 4.02 | SA |

(continued)

| Ex. No. | - B | obs MS [M+1] | tR (min) | Measurement Method |
|---|---|---|---|---|
| AJ13 | 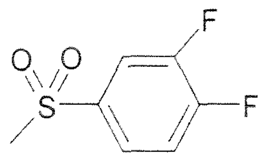 | 598.6 | 4.06 | SA |
| AJ14 | 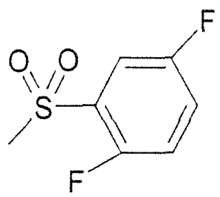 | 598.5 | 4 | SA |
| AJ15 | 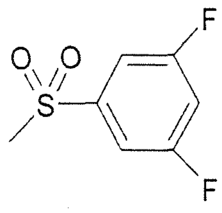 | 598.6 | 4.09 | SA |
| AJ16 | 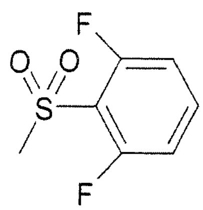 | 598.6 | 3.91 | SA |

[Table 124]

| Ex. No. | -B | obs MS [M+1] | tR (min) | Measurement Method |
|---|---|---|---|---|
| AJ17 | 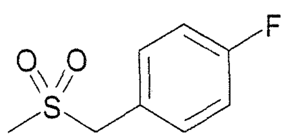 | 594.4 | 3.89 | SA |
| AJ18 | 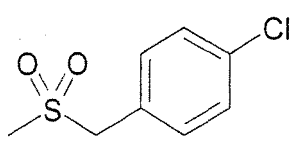 | 610.3 | 4.05 | SA |
| AJ19 | 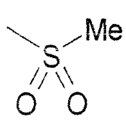 | 500.5 | 3.33 | SA |
| AJ20 | 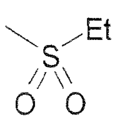 | 514.5 | 3.47 | SA |

(continued)

| Ex. No. | -B | obs MS [M+1] | tR (min) | Measurement Method |
|---|---|---|---|---|
| AJ21 | 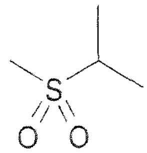 | 528.5 | 3.58 | SA |
| AJ22 | 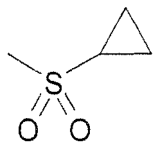 | 526.5 | 3.54 | SA |
| AJ23 | 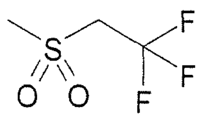 | 568.3 | 3.76 | SA |
| AJ24 | 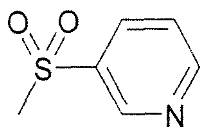 | 563.6 | 3.46 | SA |

[Table 125]

| Ex. No. | -B | obs MS [M+1] | tR (min) | Measurement Method |
|---|---|---|---|---|
| AJ25 | 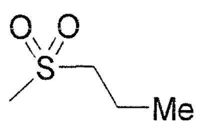 | 528.6 | 3.67 | SA |

[Table 126]

| Ex. No. | - B | obs MS [M+1] | tR (min) | Measurement Method |
|---|---|---|---|---|
| AK1 | 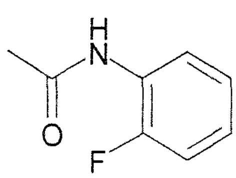 | 559.7 | 3.78 | SB |
| AK2 | 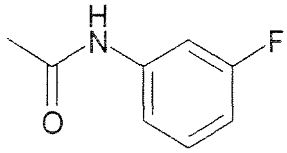 | 559.7 | 3.86 | SB |

(continued)

| Ex. No. | - B | obs MS [M+1] | tR (min) | Measurement Method |
|---|---|---|---|---|
| AK3 | 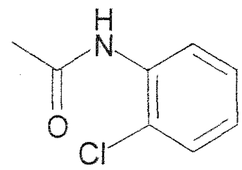 | 575.7 | 3.95 | SB |
| AK4 | 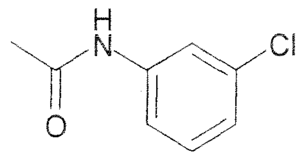 | 575.7 | 0.69 | SB |
| AK5 | 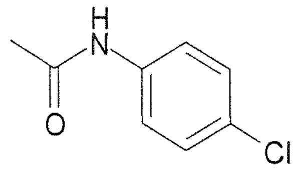 | 575.4 | 3.38 | SB |
| AK6 | 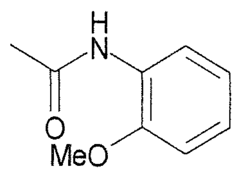 | 571.7 | 3.9 | SB |
| AK7 | 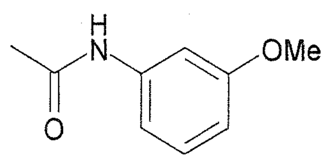 | 571.7 | 3.78 | SB |
| AK8 | 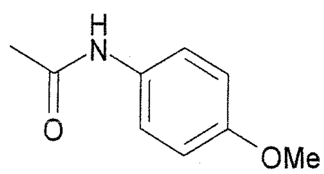 | 571.7 | 3.72 | SB |

[Table 127]

| Ex. No. | - B | obs MS [M+1] | tR (min) | Measurement Method |
|---|---|---|---|---|
| AK9 | 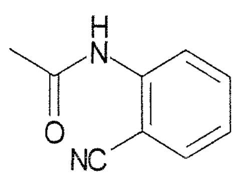 | 566.7 | 3.65 | SB |

(continued)

| Ex. No. | - B | obs MS [M+1] | tR (min) | Measurement Method |
|---|---|---|---|---|
| AK10 | 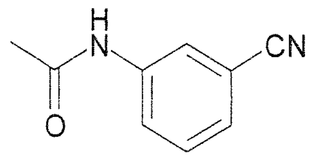 | 566.7 | 3.8 | SB |
| AK11 | 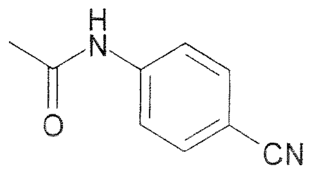 | 566.7 | 3.76 | SB |
| AK12 | 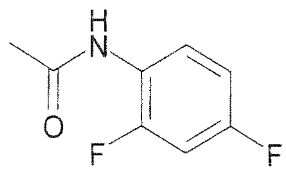 | 577.9 | 3.8 | SB |
| AK13 | 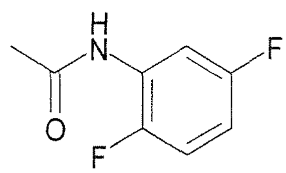 | 577.9 | 3.84 | SB |
| AK14 | 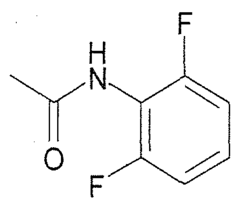 | 577.9 | 0.65 | SB |
| AK15 | 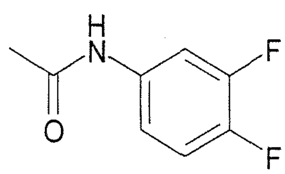 | 577.9 | 0.65 | SB |

[Table 128]

| Ex. No. | -B | obs MS [M+1] | tR (min) | Measurement Method |
|---|---|---|---|---|
| AK17 | 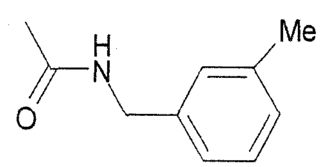 | 569.5 | 3.32 | SB |

(continued)

| Ex. No. | -B | obs MS [M+1] | tR (min) | Measurement Method |
|---|---|---|---|---|
| AK18 | 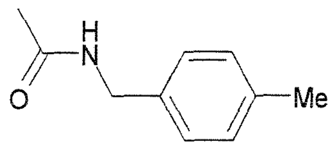 | 569.5 | 3.3 | SB |
| AK19 | 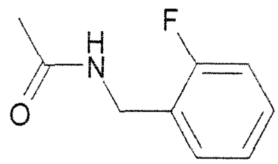 | 573.4 | 3.19 | SB |
| AK20 | 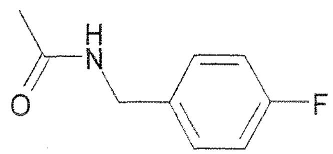 | 573.4 | 3.21 | SB |
| AK21 | 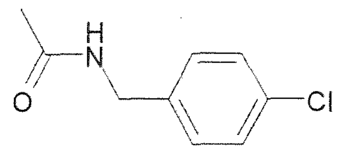 | 589.4 | 3.36 | SB |
| AK22 | 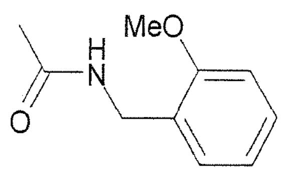 | 585.2 | 3.21 | SB |
| AK23 | 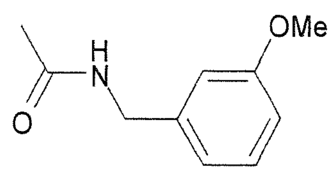 | 585.2 | 3.17 | SB |
| AK24 | 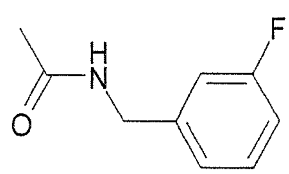 | 573.4 | 3.24 | SB |

[Table 129]

| Ex. No. | -B | obs MS [M+1] | tR (min) | Measurement Method |
|---|---|---|---|---|
| AK25 | 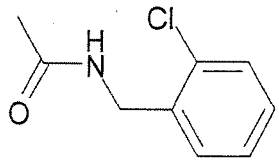 | 589.4 | 3.32 | SB |
| AK26 | 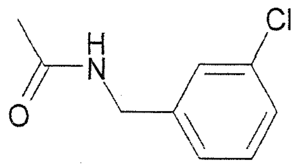 | 589.4 | 3.36 | SB |
| AK27 | 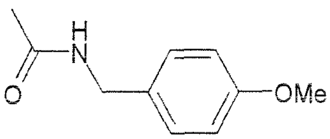 | 585.2 | 3.17 | SB |
| AK28 | 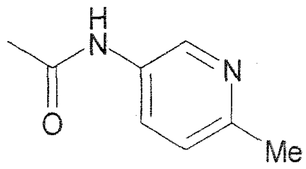 | 556.3 | 2.44 | SB |
| AK29 | 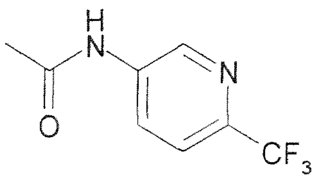 | 610.4 | 3.3 | SB |
| AK30 | 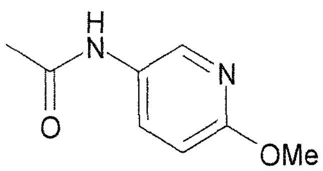 | 572.3 | 2.82 | SB |
| AK31 | 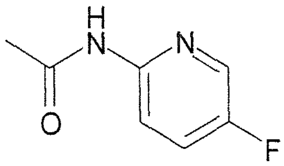 | 560.2 | 2.9 | SB |
| AK32 | 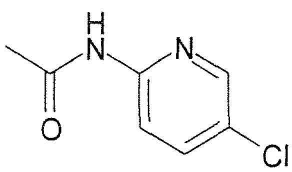 | 576.2 | 3.11 | SB |

[Table 130]

| Ex. No. | - B | obs MS [M+1] | tR (min) | Measurement Method |
|---|---|---|---|---|
| AK33 | 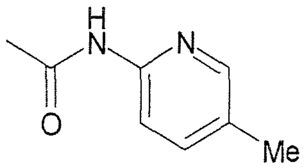 | 556.3 | 2.51 | SB |
| AK34 | 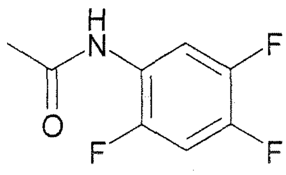 | 595.3 | 3.35 | SB |
| AK35 | 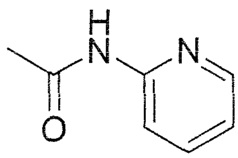 | 542.3 | 2.44 | SB |

[Table 131]

| Ex. No. | -B | obs MS [M+1] | tR (min) | Measurement Method |
|---|---|---|---|---|
| AL1 | 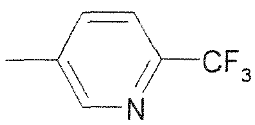 | 567.3 | 3.69 | SB |
| AL2 | 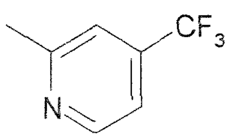 | 567.3 | 3.42 | SB |
| AL3 | 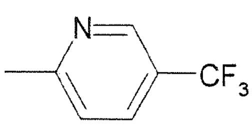 | 567.3 | 3.65 | SB |
| AL4 | 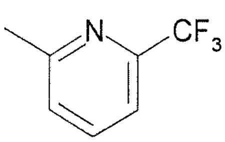 | 567.5 | 3.97 | SB |
| AL5 | 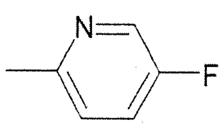 | 517.4 | 2.94 | SB |

(continued)

| Ex. No. | -B | obs MS [M+1] | tR (min) | Measurement Method |
|---|---|---|---|---|
| AL6 | 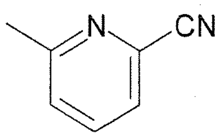 | 524.4 | 3.57 | SB |
| AL7 | 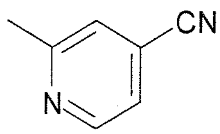 | 524.4 | 3.32 | SB |
| AL8 | 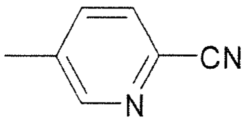 | 524.4 | 3.28 | SB |

[Table 132]

| Ex. No. | - B | obs MS [M+1] | tR (min) | Measurement Method |
|---|---|---|---|---|
| AL9 | 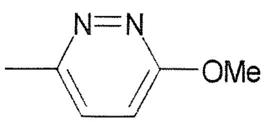 | 530.3 | 2.40 | SB |
| AL10 | 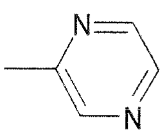 | 500.3 | 2.99 | SB |
| AL11 | 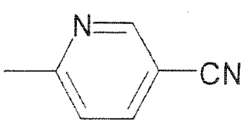 | 524.4 | 3.40 | SB |

[Chemical formula 121]

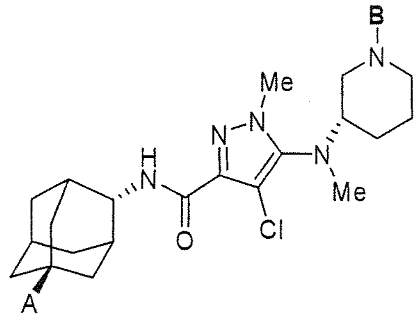

[Table 133]

| Ex. No. | -B | obs MS [M+1] | tR (min) | Measurement Method |
|---|---|---|---|---|
| AM1 | 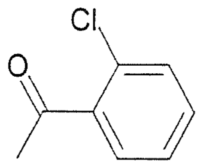 | 560.6 | 3.84 | SA |
| AM2 | 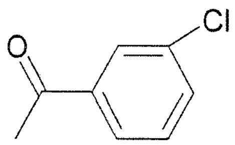 | 560.5 | 3.74 | SA |
| AM3 | 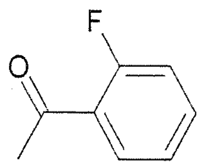 | 544.5 | 3.56 | SA |
| AM4 | 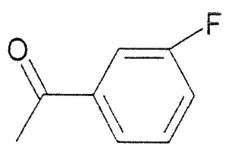 | 544.5 | 3.58 | SA |
| AM5 | 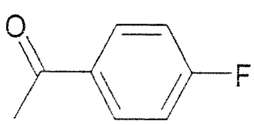 | 544.5 | 3.56 | SA |
| AM6 | 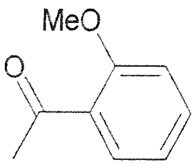 | 556.5 | 3.5 | SA |

(continued)

| Ex. No. | -B | obs MS [M+1] | tR (min) | Measurement Method |
|---|---|---|---|---|
| AM7 | 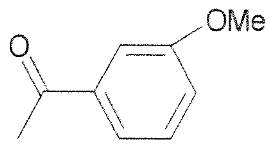 | 556.5 | 3.56 | SA |
| AM8 | 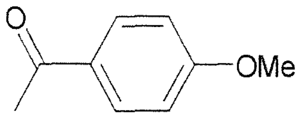 | 556.5 | 3.54 | SA |

[Table 134]

| Ex. No. | -B | obs MS [M+1] | tR (min) | Measurement Method |
|---|---|---|---|---|
| AM9 | 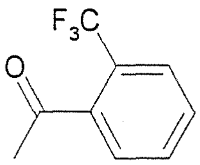 | 594.4 | 3.77 | SA |
| AM10 | 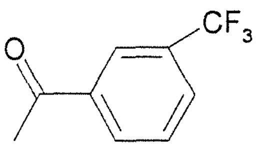 | 594.4 | 3.87 | SA |
| AM11 | 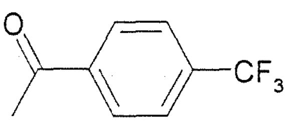 | 594.4 | 3.88 | SA |
| AM12 | 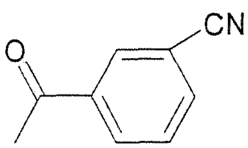 | 551.5 | 3.46 | SA |
| AM13 | 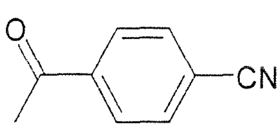 | 551.6 | 3.48 | SA |
| AM14 | 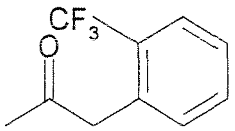 | 608.5 | 3.92 | SA |

(continued)

| Ex. No. | -B | obs MS [M+1] | tR (min) | Measurement Method |
|---|---|---|---|---|
| AM15 | 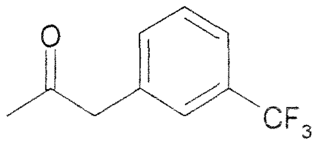 | 608.5 | 3.97 | SA |
| AM16 | 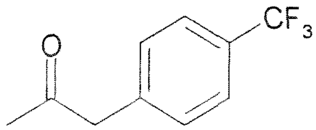 | 608.5 | 3.95 | SA |

[Table 135]

| Ex. No. | - B | obs MS [M+1] | tR (min) | Measurement Method |
|---|---|---|---|---|
| AM17 | 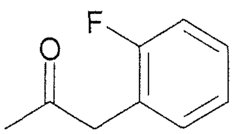 | 558.6 | 3.68 | SA |
| AM18 | 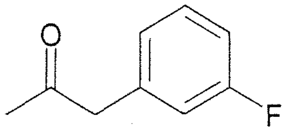 | 558.5 | 3.66 | SA |
| AM19 | 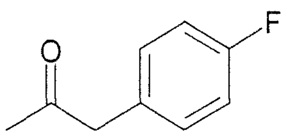 | 558.6 | 3.64 | SA |
| AM20 | 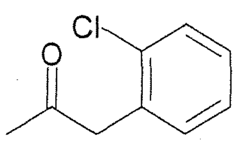 | 574.6 | 3.8 | SA |
| AM21 | 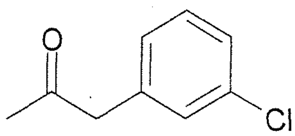 | 574.6 | 3.82 | SA |
| AM22 | 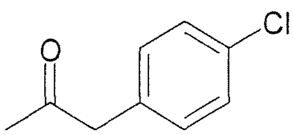 | 574.6 | 3.79 | SA |
| AM23 | 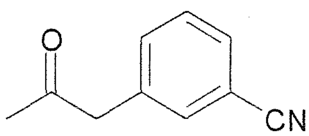 | 565.5 | 3.55 | SA |

(continued)

| Ex. No. | - B | obs MS [M+1] | tR (min) | Measurement Method |
|---|---|---|---|---|
| AM24 | 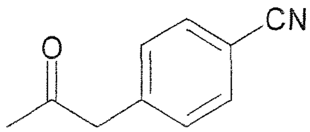 | 565.5 | 3.52 | SA |

[Table 136]

| Ex. No. | - B | obs MS [M+1] | tR (min) | Measurement Method |
|---|---|---|---|---|
| AM25 | 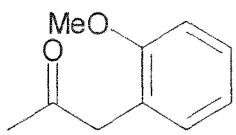 | 570.6 | 3.67 | SA |
| AM26 | 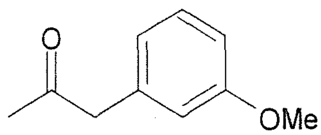 | 570.6 | 3.6 | SA |
| AM27 | 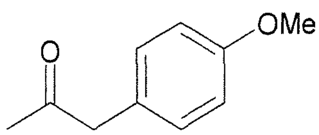 | 570.6 | 3.58 | SA |
| AM28 | 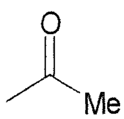 | 464.5 | 3.1 | SA |
| AM29 | 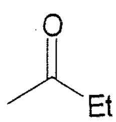 | 478.5 | 3.27 | SA |
| AM30 | 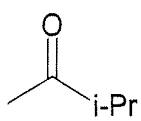 | 492.5 | 3.42 | SA |
| AM31 | 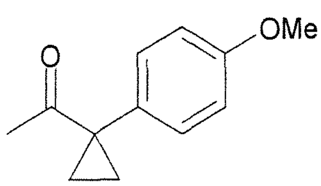 | 596.5 | 3.73 | SA |
| AM32 | 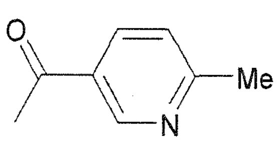 | 541.5 | 2.91 | SA |

[Table 137]

| Ex. No. | -B | obs MS [M+1] | tR (min) | Measurement Method |
|---|---|---|---|---|
| AM33 | 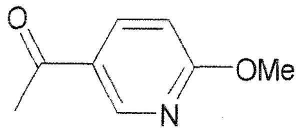 | 557.5 | 3.38 | SA |
| AM34 | 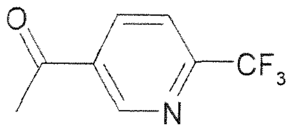 | 595.4 | 3.62 | SA |
| AM35 | 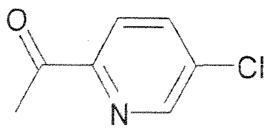 | 561.6 | 3.49 | SA |
| AM36 | 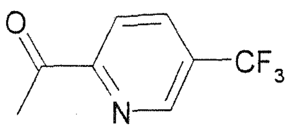 | 595.5 | 3.68 | SA |
| AM37 | 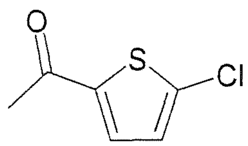 | 566.4 | 3.83 | SA |
| AM38 | 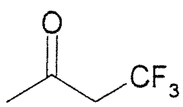 | 532.6 | 3.51 | SA |
| AM39 | 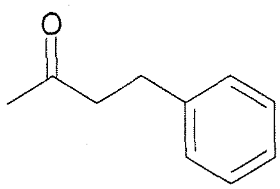 | 554.6 | 3.78 | SA |

[Table 138]

| Ex. No. | - B | obs MS [M+1] | tR (min) | Measurement Method |
|---|---|---|---|---|
| AN1 | 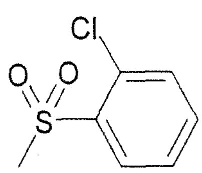 | 596.5 | 4.01 | SA |

(continued)

| Ex. No. | - B | obs MS [M+1] | tR (min) | Measurement Method |
|---|---|---|---|---|
| AN2 | 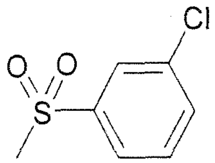 | 596.5 | 4.13 | SA |
| AN3 | 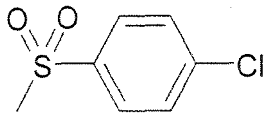 | 596.4 | 4.14 | SA |
| AN4 | 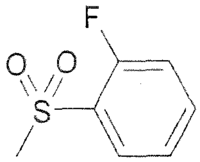 | 580.4 | 3.91 | SA |
| AN5 | 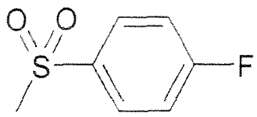 | 580.4 | 3.97 | SA |
| AN6 | 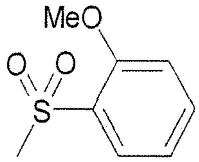 | 592.4 | 3.81 | SA |
| AN7 | 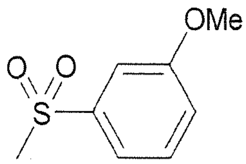 | 592.4 | 3.95 | SA |
| AN8 | 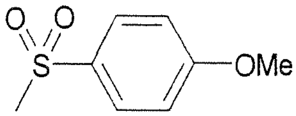 | 592.6 | 3.92 | SA |

[Table 139]

| Ex. No. | - B | obs MS [M+1] | tR (min) | Measurement Method |
|---|---|---|---|---|
| AN9 | 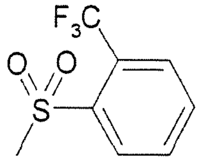 | 630.5 | 4.1 | SA |

(continued)

| Ex. No. | - B | obs MS [M+1] | tR (min) | Measurement Method |
|---|---|---|---|---|
| AN10 | 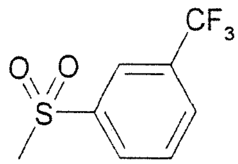 | 630.5 | 4.21 | SA |
| AN11 | 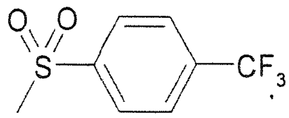 | 630.3 | 4.25 | SA |
| AN 12 | 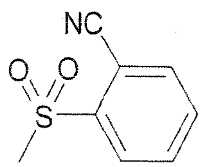 | 587.6 | 3.76 | SA |
| AN13 | 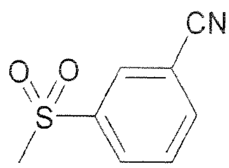 | 587.6 | 3.82 | SA |
| AN14 | 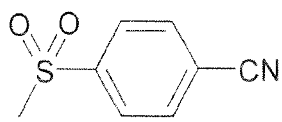 | 587.4 | 3.84 | SA |
| AN15 | 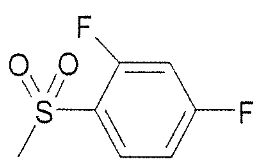 | 598.6 | 3.99 | SA |
| AN16 | 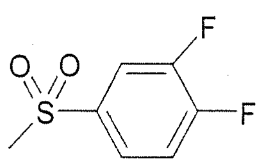 | 598.6 | 4.06 | SA |

[Table 140]

| Ex. No. | -B | obs MS [M+1] | tR (min) | Measurement Method |
|---|---|---|---|---|
| AN17 | 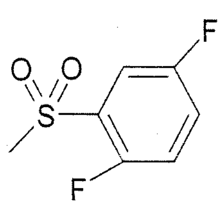 | 598.3 | 3.99 | SA |

(continued)

| Ex. No. | -B | obs MS [M+1] | tR (min) | Measurement Method |
|---|---|---|---|---|
| AN18 | 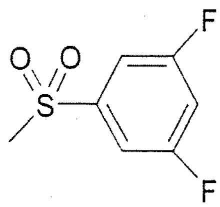 | 598.5 | 4.08 | SA |
| AN19 | 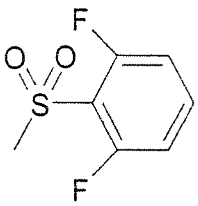 | 598.6 | 3.9 | SA |
| AN20 | 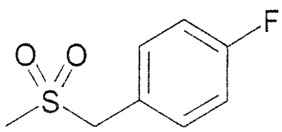 | 594.4 | 3.9 | SA |
| AN21 | 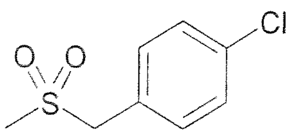 | 610.3 | 4.06 | SA |
| AN22 | 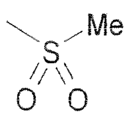 | 500.6 | 3.32 | SA |
| AN23 | 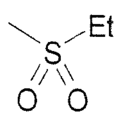 | 514.6 | 3.45 | SA |
| AN24 | 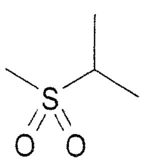 | 528.5 | 3.6 | SA |

[Table 141]

| Ex. No. | -B | obs MS [M+1] | tR (min) | Measurement Method |
|---|---|---|---|---|
| AN25 | 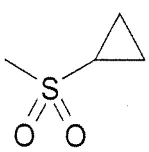 | 526.6 | 3.53 | SA |

(continued)

| Ex. No. | -B | obs MS [M+1] | tR (min) | Measurement Method |
|---|---|---|---|---|
| AN26 | 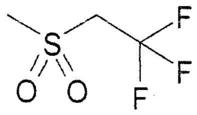 | 568.6 | 3.76 | SA |
| AN27 | 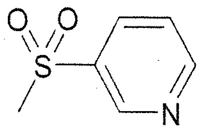 | 563.5 | 3.47 | SA |
| AN28 | 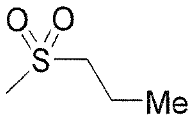 | 528.5 | 3.64 | SA |

[Table 142]

| Ex. No. | - B | obs MS [M+1] | tR (min) | Measurement Method |
|---|---|---|---|---|
| AO1 | 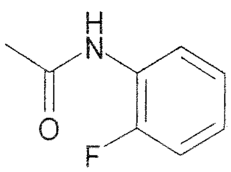 | 559.4 | 3.23 | SA |
| AO2 | 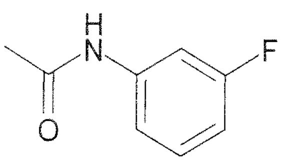 | 559.6 | 3.78 | SA |
| AO3 | 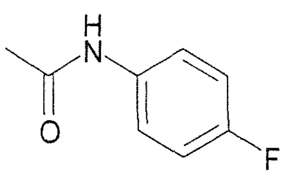 | 559.6 | 3.69 | SA |
| AO4 | 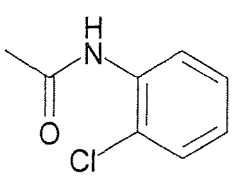 | 575.5 | 3.81 | SA |
| AO5 | 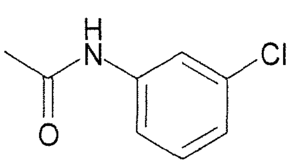 | 575.5 | 3.88 | SA |

(continued)

| Ex. No. | - B | obs MS [M+1] | tR (min) | Measurement Method |
|---|---|---|---|---|
| AO6 | 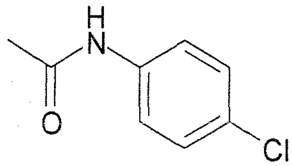 | 575.6 | 3.86 | SA |
| AO7 | 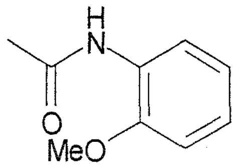 | 571.5 | 3.79 | SA |
| AO8 | 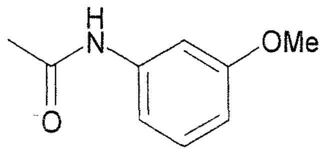 | 571.5 | 3.66 | SA |

[Table 143]

| Ex. No. | - B | obs MS [M+1] | tR (min) | Measurement Method |
|---|---|---|---|---|
| AO9 | 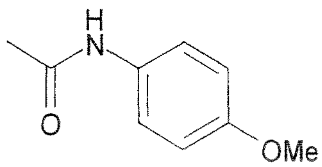 | 571.5 | 3.58 | SA |
| AO10 | 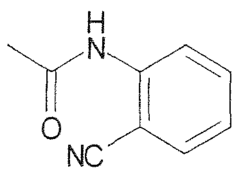 | 566.5 | 3.52 | SA |
| AO11 | 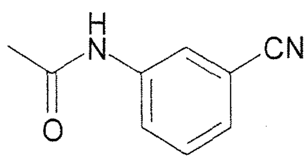 | 566.7 | 3.66 | SA |
| AO12 | 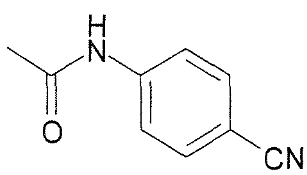 | 566.5 | 3.61 | SA |

(continued)

| Ex. No. | - B | obs MS [M+1] | tR (min) | Measurement Method |
|---|---|---|---|---|
| AO13 | 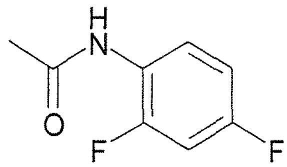 | 577.6 | 3.65 | SA |
| AO14 | 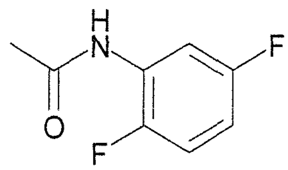 | 577.6 | 3.81 | SA |
| AO1 | 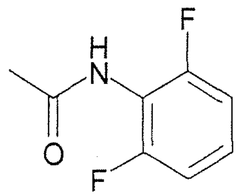 | 577.9 | 3.69 | SB |
| AO16 | 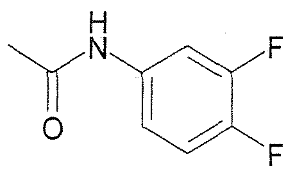 | 577.5 | 3.8 | SA |

[Table 144]

| Ex. No. | -B | obs MS [M+1] | tR (min) | Measurement Method |
|---|---|---|---|---|
| AO17 | 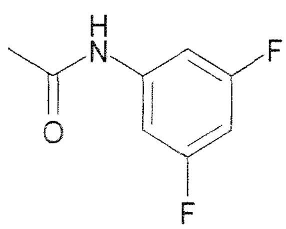 | 577.9 | 4.01 | SB |
| AO18 | 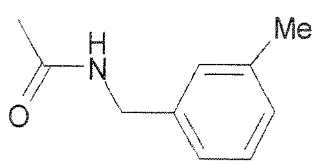 | 569.8 | 3.9 | SB |
| AO19 | 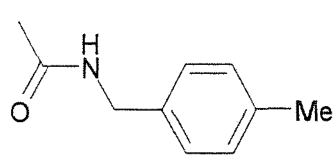 | 569.8 | 3.95 | SB |

(continued)

| Ex. No. | -B | obs MS [M+1] | tR (min) | Measurement Method |
|---|---|---|---|---|
| AO20 | 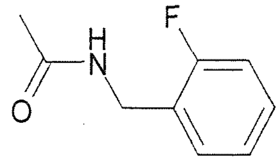 | 573.7 | 3.8 | SB |
| AO21 | 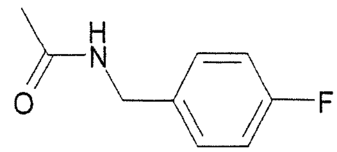 | 573.7 | 3.82 | SB |
| AO22 | 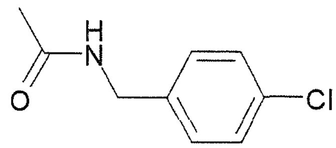 | 589.4 | 4.01 | SB |
| AO23 | 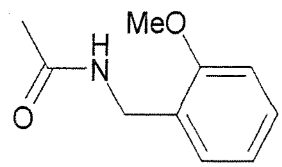 | 585.7 | 3.86 | SB |
| AO24 | 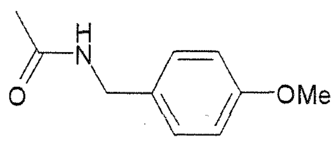 | 585.5 | 3.17 | SB |

[Table 145]

| Ex. No. | - B | obs MS [M+1] | tR (min) | Measurement Method |
|---|---|---|---|---|
| AO25 | 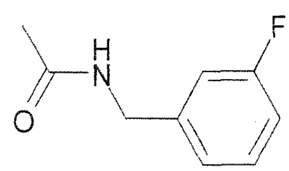 | 573.4 | 3.24 | SB |
| AO26 | 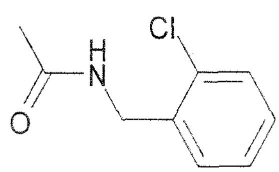 | 589.4 | 3.32 | SB |

(continued)

| Ex. No. | - B | obs MS [M+1] | tR (min) | Measurement Method |
|---|---|---|---|---|
| AO27 | 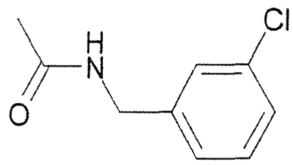 | 589.4 | 3.36 | SB |
| AO28 | 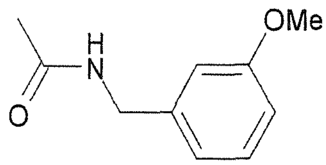 | 585.7 | 3.76 | SB |
| A029 | 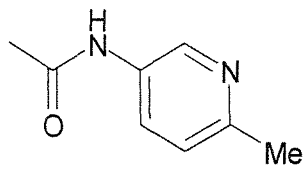 | 556.3 | 2.42 | SB |
| AO30 | 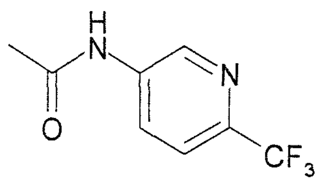 | 610.4 | 3.28 | SB |
| AO31 | 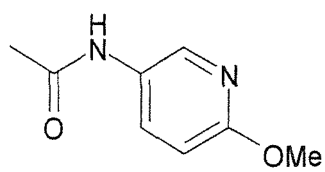 | 572.3 | 2.82 | SB |
| AO32 | 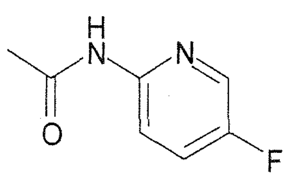 | 560.5 | 2.9 | SB |

[Table 146]

| Ex. No. | -B | obs MS [M+1] | tR (min) | Measurement Method |
|---|---|---|---|---|
| A033 | 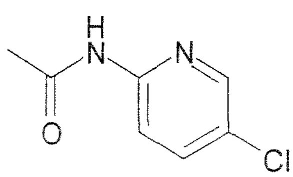 | 576.2 | 3.11 | SB |

(continued)

| Ex. No. | -B | obs MS [M+1] | tR (min) | Measurement Method |
|---|---|---|---|---|
| AO34 | 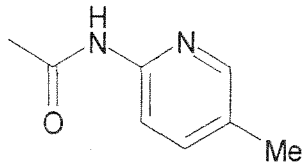 | 556.3 | 2.51 | SB |
| AO35 | 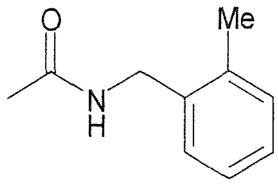 | 569.5 | 3.29 | SB |
| AO36 | 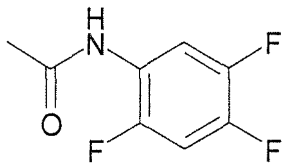 | 595.3 | 3.36 | SB |
| AO37 | 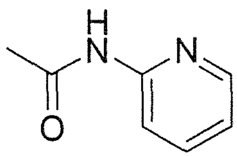 | 542.3 | 2.46 | SB |

[Table 147]

| Ex. No. | - B | obs MS [M+1] | tR (min) | Measurement Method |
|---|---|---|---|---|
| AP1 | 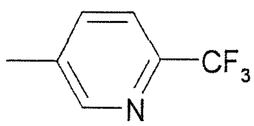 | 567.3 | 3.63 | SB |
| AP2 | 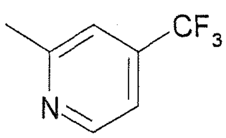 | 567.3 | 3.42 | SB |
| AP3 | 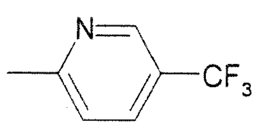 | 567.3 | 3.65 | SB |
| AP4 | 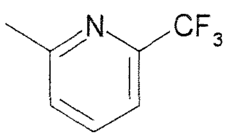 | 567.3 | 3.97 | SB |

(continued)

| Ex. No. | - B | obs MS [M+1] | tR (min) | Measurement Method |
|---|---|---|---|---|
| AP5 | 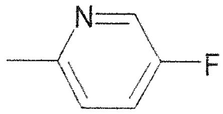 | 517.4 | 2.92 | SB |
| AP6 | 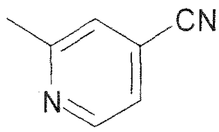 | 524.4 | 3.32 | SB |
| AP7 | 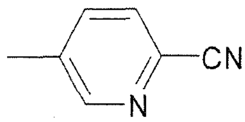 | 524.4 | 3.36 | SB |
| AP8 | 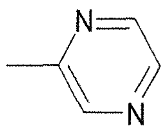 | 500.3 | 2.99 | SB |

[Table 148]

| Ex. No. | - B | obs MS [M+1] | tR (min) | Measurement Method |
|---|---|---|---|---|
| AP9 | 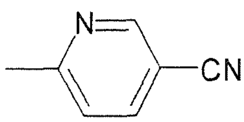 | 524.4 | 3.40 | SB |

[Chemical formula 122]

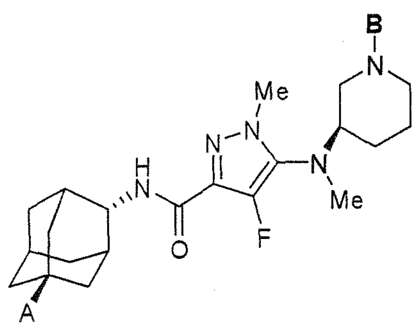

[Table 149]

| Ex. No. | -B | obs MS [M+1] | tR (min) | Measurement Method |
|---|---|---|---|---|
| AQ1 | 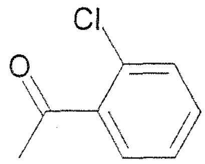 | 544.5 | 3.58 | SA |
| AQ2 | 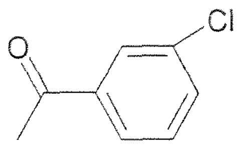 | 544.5 | 3.68 | SA |
| AQ3 | 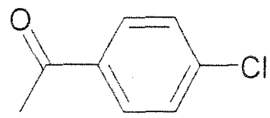 | 544.5 | 3.65 | SA |
| AQ4 | 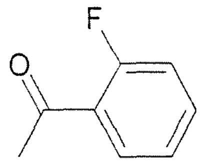 | 528.6 | 3.48 | SA |
| AQ5 | 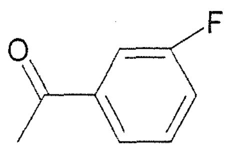 | 528.6 | 3.51 | SA |
| AQ6 | 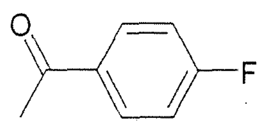 | 528.6 | 3.5 | SA |
| AQ7 | 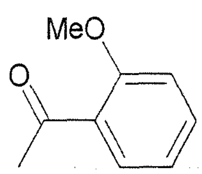 | 540.6 | 3.43 | SA |
| AQ8 | 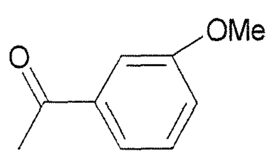 | 540.6 | 3.48 | SA |

[Table 150]

| Ex. No. | -B | obs MS [M+1] | tR (min) | Measurement Method |
|---|---|---|---|---|
| AQ9 | 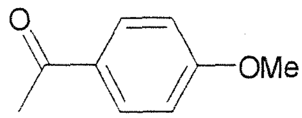 | 540.6 | 3.47 | SA |
| AQ10 | 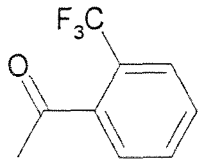 | 578.5 | 3.72 | SA |
| AQ11 | 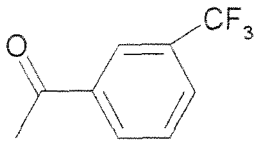 | 578.5 | 3.81 | SA |
| AQ12 | 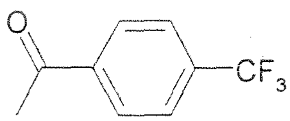 | 578.6 | 3.83 | SA |
| AQ13 | 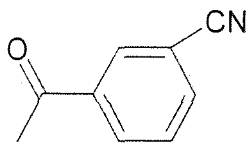 | 535.7 | 3.39 | SA |
| AQ14 | 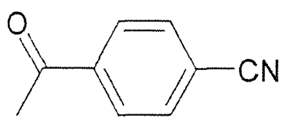 | 535.5 | 3.39 | SA |
| AQ15 | 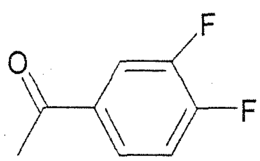 | 546.7 | 3.6 | SA |
| AQ16 | 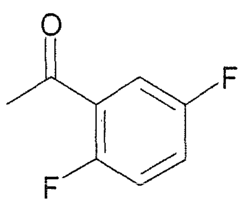 | 546.7 | 3.57 | SA |

[Table 151]

| Ex. No. | - B | obs MS [M+1] | tR (min) | Measurement Method |
|---|---|---|---|---|
| AQ17 | 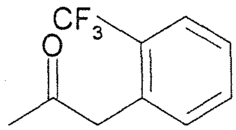 | 592.5 | 3.85 | SA |
| AQ18 | 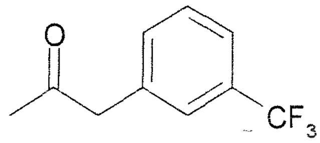 | 592.5 | 3.92 | SA |
| AQ19 | 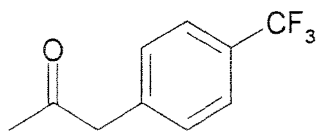 | 592.4 | 3.9 | SA |
| AQ20 | 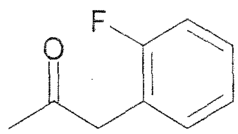 | 542.4 | 3.61 | SA |
| AQ21 | 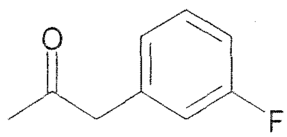 | 542.5 | 3.6 | SA |
| AQ22 | 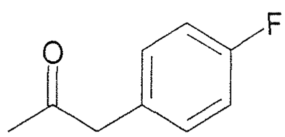 | 542.5 | 3.6 | SA |
| AQ23 | 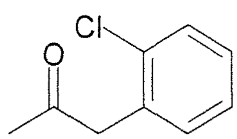 | 558.6 | 3.7 | SA |
| AQ24 | 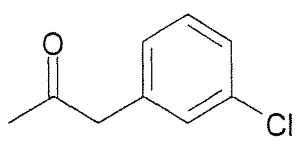 | 558.6 | 3.77 | SA |

[Table 152]

| Ex. No. | - B | obs MS [M+1] | tR (min) | Measurement Method |
|---|---|---|---|---|
| AQ25 | 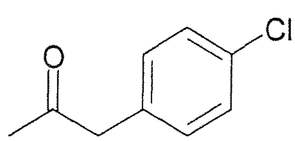 | 558.6 | 3.76 | SA |

(continued)

| Ex. No. | - B | obs MS [M+1] | tR (min) | Measurement Method |
|---|---|---|---|---|
| AQ26 | 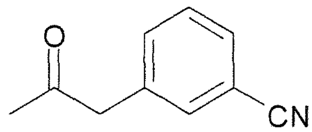 | 549.5 | 3.49 | SA |
| AQ27 | 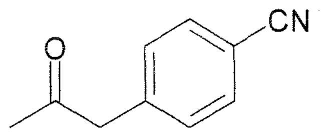 | 549.6 | 3.46 | SA |
| AQ28 | 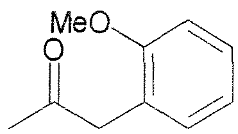 | 554.6 | 3.6 | SA |
| AQ29 | 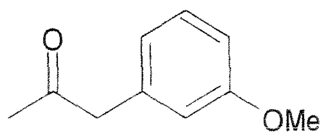 | 554.6 | 3.54 | SA |
| AQ30 | 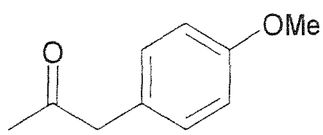 | 554.6 | 3.53 | SA |
| AQ31 | 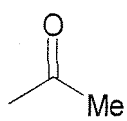 | 448.6 | 3.03 | SA |
| AQ32 | 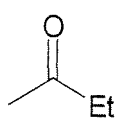 | 462.4 | 3.2 | SA |

[Table 153]

| Ex. No. | - B | obs MS [M+1] | tR (min) | Measurement Method |
|---|---|---|---|---|
| AQ33 | 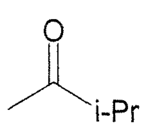 | 476.4 | 3.49 | SA |
| AQ34 | 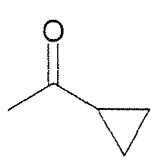 | 474.6 | 3.28 | SA |

(continued)

| Ex. No. | - B | obs MS [M+1] | tR (min) | Measurement Method |
|---|---|---|---|---|
| AQ35 | 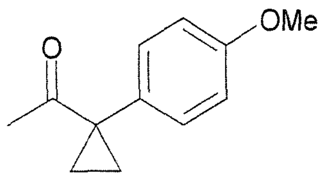 | 580.5 | 3.61 | SA |
| AQ36 | 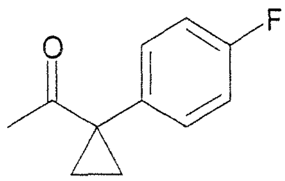 | 568.6 | 3.74 | SA |
| AQ37 | 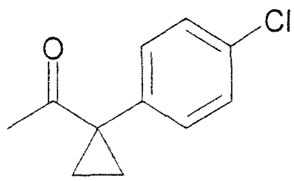 | 584.5 | 3.9 | SA |
| AQ38 | 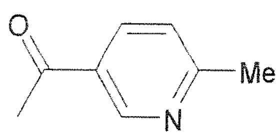 | 525.6 | 2.75 | SA |
| AQ39 | 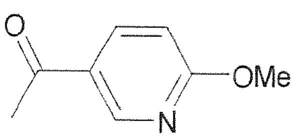 | 541.7 | 3.3 | SA |
| AQ40 | 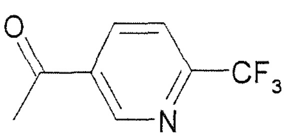 | 579.6 | 3.59 | SA |

[Table 154]

| Ex. No. | -B | obs MS [M+1] | tR (min) | Measurement Method |
|---|---|---|---|---|
| AQ41 | 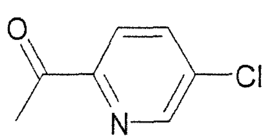 | 545.6 | 3.43 | SA |
| AQ42 | 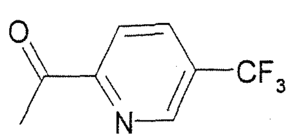 | 579.6 | 3.62 | SA |

(continued)

| Ex. No. | -B | obs MS [M+1] | tR (min) | Measurement Method |
|---|---|---|---|---|
| AQ43 | 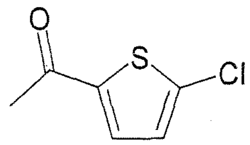 | 550.4 | 3.76 | SA |
| AQ44 | 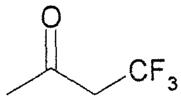 | 516.7 | 3.43 | SA |

[Table 155]

| Ex. No. | -B | obs MS [M+1] | tR (min) | Measurement Method |
|---|---|---|---|---|
| AR1 | 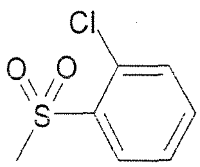 | 580.4 | 3.93 | SA |
| AR2 | 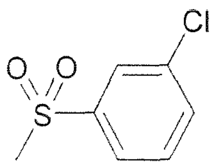 | 580.4 | 4.05 | SA |
| AR3 | 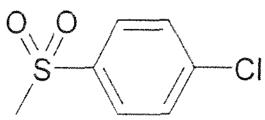 | 580.4 | 4.04 | SA |
| AR4 | 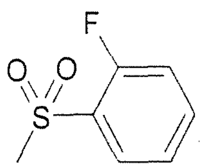 | 564.5 | 3.83 | SA |
| AR5 | 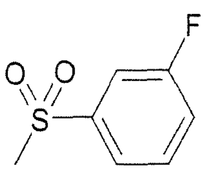 | 564.6 | 3.88 | SA |
| AR6 | 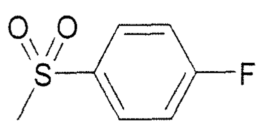 | 564.5 | 3.88 | SA |

(continued)

| Ex. No. | -B | obs MS [M+1] | tR (min) | Measurement Method |
|---|---|---|---|---|
| AR7 | 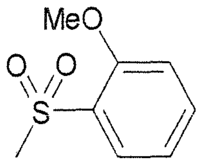 | 576.4 | 3.73 | SA |
| AR8 | 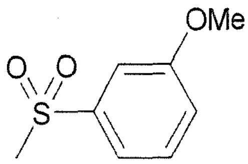 | 576.4 | 3.87 | SA |

[Table 156]

| Ex. No. | - B | obs MS [M+1] | tR (min) | Measurement Method |
|---|---|---|---|---|
| AR9 | 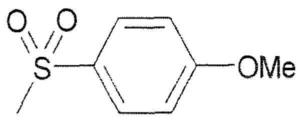 | 576.5 | 3.84 | SA |
| AR10 | 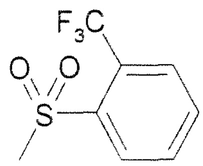 | 614.3 | 4.02 | SA |
| AR11 | 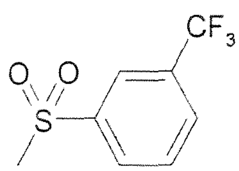 | 614.4 | 4.14 | SA |
| AR12 | 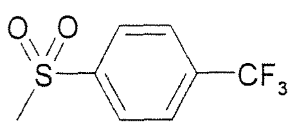 | 614.4 | 4.17 | SA |
| AR13 | 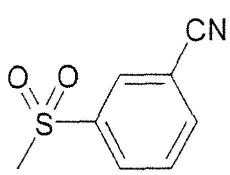 | 571.5 | 3.76 | SA |
| AR14 | 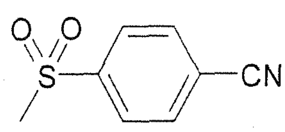 | 571.5 | 3.79 | SA |

(continued)

| Ex. No. | - B | obs MS [M+1] | tR (min) | Measurement Method |
|---|---|---|---|---|
| AR15 | 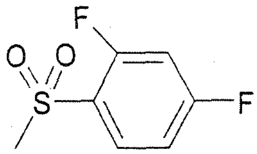 | 582.5 | 3.92 | SA |
| AR16 | 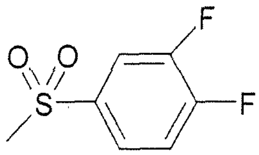 | 582.5 | 3.97 | SA |

[Table 157]

| Ex. No. | -B | obs MS [M+1] | tR (min) | Measurement Method |
|---|---|---|---|---|
| AR17 | 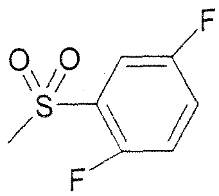 | 582.2 | 3.92 | SA |
| AR18 | 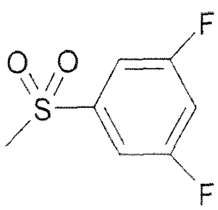 | 582.4 | 3.99 | SA |
| AR19 | 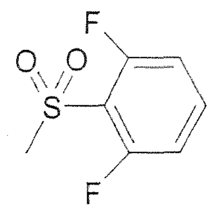 | 582.4 | 3.82 | SA |
| AR20 | 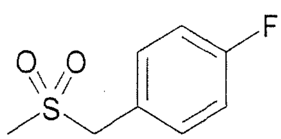 | 578.5 | 3.82 | SA |
| AR21 | 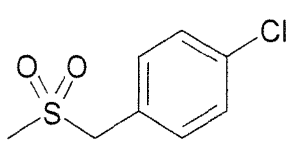 | 594.4 | 3.98 | SA |

(continued)

| Ex. No. | -B | obs MS [M+1] | tR (min) | Measurement Method |
|---|---|---|---|---|
| AR22 | 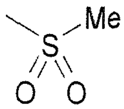 | 484.4 | 3.23 | SA |
| AR23 | 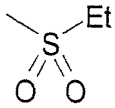 | 498.6 | 3.37 | SA |
| AR24 | 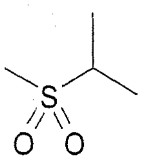 | 512.7 | 3.52 | SA |

[Table 158]

| Ex. No. | -B | obs MS [M+1] | tR (min) | Measurement Method |
|---|---|---|---|---|
| AR25 | 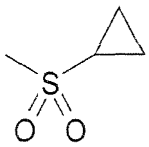 | 510.7 | 3.44 | SA |
| AR26 | 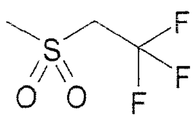 | 552.6 | 3.69 | SA |
| AR27 | 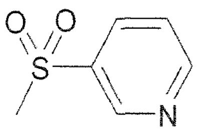 | 547.6 | 3.4 | SA |

[Table 159]

| Ex. No. | -B | obs MS [M+1] | tR (min) | Measurement Method |
|---|---|---|---|---|
| AS1 | 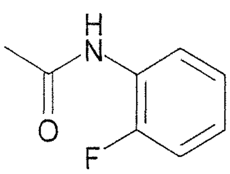 | 543.4 | 3.11 | SB |

(continued)

| Ex. No. | -B | obs MS [M+1] | tR (min) | Measurement Method |
|---|---|---|---|---|
| AS2 | 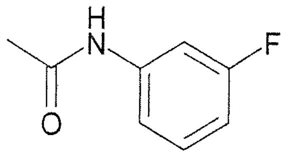 | 543.4 | 3.24 | SB |
| AS3 | 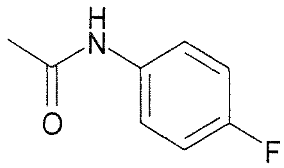 | 543.4 | 3.17 | SB |
| AS4 | 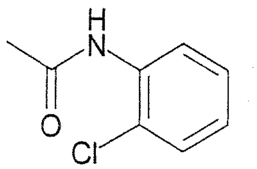 | 559.4 | 3.27 | SB |
| AS5 | 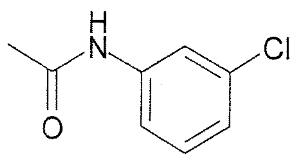 | 559.4 | 3.38 | SB |
| AS6 | 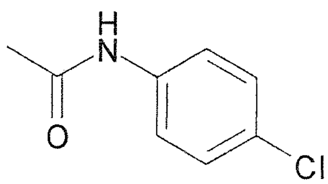 | 559.4 | 3.34 | SB |
| AS7 | 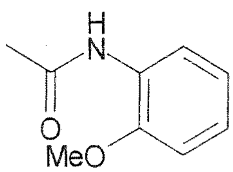 | 555.5 | 3.27 | SB |

[Table 160]

| Ex. No. | - B | obs MS [M+1] | tR (min) | Measurement Method |
|---|---|---|---|---|
| AS9 | 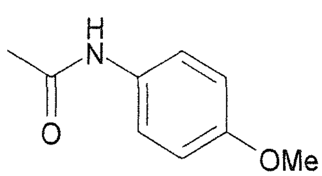 | 555.2 | 3.07 | SB |

(continued)

| Ex. No. | - B | obs MS [M+1] | tR (min) | Measurement Method |
|---|---|---|---|---|
| AS10 | 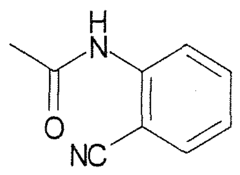 | 550.4 | 3.02 | SB |
| AS11 | 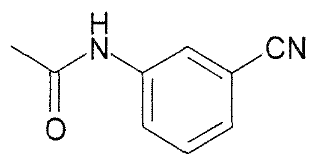 | 550.4 | 3.15 | SB |
| AS12 | 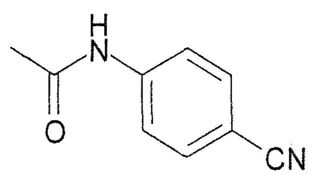 | 550.4 | 3.13 | SB |
| AS13 | 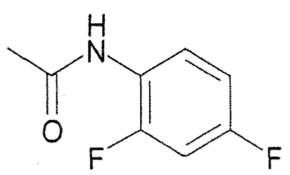 | 561.4 | 3.15 | SB |
| AS14 | 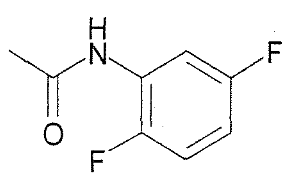 | 561.4 | 3.25 | SB |
| A15 | 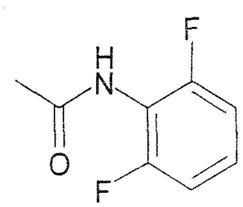 | 561.4 | 3.03 | SB |

[Table 161]

| Ex. No. | - B | obs MS [M+1] | tR (min) | Measurement Method |
|---|---|---|---|---|
| AS17 | 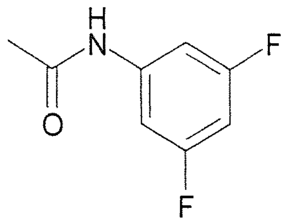 | 561.4 | 3.38 | SB |

(continued)

| Ex. No. | - B | obs MS [M+1] | tR (min) | Measurement Method |
|---|---|---|---|---|
| AS18 | 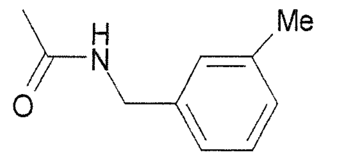 | 553.2 | 3.24 | SB |
| AS19 | 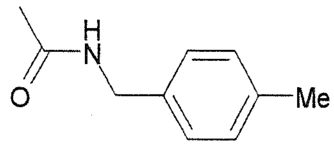 | 553.5 | 3.24 | SB |
| AS20 | 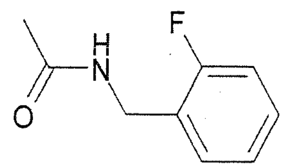 | 557.4 | 3.15 | SB |
| AS21 | 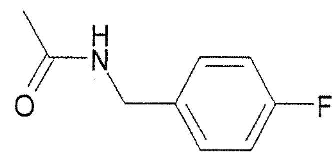 | 557.4 | 3.15 | SB |
| AS22 | 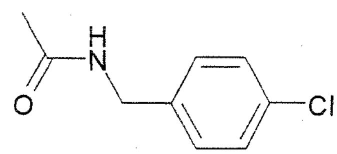 | 573.4 | 3.3 | SB |
| AS23 | 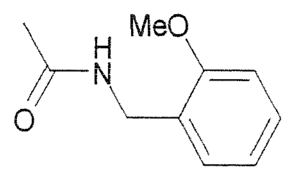 | 569.5 | 3.21 | SB |

[Table 162]

| Ex. No. | -B | obs MS [M+1] | tR (min) | Measurement Method |
|---|---|---|---|---|
| AS25 | 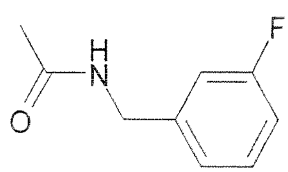 | 557.4 | 3.15 | SB |

(continued)

| Ex. No. | -B | obs MS [M+1] | tR (min) | Measurement Method |
|---|---|---|---|---|
| AS26 | 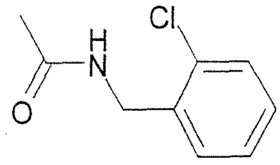 | 573.4 | 3.27 | SB |
| AS27 | 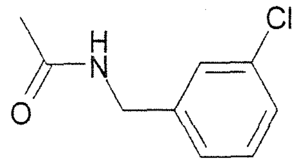 | 573.4 | 3.28 | SB |
| AS28 | 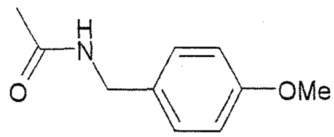 | 569.5 | 3.17 | SB |
| AS29 | 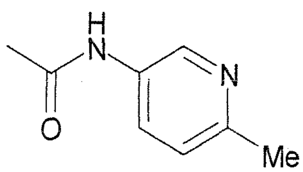 | 540.4 | 3.5 | SB |
| AS30 | 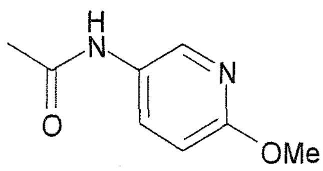 | 556.3 | 2.78 | SB |
| AS31 | 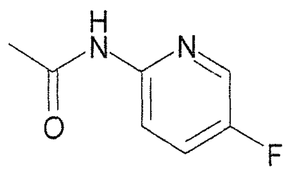 | 544.3 | 2.85 | SB |
| AS32 | 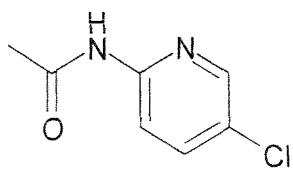 | 560.5 | 3.14 | SB |

[Table 163]

| Ex. No. | - B | obs MS [M+1] | tR (min) | Measurement Method |
|---|---|---|---|---|
| AS33 | 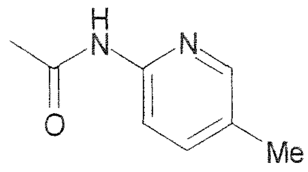 | 540.4 | 3.47 | SB |
| AS34 | 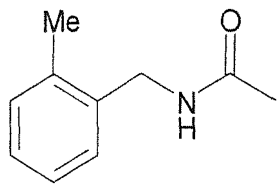 | 553.2 | 3.19 | SB |
| AS35 | 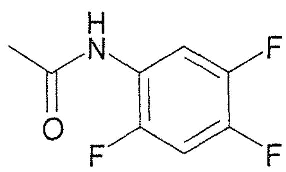 | 579.3 | 3.29 | SB |
| AS36 | 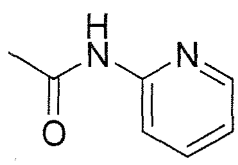 | 526.4 | 2.4 | SB |

[Table 164]

| Ex. No. | -B | obs MS [M+1] | tR (min) | Measurement Method |
|---|---|---|---|---|
| AT1 | 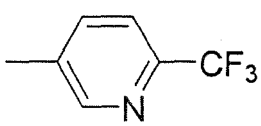 | 551.3 | 3.55 | SB |
| AT2 | 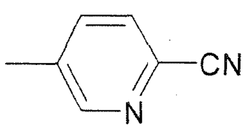 | 508.4 | 3.19 | SB |
| AT3 | 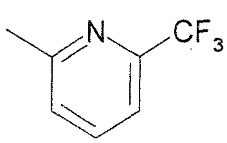 | 551.3 | 3.86 | SB |
| AT4 | 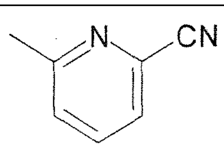 | 508.4 | 2.92 | SB |

(continued)

| Ex. No. | -B | obs MS [M+1] | tR (min) | Measurement Method |
|---|---|---|---|---|
| AT5 | 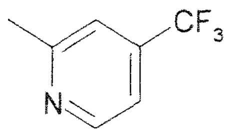 | 551.3 | 3.30 | SB |
| AT6 | 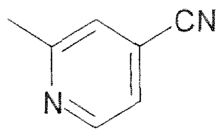 | 508.4 | 3.21 | SB |
| AT7 | 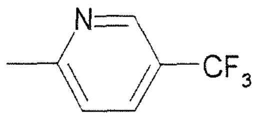 | 551.3 | 3.21 | SB |
| AT8 | 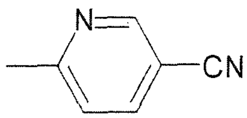 | 508.4 | 3.32 | SB |

[Table 165]

| Ex. No. | -B | obs MS [M+1] | tR (min) | Measurement Method |
|---|---|---|---|---|
| AT9 | 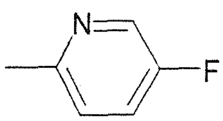 | 501.4 | 2.82 | SB |
| AT10 | 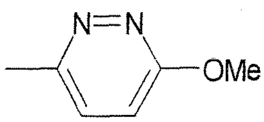 | 514.6 | 2.36 | SB |
| AT11 | 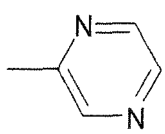 | 484.6 | 2.90 | SB |

[Chemical formula 123]

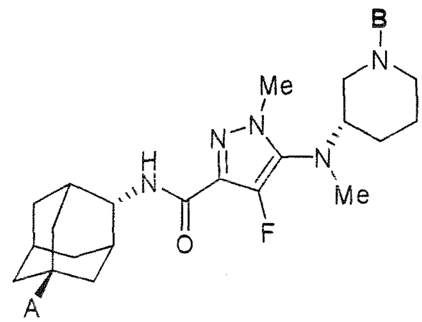

[Table 166]

| Ex. No. | -B | obs MS [M+1] | tR (min) | Measurement Method |
|---|---|---|---|---|
| AU1 | 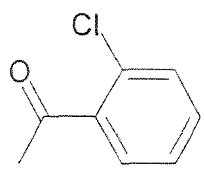 | 544.4 | 3.58 | SA |
| AU2 | 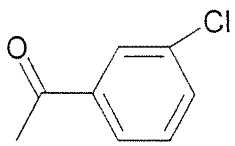 | 544.6 | 3.68 | SA |
| AU3 | 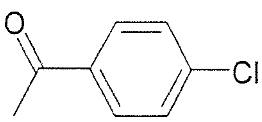 | 544.5 | 3.67 | SA |
| AU4 | 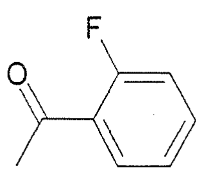 | 528.6 | 3.5 | SA |
| AU5 | 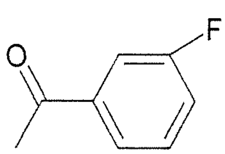 | 528.6 | 3.53 | SA |
| AU6 | 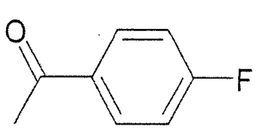 | 528.6 | 3.51 | SA |

(continued)

| Ex. No. | -B | obs MS [M+1] | tR (min) | Measurement Method |
|---|---|---|---|---|
| AU7 | 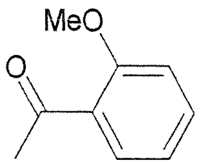 | 540.6 | 3.44 | SA |
| AU8 | 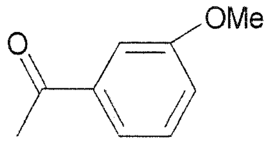 | 540.6 | 3.49 | SA |

[Table 167]

| Ex. No. | -B | obs MS [M+1] | tR (min) | Measurement Method |
|---|---|---|---|---|
| AU9 | 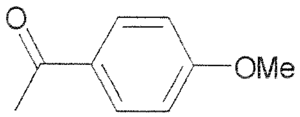 | 540.6 | 3.48 | SA |
| AU10 | 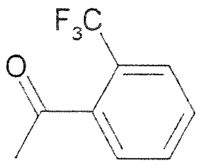 | 578.5 | 3.72 | SA |
| AU11 | 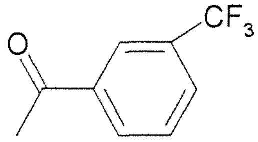 | 578.5 | 3.82 | SA |
| AU12 | 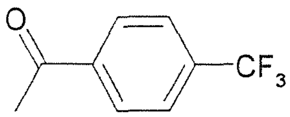 | 578.5 | 3.83 | SA |
| AU13 | 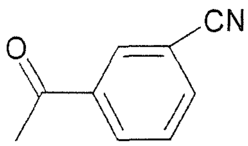 | 535.7 | 3.4 | SA |
| AU14 | 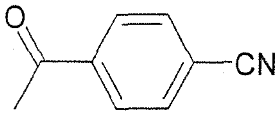 | 535.6 | 3.39 | SA |

(continued)

| Ex. No. | -B | obs MS [M+1] | tR (min) | Measurement Method |
|---|---|---|---|---|
| AU15 | 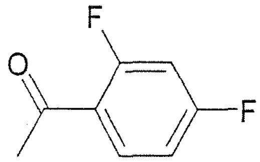 | 546.7 | 3.58 | SA |
| AU16 | 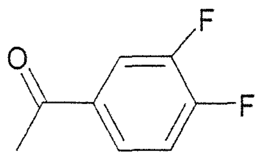 | 546.7 | 3.76 | SA |

[Table 168]

| Ex. No. | -B | obs MS [M+1] | tR (min) | Measurement Method |
|---|---|---|---|---|
| AU17 | 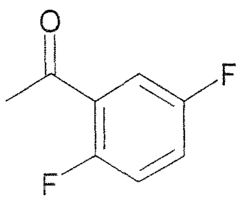 | 546.7 | 3.59 | SA |
| AU18 | 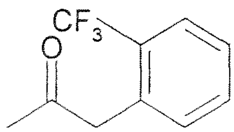 | 592.5 | 3.85 | SA |
| AU19 | 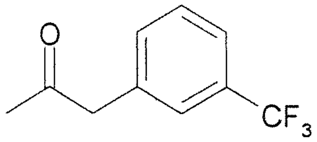 | 592.5 | 3.91 | SA |
| AU20 | 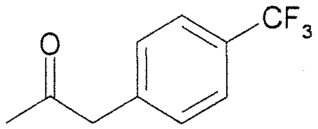 | 592.5 | 3.87 | SA |
| AU21 | 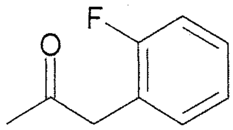 | 542.5 | 3.61 | SA |
| AU22 | 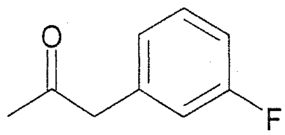 | 542.5 | 3.62 | SA |

(continued)

| Ex. No. | -B | obs MS [M+1] | tR (min) | Measurement Method |
|---|---|---|---|---|
| AU23 | 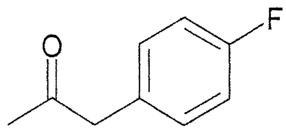 | 542.5 | 3.6 | SA |
| AU24 | 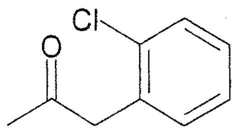 | 558.6 | 3.88 | SA |

[Table 169]

| Ex. No. | -B | obs MS [M+1] | tR (min) | Measurement Method |
|---|---|---|---|---|
| AU25 | 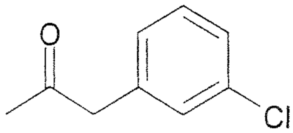 | 558.6 | 3.77 | SA |
| AU26 | 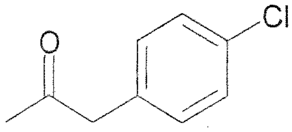 | 558.5 | 3.76 | SA |
| AU27 | 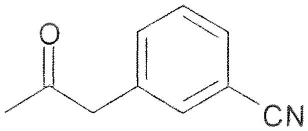 | 549.7 | 3.47 | SA |
| AU28 | 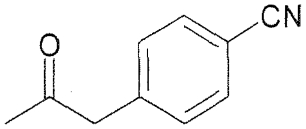 | 549.6 | 3.45 | SA |
| AU29 | 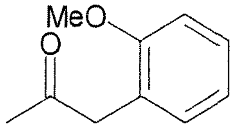 | 554.6 | 3.61 | SA |
| AU30 | 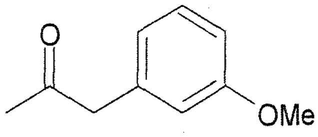 | 554.6 | 3.55 | SA |
| AU31 | 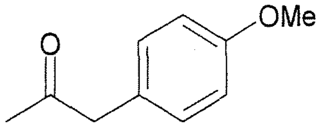 | 554.6 | 3.53 | SA |

(continued)

| Ex. No. | -B | obs MS [M+1] | tR (min) | Measurement Method |
|---|---|---|---|---|
| AU32 | 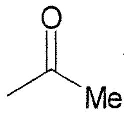 | 448.6 | 3.05 | SA |

[Table 170]

| Ex. No. | -B | obs MS [M+1] | tR (min) | Measurement Method |
|---|---|---|---|---|
| AU33 | 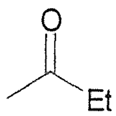 | 462.5 | 3.21 | SA |
| AU34 | 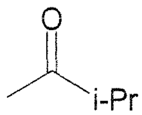 | 476.4 | 3.37 | SA |
| AU35 | 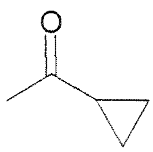 | 474.4 | 3.28 | SA |
| AU36 | 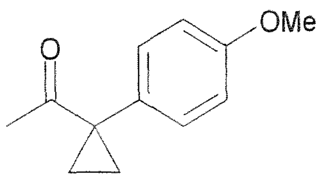 | 580.5 | 3.61 | SA |
| AU37 | 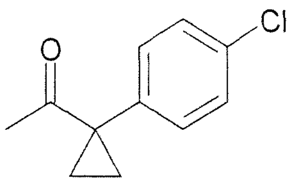 | 584.5 | 3.9 | SA |
| AU38 | 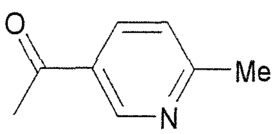 | 525.7 | 2.76 | SA |
| AU39 | 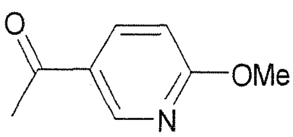 | 541.5 | 3.3 | SA |
| AU40 | 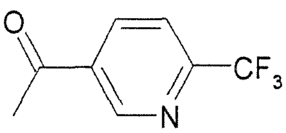 | 579.6 | 3.55 | SA |

[Table 171]

| Ex. No. | -B | obs MS [M+1] | tR (min) | Measurement Method |
|---|---|---|---|---|
| AU41 | 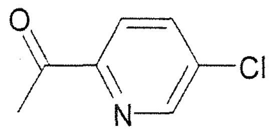 | 545.6 | 3.43 | SA |
| AU42 | 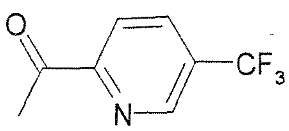 | 579.6 | 3.63 | SA |
| AU43 | 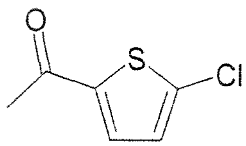 | 550.5 | 3.75 | SA |
| AU44 | 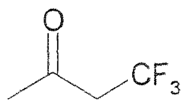 | 516.7 | 3.42 | SA |

[Table 172]

| Ex. No. | -B | obs MS [M+1] | tR (min) | Measurement Method |
|---|---|---|---|---|
| AV1 | 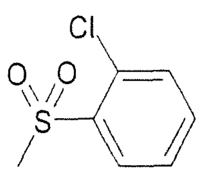 | 580.4 | 3.94 | SA |
| AV2 | 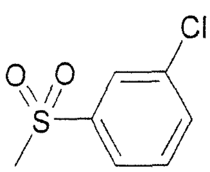 | 580.4 | 4.07 | SA |
| AV3 | 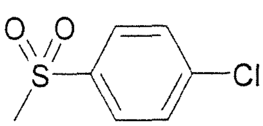 | 580.4 | 4.06 | SA |
| AV4 | 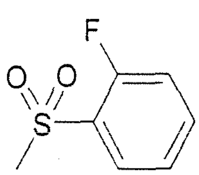 | 564.4 | 3.83 | SA |

(continued)

| Ex. No. | -B | obs MS [M+1] | tR (min) | Measurement Method |
|---|---|---|---|---|
| AV5 | 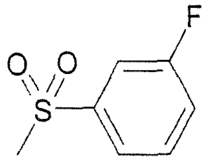 | 564.4 | 3.9 | SA |
| AV6 | 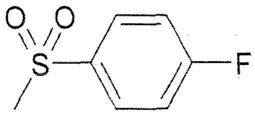 | 564.6 | 3.88 | SA |
| AV7 | 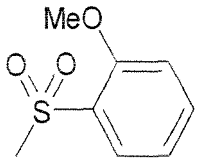 | 576.5 | 3.74 | SA |
| AV8 | 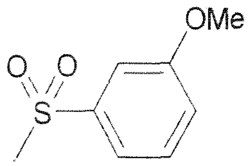 | 576.5 | 3.88 | SA |

[Table 173]

| Ex. No. | -B | obs MS [M+1] | tR (min) | Measurement Method |
|---|---|---|---|---|
| AV9 | 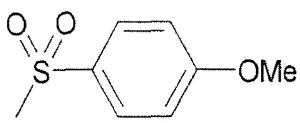 | 576.5 | 3.84 | SA |
| AV10 | 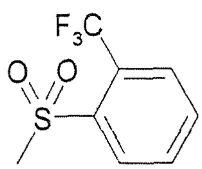 | 614.4 | 4.03 | SA |
| AV11 | 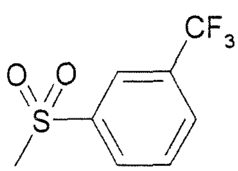 | 614.3 | 4.14 | SA |
| AV12 | 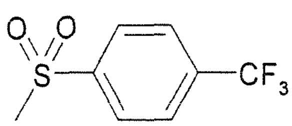 | 614.4 | 4.17 | SA |

(continued)

| Ex. No. | -B | obs MS [M+1] | tR (min) | Measurement Method |
|---|---|---|---|---|
| AV13 | 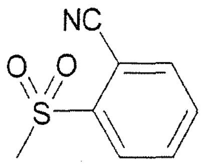 | 571.5 | 3.71 | SA |
| AV14 | 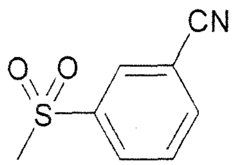 | 571.5 | 3.76 | SA |
| AV15 | 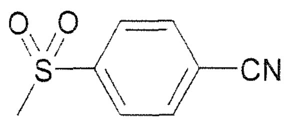 | 571.5 | 3.79 | SA |
| AV16 | 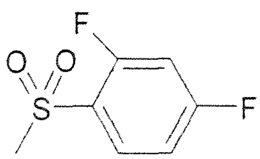 | 582.3 | 3.92 | SA |

[Table 174]

| Ex. No. | - B | obs MS [M+1] | tR (min) | Measurement Method |
|---|---|---|---|---|
| AV17 | 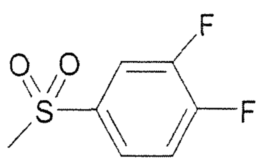 | 582.3 | 3.98 | SA |
| AV18 | 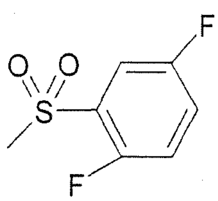 | 582.3 | 3.93 | SA |
| AV19 | 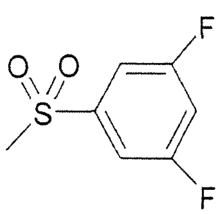 | 582.4 | 4.01 | SA |

(continued)

| Ex. No. | - B | obs MS [M+1] | tR (min) | Measurement Method |
|---|---|---|---|---|
| AV20 | 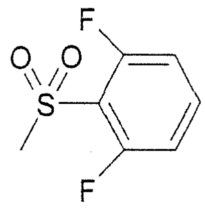 | 582.3 | 3.97 | SA |
| AV21 | 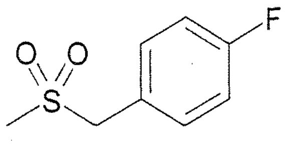 | 578.5 | 3.83 | SA |
| AV22 | 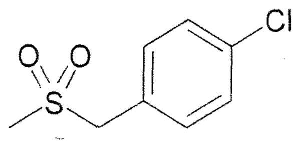 | 594.4 | 4 | SA |
| AV23 | 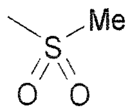 | 484.5 | 3.4 | SA |
| AV24 | 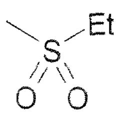 | 498.6 | 3.37 | SA |

[Table 175]

| Ex. No. | - B | obs MS [M+1] | tR (min) | Measurement Method |
|---|---|---|---|---|
| AV25 | 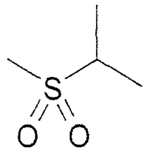 | 512.7 | 3.51 | SA |
| AV26 | 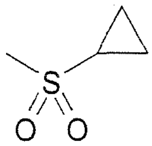 | 510.5 | 3.46 | SA |
| AV27 | 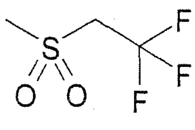 | 552.4 | 3.7 | SA |

(continued)

| Ex. No. | - B | obs MS [M+1] | tR (min) | Measurement Method |
|---|---|---|---|---|
| AV28 | 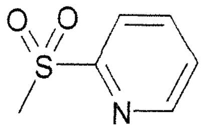 | 547.6 | 3.4 | SA |

[Table 176]

| Ex. No. | - B | obs MS [M+1] | tR (min) | Measurement Method |
|---|---|---|---|---|
| AW1 | 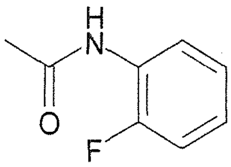 | 543.4 | 3.11 | SB |
| AW2 | 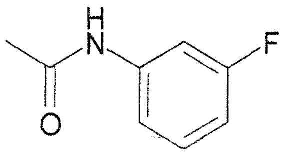 | 343.4 | 3.24 | SB |
| AW3 | 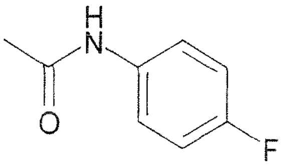 | 543.4 | 3.15 | SB |
| AW4 | 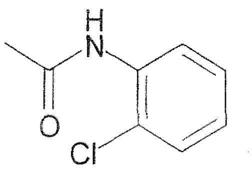 | 559.6 | 3.74 | SA |
| AW5 | 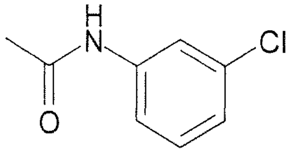 | 559.5 | 3.81 | SA |
| AW6 | 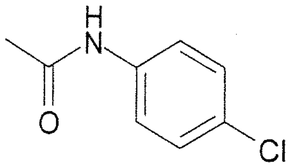 | 559.5 | 3.79 | SA |

(continued)

| Ex. No. | - B | obs MS [M+1] | tR (min) | Measurement Method |
|---|---|---|---|---|
| AW7 | 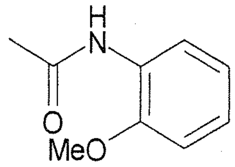 | 555.2 | 3.21 | SB |

[Table 177]

| Ex. No. | -B | obs MS [M+1] | tR (min) | Measurement Method |
|---|---|---|---|---|
| AW9 | 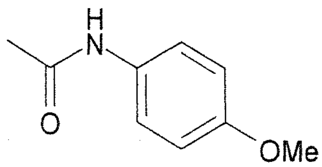 | 555.5 | 3.07 | SB |
| AW10 | 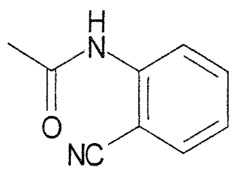 | 550.7 | 3.08 | SA |
| AW11 | 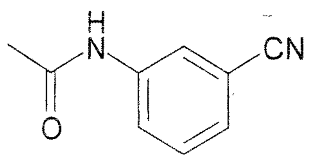 | 550.7 | 3.18 | SA |
| AW12 | 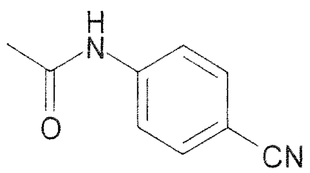 | 550.7 | 3.69 | SA |
| AW13 | 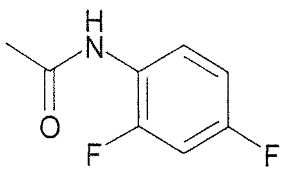 | 561.7 | 3.58 | SA |
| AW14 | 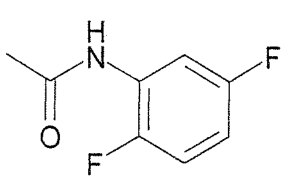 | 561.4 | 3.32 | SA |

(continued)

| Ex. No. | -B | obs MS [M+1] | tR (min) | Measurement Method |
|---|---|---|---|---|
| AW15 | 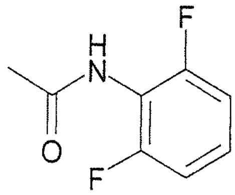 | 561.4 | 3.13 | SB |

[Table 178]

| Ex. No. | - B | obs MS [M+1] | tR (min) | Measurement Method |
|---|---|---|---|---|
| AW17 | 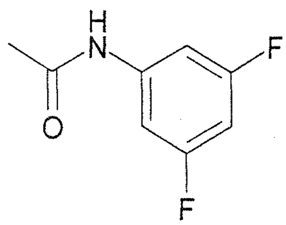 | 561.7 | 3.96 | SB |
| AW18 | 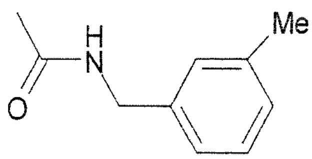 | 553.8 | 3.9 | SB |
| AW19 | 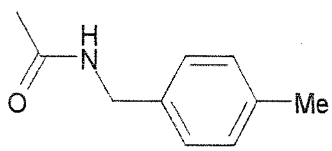 | 553.5 | 3.86 | SB |
| AW20 | 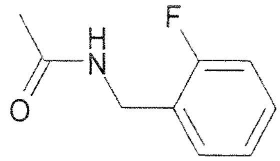 | 557.7 | 3.72 | SB |
| AW21 | 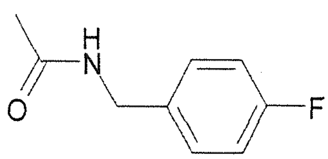 | 557.7 | 3.76 | SB |
| AW22 | 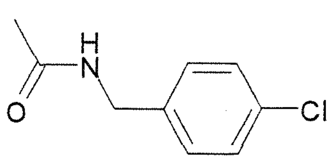 | 573.4 | 3.28 | SB |

(continued)

| Ex. No. | - B | obs MS [M+1] | tR (min) | Measurement Method |
|---|---|---|---|---|
| AW23 | 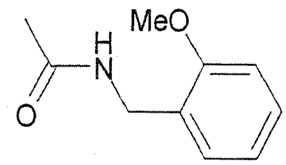 | 569.5 | 3.15 | SB |

[Table 179]

| Ex. No. | - B | obs MS [M+1] | tR (min) | Measurement Method |
|---|---|---|---|---|
| AW25 | 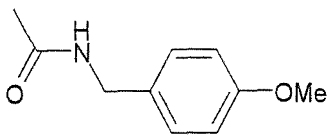 | 569.5 | 3.1 | SB |
| AW26 | 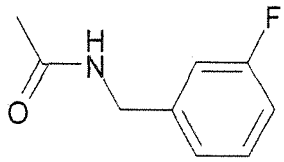 | 557.4 | 3.23 | SB |
| AW27 | 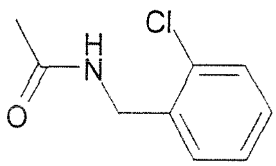 | 573.4 | 3.26 | SB |
| AW28 | 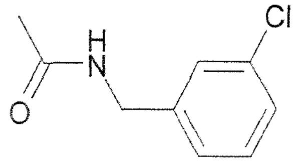 | 573.4 | 3.29 | SB |
| AW29 | 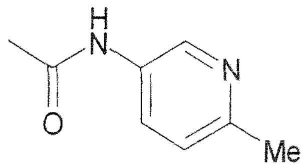 | 540.4 | 2.4 | SB |
| AW30 | 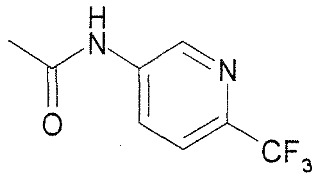 | 594.4 | 3.21 | SB |

(continued)

| Ex. No. | - B | obs MS [M+1] | tR (min) | Measurement Method |
|---|---|---|---|---|
| AW31 | 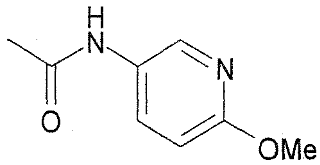 | 556.3 | 2.73 | SB |
| AW32 | 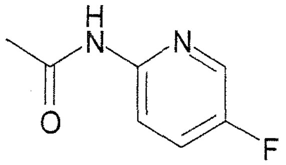 | 544.6 | 2.86 | SB |

[Table 180]

| Ex. No. | -B | obs MS [M+1] | tR (min) | Measurement Method |
|---|---|---|---|---|
| AW33 | 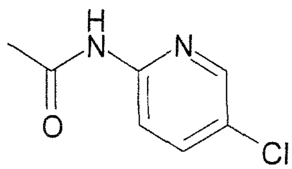 | 560.5 | 3.05 | SB |
| AW34 | 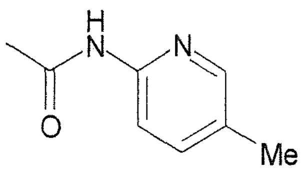 | 540.4 | 2.44 | SB |
| AW35 | 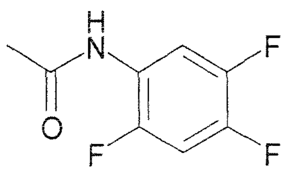 | 579.3 | 3.34 | SB |
| AW36 | 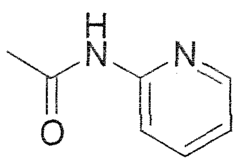 | 526.4 | 2.38 | SB |
| AW37 | 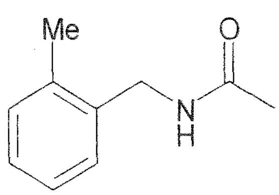 | 553.2 | 3.21 | SB |

[Table 181]

| Ex. No. | - B | obs MS [M+1] | tR (min) | Measurement Method |
|---|---|---|---|---|
| AX1 | 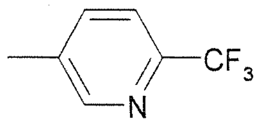 | 551.3 | 3.55 | SB |
| AX2 | 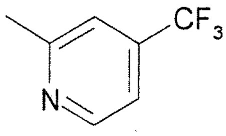 | 551.3 | 3.30 | SB |
| AX3 | 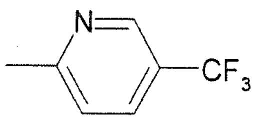 | 551.3 | 3.54 | SB |
| AX4 | 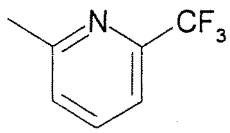 | 551.3 | 3.84 | SB |
| AX5 | 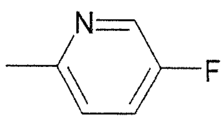 | 501.4 | 2.82 | SB |
| AX6 | 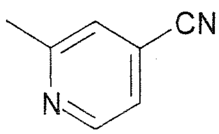 | 508.4 | 3.19 | SB |
| AX7 | 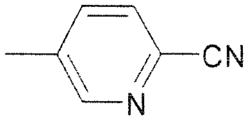 | 508.4 | 3.20 | SB |
| AX8 | 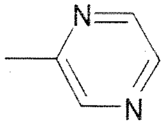 | 484.6 | 2.90 | SB |

[Table 182]

| Ex. No. | -B | obs MS [M+1] | tR (min) | Measurement Method |
|---|---|---|---|---|
| AX9 | 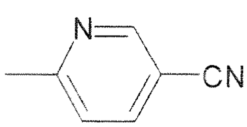 | 508.4 | 3.30 | SB |

Comparative Example 1A

[Chemical Formula 124]

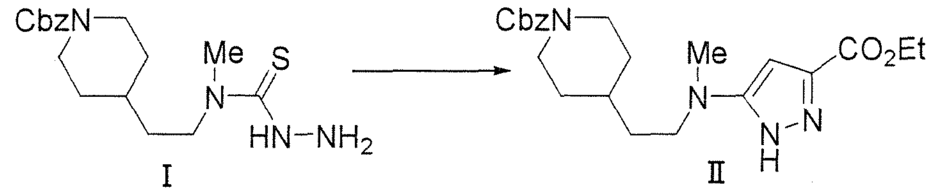

To a solution of Compound I (6.91 g) in 99.5% ethanol (100 mL) was added sodium bicarbonate (3.31 g), and then thereto was added ethyl bromopyruvate (3.0 mL) and then the mixture was stirred at 80˚C. After 4 hours, thereto was added acetic acid (50 mL), and the mixture was stirred at 120˚C. The mixture was stirred overnight, and then cooled to room temperature and concentrated *in vacuo*. To the residue was added saturated sodium bicarbonate water, and the mixture was extracted with ethyl acetate. The organic layer was dried over sodium sulfate and concentrated *in vacuo*. The residue was purified by silica gel chromatography (eluent: hexane/ethyl acetate = 1/1 to chloroform/methanol = 10/1) to give the titled Compound II (2.48 g) (30.4% yields).

Comparative Example 1B

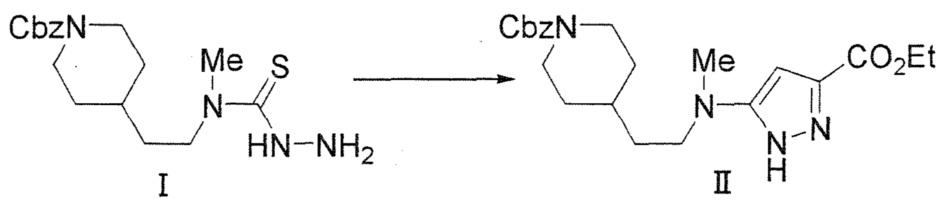

To a solution of Compound I (6.91 g) in 99.5% ethanol (100 mL) was added sodium bicarbonate (3.31 g). Then, thereto was added ethyl bromopyruvate (3.0 mL), and then the mixture was stirred at 80˚C. After 4 hours, thereto was added acetic acid (50 mL), and the mixture was stirred at 120˚C. The mixture was stirred overnight, and then cooled to room temperature and concentrated *in vacuo*. To the residue was added saturated sodium bicarbonate water, and the mixture was extracted with ethyl acetate. The organic layer was dried over sodium sulfate and concentrated *in vacuo*. The residue was purified by silica gel chromatography (eluent: hexane/ethyl acetate = 1/1 to chloroform/methanol = 10/1) to give the titled Compound II (2.48 g) (30.4% yields).

Comparative Example 2

[Chemical Formula 125]

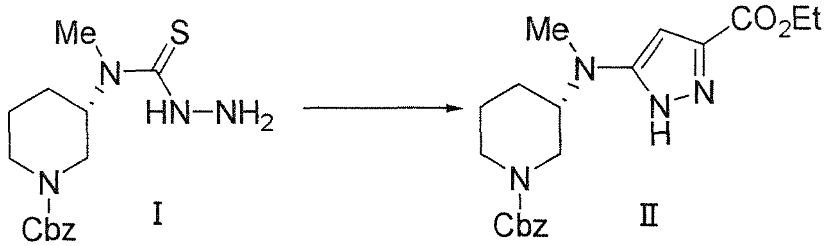

I II

To an ice-cooled mixed solution of Compound I (24.9 g), sodium bicarbonate (9.66 g) and 95% ethanol (350 mL) was added ethyl bromopyruvate (11.9 mL). The mixture was stirred at 80°C for 4 hours, and the reaction solution was evaporated to concentrate *in vacuo*. To the residue was added acetic acid (150 mL), and the mixture was stirred at 130°C for 18 hours and then concentrated *in vacuo*. To the residue was added saturated sodium bicarbonate water, and the mixture was extracted with ethyl acetate. The organic layer was dried over sodium sulfate and concentrated *in vacuo*. The residue was purified by silica gel chromatography (eluent: hexane/ethyl acetate = 1/1) to give the titled Compound II (14.6 g) (49% yields).

Comparative Example 3

[Chemical Formula 126]

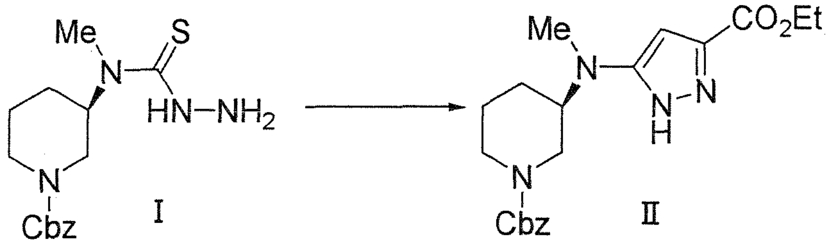

I II

To an ice-cooled solution of Compound I (16.44 g) in 99.5% ethanol (125 mL) was added ethyl bromopyruvate (7.83 mL), and then the mixture was stirred at 80°C. After 4 hours, the reaction solution was evaporated to concentrate *in vacuo*. To the residue was added acetic acid (150 mL), and the mixture was stirred at 130°C for 18 hours and then concentrated *in vacuo*. To the residue was added saturated sodium bicarbonate water, and the mixture was extracted with ethyl acetate. The organic layer was dried over sodium sulfate and concentrated *in vacuo*. The residue was purified by silica gel chromatography (eluent: hexane/ethyl acetate = 1/1) to give the titled Compound II (1.43 g) (7% yields).

Experiment 1 (Preparation of human 11βHSD1 enzyme source)

A sequence containing ORF of human 11βHSD1 gene (GenBank Accession No. BC012593) was amplified according to PCR technique which was one of conventional methods, and digested by a restriction enzyme BamHI/XhoI. 2kb of DNA fragments obtained from agarose gels were inserted into pCMV-Tag 2B plasmids (Stratagene) according to a conventional method. The plasmids prepared in Escherichia coli in large amounts were transformed into CHO-K1 cells, and then cells stably expressing human 11βHSD1 genes were selected by medium containing 400 μg/ml G-418 solutions (GIBCO, Inc.) containing media. The resulting stably expressing cells were incubated up to -90% confluent in F-12 medium (Nacalai Tesque, Inc.) containing 10% charcoal-dextran treated fetal bovine serum (Hyclone), 1% penicillin-streptomycin (Nacalai Tesque, Inc.) and 400 μg/ml G-418. The resultant cells were treated by tripsin, and the obtained cells were suspended in the above media (1 L) and seeded in cell stack 10 chamber (Corning) in total amounts. The mixture was incubated in $CO_2$ incubator (5% $CO_2$, 37°C) for 4-5 days, and then treated by tripsin. Total amounts of the obtained cells were washed with PBS buffer (GIBCO, Inc.), and stored at -80°C. The cells were suspended in 8 ml of buffer (50 mM HEPES pH7.3, 5% glycerol, 1 mM EDTA, protease inhibitor cocktail (Roche)), and then disrupted. The resulting solution was centrifuged at 1,500 rpm for 10 minutes, and then the supernatant was ultracentrifuged at 100,000 g for 1 hour. The precipitate after the ultracentrifugation was collected and suspended in buffer (50 mM HEPES

pH7.3, 5% glycerol, 1 mM EDTA), and then dispensed to store at -80°C. The resulting enzyme fractions were used as human 11βHSD1 enzyme fractions in the following Experiments.

Experiment 2 (Measurement of human 11βHSD1 inhibitory activity)

A test compound and cortisone (Sigma) was diluted with buffer (50 mM HEPES pH7.3, 150 mM NaCl, 1 mM EDTA) to prepare a substrate solution containing a test compound (50 mM HEPES pH7.3, 150 mM NaCl, 1 mM EDTA, 1 mM NADPH, 20 nM cortisone) (2% DMSO solution), and the solution was added to 384-well low-volume plate (manufactured by Greiner, No. 3782086) in 4 μl/well. Then, human 11βHSD1 enzyme fractions obtained in Experiment 1 were diluted with buffer (50 mM HEPES pH7.3, 150 mM NaCl, 1 mM EDTA, 5% glycerol) to be 60-100 μg/ml of post-assay concentrations. Human 11βHSD1 enzyme fractions after the dilution was added to each well in 4 μl/well and gently stirred, and then spun down to react at 37°C for 2 hours. After the enzyme reaction, the produced cortisol was detected by Homogeneous time-resolved fluorescence (HTRF) to deteremine enzyme inhibitory activities. Then, thereto were added XL-665 labeled cortisol (or d2-labeled cortisol) containing 400 μM carbenoxolone (Sigma) and cryptate-labeled cortisol antibody (Cisbio International) in 4 μl/well each, and the mixture was gently stirred, and then spun down to store at room temperature for 2 or more hours. A fluorescence intensity was determined by 2120 EnVision® Multilabel counter (PerkinElmer) to calculate enzyme inhibitory activities from 2 wavelengths of fluorescence intensity ratios (665 nm/620 nm).
Each inhibitory activity (%) of each test compound was calculated from the avarage value (%) of inhibitory activities of 4 wells under the same condition. An inhibition rate in a well wherein DMSO was added instead of a test compound was 0% and an inhibition rate in a well without human 11βHSD1 enzyme fractions was 100%. A concentration ($IC_{50}$ value) of a test compound required to inhibit 50% of human 11βHSD1 was calculated.
The result was shown in Table 1.
[Table 183]

| | Example No. | $IC_{50}$ (nM) |
|---|---|---|
| 1 | 11 | 32 |
| 2 | 15 | 39 |
| 3 | 19 | 25 |

Experiment 3 (Inhibitory activity assay for cortisone reducing activity of cultured human adipocytes)

Normal human proadipocytes (HPrAD-vis, Cambrex) were inoculated on 48 well plate, and differentiated according to a protocol attached to a kit. Media for cells on 9-11th day of differentiation were changed to 0.2 ml of D-MEM media (GIBCO, Inc.) containing 100 nM [1,2-$^3$H] cortisone (1 μCi/well, Muromati Yakuhin), 0.5% DMSO, test compound (DMSO only for test compound-addition districts and test compound additive-free districts). After incubation at 37°C for 3 hours, all media were collected. As background districts, cell additive-free media were used. Media were mixed with ethyl acetate (0.1 ml) in Eppendorf tube. The mixture was vortexed, and then centrifuged at 5,000 rpm x 1 minute at room temperature to separate ethyl acetate (upper layer). Ethyl acetate (10 μl) was spotted on aluminum plate for thin-layer chromatography (silica gel 60 angstrom, Merck, referred to as TLC plate hereinafter). To a sealed vessel was added chloroform/methanol (90:10, v/v) as an eluent, and TLC plate was developed and then dried at room temperature. To the dried TLC plate was exposed an imaging plate (TR-2040, FUJIFILM) over 16 or more hours. After exposure, the imaging plate was analyzed by Bioimage analyzer (BAS2500, FUJIFILM), and [$^3$H] radioactivity of the spot corresponding to cortisol on TLC plate was determined. Inhibitory activities of cortisone reducing activities of test compounds were calculated as below.

(Inhibitory activity (%)) = 100 x ((Test compound additive-free districts) - (Test compound-addition districts))/((Test compound additive-free districts) - (Background districts))

$IC_{50}$ values were calculated by a linear regression of logarithmic values of analyte concentrations and inhibitory activity values using 2-point data wherein inhibitory activities indicated values around 50%. $IC_{50}$ values for human adipocyte cortisone reducing activities of the inventive compound usually exist within the range of 0.01-1000 nM. $IC_{50}$ values for

human adipocyte cortisone reducing activities of the following inventive compounds were determined.
[Table 184]

| | Example No. | $IC_{50}$ (nM) |
|---|---|---|
| 1 | 8 | 8.7 |
| 2 | 22 | 1.8 |
| 3 | 65 | 9.1 |
| 4 | 66 | 0.3 |
| 5 | 70 | 39 |
| 6 | 82 | 43 |
| 7 | 129 | 3.5 |
| 8 | 153 | 22 |
| 9 | 160 | 5.6 |
| 10 | 166 | 6.4 |
| 11 | K16 | 11 |
| 12 | I26 | 3.1 |
| 13 | V18 | 21 |
| 14 | AN2 | 6.7 |

According to the experiment of Table 2, the inventive compound group is expected to inhibit an 11βHSD1 activity and cortisol production in the target organ human adipocyte.

Experiment 4 (Inhibitory activity assay for cortisone reducing activity of mouse primary adipocytes)

Adipose tissues (referred to as visceral fat tissues hereinafter) adhered around mesenteries and testicles of 10 of 9-11 week-old ICR male mice (Japan SLC, Inc.) were soaked in phosphate buffer (0.20 g/L KCl, 0.20 g/L $KH_2PO_4$, 8.00 g/L NaCl, 2.16 g/L $Na_2HPO_4 \cdot 7H_2O$, 100 unit/ml penicillin (GIBCO, Inc.), 100 μg/ml streptomycin (GIBCO, Inc.), 250 ng/ml amphotericin (GIBCO, Inc.)) (about 100 ml) and washed at room temperature.
The visceral fat tissues removed as above were cut out in about 5 x 5 mm by scissors in Dulbecco's Modified Eagle Media (containing 4.5 g/L D-glucose and 584 mg/L L-glutamine, GIBCO, Inc.) (about 50 ml) wherein collagenase (type II, Sigma), penicillin (GIBCO, Inc.), streptomycin (GIBCO, Inc.) and amphotericin (GIBCO, Inc.) were added until the final concentration of 1 mg/ml, 100 unit/ml, 100 μg/ml and 250 ng/ml each. Then, the tissues were shaken at 37°C for 30 minutes (about 170 rpm) and filtered through nylon mesh (80S [250 μm mesh], SANSHIN INDUSTRIAL CO., LTD.) to give a filtrate (cell suspension). The filtrate was centrifuged at room temperature at 1800 rpm for 5 minutes, and then the liquid layer was gently removed by decantation to give a precipitate. The precipitate was suspended in Dulbecco's Modified Eagle Media (containing 4.5 g/L D-glucose and 584 mg/L L-glutamine, GIBCO, Inc., also referred to as FBS-containing media hereinafter) (30 ml) wherein fetal bovine serum (referred to as FBS hereinafter) (GIBCO, Inc.), ascorbic acid (Wako Pure Chemical Industries, Ltd.), penicillin (GIBCO, Inc.), streptomycin (GIBCO, Inc.) and amphotericin (GIBCO, Inc.) were added until the final concentration of 10%, 200 μM, 100 unit/ml, 100 μg/ml and 250 ng/ml each, and the suspension was filtered through nylon mesh (420S [25 μm mesh], SANSHIN INDUSTRIAL CO., LTD.). The filtrate was collected and centrifuged at room temperature at 1800 rpm for 5 minutes, and then the liquid layer was gently removed by decantation and the precipitate was suspended again in FBS-containing media (30 ml). The similar treatment of centrifugation, removal of liquid layer and suspension in FBS-containing media was further carried out twice for the resulting suspension to prepare the suspension (90 ml). The suspension was dispensed in flasks for cell incubation (T150 for adhered cells, IWAKI GLASS) by 30 ml each, and incubated at 37°C in the presence of 5% $CO_2$. 5-6 hours after starting incubation, media were removed and flask walls were washed with the phosphate buffer (15 ml). The washing was removed and the washing operation was carried out again. Then, the phosphate buffer was removed, and FBS-containing media (30 ml) was added to flasks and incubated at 37°C in the presence of 5% $CO_2$. 1 or 2 days after starting incubation, media were removed and flask walls were washed with the phosphate buffer (15 ml) once. Then, to

the flask was added tripsin-ethylene diamine tetracetic acid (referred to as tripsin-EDTA hereinafter) solution (0.05% tripsin, 0.53 mM EDTA·4Na, GIBCO, Inc.) so that cells were soaked, and the mixture was incubated at 37°C for 5 minutes. Then, thereto were added FBS-containing media in about tenfold amounts of tripsin-EDTA solution, and the cell suspension was obtained.

The cell suspension was diluted by the addition of FBS-containing media so that the number of cells in the cell suspension was determined by a counting chamber to be 1.4 x $10^5$ cells/ml. The resulting diluent was dispensed in 48 well plate (for incubation of adherent cells, IWAKI GLASS) by 300 μl/well each, and incubated at 37°C for 1-2 days in the presence of 5% $CO_2$. Media were removed from each well of 48 well plate, and FBS-containing media (300 μl) containing 10 μg/ml insulin (Sigma), 0.25 μM dexamethazone (Wako Pure Chemical Industries, Ltd.), 0.5 mM 3-isobutyl-1-methylxanthin (Sigma) and 5 μM 15-deoxy-$\Delta^{12,14}$-prostaglandin $J_2$ (Cayman) were added to each well and incubated at 37°C for 3 days in the presence of 5% $CO_2$. Then, media in each well were removed, and FBS-containing media (300 μl) containing 10 μg/ml insulin and 5 μM 15-deoxy-$\Delta^{12,14}$-prostaglandin $J_2$ were added to each well and incubated for 2 days. Further, media in each well were removed, and FBS-containing media (300 μl) containing 10 μg/ml insulin and 5 μM 15-deoxy-$\Delta^{12,14}$-prostaglandin $J_2$ were added to each well and incubated for 2 days.

Media for adipocyte as differentiated above were changed to 0.2 ml of D-MEM media (GIBCO, Inc.) containing 100 nM [1,2-$^3$H] cortisone (1 μCi/well, Muromati Yakuhin), 0.5% DMSO, test compound (DMSO only for test compound-addition districts and test compound additive-free districts). After incubation at 37°C for 3 hours, all media were removed. As background districts, cell additive-free media were used. Media were combined with ethyl acetate (0.1 ml) in Eppendorf tube. The mixture was vortexed, and then centrifuged at 5,000 rpm x 1 minute at room temperature to separate ethyl acetate (upper layer). Ethyl acetate (10 μl) was spotted on aluminum plate for thin-layer chromatography (silica gel 60 angstrom, Merck, referred to as TLC plate hereinafter). To a sealed vessel was added chloroform/methanol (90:10, v/v) as an eluent, and TLC plate was developed and then dried at room temperature. To the dried TLC plate was exposured an imaging plate (TR-2040, FUJIFILM) over 16 or more hours. After exposure, the imaging plate was analyzed by Bioimage analyzer (BAS2500, FUJIFILM), and [$^3$H] radioactivity of the spot corresponding to cortisol on TLC plate was determined. Inhibitory activities of cortisone reducing activities of test compounds were calculated as below.

(Inhibitory activity (%)) = 100 x ((Test compound additive-free districts) - (Test compound-addition districts))/((Test compound additive-free districts) - (Background districts))

$IC_{50}$ values were calculated by a linear regression of logarithmic values of analyte concentrations and inhibitory activity values using 2-point data wherein inhibitory activities indicated values around 50%. $IC_{50}$ values for mouse adipocyte cortisone reducing activities of the inventive compound usually exist within the range of 0.01-1000 nM. $IC_{50}$ values for mouse adipocyte cortisone reducing activities of the following inventive compounds were determined. The results are shown below.

[Table 185]

| | Example No. | $IC_{50}$ (nM) |
|---|---|---|
| 1 | 51 | 5.6 |
| 2 | 62 | 47 |
| 3 | 64 | 2.4 |
| 4 | 66 | 0.6 |
| 5 | 74 | 4.0 |
| 6 | 93 | 1.5 |
| 7 | 96 | 55 |
| 8 | 169 | 4 |
| 9 | A41 | <10 |
| 10 | Y9 | 4.2 |

The inventive compound has good properties as a medicinal product. The properties include solubility which

may be measured according to methods of Experiments 5-1 and 5-2 or other known methods.

Experiment 5-1 (Elution Method)

1.75% aqueous disodium hydrogen phosphate solution was mixed with 5.53% aqueous citric acid solution with monitoring by pH indicator to prepare isotonic buffer solutions of pH = 7.4 and 6.0. A buffer of pH = 1.2 (Pharmacopeia Solution 1) was prepared according to Pharmacopeia. Then, a standard solution was prepared. A test compound (about 1 mg) was precisely weighed in 10 ml measuring flask and dissolved in HPLC carrier (0.1% TFA water/acetonitrile = 1/1) to prepare 100 μg/ml standard solution. An elution condition for a test compound was set by the standard solution in ODS column (ChemcoPack Quicksorb: 4.6 mmφ x 150 mm, 5 μm) at 5-10 min. Detection was carried out by UV at both 254 and 230 nm of wavelengths. Quantification was carried out on the basis of the former detected data, and in case of a low sensitibity, the latter detected data was adopted. Dissolution and analysis were carried out as follows. A test compound (about 1 mg) was weighed in 1 ml glass sample tube, and thereto was added each pH of isotonic buffer solution (0.4 ml) by PIPETMAN® and the mixture was shaken at room temperature for 1.5 hours (Conditions: RECIPRO SHAKER SR-1N manufactured by TAITEC, Speed = 8). Then, the solution was transferred to 1.5 ml Eppendorf tube, and centrifuged by a compact high-speed centrifuge at 15.000 rpm for 5 minutes to separate an insoluble. The supernatant was analyzed by HPLC without any purification to calculate a concentration (solubility) by area ratios with a standard solution.

Experiment 5-2 (Dimethylsulfoxide (abbreviated as DMSO hereinafter) Deposition Method)

1.75% aqueous disodium hydrogen phosphate solution was mixed with 5.53% aqueous citric acid solution with monitoring by pH indicator to prepare each isotonic buffer solution of pH = 7.4. A buffer of pH = 1.2 (Pharmacopeia Solution 1) was prepared according to Pharmacopeia. Then, a standard solution was prepared. A test compound (2 μL, 10 mM DMSO solution) was dispensed in 96 well plate and diluted with 50% acetonitrile (198 μL). A HPLC analysis condition was determined by the standard solution. The analysis was carried out under UPLC (Column: ACQUITY UPLC BEH® C18 1.7 μm 2.1 mm x 50 mm, Guard column: VanGuard® Pre-column 2.1 x 5 mm, Mobile phase: solution A; 0.1% TFA aqueous solution, solution B; 0.1% TFA acetonitrile solution, Gradient: 0.00 min-solution B: 5%, 2.00 min-solution B: 100%, 2.71 min-solution B: 5%, 3.50 min-stop, Column temperature: 40˚C, flow rate: 0.4 mL/min, Detection wavelength: 254 or 230 nm, Sample injection: 5 μL), and a measurement wavelength and injection amounts of analysis were determined by the result. Dissolution and analysis were carried out as follows. Samples (10 mM DMSO solution) were dispensed in four Utube on 96 well rack by 15 μL, and evaporate to dryness by centrifugal evaporation at 40˚C for 90 minutes. Thereto was added DMSO (3 μL) to dissolve again, and then buffers of pH7.4 and 1.2 were added to 2 wells each in 300 μL each. After shaking at 25˚C at 110 rpm for 90 minutes, the mixture let stand for 16-20 hours and centrifuged at 2000 g for 15 minutes to separate an insoluble and collect a supernatant (100 μL) in 96 well plate. A test compound (2 μL, 10 mM DMSO solution) was dispensed in separate 96 well plate and diluted with 50% acetonitrile (198 μL) to prepare 100 μM standard solution. Additionally, 100 μM standard solution was tenfold diluted with 50% acetonitrile to prepare 10 μM standard solution. The sample for measuring solubility and two standard solutions were analyzed under the measurement condition determined in pre-investigation to calculate solubilities by area ratios with a standard solution.

INDUSTRIAL APPLICABILITY

The inventive compound is useful as a preventive and/or therapeutic agent for a disease including type II diabetes, abnormal glucose tolerance, hyperglycemia, insulin resistance, hypo-HDL-emia, hyper-LDL-emia, dyslipidemia, hyperlipidemia, hypertriglyceridemia, hypercholesterolemia, hypertension, arteriosclerosis, angiostenosis, atherosclerosis, obesity, cognitive disorder, glaucoma, retinopathy, dementia, Alzheimer disease, osteoporosis, immune disorder, syndrome X, depression, cardiovascular disease, neurodegenerative disease, etc..

## Claims

**1.** A compound of formula (1):

[Chemical Formula 1]

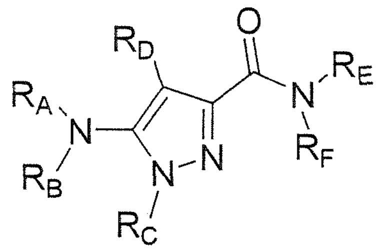

(1)

wherein $R_A$ and $R_B$ are each independently optionally substituted alkyl, optionally substituted cycloalkyl, optionally substituted heterocycloalkyl, or a group of formula: -Rw-Rx-Ry-Rz;

Rw is, independently when it exists more than one, optionally substituted alkylene or optionally substituted cycloalkylene;

Rx is, independently when it exists more than one, a single bond, oxygen atom, or a group of formula: $-S(O)_n-$, -C(O)-, $-NR^3-$, -OC(O)-, -C(O)O-, $-CONR^3-$, $-NR^3CO-$, $-SO_2NR^3-$, $-NR_2SO_2-$ or - $NR^3CONR^4-$;

Ry is, independently when it exists more than one, a single bond or optionally substituted alkylene;

Rz is, independently when it exists more than one, optionally substituted alkyl, optionally substituted cycloalkyl, optionally substituted aryl, optionally substituted heteroaryl or optionally substituted heterocycloalkyl;

$R^3$ and $R^4$ are each independently hydrogen atom or optionally substituted alkyl;

n is 0, 1 or 2;

$R_C$ is optionally substituted alkyl, optionally substituted cycloalkyl or optionally substituted cycloalkylalkyl;

$R_D$ is hydrogen atom, halogen atom, cyano or optionally substituted alkyl;

$R_E$ is hydrogen atom or optionally substituted alkyl;

$R_F$ is a group selected from the following formulae (G1):

[Chemical Formula 2]

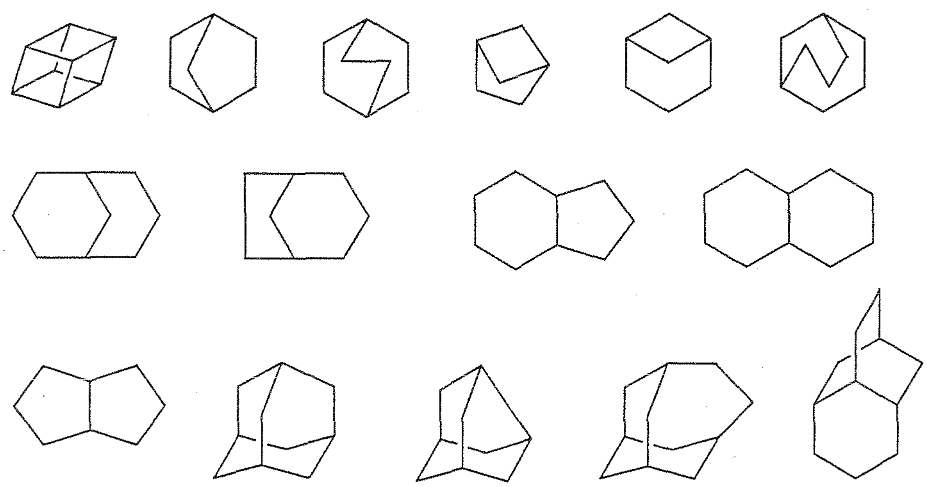

**(G1)**

,

wherein one of hydrogen atoms is a bond, which may be optionally substituted;

provided that if both $R_A$ and $R_B$ are selected from the following group X, then $R_F$ is a group of the following formula (2):

[Chemical Formula 3]

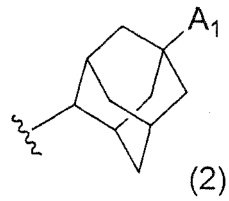

(2),

$A_1$ is $COOR^1$, $CONR^1R^2$, $SO_2NR^1R^2$, $COOR^1$-substituted alkyl, $CONR^1R^2$-substituted alkyl, or $SO_2NR^1R^2$-substituted alkyl, $R^1$ and $R^2$ are each independently hydrogen atom or optionally substituted alkyl, and $R^1$ and $R^2$ may combine each other and together with the adjacent nitrogen atom, to which they are bonded, to form optionally substituted saturated heterocycle;
the group X is optionally substituted alkyl, optionally substituted piperidinyl, optionally substituted pyrrolidinyl, optionally substituted arylalkyl, optionally substituted heteroarylalkyl, optionally substituted piperidinylalkyl or optionally substituted pyrrolidinylalkyl, wherein the substituent is hydroxyl, oxo, halogen atom, cyano, nitro, alkyl, alkoxy, amino which may be optionally substituted by alkyl or arylalkyl, methylenedioxy, trihalomethyl, or trihalomethoxy; or a pharmaceutically acceptable salt thereof.

**2.** The compound as claimed in claim 1, which is represented by formula (3):

[Chemical Formula 4]

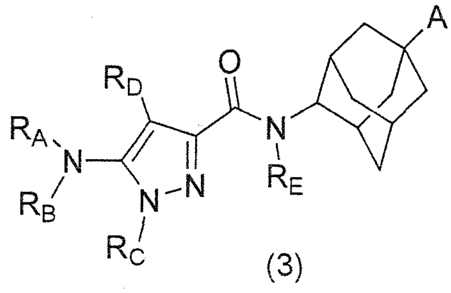

(3)

wherein $R_A$ and $R_B$ are each independently optionally substituted alkyl, optionally substituted cycloalkyl, optionally substituted heterocycloalkyl, or a group of formula: -Rw-Rx-Ry-Rz;
Rw is, independently when it exists more than one, optionally substituted alkylene or optionally substituted cycloalkylene;
Rx is, independently when it exists more than one, a single bond, oxygen atom, or a group of formula: $-S(O)_n-$, -C(O)-, $-NR^3-$, -OC(O)-, -C(O)O-, $-CONR^3-$, $-NR^3CO-$, $-SO_2NR^3-$, $-NR^3SO_2-$ or $-NR^3CONR^4-$;
Ry is, independently when it exists more than one, a single bond or optionally substituted alkylene;
Rz is, independently when it exists more than one, optionally substituted alkyl, optionally substituted cycloalkyl, optionally substituted aryl, optionally substituted heteroaryl or optionally substituted heterocycloalkyl;
$R^3$ and $R^4$ are each independently hydrogen atom or optionally substituted alkyl;
n is 0, 1 or 2;
$R_C$ is optionally substituted alkyl, optionally substituted cycloalkyl or optionally substituted cycloalkylalkyl;
$R_D$ is hydrogen atom, halogen atom, cyano or optionally substituted alkyl;
$R_E$ is hydrogen atom or optionally substituted alkyl;
A is hydrogen atom, halogen atom, hydroxyl, cyano, or a group of formula: $COOR^1$, $CONR^1R^2$, $SO_2NR^1R^2$, $COOR^1$-substituted alkyl, $CONR^1R^2$-substituted alkyl, or $SO_2NR^1R^2$-substituted alkyl, $R^1$ and $R^2$ are each independently hydrogen atom or optionally substituted alkyl, or $R^1$ and $R^2$ may combine each other and together with the adjacent nitrogen atom, to which they are bonded, to form optionally substituted saturated heterocycle;
provided that if both $R_A$ and $R_B$ are selected from the following group X, then A is $COOR^1$, $CONR^1R^2$, $SO_2NR^1R^2$, $COOR^1$-substituted alkyl, $CONR^1R^2$-substituted alkyl, or $SO_2NR^1R^2$-substituted alkyl;
the group X is optionally substituted alkyl, optionally substituted piperidinyl, optionally substituted pyrrolidinyl, optionally substituted arylalkyl, optionally substituted heteroarylalkyl, optionally substituted piperidinylalkyl, or optionally substituted pyrrolidinylalkyl, wherein the substituent is hydroxyl, oxo, halogen atom, cyano, nitro,

alkyl, alkoxy, amino which may be optionally substituted by alkyl or arylalkyl, methylenedioxy, trihalomethyl, or trihalomethoxy; or a pharmaceutically acceptable salt thereof.

**3.** The compound as claimed in claim 2, wherein $R_C$ is optionally substituted alkyl, $R_D$ is hydrogen atom, halogen atom or optionally substituted alkyl, $R_E$ is hydrogen atom, A is halogen atom, hydroxyl, cyano, or a group of formula: $COOR^1$, $CONR^1R^2$, $SO_2NR^1R^2$, $COOR^1$-substituted alkyl, $CONR^1R^2$-substituted alkyl or $SO_2NR^1R^2$-substituted alkyl, $R^1$ and $R^2$ are each independently hydrogen atom or optionally substituted alkyl, or $R^1$ and $R^2$ may combine each other and together with the adjacent nitrogen atom, to which they are bonded, to form optionally substituted saturated heterocycle, or a pharmaceutically acceptable salt thereof.

**4.** The compound as claimed in any one of claim 2 or 3, wherein $R_A$ and $R_B$ are each independently optionally substituted alkyl, optionally substituted cycloalkyl, optionally substituted cycloalkylalkyl or optionally substituted heterocycloalkyl, A is a group of formula: $COOR^1$, $CONR^1R^2$ or $SO_2$-$NR^1R^2$, $R^1$ and $R^2$ are each independently hydrogen atom or optionally substituted alkyl, $R_A$ is optionally substituted cycloalkyl or optionally substituted cycloalkylalkyl, $R_B$ is optionally substituted alkyl, optionally substituted cycloalkyl, optionally substituted heterocycloalkyl, or a group of formula: - Rw-Rx-Ry-Rz wherein Rw, Rx, Ry and Rz are the same as defined in claim 2; or $R_A$ is optionally substituted alkyl, $R_B$ is a group of formula: -Rw-Rx-Ry-Rz wherein Rw, Rx, Ry and Rz are the same as defined in claim 2, or a pharmaceutically acceptable salt thereof.

**5.** The compound as claimed in any one of claims 2 to 4, wherein $R_A$ and $R_B$ are each independently optionally substituted alkyl, optionally substituted cycloalkyl or optionally substituted cycloalkylalkyl, A is a group of formula: $COOR^1$, $CONR^1R^2$ or $SO_2NR^1R^2$, $R^1$ and $R^2$ are each independently hydrogen atom or optionally substituted alkyl, or a pharmaceutically acceptable salt thereof.

**6.** The compound as claimed in any one of claim 4 or 5, wherein A is a group of formula: $CONR^1R^2$, $R^1$ and $R^2$ are each independently hydrogen atom or alkyl which may be optionally substituted by hydroxyl, alkoxy, benzenesulfonyl or pyridyl, or a pharmaceutically acceptable salt thereof.

**7.** The compound as claimed in claim 6, wherein A and nitrogen atom on which adamantyl group is substituted are arranged in *E*-configuration, or a pharmaceutically acceptable salt thereof.

**8.** The compound as claimed in any one of claims 2 to 4, wherein $R_A$ is optionally substituted cycloalkyl or optionally substituted cycloalkylalkyl, $R_B$ is optionally substituted alkyl, optionally substituted cycloalkyl, optionally substituted heterocycloalkyl, or a group of formula: -Rw-Rx-Ry-Rz wherein Rw, Rx, Ry and Rz are the same as defined in claim 2, or a pharmaceutically acceptable salt thereof.

**9.** The compound as claimed in claim 8, wherein $R_B$ is optionally substituted alkyl, optionally substituted heterocycloalkyl, or a group of formula: -Rw-Rx-Ry-Rz wherein Rw is optionally substituted alkylene, Rx is a single bond, oxygen atom, or a group of formula: $-S(O)_n-$, Ry is a single bond, Rz is optionally substituted aryl or optionally substituted heterocycloalkyl, or a pharmaceutically acceptable salt thereof.

**10.** The compound as claimed in any one of claims 2 to 4, wherein $R_A$ is optionally substituted alkyl, $R_B$ is a group of formula: -Rw-Rx-Ry-Rz wherein Rw, Rx, Ry and Rz are the same as defined in claim 2, or a pharmaceutically acceptable salt thereof.

**11.** The compound as claimed in claim 10, wherein Rx is a group of formula: $-S(O)_n-$, -C(O)-, - $NR^3$-, -OC(O)-, -C(O)O-, $-CONR^3-$, $-NR^3CO-$, $-SO_2NR^3-$, $-NR^3SO_2-$ or $-NR^3CONR^4-$, $R^3$ and $R^4$ are each independently hydrogen atom or optionally substituted alkyl, n is 0, 1 or 2, or a pharmaceutically acceptable salt thereof.

**12.** The compound as claimed in claim 11, wherein Rw is optionally substituted alkylene, Rx is a group of formula: $-S(O)_n-$, Ry is a single bond, Rz is optionally substituted alkyl, or a pharmaceutically acceptable salt thereof.

**13.** The compound as claimed in claim 10, wherein Rx is oxygen atom, Rz is optionally substituted cycloalkyl, optionally substituted aryl, optionally substituted heteroaryl or optionally substituted heterocycloalkyl, or a pharmaceutically acceptable salt thereof.

**14.** The compound as claimed in claim 13, wherein Rw is optionally substituted alkylene, Ry is a single bond, Rz is optionally substituted aryl or optionally substituted heterocycloalkyl, or a pharmaceutically acceptable salt thereof.

**15.** The compound as claimed in claim 10, wherein Rx is a single bond, Rz is optionally substituted cycloalkyl or optionally substituted heterocycloalkyl, or a pharmaceutically acceptable salt thereof.

**16.** The compound as claimed in claim 15, wherein Rw is optionally substituted alkylene, Ry is a single bond, Rz is optionally substituted cycloalkyl or optionally substituted heterocycloalkyl, or a pharmaceutically acceptable salt thereof.

**17.** The compound as claimed in claim 10, wherein Rx is a single bond, Rz is substituted aryl, substituted heteroaryl or substituted heterocycloalkyl, in which the substituent is $-COR^5$, $-S(O)_nR^5$, $-NR^{7a}COR^5$, $-SO_2NR^{7a}R^{7b}$, $-NR^{7a}CONR^{7b}R^5$, $-OR^6$ or $-(CH_2)_mR^6$, $R^5$ is alkyl, cycloalkyl, aryl, heteroaryl or heterocycloalkyl, $R^6$ is cycloalkyl, aryl, heteroaryl or heterocycloalkyl, the alkyl, cycloalkyl, aryl, heteroaryl and heterocycloalkyl groups in $R^5$ and $R^6$ may be further optionally substituted by halogen atom, haloalkyl, haloalkoxy, alkyl, hydroxyl, alkoxy, $-NR^{8a}R^{8b}$, alkylsulfonyl, cyano, cycloalkyl, cycloalkylsulfonyl, alkoxyalkoxy, hydroxyalkoxy, cycloalkyloxyalkyl, cycloalkyloxy, haloalkoxyalkyl, hydroxyalkyl, alkoxyalkyl, $NR^{8a}R^{8b}$-substituted alkyl, alkylsulfonylalkyl, cyanoalkyl, cycloalkylalkyl, cycloalkylsulfonylalkyl, alkoxyalkoxyalkyl, hydroxyalkoxyalkyl or nitrogen-containing saturated heterocycle, $R^{7a}$, $R^{7b}$, $R^{8a}$ and $R^{8b}$ are each independently hydrogen atom or alkyl, n and m are each independently 0, 1 or 2, or a pharmaceutically acceptable salt thereof.

**18.** The compound as claimed in claim 17, wherein Rw is optionally substituted alkylene, Ry is a single bond, Rz is substituted aryl or substituted heterocycloalkyl, in which the substituent is $-COR^5$ or $-S(O)_nR^5$, or a pharmaceutically acceptable salt thereof.

**19.** The compound as claimed in claim 10, wherein Rw is optionally substituted cycloalkylene, Rx is a single bond, Ry is a single bond, Rz is optionally substituted aryl, or a pharmaceutically acceptable salt thereof.

**20.** The compound as claimed in claim 2, wherein $R_A$ is tetrahydropyranyl, $R_B$ is alkyl or cycloalkyl, or a pharmaceutically acceptable salt thereof.

**21.** The compound as claimed in claim 2, which is represented by formula (4):

[Chemical Formula 5]

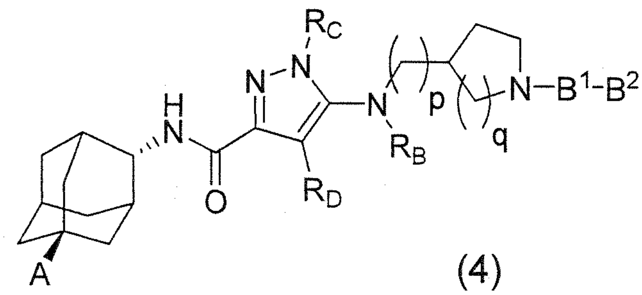

(4)

wherein p is 0, 1 or 2, q is 1 or 2, $B^1$ is a single bond, carbonyl or sulfonyl, $B^2$ is optionally substituted alkyl, optionally substituted cycloalkyl, optionally substituted heterocycloalkyl, optionally substituted aryl, optionally substituted heteroaryl, optionally substituted alkylamino, optionally substituted dialkylamino, optionally substituted cycloalkylamino, optionally substituted heterocycloalkylamino, optionally substituted arylamino or optionally substituted heteroarylamino, provided that if $B^1$ is a single bond, then $B^2$ is optionally substituted aryl or optionally substituted heteroaryl, or a pharmaceutically acceptable salt thereof.

**22.** The compound as claimed in claim 21, wherein $B^1$ is a single bond, $B^2$ is optionally substituted aryl or optionally substituted heteroaryl, or a pharmaceutically acceptable salt thereof.

**23.** The compound as claimed in claim 22, wherein $B^2$ is optionally substituted aryl, or a pharmaceutically acceptable salt thereof.

**24.** The compound as claimed in claim 22, wherein $B^2$ is optionally substituted heteroaryl, or a pharmaceutically acceptable salt thereof.

**25.** The compound as claimed in claim 24, wherein $B^2$ is optionally substituted pyridyl, or a pharmaceutically acceptable salt thereof.

**26.** The compound as claimed in claim 21, wherein $B^1$ is carbonyl, $B^2$ is optionally substituted aryl, optionally substituted alkyl, optionally substituted cycloalkyl or optionally substituted heteroaryl, or a pharmaceutically acceptable salt thereof.

**27.** The compound as claimed in claim 26, wherein the optionally substituted alkyl group in $B^2$ is optionally substituted benzyl, or a pharmaceutically acceptable salt thereof.

**28.** The compound as claimed in claim 26, wherein the optionally substituted cycloalkyl group in $B^2$ is cyclopropyl or cyclobutyl, and substituted by optionally substituted aryl, or a pharmaceutically acceptable salt thereof.

**29.** The compound as claimed in claim 26, wherein $B^2$ is optionally substituted aryl, or a pharmaceutically acceptable salt thereof.

**30.** The compound as claimed in claim 26, wherein $B^2$ is optionally substituted heteroaryl, or a pharmaceutically acceptable salt thereof.

**31.** The compound as claimed in claim 26, wherein the optionally substituted heteroaryl group in $B^2$ is optionally substituted pyridyl, or a pharmaceutically acceptable salt thereof.

**32.** The compound as claimed in claim 26, wherein $B^2$ is fluorine-substituted alkyl, or a pharmaceutically acceptable salt thereof.

**33.** The compound as claimed in claim 21, wherein $B^1$ is sulfonyl, $B^2$ is optionally substituted alkyl, optionally substituted cycloalkyl, optionally substituted heterocycloalkyl, optionally substituted aryl or optionally substituted heteroaryl, or a pharmaceutically acceptable salt thereof.

**34.** The compound as claimed in claim 33, wherein $B^2$ is optionally substituted aryl, or a pharmaceutically acceptable salt thereof.

**35.** The compound as claimed in claim 33, wherein the optionally substituted alkyl group in $B^2$ is fluorine-substituted alkyl, or a pharmaceutically acceptable salt thereof.

**36.** The compound as claimed in claim 33, wherein the optionally substituted heteroaryl group in $B^2$ is optionally substituted pyridyl, or a pharmaceutically acceptable salt thereof.

**37.** The compound as claimed in claim 33, wherein the optionally substituted alkyl group in $B^2$ is optionally substituted benzyl, or a pharmaceutically acceptable salt thereof.

**38.** The compound as claimed in claim 21, wherein $B^1$ is carbonyl, $B^2$ is optionally substituted alkylamino, optionally substituted dialkylamino, optionally substituted cycloalkylamino, optionally substituted heterocycloalkylamino, optionally substituted arylamino or optionally substituted heteroarylamino, or a pharmaceutically acceptable salt thereof.

**39.** The compound as claimed in claim 38, wherein $B^2$ is optionally substituted arylamino or optionally substituted heteroarylamino, or a pharmaceutically acceptable salt thereof.

**40.** The compound as claimed in claim 38, wherein $B^2$ is optionally substituted arylamino, or a pharmaceutically acceptable salt thereof.

**41.** The compound as claimed in claim 38, wherein the optionally substituted heteroarylamino group in $B^2$ is optionally substituted pyridylamino, or a pharmaceutically acceptable salt thereof.

**42.** The compound as claimed in claim 38, wherein the optionally substituted alkylamino group in $B^2$ is optionally substituted benzylamino, or a pharmaceutically acceptable salt thereof.

**43.** The compound as claimed in any one of claims 21 to 42, wherein p is 0 and q is 1, or a pharmaceutically acceptable

salt thereof.

**44.** The compound as claimed in any one of claims 21 to 42, represented by formula (5):

[Chemical Formula 6]

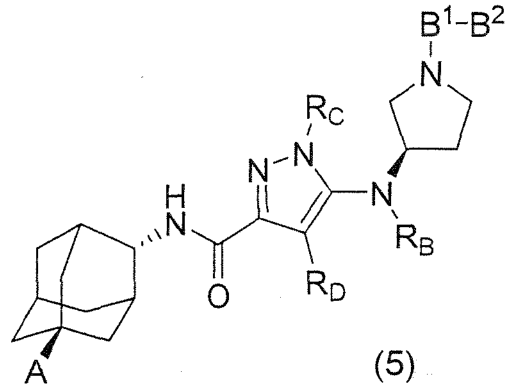

(5)

or a pharmaceutically acceptable salt thereof.

**45.** The compound as claimed in any one of claims 21 to 42, represented by formula (6):

[Chemical Formula 7]

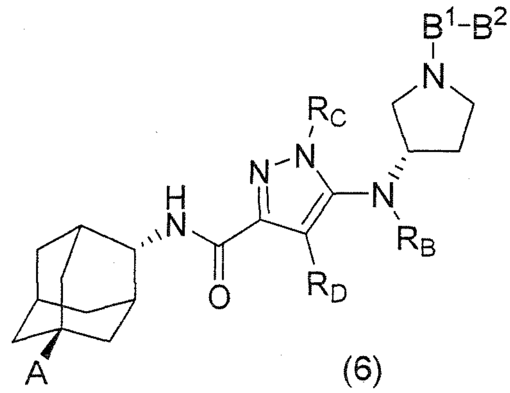

(6)

,

or a pharmaceutically acceptable salt thereof.

**46.** The compound as claimed in any one of claims 21 to 42, wherein p is 1 and q is 2, or a pharmaceutically acceptable salt thereof.

**47.** The compound as claimed in any one of claims 21 to 42, wherein p is 2 and q is 2, or a pharmaceutically acceptable salt thereof.

**48.** The compound as claimed in any one of claims 21 to 42, wherein p is 0 and q is 2, or a pharmaceutically acceptable salt thereof.

**49.** The compound as claimed in any one of claims 21 to 42, represented by formula (7):

[Chemical Formula 8]

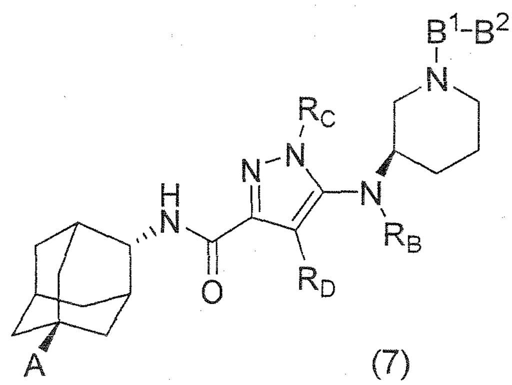

(7)

,

or a pharmaceutically acceptable salt thereof.

**50.** The compound as claimed in any one of claims 21 to 42, represented by formula (8):

[Chemical Formula 9]

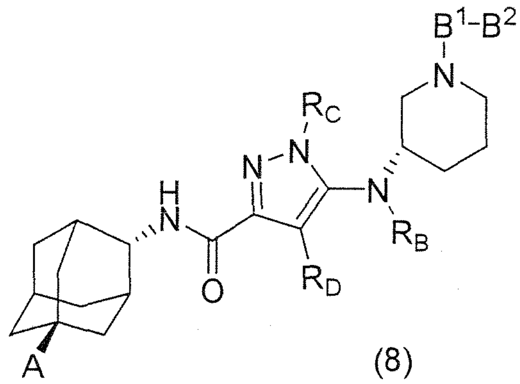

(8)

,

or a pharmaceutically acceptable salt thereof.

**51.** The compound as claimed in any one of claims 21 to 50, wherein $R_B$ is methyl or ethyl, or a pharmaceutically acceptable salt thereof.

**52.** The compound as claimed in any one of claims 8 to 51, wherein A is hydroxyl, or a pharmaceutically acceptable salt thereof.

**53.** The compound as claimed in any one of claims 8 to 51, wherein A is carbamoyl, or a pharmaceutically acceptable salt thereof.

**54.** The compound as claimed in any one of claims 4 to 53, wherein $R_D$ is chlorine atom, fluorine atom or methyl, or a pharmaceutically acceptable salt thereof.

**55.** The compound as claimed in claim 54, wherein $R_C$ is alkyl, or a pharmaceutically acceptable salt thereof

**56.** The compound as claimed in claim 54, wherein $R_C$ is methyl or ethyl, or a pharmaceutically acceptable salt thereof.

**57.** The compound as claimed in claim 56, wherein $R_E$ is hydrogen atom, or a pharmaceutically acceptable salt thereof.

**58.** The compound as claimed in any one of claims 4 to 57, wherein A and nitrogen atom on which adamantyl group is substituted are arranged in *E*-configuration, or a pharmaceutically acceptable salt thereof.

**59.** A medicament, comprising as the active ingredient the compound as claimed in any one of claims 1 to 58 as an active ingredient, or a pharmaceutically acceptable salt thereof.

**60.** A therapeutic agent for type II diabetes, abnormal glucose tolerance, hyperglycemia, insulin resistance, dyslipidemia, hypertension, arteriosclerosis, angiostenosis, obesity, cognitive disorder, dementia, Alzheimer disease, syndrome X, depression, cardiovascular disease or atherosclerosis, comprising the compound as claimed in any one of claims 1 to 58 as an active ingredient, or a pharmaceutically acceptable salt thereof.

**61.** A therapeutic agent for diabetes, insulin resistance or type II diabetes, comprising the compound as claimed in any one of claims 1 to 58 as an active ingredient, or a pharmaceutically acceptable salt thereof.

**62.** A therapeutic agent for arteriosclerosis or atherosclerosis, comprising the compound as claimed in any one of claims 1 to 58 as an active ingredient, or a pharmaceutically acceptable salt thereof.

**63.** A therapeutic agent for syndrome X, comprising the compound as claimed in any one of claims 1 to 58 as an active ingredient, or a pharmaceutically acceptable salt thereof.

**64.** A therapeutic agent for obesity, comprising the compound as claimed in any one of claims 1 to 58 as an active ingredient, or a pharmaceutically acceptable salt thereof.

**65.** A therapeutic agent for cognitive disorder, dementia, Alzheimer disease or depression, comprising the compound as claimed in any one of claims 1 to 58 as an active ingredient, or a pharmaceutically acceptable salt thereof.

**66.** A therapeutic agent for dyslipidemia, comprising the compound as claimed in any one of claims 1 to 58 as an active ingredient, or a pharmaceutically acceptable salt thereof

**67.** A therapeutic agent for hypertension, comprising the compound as claimed in any one of claims 1 to 58 as an active ingredient, or a pharmaceutically acceptable salt thereof.

**INTERNATIONAL SEARCH REPORT**

International application No.
PCT/JP2008/064085

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
C07D231/38, A61K31/415, A61K31/4155, A61K31/445, A61K31/501, A61P3/00, A61P3/04, A61P3/06, A61P3/10, A61P9/10, A61P9/12, A61P25/24, A61P25/28, C07D401/12, C07D401/14, C07D403/12, C07D405/12, C07D409/14, C07D413/14

Documentation searched other than minimum documentation to the extent that such documents are included in the fields searched

Electronic data base consulted during the international search (name of data base and, where practicable, search terms used)
JSTPlus/JST7580(JDreamII), CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y/A | WO 2005/016877 A2 (Merck & Co., Inc.), 24 February, 2005 (24.02.05), Full text & JP 2007-501789 A & EP 1653949 A1 | 1-7,10, 59-67/8,9, 11-58 |
| Y/A | WO 2006/024627 A2 (Janssen Pharmaceutica N.V.), 09 March, 2006 (09.03.06), Claim 1, substituent R4 & US 2008/0064693 A1 & EP 1796657 A1 & KR 10-2007-0049224 A | 1-7,10, 59-67/8,9, 11-58 |
| A | WO 2004/089470 A2 (Novo Nordisk A/S), 21 October, 2004 (21.10.04), & JP 2006-522745 A & US 2007/0270408 A1 & EP 1787982 A2 | 1-67 |

☒ Further documents are listed in the continuation of Box C.　☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 August, 2008 (25.08.08) | 02 September, 2008 (02.09.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2008/064085

C (Continuation). DOCUMENTS CONSIDERED TO BE RELEVANT

| Category* | Citation of document, with indication, where appropriate, of the relevant passages | Relevant to claim No. |
|---|---|---|
| A | WO 2005/095350 A1 (Amgen Inc.), 13 October, 2005 (13.10.05), & JP 2007-530549 A & US 2005/0272793 A1 & EP 1735282 A1 | 1-67 |

Form PCT/ISA/210 (continuation of second sheet) (April 2007)

**INTERNATIONAL SEARCH REPORT**

International application No.
PCT/JP2008/064085

Continuation of A. CLASSIFICATION OF SUBJECT MATTER
(International Patent Classification (IPC))

*C07D231/38*(2006.01)i, *A61K31/415*(2006.01)i, *A61K31/4155*(2006.01)i, *A61K31/445*(2006.01)i, *A61K31/501*(2006.01)i, *A61P3/00*(2006.01)i, *A61P3/04*(2006.01)i, *A61P3/06*(2006.01)i, *A61P3/10*(2006.01)i, *A61P9/10*(2006.01)i, *A61P9/12*(2006.01)i, *A61P25/24*(2006.01)i, *A61P25/28*(2006.01)i, *C07D401/12*(2006.01)i, *C07D401/14*(2006.01)i, *C07D403/12*(2006.01)i, *C07D405/12*(2006.01)i, *C07D409/14*(2006.01)i, *C07D413/14*(2006.01)i

(According to International Patent Classification (IPC) or to both national classification and IPC)

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005016877 A
- WO 2004089470 A


### Non-patent literature cited in the description


- Greene's Protective Groups in Organic Synthesis. John Wiley & Sons Inc, 1981
- The Experimental Chemistry. Maruzen, vol. 22
- **R.C. Larock.** *Comprehensive Organic Transformations,* 1989
- **C. D. Jones ; M. Kaselj et al.** *J. Org. Chem.,* 1998, vol. 63, 2758-2760
- *The Journal of Organic Chemistry,* 1983, vol. 48, 1099